# EUROPEAN PATENT APPLICATION

(11) **EP 1 820 515 A1**
(43) Date of publication of application: **22.08.2007**
(21) Application number: 05811303.6
(22) Date of filing: 29.11.2005
(51) Int. Cl.: A61K 45/00

(54) **NITROGEN-CONTAINING FUSED RING COMPOUND AND USE THEREOF**

(30) Priority: 29.11.2004 JP 2004344563; 07.12.2004 US 634223 P
(71) Applicant: Japan Tobacco, Inc., Tokyo 105-8422 (JP)
(72) Inventor: HIRATA, Kazuyuki C.Pharm.Res.Inst. of Jp. Tobacco, Takatsuki-shi, Osaka 569-1125 (JP); OGAWA, Naoki C.Pharm.Res.Inst. of Jp. Tobacco, Takatsuki-shi, Osaka 569-1125 (JP); SHINAGAWA, Yuko C.Pharm.Res.Inst. of Jp. Tobacco, Takatsuki-shi, Osaka 569-1125 (JP); KIGUCHI, Toshihiro C.Pharm.Res.Inst. of Jp.Tobacco, Takatsuki-shi, Osaka 569-1125 (JP); INOUE, Teruhiko C.Pharm.Res.Inst. of Jp. Tobacco, Takatsuki-shi, Osaka 569-1125 (JP); MATSUO, Akira C.Pharm.Res.Inst. of Jp. Tobacco, Takatsuki-shi, Osaka 569-1125 (JP); KOSUGI, Yoshinori C.Pharm.Res.Inst. of Jp. Tobacco, Takatsuki-shi, Osaka 569-1125 (JP); NOMURA, Yukihiro C.Pharm.Res.Inst. of Jp. Tobacco, Takatsuki-shi, Osaka 569-1125 (JP); KAWAHARA, Iichiro C.Pharm.Res.Inst. of Jp. Tobacco, Takatsuki-shi, Osaka 569-1125 (JP); UEHARA, Hideto C.Pharm.Res.Inst. of Jp. Tobacco, Takatsuki-shi, Osaka 569-1125 (JP)
(74) Representative: Vossius & Partner
(86) International application number: PCT/JP2005/022259
(87) International publication number: WO 2006/057460

(57) **Abstract**

A URAT1 activity inhibitor containing a nitrogen-containing fused ring compound represented by the following formula [1]: 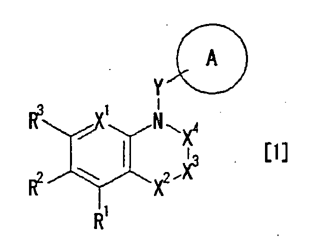 wherein each symbol is as defined in the description. The present invention is useful for the prophylaxis or treatment of pathology showing involvement of uric acid, such as hyperuricemia, gouty tophus, acute gouty arthritis, chronic gouty arthritis, gouty kidney, urolithiasis, renal function disorder, coronary artery disease, ischemic heart disease and the like.

## Description

### Technical Field

The present invention relates to a nitrogen-containing fused ring compounds and use thereof.

### Background Art

Uric acid is a substance having a molecular weight of 168 and a dissociation constant (pKa value) of 5.75, which is present in the form of uric acid or a conjugate base (urate) thereof in the body fluid depending on the pH of the body fluid. In human, since the function of urate oxidase (uricase) of the liver is lack by mutation, uric acid is the final metabolite of purine form. To be specific, dietarily or endogenously produced purine form becomes inosine from adenosine, then hypoxanthine, and then xanthine, or becomes guanine from guanosine, and then xanthine, and this xanthine is subject to oxidization by xanthine oxidase or xanthine dehydrogenase to become uric acid. Uric acid is mainly excreted from the kidney.

Hyperuricemia becomes severe, and when the blood uric acid level exceeds the upper limit of solubility, sodium urate crystal forms in the cartilage tissues and joints and then sediments called gouty tophus (tophi) are produced. This gouty tophus causes acute gouty arthritis, which progresses into chronic gouty arthritis. Besides these, it has been clarified that nephropathy (gouty kidney) and urolithiasis occur as complications of sodium urate crystal deposition due to hyperuricemia, and that hyperuricemia itself induces renal function disorder (see Johnson RJ, Kivilighn SD, Kim YG et al. Reappraisal of the pathogenesis and consequences of hyperuricemia in hypertension, cardiovascular disease, and renal disease. Am. J. Kidney Dis. 33, 225-234(1999)).

Many hyperuricemia patients have complications such as hyperlipidemia, diabetes, hypertension, obesity and the like. While these complications are each a risk factor for coronary artery disease and death rates, hyperuricemia patients have long been known to show significantly high complication rate of coronary artery diseases and short survival, as compared to patient groups having normal blood uric acid level. Fang et al. conducted a large-scale investigation on the death rates of coronary artery disease in 5926 cases ranging from 25 to 74 years old whose blood uric acid level was measured during the period of 22 years from 1971 to 1992, and clarified that increased blood uric acid level alone can be a risk for ischemic heart diseases (see Fang J., Alderman MH. JAMA 283(18) 2404-2410 (2000)). It has been also reported that a treatment aiming at decreasing the blood uric acid level itself, along with the treatment of complications, is useful for preventing the onset of and decreasing the death rates of coronary artery disease, and a treatment to decrease the blood uric acid level should be positively employed for asymptomatic hyperuricemia, too. (see Tohru Nakamura, Hyperuricemia and Gout, 9(1) 61-65 (2001)). Recently, it has become determinative that hyperuricemia associated with hypertension is an independent risk factor for cardiovascular diseases (see Ichiro Hisatome, Nikkei Medical, January 2004, 100-101). According to Guideline for the management of hyperuricemia and gout (1st Edit.), Gout and Nucleic acid metabolism, vol. 26, suppl. 1 (2002), Japanese Society of Gout and Nucleic Acid Metabolism, moreover, it has been also described that 1) nephropathy (also referred to as gouty kidney) and urolithiasis, which are complications associated with sodium urate crystal deposition, highly frequently occur, 2) control of blood uric acid level is important from the aspect of prevention of a recurrence of cerebral or cardiovascular incidents, 3) patients with hyperuricemia or gout show frequent complication of hyperlipidemia, 4) obesity should not be ignored as etiology or exacerbation factor of hyperuricemia, 5) uricosuric agents are basically used for decreased uric acid excretion hyperuricemia, 6) hyperuricemia associated with hypertension is highly likely an independent risk factor of cardiovascular incidents, and the like. Therefore, it is almost certain that decreasing the blood uric acid level is not the only effective measure for the prophylaxis or treatment of the above-mentioned diseases, but so is combined use of a pharmaceutical agent that decreases the blood uric acid level with therapeutic or prophylactic agents for these above-mentioned diseases.

While uric acid is mainly excreted from the kidney, uric acid in blood is once filtered off almost completely by renal glomerulus, after which uric acid is mostly reabsorbed by proximal renal tubule. Therefore, only a small amount of uric acid is excreted into urine. The proximal reabsorption of uric acid has been clarified to be a transport via a transporter by an experiment using membrane vesicle prepared from renal cortex (see Sica DA and Schoolwerth AC. The Kidney, 6th edition, pp. 680-700, Saunders, Philadelphia (2000)), and its substrate selectivity, inhibitors thereof and the like have also been elucidated (see Roch-Ramel F., Werner D., and Guisan B. Am. J. Physiol. 266, F797-F805 (1994), Roch-Ramel F., Guisan B., and Diezi J. J. Pharmacol. Exp. Ther. 280, 839-845 (1997)).

In recent years, a gene encoding a human kidney uric acid transporter (SLC22A12) has been identified (see Enomoto A. et al., Nature 417, 447-452 (2002)). The transporter (urate transporter 1, URAT1) encoded by this gene is a 12-spanning transmembrane molecule belonging to the organic anion transporter (OAT) family, and Northern blot using the full-length cDNA thereof as a probe has revealed that it specifically expresses in adult and embryo kidneys. It has been also confirmed by immunostaining of human renal tissue section conducted using a polyclonal antibody specific to C-terminal peptide thereof that it is localized on the lumen of the proximal renal tubule in the cortex. In an experiment using a Xenopus laevis oocyte expression system, uric acid uptake via URAT1 increased in a time-dependent manner, and the uric acid uptake showed saturation at high uric acid concentration, which is characteristic of carrier transport. Moreover, it has been clarified that the uptake is based on the exchange with organic anion such as lactic acid, pyrazine carboxylic acid, nicotinic acid and the like, and that the uptake is inhibited by uricosuric agents such as probenecid, benzbromarone and the like, and URAT1 has been demonstrated to be the transporter being elucidated by experiments using the above-mentioned membrane vesicle (see Naohiko Anzai et. al., Biochemical 76(2) 101-110 (2004)). In other words, URAT1 has been clarified to be a main transporter responsible for reabsorption of uric acid in the kidney.

Furthermore, gene mutations of URAT1 have been identified by gene analysis of idiopathic renal hypouricemia patients, and when these mutant URAT1 were expressed in Xenopus laevis oocyte, the uric acid transport activity had been lost. These facts also clarify that URAT1 is involved in the control of blood uric acid level (see Enomoto A. et al., Nature 417, 447-452 (2002)).

With regard to the relationship between URAT1 and diseases, moreover, many reports have documented that probenecid and benzbromarone that inhibit the uric acid transport activity of URAT1 are therapeutic drugs for hyperuricemia, and useful as drugs for the prophylaxis or treatment of pathology exhibiting high blood uric acid level, such as hyperuricemia, gouty tophus, gout arthritis, gouty kidney, urolithiasis and renal function disorder (see Hisashi Yamanaka, Diagnosis and Treatment, vol. 92, No. 1, 125-128 (2004)).

Furthermore, since some of the drugs of nucleic acid metabolic antagonists, hypotensive diuretics, antituberculosis, anti-inflammatory analgesic drugs, hyperlipidemic drugs, therapeutic drugs for asthma, immunosuppressants and the like increase blood uric acid level, thus creating the problems of progress into or exacerbation of pathology caused by increase in the above-mentioned blood uric acid level.

Therefore, a substance having an inhibitory action on URAT1 activity would be useful as a drug for the prophylaxis or treatment of pathology suggesting the involvement of uric acid, such as pathology suggesting the involvement of high blood uric acid level, specifically, hyperuricemia, gouty tophus, gout arthritis, gouty kidney, urolithiasis, renal function disorder and the like, and further as a drug for the prophylaxis or treatment of hyperlipidemia, diabetes, obesity or cardiovascular diseases (e.g., hypertension, coronary arterial disease, vascular endothelial disorder, ischemic heart disease etc.) because it decreases the blood uric acid level. A concurrent use of these other prophylactic or therapeutic drugs with the substance having an inhibitory action on URAT1 activity would be useful for more effective prophylaxis or treatment of these diseases.

A substance having an inhibitory action on URAT1 activity can be said to be useful because it can prevent increase in the blood uric acid level when concurrently used together with a pharmaceutical agent that prevents increase in the blood uric acid level, such as nucleic acid metabolic antagonist, hypotensive diuretic, anti-tuberculosis, anti-inflammatory analgesic drugs, hyperlipidemic drugs, therapeutic drugs for asthma, immunosuppressants and the like.

As a compound having a structure similar to that of the present invention, the following compounds are known. For example, JP-B-7-76214 describes, as a compound showing a vasopressin antagonistic action, a compound represented by the following formula: 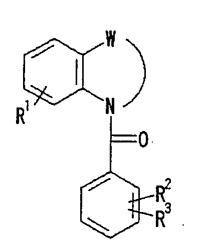 wherein R¹ is a hydrogen atom, a halogen atom, a lower alkyl group, an amino group optionally having lower alkyl group(s) as substituent(s), or lower alkoxy group, R² is a hydrogen atom, a halogen atom, a lower alkoxy group, a phenyl-lower alkoxy group, a hydroxyl group, a lower alkyl group, an amino group optionally having lower alkyl group(s) as substituent(s), a carbamoyl-substituted lower alkoxy group, an amino group-substituted lower alkoxy group optionally having lower alkyl group(s) as substituent(s), or a benzoyloxy group optionally having halogen atom(s) on the phenyl ring as substituent(s), R³ is -NR⁴R⁵ or - CONR¹¹R¹², and W is a group: -(CH₂)p- (p is an integer of 3-5) or a group: -CH=CH-(CH₂)q- (q is 1 or 2).

However, the compound has a different structure from that of the compound of the present invention, and no description suggestive of the compound of the present invention can be found.

Now, known uricosuric agents and compounds considered to be comparatively similar to the compound of the present invention are described below. For example, USP 265559 describes, as a compound showing a uric acid excretion promoting action, the following compound, or benzbromarone.

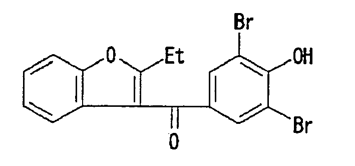

However, this compound has a different structure from that of the compound of the present invention, and no description suggestive of the compound of the present invention can be found. In addition, this compound is suggested to potentially cause pharmacokinetic drug interaction because it has a strong inhibitory action on CYP2C8, CYP2C9, CYP2C19 and CYP3A4, molecular species of cytochrome P450 (CYP), which is a drug-metabolizing enzyme (see Chiyoko Kunishima et. al., J. Saitama Med School, vol. 30, No. 4, p187-194 (2004)).

### Disclosure of Invention

At present, as an agent for the prophylaxis or treatment of hyperuricemia, a uricosuric agent, benzbromarone, having an inhibitory action on URAT1 activity is used. However, the inhibitory action on URAT1 activity of benzbromarone is not sufficient. Moreover, a possibility of inducing a pharmacokinetic drug interaction has been suggested in view of its CYP inhibitory action. Therefore, there is a strong demand for the development of an agent for the prophylaxis or treatment of hyperuricemia, which has more potent inhibitory action on URAT1 activity and has no or very weak CYP inhibitory action. The present invention aims at providing an agent for the prophylaxis or treatment of hyperuricemia, which has more potent inhibitory action on URAT1 activity and has no or very weak CYP inhibitory action, namely, a URAT1 activity inhibitor and the like.

The present inventors have conducted intensive studies in an attempt to develop a new agent for the prophylaxis or treatment of hyperuricemia, which takes the place of conventional agents for the prophylaxis or treatment of hyperuricemia, and found a nitrogen-containing fused ring compound having a superior inhibitory action on URAT1 activity, which resulted in the completion of the present invention.

Accordingly, the present invention provides the following.

### <1>

A URAT1 activity inhibitor comprising a substance that inhibits URAT1 activity and does not substantially inhibit CYP.

### <2>

The inhibitor of the above-mentioned <1>, wherein CYP is CYP2C9.

### <3>

The inhibitor of the above-mentioned <1> or <2>, wherein the concentration of the substance necessary for inhibiting CYP2C9 by 50% is not less than 1 _{µ}M.

### <4>

A URAT1 activity inhibitor comprising a nitrogen-containing fused ring compound represented by the following formula [1]: 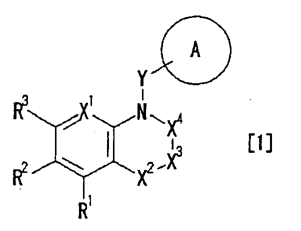 wherein
R¹, R² and R³ are the same or different and each is
1) a hydrogen atom, or
2) a group selected from group A below, or
3) R¹ and R² may form, together with the carbon atoms they are bonded to, a saturated or unsaturated carbon ring having 3 to 14 carbon atoms optionally substituted by one or more, the same or different substituents selected from group A below, or
4) R² and R³ may form, together with the carbon atoms they are bonded to, a saturated or unsaturated carbon ring having 3 to 14 carbon atoms optionally substituted by one or more, the same or different substituents selected from group A below;
Y is
1) -CO-,
2) -CS-, or
3) -S(=O)₂-;
X¹ is
1) a nitrogen atom, or
2) CR⁴ wherein R⁴ is
   (a) a hydrogen atom, or
   (b) a group selected from group A below, or
   (c) R³ and R⁴ may form, together with the carbon atoms they are bonded to, a saturated or unsaturated carbon ring having 3 to 14 carbon atoms optionally substituted by one or more, the same or different substituents selected from group A below;
X² is
1) an oxygen atom,
2) -N(R⁵)- wherein R⁵ is
   (a) a hydrogen atom, or
   (b) a C₁₋₆ alkyl group optionally substituted by one or more, the same or different substituents selected from group B below,
3) -N(COR⁶)-wherein R⁶ is
   (a) a hydroxyl group,
   (b) a C₁₋₆ alkyl group optionally substituted by one or more, the same or different substituents selected from group B below,
   (c) a C₁₋₆ alkoxy group optionally substituted by one or more, the same or different substituents selected from (i) and (ii) :
      (i) a substituent selected from group B below,
      (ii) a C₁₋₆ alkyl group optionally substituted by one or more, the same or different substituents selected from group B below,
   (d) a saturated or unsaturated carbon ring group having 3 to 14 carbon atoms optionally substituted by one or more, the same or different substituents selected from group A below,
   (e) a cycloalkylalkoxy group optionally substituted by one or more, the same or different substituents selected from group A below,
   (f) an aralkyl group optionally substituted by one or more, the same or different substituents selected from group A below, or
   (g) an aralkoxy group optionally substituted by one or more, the same or different substituents selected from group A below,
4) -N(S(=O)₂R⁶)- wherein R⁶ is as defined above,
5) -N(CONR⁷R⁸)- wherein R⁷ and R⁸ are the same or different and each is
   (a) a hydrogen atom, or
   (b) a C₁₋₆ alkyl group optionally substituted by one or more, the same or different substituents selected from group B below, or
   (c) R⁷ and R⁸ may form, together with the nitrogen atom they are bonded to, a monocyclic nitrogen-containing saturated heterocycle optionally substituted by one or more, the same or different substituents selected from group A below,
6) a sulfur atom,
7) -S(=O)-,
8) -S (=O)₂-, or
9) -CR⁹R¹⁰- wherein R⁹ and R¹⁰ are the same or different and each is
   (a) a hydrogen atom, or
   (b) a C₁₋₆ alkyl group optionally substituted by one or more, the same or different substituents selected from group B below, or
   (c) R⁹ and R¹⁰ may in combination form an oxo group;
-X³-X⁴- is
- (CR¹¹R¹²) n- wherein n is an integer of 1 to 3, and R¹¹ and R¹² each in the number of n are the same or different and each is
   (a) a hydrogen atom, or
   (b) a C₁₋₆ alkyl group optionally substituted by one or more, the same or different substituents selected from group B below, or
   (c) R¹¹ and R¹² bonded to a single carbon atom may in combination form an oxo group, or
   (d) two of R¹¹ and R¹² each in the number of n, which are bonded to a single carbon atom or two adjacent carbon atoms, may form, together with the carbon atom(s), a saturated or unsaturated carbon ring having 3 to 14 carbon atoms optionally substituted by one or more, the same or different substituents selected from group A below; and
ring A is
1) a saturated or unsaturated carbon ring group having 3 to 14 carbon atoms optionally substituted by one or more, the same or different substituents selected from group A below, or
2) a saturated or unsaturated heterocyclic group containing at least one heteroatom selected from a nitrogen atom, an oxygen atom and a sulfur atom, optionally substituted by one or more, the same or different substituents selected from group A below,
or a pharmaceutically acceptable salt thereof:
[group A]
   1) a halogen atom,
   2) -OR¹³ wherein R¹³ is
      (a) a hydrogen atom,
      (b) a C₁₋₆ alkyl group optionally substituted by one or more, the same or different substituents selected from group B below, or
      (c) -COR¹⁴ wherein R¹⁴ is
         a) a hydrogen atom,
         b) a hydroxyl group,
         c) a C₁₋₆ alkyl group optionally substituted by one or more, the same or different substituents selected from group B below,
         d) a C₁₋₆ alkoxy group optionally substituted by one or more, the same or different substituents selected from (i) and (ii) :
            (i) a substituent selected from group B below,
            (ii) a C₁₋₆ alkyl group optionally substituted by one or more, the same or different substituents selected from group B below,
         e) a saturated or unsaturated carbon ring group having 3 to 14 carbon atoms optionally substituted by one or more, the same or different substituents selected from (i) and (ii):
            (i) a substituent selected from group B below,
            (ii) a C₁₋₆ alkyl group optionally substituted by one or more, the same or different substituents selected from group B below,
         f) a cycloalkylalkoxy group optionally substituted by one or more, the same or different substituents selected from (i) and (ii) :
            (i) a substituent selected from group B below,
            (ii) a C₁₋₆ alkyl group optionally substituted by one or more, the same or different substituents selected from group B below,
         g) an aralkyl group optionally substituted by one or more, the same or different substituents selected from
            (i) and (ii) :

            (i) a substituent selected from group B below,
            (ii) a C₁₋₆ alkyl group optionally substituted by one or more, the same or different substituents selected from group B below, or
         h) an aralkoxy group optionally substituted by one or more, the same or different substituents selected from (i) and (ii) :
            (i) a substituent selected from group B below,
            (ii) a C₁₋₆ alkyl group optionally substituted by one or more, the same or different substituents selected from group B below,
   3) a C₁₋₆ alkyl group optionally substituted by one or more, the same or different substituents selected from group B below,
   4) a cycloalkylalkoxy group optionally substituted by one or more, the same or different substituents selected from (a) and (b) :
      (a) a substituent selected from group B below,
      (b) a C₁₋₆ alkyl group optionally substituted by one or more, the same or different substituents selected from group B below,
   5) an aralkyl group optionally substituted by one or more, the same or different substituents selected from (a) and (b):
      (a) a substituent selected from group B below,
      (b) a C₁₋₆ alkyl group optionally substituted by one or more, the same or different substituents selected from group B below,
   6) an aralkoxy group optionally substituted by one or more, the same or different substituents selected from (a) and (b):
      (a) a substituent selected from group B below,
      (b) a C₁₋₆ alkyl group optionally substituted by one or more, the same or different substituents selected from group B below,
   7) -COR¹⁴ wherein R¹⁴ is as defined above,
   8) -NR¹⁵R¹⁶ wherein R¹⁵ and R¹⁶ are the same or different and each is
      (a) a hydrogen atom, or
      (b) a C₁₋₆ alkyl group optionally substituted by one or more, the same or different substituents selected from group B below, or
      (c) R¹⁵ and R¹⁶ may form, together with the nitrogen atom they are bonded to, a monocyclic nitrogen-containing saturated heterocycle optionally substituted by one or more, the same or different substituents selected from (i) and (ii):
         (i) a substituent selected from group B below,
         (ii) a C₁₋₆ alkyl group optionally substituted by one or more, the same or different substituents selected from group B below,
   9) -CONR¹⁵R¹⁶ wherein R¹⁵ and R¹⁶ are as defined above,
   10) -NR¹⁷COR¹⁴ wherein R¹⁴ is as defined above, and R¹⁷ is
      (a) a hydrogen atom, or
      (b) a C₁₋₆ alkyl group optionally substituted by one or more, the same or different substituents selected from group B below,
   11) -NR¹⁷S (=O)₂R¹⁴ wherein R¹⁴ and R¹⁷ are as defined above,
   12) -NR¹⁷CONR¹⁵R¹⁶ wherein R¹⁵, R¹⁶ and R¹⁷ are as defined above,
   13) -SR¹³ wherein R¹³ is as defined above,
   14) -S(=O)R¹⁴ wherein R¹⁴ is as defined above,
   15) -S (=O)₂R¹⁴ wherein R¹⁴ is as defined above,
   16) -S(=O)₂NR¹⁵R¹⁶ wherein R¹⁵ and R¹⁶ are as defined above,
   17) a saturated or unsaturated carbon ring group having 3 to 14 carbon atoms optionally substituted by one or more, the same or different substituents selected from (a) and (b):
      (a) a substituent selected from group B below,
      (b) a C₁₋₆ alkyl group optionally substituted by one or more, the same or different substituents selected from group B below,
   18) a saturated or unsaturated heterocyclic group containing at least one heteroatom selected from a nitrogen atom, an oxygen atom and a sulfur atom, optionally substituted by one or more, the same or different substituents selected from (a) and (b):
      (a) a substituent selected from group B below,
      (b) a C₁₋₆ alkyl group optionally substituted by one or more, the same or different substituents selected from group B below,
   19) an aryloxy group optionally substituted by one or more, the same or different substituents selected from (a) and (b):
      (a) a substituent selected from group B below,
      (b) a C₁₋₆ alkyl group optionally substituted by one or more, the same or different substituents selected from group B below,
   20) a cyano group, and
   21) a nitro group,
[group B]
   1) a halogen atom,
   2) a hydroxyl group,
   3) a C₁₋₆ alkoxy group,
   4) -NR¹⁸R¹⁹ wherein R¹⁸ and R¹⁹ are the same or different and each is
      (a) a hydrogen atom, or
      (b) a C₁₋₆ alkyl group, or
      (c) R¹⁸ and R¹⁹ may form, together with the nitrogen atom they are bonded to, a monocyclic nitrogen-containing saturated heterocycle,
   5) -CONR¹⁸R¹⁹ wherein R¹⁸ and R¹⁹ are as defined above,
   6) -COR²⁰ wherein R²⁰ is
      (a) a hydrogen atom,
      (b) a hydroxyl group,
      (c) a C₁₋₆ alkyl group, or
      (d) a C₁₋₆ alkoxy group,
   7) -NR²¹COR²⁰ wherein R²⁰ is as defined above, and R²¹ is
      (a) a hydrogen atom, or
      (b) a C₁₋₆ alkyl group,
   8) -NR²¹CONR¹⁸R¹⁹ wherein R¹⁸, R¹⁹ and R²¹ are as defined above,
   9) -NR²¹S (=O)₂R²² wherein R²¹ is as defined above, and R²² is a C₁₋₆ alkyl group, and
   10) -S(=O)₂R²² wherein R²² is as defined above,
wherein the C₁₋₆ alkyl group and C₁₋₆ alkoxy group in 3) to 10) above are optionally further substituted by one or more, the same or different substituents selected from group B, and the monocyclic nitrogen-containing saturated heterocycle in 4), 5) and 8) above are optionally further substituted by one or more substituents selected from group B and a C₁₋₆ alkyl group.

### <5>

A nitrogen-containing fused ring compound represented by the following formula [2]: 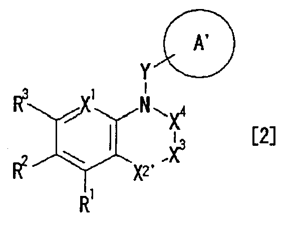 wherein R¹, R², R³, Y, X¹, X³ and X⁴ are as defined in the above-mentioned <4>,
ring A' is
1) a saturated or unsaturated carbon ring group having 3 to 14 carbon atoms optionally substituted by one or more, the same or different substituents selected from the group C below, or
2) a saturated or unsaturated heterocyclic group containing at least one heteroatom selected from a nitrogen atom, an oxygen atom and a sulfur atom, optionally substituted by one or more, the same or different substituents selected from the group C below,
the ring A' is substituted by at least one -OR^{13'} wherein R^{13'} is as defined in the group C below;
X²' is
1) an oxygen atom,
2) -N(R⁵)- wherein R⁵ is
   (a) a hydrogen atom, or
   (b) a C₁₋₆ alkyl group optionally substituted by one or more, the same or different substituents selected from group B below,
3) -N(COR⁶)- wherein R⁶ is
   (a) a hydroxyl group,
   (b) a C₁₋₆ alkyl group optionally substituted by one or more, the same or different substituents selected from group B below,
   (c) a C₁₋₆ alkoxy group optionally substituted by one or more, the same or different substituents selected from (i) and (ii):
      (i) a substituent selected from group B below,
      (ii) a C₁₋₆ alkyl group optionally substituted by one or more, the same or different substituents selected from group B below,
   (d) a saturated or unsaturated carbon ring group having 3 to 14 carbon atoms optionally substituted by one or more, the same or different substituents selected from group A below,
   (e) a cycloalkylalkoxy group optionally substituted by one or more, the same or different substituents selected from group A below,
   (f) an aralkyl group optionally substituted by one or more, the same or different substituents selected from group A below, or
   (g) an aralkoxy group optionally substituted by one or more, the same or different substituents selected from group A below,
4) -N (S (=_{O}) ₂R⁶) - wherein R⁶ is as defined above,
5) -N(CONR⁷R⁸)- wherein R⁷ and R⁸ are the same or different and each is
   (a) a hydrogen atom, or
   (b) a C₁₋₆ alkyl group optionally substituted by one or more, the same or different substituents selected from group B below, or
   (c) R⁷ and R⁸ may form, together with the nitrogen atom they are bonded to, a monocyclic nitrogen-containing saturated heterocycle optionally substituted by one or more, the same or different substituents selected from group A below,
6) a sulfur atom,
7) -S(=O)-,
8) -S(=O)₂-, or
9) -CH₂-,
(provided that when X²_{,} is -CH₂-,
then -X³-X⁴- should be
- (CR¹¹R¹²)n- wherein n is an integer of 1 to 3, and R¹¹ and R¹² each in the number of n are the same or different and each is
   (a) a hydrogen atom, or
   (b) R¹¹ and R¹² bonded to a single carbon atom may in combination form an oxo group, or
   (c) two of R¹¹ and R¹² each in the number of n, which are bonded to a single carbon atom or two adjacent carbon atoms, may form, together with the carbon atom(s), a saturated or unsaturated carbon ring having 3 to 14 carbon atoms optionally substituted by one or more, the same or different substituents selected from group A below;
R¹³' should be a hydrogen atom; and
ring A' should be further substituted by at least one a halogen atom;
provided that when both R¹¹ and R¹² are hydrogen atoms, and n is 2, then all of R¹, R² and R³ should be hydrogen atoms) ,
or a pharmaceutically acceptable salt thereof:
[group A]
   1) a halogen atom,
   2) -OR¹³ wherein R¹³ is
      (a) a hydrogen atom,
      (b) a C₁₋₆ alkyl group optionally substituted by one or more, the same or different substituents selected from group B below, or
      (c) -COR¹⁴ wherein R¹⁴ is
         a) a hydrogen atom,
         b) a hydroxyl group,
         c) a C₁₋₆ alkyl group optionally substituted by one or more, the same or different substituents selected from group B below,
         d) a C₁₋₆ alkoxy group optionally substituted by one or more, the same or different substituents selected from (i) and (ii):
            (i) a substituent selected from group B below,
            (ii) a C₁₋₆ alkyl group optionally substituted by one or more, the same or different substituents selected from group B below,
         e) a saturated or unsaturated carbon ring group having 3 to 14 carbon atoms optionally substituted by one or more, the same or different substituents selected from (i) and (ii) :
            (i) a substituent selected from group B below,
            (ii) a C₁₋₆ alkyl group optionally substituted by one or more, the same or different substituents selected from group B below,
         f) a cycloalkylalkoxy group optionally substituted by one or more, the same or different substituents selected from (i) and (ii):
            (i) a substituent selected from group B below,
            (ii) a C₁₋₆ alkyl group optionally substituted by one or more, the same or different substituents selected from group B below,
         g) an aralkyl group optionally substituted by one or more, the same or different substituents selected from (i) and (ii) :
            (i) a substituent selected from group B below,
            (ii) a C₁₋₆ alkyl group optionally substituted by one or more, the same or different substituents selected from group B below, or
         h) an aralkoxy group optionally substituted by one or more, the same or different substituents selected from (i) and (ii) :
            (i) a substituent selected from group B below,
            (ii) a C₁₋₆ alkyl group optionally substituted by one or more, the same or different substituents selected from group B below,
   3) a C₁₋₆ alkyl group optionally substituted by one or more, the same or different substituents selected from group B below,
   4) a cycloalkylalkoxy group optionally substituted by one or more, the same or different substituents selected from (a) and (b) :
      (a) a substituent selected from group B below,
      (b) a C₁₋₆ alkyl group optionally substituted by one or more, the same or different substituents selected from group B below,
   5) an aralkyl group optionally substituted by one or more, the same or different substituents selected from (a) and (b):
      (a) a substituent selected from group B below,
      (b) a C₁₋₆ alkyl group optionally substituted by one or more, the same or different substituents selected from group B below,
   6) an aralkoxy group optionally substituted by one or more, the same or different substituents selected from (a) and (b):
      (a) a substituent selected from group B below,
      (b) a C₁₋₆ alkyl group optionally substituted by one or more, the same or different substituents selected from group B below,
   7) -COR¹⁴ wherein R¹⁴ is as defined above,
   8) -NR¹⁵R¹⁶ wherein R¹⁵ and R¹⁶ are the same or different and each is
      (a) a hydrogen atom, or
      (b) a C₁₋₆ alkyl group optionally substituted by one or more, the same or different substituents selected from group B below, or
      (c) R¹⁵ and R¹⁶ may form, together with the nitrogen atom they are bonded to, a monocyclic nitrogen-containing saturated heterocycle optionally substituted by one or more, the same or different substituents selected from (i) and (ii) :
         (i) a substituent selected from group B below,
         (ii) a C₁₋₆ alkyl group optionally substituted by one or more, the same or different substituents selected from group B below,
   9) -CONR¹⁵R¹⁶ wherein R¹⁵ and R¹⁶ are as defined above,
   10) -NR¹⁷COR¹⁴ wherein R¹⁴ is as defined above, and R¹⁷ is
      (a) a hydrogen atom, or
      (b) a C₁₋₆ alkyl group optionally substituted by one or more, the same or different substituents selected from group B below,
   11) -NR¹⁷S(=O)₂R¹⁴ wherein R¹⁴ and R¹⁷ are as defined above,
   12) -NR¹⁷CONR¹⁵R¹⁶ wherein R¹⁵, R¹⁶ and R¹⁷ are as defined above,
   13) -SR¹³ wherein R¹³ is as defined above,
   14) -S(=O)R¹⁴ wherein R¹⁴ is as defined above,
   15) -S(=O)₂R¹⁴wherein R¹⁴ is as defined above,
   16) -S(=O)₂NR¹⁵R¹⁶ wherein R¹⁵ and R¹⁶ are as defined above,
   17) a saturated or unsaturated carbon ring group having 3 to 14 carbon atoms optionally substituted by one or more, the same or different substituents selected from (a) and (b):
      (a) a substituent selected from group B below,
      (b) a C₁₋₆ alkyl group optionally substituted by one or more, the same or different substituents selected from group B below,
   18) a saturated or unsaturated heterocyclic group containing at least one heteroatom selected from a nitrogen atom, an oxygen atom and a sulfur atom, optionally substituted by one or more, the same or different substituents selected from (a) and (b):
      (a) a substituent selected from group B below,
      (b) a C₁₋₆ alkyl group optionally substituted by one or more, the same or different substituents selected from group B below,
   19) an aryloxy group optionally substituted by one or more, the same or different substituents selected from (a) and (b):
      (a) a substituent selected from group B below,
      (b) a C₁₋₆ alkyl group optionally substituted by one or more, the same or different substituents selected from group B below,
   20) a cyano group, and
   21) a nitro group,
[group B]
   1) a halogen atom,
   2) a hydroxyl group,
   3) a C₁₋₆ alkoxy group,
   4) -NR¹⁸R¹⁹ wherein R¹⁸ and R¹⁹ are the same or different and each is
      (a) a hydrogen atom, or
      (b) a C₁₋₆ alkyl group, or
      (c) R¹⁸ and R¹⁹ may form, together with the nitrogen atom they are bonded to, a monocyclic nitrogen-containing saturated heterocycle,
   5) -CONR¹⁸R¹⁹ wherein R¹⁸ and R¹⁹ are as defined above,
   6) -COR²⁰ wherein R²⁰ is
      (a) a hydrogen atom,
      (b) a hydroxyl group,
      (c) a C₁₋₆ alkyl group, or
      (d) a C₁₋₆ alkoxy group,
   7) -NR²¹COR²⁰ wherein R²⁰ is as defined above, and R²¹ is
      (a) a hydrogen atom, or
      (b) a C₁₋₆ alkyl group,
   8) -NR²¹CONR¹⁸R¹⁹ wherein R¹⁸, R¹⁹ and R²¹ are as defined above,
   9) -NR²¹S(=O)₂R²² wherein R²¹ is as defined above, and R²² is a C₁₋₆ alkyl group, and
   10) -S (=O) _{2R}²² wherein R²² is as defined above,
   wherein the C₁₋₆ alkyl group and C₁₋₆ alkoxy group in 3) to 10) above are optionally further substituted by one or more, the same or different substituents selected from group B, and the monocyclic nitrogen-containing saturated heterocycle in 4), 5) and 8) above are optionally further substituted by one or more substituents selected from group B and a C₁₋₆ alkyl group,
[group C]
   1) a halogen atom,
   2) -OR^{13'} wherein R^{13'} is
      (a) a hydrogen atom,
      (b) a C₁₋₆ alkyl group optionally substituted by one or more, the same or different substituents selected from group B above, or
      (c) -COR¹⁴' wherein R¹⁴' is
         a) a hydrogen atom,
         b) a hydroxyl group,
         c) a C₁₋₆ alkyl group optionally substituted by one or more, the same or different substituents selected from group B above,
         d) a C₁₋₆ alkoxy group optionally substituted by one or more, the same or different substituents selected from (i) and (ii):
            (i) a substituent selected from group B above,
            (ii) a C₁₋₆ alkyl group optionally substituted by one or more, the same or different substituents selected from group B above,
         e) a saturated or unsaturated carbon ring group having 3 to 14 carbon atoms optionally substituted by one or more, the same or different substituents selected from (i) and (ii) :
            (i) a substituent selected from group B above,
            (ii) a C₁₋₆ alkyl group optionally substituted by one or more, the same or different substituents selected from group B above,
         f) a cycloalkylalkoxy group optionally substituted by one or more, the same or different substituents selected from (i) and (ii) :
            (i) a substituent selected from group B above,
            (ii) a C₁₋₆ alkyl group optionally substituted by one or more, the same or different substituents selected from group B above,
         g) an aralkyl group optionally substituted by one or more, the same or different substituents selected from (i) and (ii) :
            (i) a substituent selected from group B above,
            (ii) a C₁₋₆ alkyl group optionally substituted by one or more, the same or different substituents selected from group B above, or
         h) an aralkoxy group optionally substituted by one or more, the same or different substituents selected from (i) and (ii) :
            (i) a substituent selected from group B above,
            (ii) a C₁₋₆ alkyl group optionally substituted by one or more, the same or different substituents selected from group B above,
   3) a C₁₋₆ alkyl group optionally substituted by one or more, the same or different substituents selected from group B above,
   4) a cycloalkylalkoxy group optionally substituted by one or more, the same or different substituents selected from (a) and (b) :
      (a) a substituent selected from group B above,
      (b) a C₁₋₆ alkyl group optionally substituted by one or more, the same or different substituents selected from group B above,
   5) an aralkyl group optionally substituted by one or more, the same or different substituents selected from (a) and (b):
      (a) a substituent selected from group B above,
      (b) a C₁₋₆ alkyl group optionally substituted by one or more, the same or different substituents selected from group B above,
   6) an aralkoxy group optionally substituted by one or more, the same or different substituents selected from (a) and (b):
      (a) a substituent selected from group B above,
      (b) a C₁₋₆ alkyl group optionally substituted by one or more, the same or different substituents selected from group B above,
   7) -COR¹⁴' wherein R¹⁴' is as defined above,
   8) -NR¹⁵'R¹'^{,} wherein R¹⁵' and R¹⁶' are the same or different and each is
      (a) a hydrogen atom, or
      (b) a C₁₋₆ alkyl group optionally substituted by one or more, the same or different substituents selected from group B above, or
      (c) R¹⁵' and R¹⁶' may form, together with the nitrogen atom they are bonded to, a monocyclic nitrogen-containing saturated heterocycle optionally substituted by one or more, the same or different substituents selected from (i) and (ii) :
         (i) a substituent selected from group B above,
         (ii) a C₁₋₆ alkyl group optionally substituted by one or more, the same or different substituents selected from group B above,
   9) -NR¹⁷' COR¹⁴' wherein R¹⁴' is as defined above, and R¹⁷' is
      (a) a hydrogen atom, or
      (b) a C₁₋₆ alkyl group optionally substituted by one or more, the same or different substituents selected from group B above,
   10) -NR¹⁷' S (=O) ₂R¹⁴' wherein R¹⁴' and R¹⁷' are as defined above,
   11) -NR¹⁷' CONR¹⁵' R¹⁶_{'} wherein R¹⁵', R¹⁶' and R¹⁷' are as defined above,
   12) -SR¹³' wherein R¹³' is as defined above,
   13) -S(=O)R¹⁴' wherein R¹⁴' is as defined above,
   14) -S(=O)₂R¹⁴' wherein R¹⁴' is as defined above,
   15) -S(=O)₂NR¹⁵'R¹⁶' wherein R¹⁵' and R¹⁶' are as defined above,
   16) a saturated or unsaturated carbon ring group having 3 to 14 carbon atoms optionally substituted by one or more, the same or different substituents selected from (a) and (b):
      (a) a substituent selected from group B above,
      (b) a C₁₋₆ alkyl group optionally substituted by one or more, the same or different substituents selected from group B above,
   17) a saturated or unsaturated heterocyclic group containing at least one heteroatom selected from a nitrogen atom, an oxygen atom and a sulfur atom, optionally substituted by one or more, the same or different substituents selected from (a) and (b):
      (a) a substituent selected from group B above,
      (b) a C₁₋₆ alkyl group optionally substituted by one or more, the same or different substituents selected from group B above,
   18) an aryloxy group optionally substituted by one or more, the same or different substituents selected from (a) and (b):
      (a) a substituent selected from group B above,
      (b) a C₁₋₆ alkyl group optionally substituted by one or more, the same or different substituents selected from group B above,
   19) a cyano group, and
   20) a nitro group.

### <6>

The nitrogen-containing fused ring compound of the above-mentioned <5>, wherein
(A) when X²' is
   1) -N(R⁵)- wherein R⁵ is
      (a) a hydrogen atom, or
      (b) a C₁₋₆ alkyl group optionally substituted by one or more, the same or different substituents selected from group B above,
   2) -N(COR⁶)- wherein R⁶ is
      (a) a hydroxyl group,
      (b) a C₁₋₆ alkyl group optionally substituted by one or more, the same or different substituents selected from group B above,
      (c) a C₁₋₆ alkoxy group optionally substituted by one or more, the same or different substituents selected from (i) and (ii) :
         (i) a substituent selected from group B above,
         (ii) a C₁₋₆ alkyl group optionally substituted by one or more, the same or different substituents selected from group B above,
      (d) a saturated or unsaturated carbon ring group having 3 to 14 carbon atoms optionally substituted by one or more, the same or different substituents selected from group A above,
      (e) a cycloalkylalkoxy group optionally substituted by one or more, the same or different substituents selected from group A above,
      (f) an aralkyl group optionally substituted by one or more, the same or different substituents selected from group A above, or
      (g) an aralkoxy group optionally substituted by one or more, the same or different substituents selected from group A above,
   3) -N(S(=O)₂R⁶)- wherein R⁶ is as defined above, or
   4) -N(CONR⁷R⁸)- wherein R⁷ and R⁸ are the same or different and each is
      (a) a hydrogen atom, or
      (b) a C₁₋₆ alkyl group optionally substituted by one or more, the same or different substituents selected from group B above, or
      (c) R⁷ and R⁸ may form, together with the nitrogen atom they are bonded to, a monocyclic nitrogen-containing saturated heterocycle optionally substituted by one or more, the same or different substituents selected from group A above,
   then -X³-X⁴- should be -CH₂-CH₂-; and
   R¹³, should be a hydrogen atom;
(B) when X²' is
   1) -S(=O)-, or
   2) -S(=O)₂-,
   then -X³-X⁴- should be -CH₂-CH₂-;
(C) when X²' is a sulfur atom, and
   Y is
   1) -CO-, or
   2) -CS-,
   then -X³-X⁴- should be -CH₂-CH₂-; and
   R¹³' should be a hydrogen atom;
(D) when X²_{,} is an oxygen atom, and
   Y is
   1) -CO-, or
   2) -CS-,
      then R¹, R² and R³ should be the same or different and each is

   1) a hydrogen atom, or
   2) a group selected from group D below, or
   3) R¹ and R² may form, together with the carbon atoms they are bonded to, a saturated or unsaturated carbon ring having 3 to 14 carbon atoms optionally substituted by one or more, the same or different substituents selected from group D below, or
   4) R² and R³ may form, together with the carbon atoms they are bonded to, a saturated or unsaturated carbon ring having 3 to 14 carbon atoms optionally substituted by one or more, the same or different substituents selected from group D below; and
      -X³-X⁴- should be -CH₂-CH₂-;
(E) when X², is
   1) an oxygen atom, or
   2) a sulfur atom, and
   Y is -S(=O)₂-,
   then -X³-X⁴- should be -CH₂-CH₂-; and
   R¹³_{'} should be a hydrogen atom; or
(F) when X²' is -CH₂-,
   then -X³-X⁴- should be
   -(CR¹¹R¹²)n- wherein n is an integer of 1 to 3, and R¹¹ and R¹² each in the number of n are the same or different and each is
   (a) a hydrogen atom, or
   (b) R¹¹ and R¹² bonded to a single carbon atom may in combination form an oxo group, or
   (c) two of R¹¹ and R¹² each in the number of n, which are bonded to a single carbon atom or two adjacent carbon atoms, may form, together with the carbon atom(s), a saturated or unsaturated carbon ring having 3 to 14 carbon atoms optionally substituted by one or more, the same or different substituents selected from group A above;
   R¹³' should be a hydrogen atom; and
   ring A' should be further substituted by at least one a halogen atom;
   provided that in (F), when both R¹¹ and R¹² are hydrogen atoms, and n is 2, then all of R¹, R² and R³ should be hydrogen atoms, or a pharmaceutically acceptable salt thereof:

[group D]
   1) a halogen atom,
   2) -OR¹³'' wherein R¹³'' is
      (a) a hydrogen atom,
      (b) a C₁₋₆ alkyl group optionally substituted by one or more, the same or different substituents selected from group B above, or
      (c) -COR¹⁴'' wherein R¹⁴'' is
         a) a hydrogen atom,
         b) a hydroxyl group,
         c) a C₁₋₆ alkyl group optionally substituted by one or more, the same or different substituents selected from group B above,
         d) a C₁₋₆ alkoxy group optionally substituted by one or more, the same or different substituents selected from (i) and (ii) :
            (i) a substituent selected from group B above,
            (ii) a C₁₋₆ alkyl group optionally substituted by one or more, the same or different substituents selected from group B above,
         e) a saturated or unsaturated carbon ring group having 3 to 14 carbon atoms optionally substituted by one or more, the same or different substituents selected from (i) and (ii) :
            (i) a substituent selected from group B above,
            (ii) a C₁₋₆ alkyl group optionally substituted by one or more, the same or different substituents selected from group B above,
         f) a cycloalkylalkoxy group optionally substituted by one or more, the same or different substituents selected from (i) and (ii):
            (i) a substituent selected from group B above,
            (ii) a C₁₋₆ alkyl group optionally substituted by one or more, the same or different substituents selected from group B above,
         g) an aralkyl group optionally substituted by one or more, the same or different substituents selected from (i) and (ii) :
            (i) a substituent selected from group B above,
            (ii) a C₁₋₆ alkyl group optionally substituted by one or more, the same or different substituents selected from group B above, or
         h) an aralkoxy group optionally substituted by one or more, the same or different substituents selected from (i) and (ii) :
            (i) a substituent selected from group B above,
            (ii) a C₁₋₆ alkyl group optionally substituted by one or more, the same or different substituents selected from group B above,
   3) a C₁₋₆ alkyl group optionally substituted by one or more, the same or different substituents selected from group B above,
   4) a cycloalkylalkoxy group optionally substituted by one or more, the same or different substituents selected from (a) and (b) :
      (a) a substituent selected from group B above,
      (b) a C₁₋₆ alkyl group optionally substituted by one or more, the same or different substituents selected from group B above,
   5) an aralkyl group optionally substituted by one or more, the same or different substituents selected from (a) and (b):
      (a) a substituent selected from group B above,
      (b) a C₁₋₆ alkyl group optionally substituted by one or more, the same or different substituents selected from group B above,
   6) an aralkoxy group optionally substituted by one or more, the same or different substituents selected from (a) and (b):
      (a) a substituent selected from group B above,
      (b) a C₁₋₆ alkyl group optionally substituted by one or more, the same or different substituents selected from group B above,
   7) -COR¹⁴'' wherein R¹⁴'' is as defined above,
   8) -NR¹⁵'' R¹⁶'' wherein R¹⁵'' and R¹⁶'' are the same or different and each is
      (a) a hydrogen atom, or
      (b) a C₁₋₆ alkyl group optionally substituted by one or more, the same or different substituents selected from group B above, or
      (c) R¹⁵'' and R¹⁶'' may form, together with the nitrogen atom they are bonded to, a monocyclic nitrogen-containing saturated heterocycle optionally substituted by one or more, the same or different substituents selected from (i) and (ii) :
         (i) a substituent selected from group B above,
         (ii) a C₁₋₆ alkyl group optionally substituted by one or more, the same or different substituents selected from group B above,
   9) -CONR¹⁵''R¹⁶'' wherein R¹⁵'' and R¹⁶'' are as defined above,
   10) -NR¹⁷''COR¹⁴'' wherein R¹⁴'' is as defined above, and R¹⁷'' is
      (a) a hydrogen atom, or
      (b) a C₁₋₆ alkyl group optionally substituted by one or more, the same or different substituents selected from group B above,
   11) -NR¹⁷'' S(=O)₂R¹⁴'' wherein R¹⁴'' and R¹⁷'' are as defined above,
   12) -NR¹⁷''CONR¹⁵''R¹⁶''wherein R¹⁵'', R¹⁶'' and R¹⁷'' are as defined above,
   13) -SR¹³'' wherein R¹³'' is as defined above,
   14) -S(=O)R¹⁴'' wherein R¹⁴_{''} is as defined above,
   15) -S (=O)₂R¹⁴'' wherein R¹⁴'' is as defined above,
   16) -S(=O)₂NR¹⁵''R¹⁶'' wherein R¹⁵'' and R¹⁶'' are as defined above,
   17) a saturated or unsaturated carbon ring group having 3 to 14 carbon atoms optionally substituted by one or more, the same or different substituents selected from (a) and (b):
      (a) a substituent selected from group B above,
      (b) a C₁₋₆ alkyl group optionally substituted by one or more, the same or different substituents selected from group B above,
   18) an aryloxy group optionally substituted by one or more, the same or different substituents selected from (a) and (b):
      (a) a substituent selected from group B above,
      (b) a C₁₋₆ alkyl group optionally substituted by one or more, the same or different substituents selected from group B above,
   19) a cyano group, and
   20) a nitro group.

### <7>

The nitrogen-containing fused ring compound of the above-mentioned <5>, wherein
X^{2,} is
1) -N (R⁵) - wherein R⁵ is
   (a) a hydrogen atom, or
   (b) a C₁₋₆ alkyl group optionally substituted by one or more, the same or different substituents selected from group B above,
2) -N(COR⁶)-wherein R⁶ is
   (a) a hydroxyl group,
   (b) a C₁₋₆ alkyl group optionally substituted by one or more, the same or different substituents selected from group B above,
   (c) a C₁₋₆ alkoxy group optionally substituted by one or more, the same or different substituents selected from (i) and (ii) :
      (i) a substituent selected from group B above,
      (ii) a C₁₋₆ alkyl group optionally substituted by one or more, the same or different substituents selected from group B above,
   (d) a saturated or unsaturated carbon ring group having 3 to 14 carbon atoms optionally substituted by one or more, the same or different substituents selected from group A above,
   (e) a cycloalkylalkoxy group optionally substituted by one or more, the same or different substituents selected from group A above,
   (f) an aralkyl group optionally substituted by one or more, the same or different substituents selected from group A above, or
   (g) an aralkoxy group optionally substituted by one or more, the same or different substituents selected from group A above,
3) -N (S (=O)₂R⁶) - wherein R⁶ is as defined above, or
4) -N(CONR⁷R⁸)- wherein R⁷ and R⁸ are the same or different and each is
   (a) a hydrogen atom, or
   (b) a C₁₋₆ alkyl group optionally substituted by one or more, the same or different substituents selected from group B above, or
   (c) R⁷ and R⁸ may form, together with the nitrogen atom they are bonded to, a monocyclic nitrogen-containing saturated heterocycle optionally substituted by one or more, the same or different substituents selected from group A above;
-X³-X⁴- is -CH₂-CH₂-; and
R¹³' is a hydrogen atom;
or a pharmaceutically acceptable salt thereof.

### <8>

The nitrogen-containing fused ring compound of the above-mentioned <7>, wherein ring A' is 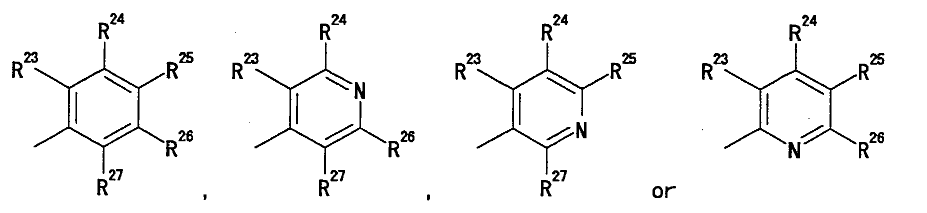 wherein
R²³ to R²⁷ are the same or different and each is
1) a hydrogen atom, or
2) a group selected from group C above, and
at least one of R²³ to R²⁷ is a hydroxyl group;
or a pharmaceutically acceptable salt thereof.

### <9>

The nitrogen-containing fused ring compound of the above-mentioned <7>, wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -COOH,
7) -CO-C₁₋₆ alkoxy group,
8) an amino group,
9) -NHS(=O)₂-C₁₋₆ alkyl group,
10) -S(=O)₂-C₁₋₆ alkyl group,
11) -S(=O)₂-NR¹⁵R¹⁶ wherein R¹⁵ and R¹⁶ are the same or different and each is
   (a) a hydrogen atom, or
   (b) a C₁₋₆ alkyl group, or
   (c) R¹⁵ and R¹⁶ may form, together with the nitrogen atom they are bonded to, a monocyclic nitrogen-containing saturated heterocycle,
12) a saturated or unsaturated carbon ring group having 3 to 14 carbon atoms, or
13) a nitro group;
Y is
1) -CO-,
2) -CS-, or
3) *-*S(=O)₂-;
X¹ is
1) a nitrogen atom, or
2) CR⁴ wherein R⁴ is
   (a) a hydrogen atom,
   (b) a hydroxyl group,
   (c) a C₁₋₆ alkyl group, or
   (d) -CO-C₁₋₆ alkoxy group, or
   (e) R³ and R⁴ may form, together with the carbon atoms they are bonded to, a saturated or unsaturated carbon ring having 3 to 14 carbon atoms;
X²' is
1) -N (R⁵) - wherein R⁵ is
   (a) a hydrogen atom, or
   (b) a C₁₋₆ alkyl group,
2) -N(COR⁶)- wherein R⁶ is
   (a) a C₁₋₆ alkyl group, or
   (b) a saturated or unsaturated carbon ring group having 3 to 14 carbon atoms optionally substituted by one or more, the same or different substituents selected from a halogen atom and a hydroxyl group, or
3) -N (S (=O) ₂-C₁₋₆ alkyl group) -;
   -X³-X³- is -CH₂-CH₂-; and
   ring A' is 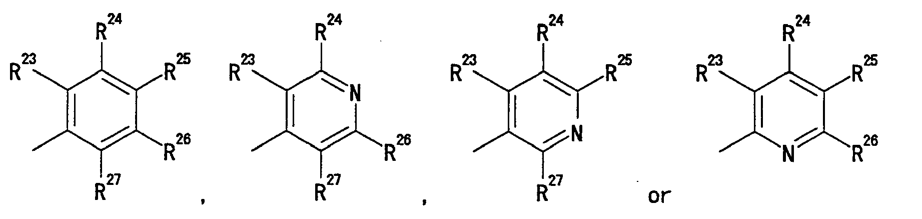 wherein
   R²³ to R²⁷ are the same or different and each is
   1) a hydrogen atom,
   2) a halogen atom,
   3) a hydroxyl group,
   4) a C₁₋₆ alkyl group optionally substituted by one or more, the same or different halogen atoms,
   5) -COOH,
   6) -CO-C₁₋₆ alkoxy group,
   7) an amino group,
   8) -NHS(=O)₂-C₁₋₆ alkyl group, or
   9) a nitro group, and
   at least one of R²³ to R²⁷ is a hydroxyl group;
   or a pharmaceutically acceptable salt thereof.

### <10>

The nitrogen-containing fused ring compound of the above-mentioned <7>, wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -CO-C₁₋₆ alkoxy group,
7) -S(=O)₂-NR¹⁵R¹⁶ wherein R¹⁵ and R¹⁶ are the same or different,
   (a) a C₁₋₆ alkyl group, or
   (b) R¹⁵ and R¹⁶ may form, together with the nitrogen atom they are bonded to, a monocyclic nitrogen-containing saturated heterocycle, or
8) a nitro group;
Y is
1) -CO-, or
2) -CS-;
X¹ is
CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X², is
1) -N(R⁵)- wherein R⁵ is
   (a) a hydrogen atom, or
   (b) a C₁₋₆ alkyl group,
2) -N(COR⁶)- wherein R⁶ is
   (a) a C₁₋₆ alkyl group, or
   (b) a saturated or unsaturated carbon ring group having 3 to 14 carbon atoms optionally substituted by one or more, the same or different substituents selected from a halogen atom and a hydroxyl group, or
3) -N (S (=O) ₂-C₁₋₆ alkyl group) -;
   -X³-X⁴- is -CH₂-CH₂-; and
   ring A' is 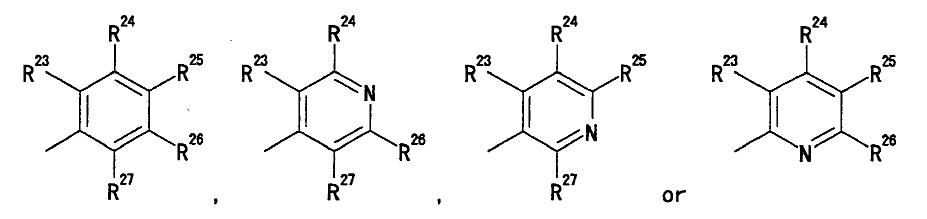
wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms, or
5) a nitro group, and
at least one of R²³ to R²⁷ is a hydroxyl group;
or a pharmaceutically acceptable salt thereof.

### <11>

The nitrogen-containing fused ring compound of the above-mentioned <5>, wherein
X²' is
1) -S (=O) -, or
2) -S (=O)₂-; and
-X³-X⁴- is -CH₂-CH₂-;
or a pharmaceutically acceptable salt thereof.

### <12>

The nitrogen-containing fused ring compound of the above-mentioned <11>, wherein ring A' is 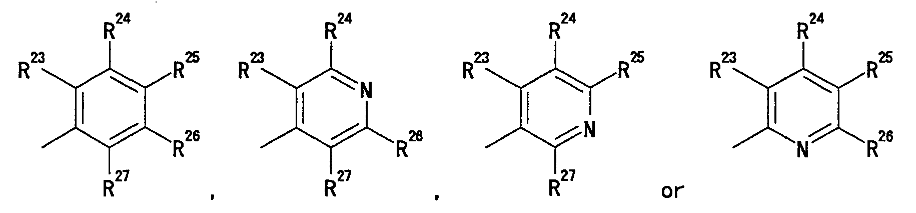 wherein
R²³ to R²⁷ are the same or different and each is
1) a hydrogen atom, or
2) a group selected from group C above, and
at least one of R²³ to R²⁷ is -OR¹³' wherein R¹³' is as defined in the above-mentioned <5>;
or a pharmaceutically acceptable salt thereof.

### <13>

The nitrogen-containing fused ring compound of the above-mentioned <11>,
wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -COOH,
7) -CO-C₁₋₆ alkoxy group,
8) an amino group,
9) -NHS (=O) ₂-C₁₋₆ alkyl group,
10) -S (=O) ₂-C₁₋₆ alkyl group,
11) -S (=O)₂-NR¹⁵R¹⁶ wherein R¹⁵ and R¹⁶ are the same or different and each is
   (a) a hydrogen atom, or
   (b) a C₁₋₆ alkyl group, or
   (c) R¹⁵ and R¹⁶ may form, together with the nitrogen atom they are bonded to, a monocyclic nitrogen-containing saturated heterocycle,
12) a saturated or unsaturated carbon ring group having 3 to 14 carbon atoms, or
13) a nitro group;
Y is
1) -CO-,
2) -CS-, or
3) -S (=O) ₂-;
X¹ is
1) a nitrogen atom, or
2) CR⁴ wherein R⁴ is
   (a) a hydrogen atom,
   (b) a hydroxyl group,
   (c) a C₁₋₆ alkyl group, or
   (d) -CO-C₁₋₆ alkoxy group, or
   (e) R³ and R⁴ may form, together with the carbon atoms they are bonded to, a saturated or unsaturated carbon ring having 3 to 14 carbon atoms;
X², is
1) -S(=O)-, or
2) -S (=O)₂-;
-X³-X⁴- is -CH₂-CH₂-; and
ring A' is 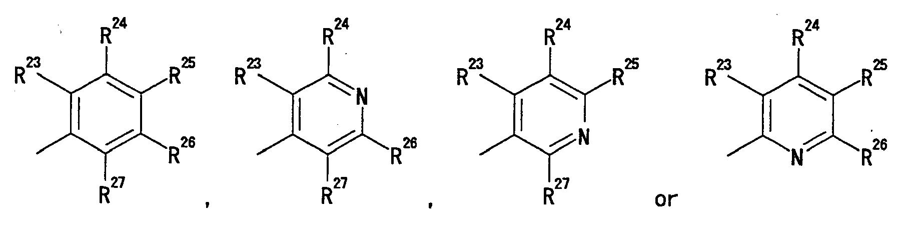 wherein
R²³ to R²⁷ are the same or different and each is
1) a hydrogen atom,
2) a halogen atom,
3) a hydroxyl group,
4) a C₁₋₆ alkoxy group optionally substituted by one or more, the same or different substituents selected from - COOH and -CO-C₁₋₆ alkoxy group,
5) -O-CO-C₁₋₆ alkyl group,
6) a C₁₋₆ alkyl group optionally substituted by one or more, the same or different halogen atoms,
7) -COOH,
8) -CO-C₁₋₆ alkoxy group,
9) an amino group,
10) -NHS(=O)₂-C₁₋₆ alkyl group, or
11) a nitro group, and
at least one of R²³ to R²⁷ is a group selected from a hydroxyl group, a C₁₋₆ alkoxy group optionally substituted by one or more, the same or different substituents selected from -COOH and -CO-C₁₋₆ alkoxy group, and -O-CO-C₁₋₆ alkyl group;
or a pharmaceutically acceptable salt thereof.

### <14>

The nitrogen-containing fused ring compound of the above-mentioned <11>, wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -CO-C₁₋₆ alkoxy group,
7) -S(=O)₂-NR¹⁵R¹⁶ wherein R¹⁵ and R¹⁶ are the same or different,
   (a) a C₁₋₆ alkyl group, or
   (b) R¹⁵ and R¹⁶ may form, together with the nitrogen atom they are bonded to, a monocyclic nitrogen-containing saturated heterocycle, or
8) a nitro group;
Y is
1) -CO-, or
2) -CS-;
X¹ is
CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X²' is
1) -S(=O)-, or
2) -S (=O)₂-;
-X³-X⁴- is -CH₂-CH₂-; and
ring A' is 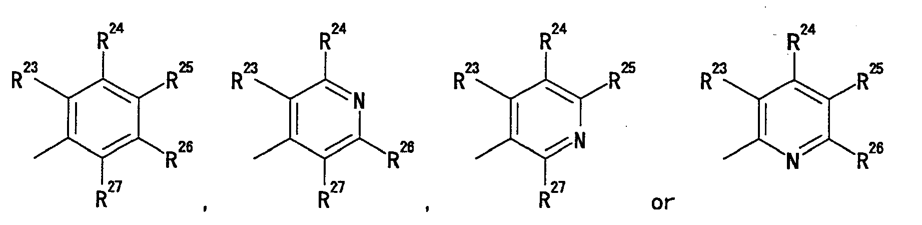 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms, or
5) a nitro group, and
at least one of R²³ to R²⁷ is a hydroxyl group;
or a pharmaceutically acceptable salt thereof.

### <15>

The nitrogen-containing fused ring compound of the above-mentioned <5>, wherein
Y is
1) -CO-, or
2) -CS-,
X², is a sulfur atom;
-X³-X⁴- is -CH₂-CH₂-; and
R¹³' is a hydrogen atom;
or a pharmaceutically acceptable salt thereof.

### <16>

The nitrogen-containing fused ring compound of the above-mentioned <15>, wherein ring A' is 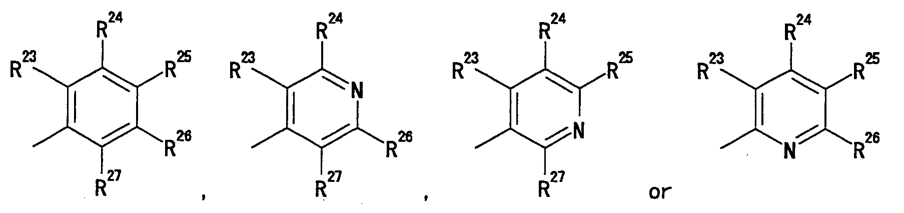 wherein
R²³ to R²⁷ are the same or different and each is
1) a hydrogen atom, or
2) a group selected from group C above, and
at least one of R²³ to R²⁷ is a hydroxyl group;
or a pharmaceutically acceptable salt thereof.

### <17>

The nitrogen-containing fused ring compound of the above-mentioned <15>, wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -COOH,
7) -CO-C₁₋₆ alkoxy group,
8) an amino group,
9) -NHS(=O)₂-C₁₋₆ alkyl group,
10) -S(=O)₂-C₁₋₆alkyl group,
11) -S(=O)₂-NR¹⁵R¹⁶ wherein R¹⁵ and R¹⁶ are the same or different and each is
   (a) a hydrogen atom, or
   (b) a C₁₋₆ alkyl group, or
   (c) R¹⁵ and R¹⁶ may form, together with the nitrogen atom they are bonded to, a monocyclic nitrogen-containing saturated heterocycle,
12) a saturated or unsaturated carbon ring group having 3 to 14 carbon atoms, or
13) a nitro group;
Y is
1) -CO-, or
2) -CS-,
X¹ is
1) a nitrogen atom, or
2) CR⁴ wherein R⁴ is
   (a) a hydrogen atom,
   (b) a hydroxyl group,
   (c) a C₁₋₆ alkyl group, or
   (d) -CO-C₁₋₆ alkoxy group, or
   (e) R³ and R⁴ may form, together with the carbon atoms they are bonded to, a saturated or unsaturated carbon ring having 3 to 14 carbon atoms;
X², is a sulfur atom;
-X³-X⁴- is -CH₂-CH₂-; and
ring A' is 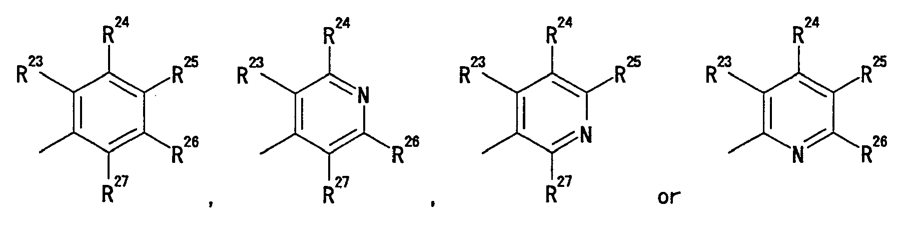 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms,
5) -COOH,
6) -CO-C₁₋₆ alkoxy group,
7) an amino group,
8) -NHS(=O)₂-C₁₋₆ alkyl group, or
9) a nitro group, and
at least one of R²³ to R²⁷ is a hydroxyl group;
or a pharmaceutically acceptable salt thereof.

### <18>

The nitrogen-containing fused ring compound of the above-mentioned <15>, wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -CO-C₁₋₆ alkoxy group,
7) -S (=O)₂-NR¹⁵R¹⁶ wherein R¹⁵ and R¹⁶ are the same or different,
   (a) a C₁₋₆ alkyl group, or
   (b) R¹⁵ and R¹⁶ may form, together with the nitrogen atom they are bonded to, a monocyclic nitrogen-containing saturated heterocycle, or
8) a nitro group;
Y is
1) -CO-, or
2) -CS-;
X¹ is
CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X²' is a sulfur atom;
-X³-X⁴- is -CH₂-CH₂-; and
ring A' is 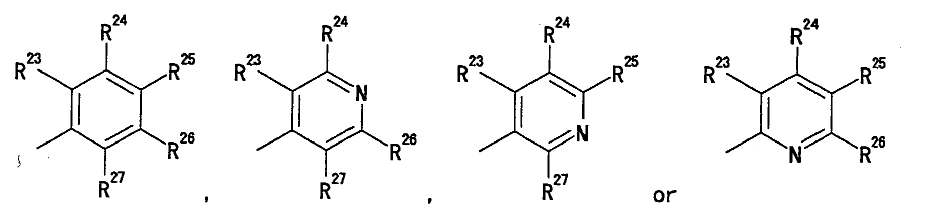 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms, or
5) a nitro group, and
at least one of R²³ to R²⁷ is a hydroxyl group;
or a pharmaceutically acceptable salt thereof.

### <19>

The nitrogen-containing fused ring compound of the above-mentioned <5>, wherein
R¹, R² and R³ are the same or different and each is
1) a hydrogen atom, or
2) a group selected from group D below, or
3) R¹ and R² may form, together with the carbon atoms they are bonded to, a saturated or unsaturated carbon ring having 3 to 14 carbon atoms optionally substituted by one or more, the same or different substituents selected from group D below, or
4) R² and R³ may form, together with the carbon atoms they are bonded to, a saturated or unsaturated carbon ring having 3 to 14 carbon atoms optionally substituted by one or more, the same or different substituents selected from group D below;
Y is
1) -CO-, or
2) -CS-;
x², is an oxygen atom; and
-X³-X⁴- is -CH₂-CH₂-;
or a pharmaceutically acceptable salt thereof:
[group D]
   1) a halogen atom,
   2) -OR¹³'' wherein R¹³'' is
      (a) a hydrogen atom,
      (b) a C₁₋₆ alkyl group optionally substituted by one or more, the same or different substituents selected from group B above, or
      (c) -COR¹⁴'' wherein R¹⁴'' is
         a) a hydrogen atom,
         b) a hydroxyl group,
         c) a C₁₋₆ alkyl group optionally substituted by one or more, the same or different substituents selected from group B above,
         d) a C₁₋₆ alkoxy group optionally substituted by one or more, the same or different substituents selected from (i) and (ii) :
            (i) a substituent selected from group B above,
            (ii) a C₁₋₆ alkyl group optionally substituted by one or more, the same or different substituents selected from group B above,
         e) a saturated or unsaturated carbon ring group having 3 to 14 carbon atoms optionally substituted by one or more, the same or different substituents selected from (i) and (ii) :
            (i) a substituent selected from group B above,
            (ii) a C₁₋₆ alkyl group optionally substituted by one or more, the same or different substituents selected from group B above,
         f) a cycloalkylalkoxy group optionally substituted by one or more, the same or different substituents selected from (i) and (ii) :
            (i) a substituent selected from group B above,
            (ii) a C₁₋₆ alkyl group optionally substituted by one or more, the same or different substituents selected from group B above,
         g) an aralkyl group optionally substituted by one or more, the same or different substituents selected from (i) and (ii) :
            (i) a substituent selected from group B above,
            (ii) a C₁₋₆ alkyl group optionally substituted by one or more, the same or different substituents selected from group B above, or
         h) an aralkoxy group optionally substituted by one or more, the same or different substituents selected from (i) and (ii) :
            (i) a substituent selected from group B above,
            (ii) a C₁₋₆ alkyl group optionally substituted by one or more, the same or different substituents selected from group B above,
   3) a C₁₋₆ alkyl group optionally substituted by one or more, the same or different substituents selected from group B above,
   4) a cycloalkylalkoxy group optionally substituted by one or more, the same or different substituents selected from (a) and (b) :
      (a) a substituent selected from group B above,
      (b) a C₁₋₆ alkyl group optionally substituted by one or more, the same or different substituents selected from group B above,
   5) an aralkyl group optionally substituted by one or more, the same or different substituents selected from (a) and (b):
      (a) a substituent selected from group B above,
      (b) a C₁₋₆ alkyl group optionally substituted by one or more, the same or different substituents selected from group B above,
   6) an aralkoxy group optionally substituted by one or more, the same or different substituents selected from (a) and (b):
      (a) a substituent selected from group B above,
      (b) a C₁₋₆ alkyl group optionally substituted by one or more, the same or different substituents selected from group B above,
   7) -COR¹⁴'' wherein R¹⁴''is as defined above,
   8) -NR¹⁵''¹⁶'' wherein R¹⁵''and R¹⁶'' are the same or different and each is
      (a) a hydrogen atom, or
      (b) a C₁₋₆ alkyl group optionally substituted by one or more, the same or different substituents selected from group B above, or
      (c) R¹⁵'' and R¹⁶'' may form, together with the nitrogen atom they are bonded to, a monocyclic nitrogen-containing saturated heterocycle optionally substituted by one or more, the same or different substituents selected from (i) and (ii) :
         (i) a substituent selected from group B above,
         (ii) a C₁₋₆ alkyl group optionally substituted by one or more, the same or different substituents selected from group B above,
   9) -CONR¹⁵''R¹⁶'' wherein R¹⁵'' and R¹⁶'' are as defined above,
   10) -NR¹⁷''COR¹⁴'' wherein R¹⁴'' is as defined above, and R¹⁷" is
      (a) a hydrogen atom, or
      (b) a C₁₋₆ alkyl group optionally substituted by one or more, the same or different substituents selected from group B above,
   11) -NR¹⁷''(=O)₂R¹⁴'' wherein R¹⁴''and R¹⁷''are as defined above,
   12) -NR¹⁷''ONR¹⁵''¹⁶'' wherein R¹⁵'' R¹⁶'' and R¹⁷'' are as defined above,
   13) -SR¹³'' wherein R¹³'' is as defined above,
   14) -S(=O)R¹⁴ '' wherein R¹⁴'' is as defined above,
   15) -S(=O)₂R¹⁴'' wherein R¹⁴ is as defined above,
   16) -S(=O)₂NR¹⁵''¹⁶'' wherein R¹⁵'' and R¹⁶'' are as defined above,
   17) a saturated or unsaturated carbon ring group having 3 to 14 carbon atoms optionally substituted by one or more, the same or different substituents selected from (a) and (b):
      (a) a substituent selected from group B above,
      (b) a C₁₋₆ alkyl group optionally substituted by one or more, the same or different substituents selected from group B above,
   18) an aryloxy group optionally substituted by one or more, the same or different substituents selected from (a) and (b):
      (a) a substituent selected from group B above,
      (b) a C₁₋₆ alkyl group optionally substituted by one or more, the same or different substituents selected from group B above,
   19) a cyano group, and
   20) a nitro group.

### <20>

The nitrogen-containing fused ring compound of the above-mentioned <19>, wherein ring A' is 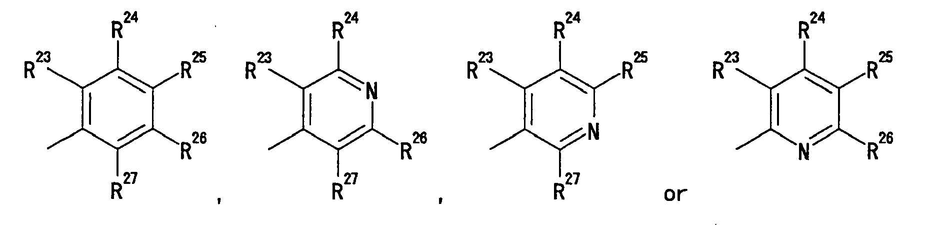 wherein
R²³ to R²⁷ are the same or different and each is
1) a hydrogen atom, or
2) a group selected from group C above, and
at least one of R²³ to R²⁷ is -OR¹³, wherein R¹³' is as defined in the above-mentioned <5>;
or a pharmaceutically acceptable salt thereof.

### <21>

The nitrogen-containing fused ring compound of the above-mentioned <19>,
wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -COOH,
7) -CO-C₁₋₆ alkoxy group,
8) an amino group,
9) -NHS(=O)₂-C₁₋₆ alkyl group,
10) -S (=O)₂-C₁₋₆ alkyl group,
11) -S (=O)₂-NR¹⁵''R¹⁶'' wherein R¹⁵'' and R¹⁶'' are the same or different and each is
   (a) a hydrogen atom, or
   (b) a C₁₋₆ alkyl group, or
   (c) R¹⁵'' and R¹⁶'' may form, together with the nitrogen atom they are bonded to, a monocyclic nitrogen-containing saturated heterocycle,
12) a saturated or unsaturated carbon ring group having 3 to 14 carbon atoms, or
13) a nitro group;
Y is
1) -CO-, or
2) -CS-;
X¹ is
1) a nitrogen atom, or
2) CR⁴ wherein R⁴ is
   (a) a hydrogen atom,
   (b) a hydroxyl group,
   (c) a C₁₋₆ alkyl group, or
   (d) -CO-C₁₋₆ alkoxy group, or
   (e) R³ and R⁴ may form, together with the carbon atoms they are bonded to, a saturated or unsaturated carbon ring having 3 to 14 carbon atoms;
X²' is an oxygen atom;
-X³-X⁴- is -CH₂-CH₂-; and
ring A' is 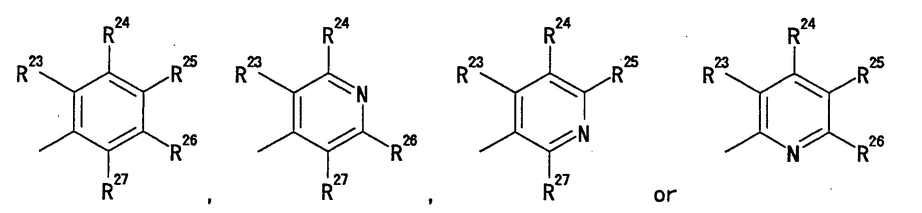 wherein
R²³ to R²⁷ are the same or different and each is
1) a hydrogen atom,
2) a halogen atom,
3) a hydroxyl group,
4) a C₁₋₆ alkoxy group optionally substituted by one or more, the same or different substituents selected from - COOH and -CO-C₁₋₆ alkoxy group,
5) -O-CO-C₁₋₆ alkyl group,
6) a C₁₋₆ alkyl group optionally substituted by one or more, the same or different halogen atoms,
7) -COOH,
8) -CO-C₁₋₆ alkoxy group,
9) an amino group,
10) -NHS (=O)₂-C₁₋₆ alkyl group, or
11) a nitro group, and
at least one of R²³ to R²⁷ is a group selected from a hydroxyl group, a C₁₋₆ alkoxy group optionally substituted by one or more, the same or different substituents selected from -COOH and -CO-C₁₋₆ alkoxy group, and -O-CO-C₁₋₆ alkyl group;
or a pharmaceutically acceptable salt thereof.

### <22>

The nitrogen-containing fused ring compound of the above-mentioned <19>, wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -CO-C₁₋₆ alkoxy group,
7) -S(=O)₂-NR¹⁵''¹⁶'' wherein R¹⁵'' and R¹⁶'' are the same or different,
   (a) a C₁₋₆ alkyl group, or
   (b) R¹⁵" and R¹⁶'' may form, together with the nitrogen atom they are bonded to, a monocyclic nitrogen-containing saturated heterocycle, or
8) a nitro group;
Y is
1) -CO-, or
2) -CS-;
X¹ is
CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X^{2,} is an oxygen atom;
^{_}X^{3_}X⁴- is -CH₂-CH₂-; and
ring A' is 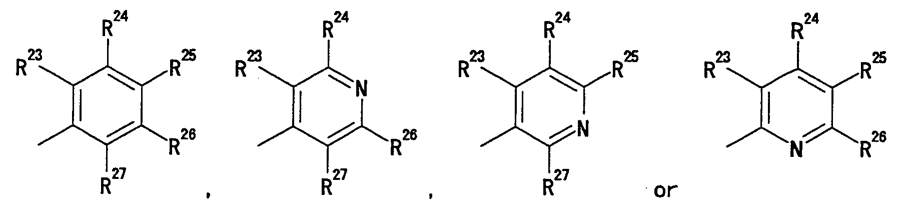 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms, or
5) a nitro group, and
at least one of R²³ to R²⁷ is a hydroxyl group;
or a pharmaceutically acceptable salt thereof.

### <23>

The nitrogen-containing fused ring compound of the above-mentioned <5>, wherein
Y is -S(=O)₂-,
X²' is
1) an oxygen atom, or
2) a sulfur atom;
-X³-X⁴- is -CH₂-CH₂-; and
R¹³' is a hydrogen atom;
or a pharmaceutically acceptable salt thereof.

### <24>

The nitrogen-containing fused ring compound of the above-mentioned <23>, wherein ring A' is 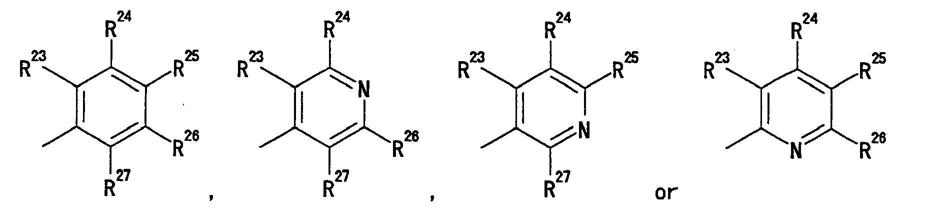 wherein
R²³ to R²⁷ are the same or different and each is
1) a hydrogen atom, or
2) a group selected from group C above, and
at least one of R²³ to R²⁷ is a hydroxyl group;
or a pharmaceutically acceptable salt thereof.

### <25>

The nitrogen-containing fused ring compound of the above-mentioned <23>, wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -COOH,
7) -CO-C₁₋₆ alkoxy group,
8) an amino group,
9) -NHS(=O)₂-C₁₋₆ alkyl group,
10) -S (=O)₂-C₁₋₆ alkyl group,
11) -S(=O)₂-NR¹⁵R¹⁶ wherein R¹⁵ and R¹⁶ are the same or different and each is
   (a) a hydrogen atom, or
   (b) a C₁₋₆ alkyl group, or
   (c) R¹⁵ and R¹⁶ may form, together with the nitrogen atom they are bonded to, a monocyclic nitrogen-containing saturated heterocycle,
12) a saturated or unsaturated carbon ring group having 3 to 14 carbon atoms, or
13) a nitro group;
Y is -S(=O)₂-,
X¹ is
1) a nitrogen atom, or
2) CR⁴ wherein R⁴ is
   (a) a hydrogen atom,
   (b) a hydroxyl group,
   (c) a C₁₋₆ alkyl group, or
   (d) -CO-C₁₋₆ alkoxy group, or
   (e) R³ and R⁴ may form, together with the carbon atoms they are bonded to, a saturated or unsaturated carbon ring having 3 to 14 carbon atoms;
X²' is
1) an oxygen atom, or
2) a sulfur atom;
-X³-X⁴- is -CH₂-CH₂- ; and
ring A' is 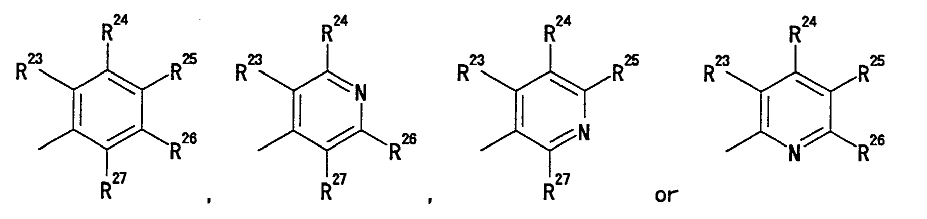 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms,
5) -COOH,
6) -CO-C₁₋₆ alkoxy group,
7) an amino group,
8) -NHS(=O)₂-C₁₋₆ alkyl group, or
9) a nitro group, and
at least one of R²³ to R²⁷ is a hydroxyl group;
or a pharmaceutically acceptable salt thereof.

### <26>

The nitrogen-containing fused ring compound of the above-mentioned <23>, wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -CO-C₁₋₆ alkoxy group,
7) -S(=O)₂-NR¹⁵R¹⁶ wherein R¹⁵ and R¹⁶ are the same or different,
   (a) a C₁₋₆ alkyl group, or
   (b) R¹⁵ and R¹⁶ may form, together with the nitrogen atom they are bonded to, a monocyclic nitrogen-containing saturated heterocycle, or
8) a nitro group;
Y is -S (=O) 2⁻,
X¹ is
CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X^{2,} is
1) an oxygen atom, or
2) a sulfur atom;
-X³-X⁴- is -CH₂-CH₂-; and
ring A' is 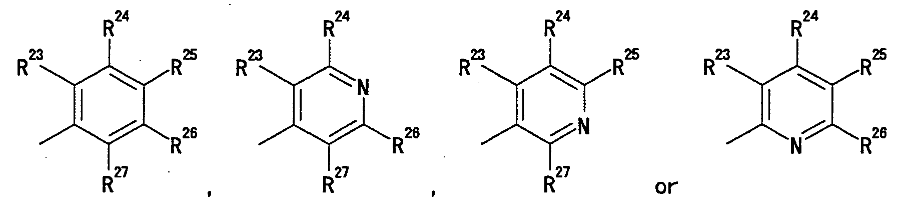 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms, or
5) a nitro group, and
at least one of R²³ to R²⁷ is a hydroxyl group;
or a pharmaceutically acceptable salt thereof.

### <27>

The nitrogen-containing fused ring compound of the above-mentioned <5>, wherein
X²' is -CH₂-;
-X³-X⁴- is
-(CR¹¹R¹²)n- wherein n is an integer of 1 to 3, and R¹¹ and R¹² each in the number of n are the same or different and each is
(a) a hydrogen atom, or
(b) R¹¹ and R¹² bonded to a single carbon atom may in combination form an oxo group, or
(c) two of R¹¹ and R¹² each in the number of n, which are bonded to a single carbon atom or two adjacent carbon atoms, may form, together with the carbon atom(s), a saturated or unsaturated carbon ring having 3 to 14 carbon atoms optionally substituted by one or more, the same or different substituents selected from group A above;
R¹³' is a hydrogen atom; and
ring A' is further substituted by at least one a halogen atom; provided that when both R¹¹ and R¹² are hydrogen atoms, and n is 2, then all of R¹, R² and R³ should be hydrogen atoms, or a pharmaceutically acceptable salt thereof.

### <28>

The nitrogen-containing fused ring compound of the above-mentioned <27>, wherein ring A' is 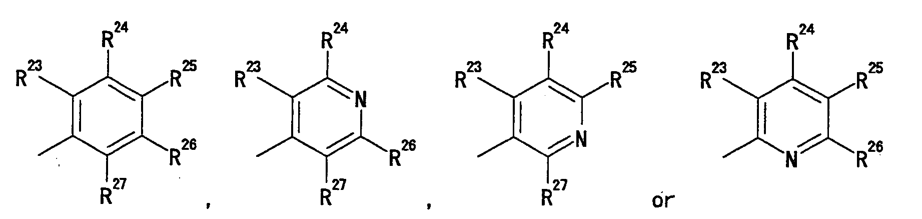 wherein
R²³ to R²⁷ are the same or different and each is
1) a hydrogen atom, or
2) a group selected from group C above, and at least one of R²³ to R²⁷ is a hydroxyl group, and at least one of R²³ to R²⁷ is a halogen atom;
or a pharmaceutically acceptable salt thereof.

### <29>

The nitrogen-containing fused ring compound of the above-mentioned <27>, wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -COOH,
7) -CO-C₁₋₆ alkoxy group,
8) an amino group,
9) -NHS(=O)₂-C₁₋₆ alkyl group,
10) -S(=O)₂-C₁₋₆ alkyl group,
11) -S(=O)₂-NR¹⁵R¹⁶ wherein R¹⁵ and R¹⁶ are the same or different and each is
   (a) a hydrogen atom, or
   (b) a C₁₋₆ alkyl group, or
   (c) R¹⁵ and R¹⁶ may form, together with the nitrogen atom they are bonded to, a monocyclic nitrogen-containing saturated heterocycle,
12) a saturated or unsaturated carbon ring group having 3 to 14 carbon atoms, or
13) a nitro group;
Y is
1) -CO-,
2) -CS-, or
3) -S(=O)₂-;
X¹ is
1) a nitrogen atom, or
2) CR⁴ wherein R⁴ is
   (a) a hydrogen atom,
   (b) a hydroxyl group,
   (c) a C₁₋₆ alkyl group, or
   (d) -CO-C₁₋₆ alkoxy group, or
   (e) R³ and R⁴ may form, together with the carbon atoms they are bonded to, a saturated or unsaturated carbon ring having 3 to 14 carbon atoms;
X^{2,} is -CH₂-;
-X³-X⁴- is
- (CR¹¹R¹²) n- wherein n is an integer of 1 to 3, and R¹¹ and R¹² each in the number of n are the same or different and each is
(a) a hydrogen atom, or
(b) R¹¹ and R¹² bonded to a single carbon atom may in combination form an oxo group, or
(c) two of R¹¹ and R¹² each in the number of n, which are bonded to a single carbon atom or two adjacent carbon atoms, may form, together with the carbon atom(s), a saturated or unsaturated carbon ring having 3 to 14 carbon atoms optionally substituted by one or more, the same or different substituents selected from group A above; and
ring A' is 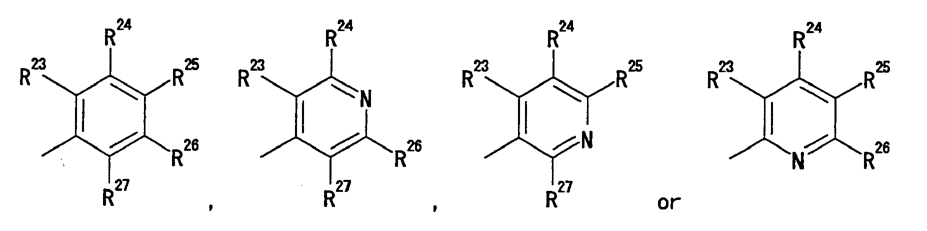 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms,
5) -COOH,
6) -CO-C₁₋₆ alkoxy group,
7) an amino group,
8) -NHS(=O)₂-C₁₋₆ alkyl group, or
9) a nitro group, and at least one of R²³ to R²⁷ is a hydroxyl group, and at least one of R²³ to R²⁷ is a halogen atom; provided that when both R¹¹ and R¹² are hydrogen atoms, and n is 2, then all of R¹, R² and R³ should be hydrogen atoms, or a pharmaceutically acceptable salt thereof.

### <30>

⁵ The nitrogen-containing fused ring compound of the above-mentioned <27>, wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -CO-C₁₋₆ alkoxy group,
7) -S(=O)₂-NR¹⁵R¹⁶ wherein R¹⁵ and R¹⁶ are the same or different,
   (a) a C₁₋₆ alkyl group, or
   (b) R¹⁵ and R¹⁶ may form, together with the nitrogen atom they are bonded to, a monocyclic nitrogen-containing saturated heterocycle, or
8) a nitro group;
Y is
1) -CO-, or
2) -CS-;
X¹ is
CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X²' is -CH₂-;
-X³-X⁴- is
- (CR¹¹R¹²) n- wherein n is an integer of 1 to 3, and R¹¹ and R¹² each in the number of n are the same or different and each
is
(a) a hydrogen atom, or
(b) R¹¹ and R¹² bonded to a single carbon atom may in combination form an oxo group, or
(c) two of R¹¹ and R¹² each in the number of n, which are bonded to a single carbon atom or two adjacent carbon atoms, may form, together with the carbon atom(s), a saturated or unsaturated carbon ring having 3 to 14 carbon atoms optionally substituted by one or more, the same or different substituents selected from group A above; and
ring A' is 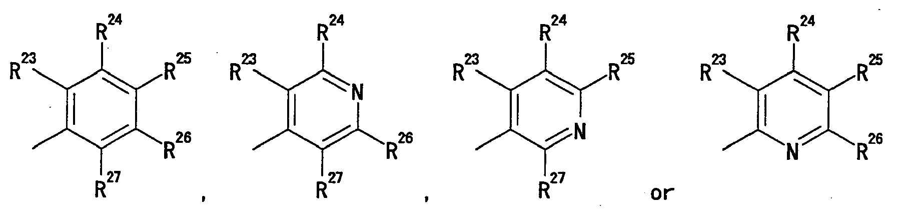 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms, or
5) a nitro group, and at least one of R²³ to R²⁷ is a hydroxyl group, and at least one of R²³ to R²⁷ should be a halogen atom;
provided that when both R¹¹ and R¹² are hydrogen atoms, and n is 2, then all of R¹, R² and R³ should be hydrogen atoms, or a pharmaceutically acceptable salt thereof.

### <31>

The nitrogen-containing fused ring compound of the above-mentioned <5>, which is selected from the group consisting of
(1) (3-chloro-4-hydroxyphenyl)-(2,3-dihydrobenzo[1,4]oxazin-4-yl)-methanone,
(2) (3-bromo-4-hydroxyphenyl)-(2,3-dihydrobenzo[1,4]oxazin-4-yl)-methanone,
(3) (3,5-dichloro-4-hydroxyphenyl)-(2,3-dihydrobenzo[1,4]oxazin-4-yl)-methanone,
(4) (3,5-dibromo-4-hydroxyphenyl)-(2,3-dihydrobenzo[1,4]oxazin-4-yl)-methanone,
(5) (2,3-dihydrobenzo[1,4]oxazin-4-yl)-(4-hydroxy-3,5-diiodophenyl)-methanone,
(6) (3,5-difluoro-4-hydroxyphenyl)-(2,3-dihydrobenzo[1,4]oxazin-4-yl)-methanone,
(7) (2,3-dihydrobenzo[1,4]oxazin-4-yl)-(4-hydroxy-3,5-dimethylphenyl)-methanone,
(8) (3,5-dichloro-4-hydroxyphenyl)-(2,3-dihydrobenzo[1,4]thiazin-4-yl)-methanone,
(9) (3,5-dichloro-4-hydroxyphenyl)-(1-oxo-2,3-dihydro-1H-1λ⁴-benzo[1,4]thiazin-4-yl)-methanone,
(10) (3,5-dichloro-4-hydroxyphenyl)-(1,1-dioxo-2,3-dihydro-1H-1λ⁶-benzo[1,4]thiazin-4-yl)-methanone,
(11) (3,5-dichloro-4-hydroxyphenyl)-(2,3-dihydrobenzo[1,4]oxazin-4-yl)-methanethione,
(12) (3,5-dichloro-4-hydroxyphenyl)-(6-methyl-2,3-dihydrobenzo[1,4]oxazin-4-yl)-methanone,
(13) (3,5-dichloro-4-hydroxyphenyl)-(7-methyl-2,3-dihydrobenzo[1,4]oxazin-4-yl)-methanone,
(14) (3,5-dichloro-4-hydroxyphenyl)-(5-methyl-2,3-dihydrobenzo[1,4]oxazin-4-yl)-methanone,
(15) (3,5-dichloro-4-hydroxyphenyl)-(8-methyl-2,3-dihydrobenzo[1,4]oxazin-4-yl)-methanone,
(16) (3,5-dichloro-4-hydroxyphenyl)-(2,3-dihydronaphtho[2,1-b][1,4]oxazin-1-yl)-methanone,
(17) (3,5-dichloro-4-hydroxyphenyl)-(6-methoxy-2,3-dihydrobenzo[1,4]oxazin-4-yl)-methanone,
(18) (3,5-dichloro-4-hydroxyphenyl)-(7-methoxy-2,3-dihydrobenzo[1,4]oxazin-4-yl)-methanone,
(19) (3,5-dichloro-4-hydroxyphenyl)-(6-hydroxy-2,3-dihydrobenzo[1,4]oxazin-4-yl)-methanone,
(20) (3,5-dichloro-4-hydroxyphenyl)-(7-hydroxy-2,3-dihydrobenzo[1,4]oxazin-4-yl)-methanone,
(21) 4-(3,5-dichloro-4-hydroxybenzoyl)-3,4-dihydro-2H-benzo[1,4]oxazine-6-sulfonic acid diethylamide,
(22) 2,6-dichloro-4-(2,3-dihydrobenzo[1,4]oxazine-4-sulfonyl)phenol,
(23) (6-tert-butyl-2,3-dihydrobenzo[1,4]oxazin-4-yl)-(3,5-dichloro-4-hydroxyphenyl)-methanone,
(24) 4-(3,5-dichloro-4-hydroxybenzoyl)-4H-benzo[1,4]oxazin-3-one,
(25) 4-(3,5-dichloro-4-hydroxybenzoyl)-3,4-dihydro-2H-benzo[1,4]oxazine-6-sulfonamide,
(26) (3,5-dichloro-4-hydroxyphenyl)-(3,4-dihydro-2H-quinolin-1-yl)-methanone,
(27) (3,5-dichloro-4-hydroxyphenyl)-(2,3,4,5-tetrahydrobenzo[b]azepin-1-yl)-methanone,
(28) (4-amino-3,5-dichlorophenyl)-(2,3-dihydrobenzo[1,4]oxazin-4-yl)-methanone,
(29) (5-chloro-6-hydroxypyridin-3-yl)-(2,3-dihydrobenzo[1,4]oxazin-4-yl)-methanone,
(30) (2,3-dihydrobenzo[1,4]oxazin-4-yl)-(4-hydroxy-3,5-dinitrophenyl)-methanone,
(31) (3-chloro-4-hydroxy-5-nitrophenyl)-(2,3-dihydrobenzo[1,4]oxazin-4-yl)-methanone,
(32) (3,5-dichloro-4-hydroxyphenyl)-(2,8-diisopropyl-2,3-dihydrobenzo[1,4]oxazin-4-yl)-methanone,
(33) (3,5-dichloro-4-hydroxyphenyl)-[6-(pyrrolidine-1-sulfonyl)-2,3-dihydrobenzo [1,4] oxazin-4-yl]-methanone,
(34) 4-(3,5-dichloro-4-hydroxybenzoyl)-3,4-dihydro-2H-benzo[1,4]oxazine-6-sulfonic acid ethylamide,
(35) 4-(3,5-dichloro-4-hydroxybenzoyl)-3,4-dihydro-2H-benzo [1,4] oxazine-6-sulfonic acid dimethylamide,
(36) (3,5-dichloro-4-hydroxyphenyl)-(2,3-dihydropyrido [3,2-b][1,4]oxazin-4-yl)-methanone,
(37) 5-(3,5-dichloro-4-hydroxybenzoyl)-1,3,4,5-tetrahydrobenzo [b] [1,4] diazepin-2-one,
(38) (3,5-dichloro-2-hydroxyphenyl)-(2,3-dihydrobenzo[1,4]oxazin-4-yl)-methanone,
(39) (2,3-dihydrobenzo[1,4]oxazin-4-yl)-(4-hydroxy-3-trifluoromethylphenyl)-methanone,
(40) (3-chloro-4-hydroxy-5-methoxyphenyl)-(2,3-dihydrobenzo[1,4]oxazin-4-yl)-methanone,
(41) (4-chloro-3-hydroxyphenyl)-(2,3-dihydrobenzo[1,4]oxazin-4-yl)-methanone,
(42) (2,6-dichloropyridin-4-yl)-(2,3-dihydrobenzo[1,4]oxazin-4-yl)-methanone,
(43) (2,3-dihydrobenzo[1,4]oxazin-4-yl)-(4-nitrophenyl)-methanone,
(44) (3,5-dichloro-4-hydroxyphenyl)-(6-fluoro-2,3-dihydrobenzo[1,4]oxazin-4-yl)-methanone,
(45) 2-(2,3-dihydrobenzo[1,4] oxazine-4-carbonyl) benzoic acid,
(46) methyl 4-(2,3-dihydrobenzo[1,4]oxazine-4-carbonyl)benzoate,
(47) 4-(2,3-dihydrobenzo[1,4]oxazine-4-carbonyl)benzoic acid,
(48) methyl 3-(2,3-dihydrobenzo[1,4]oxazine-4-carbonyl)benzoate,
(49) 3-(2,3-dihydrobenzo[1,4]oxazine-4-carbonyl)benzoic acid,
(50) (3,5-dichloro-2,4-dihydroxyphenyl)-(2,3-dihydrobenzo[1,4]oxazin-4-yl)-methanone,
(51) (6-chloro-2,3-dihydrobenzo[1,4]oxazin-4-yl)-(3,5-dichloro-4-hydroxyphenyl)-methanone,
(52) (7-chloro-2,3-dihydrobenzo[1,4]oxazin-4-yl)-(3,5-dichloro-4-hydroxyphenyl)-methanone,
(53) [4-(3,5-dichloro-4-hydroxybenzoyl)-3,4-dihydro-2H-quinoxalin-1-yl]-(3,5-dichloro-4-hydroxyphenyl)-methanone,
(54) (3,5-dichloro-4-hydroxyphenyl)-(3,4-dihydro-2H-quinoxalin-1-yl)-methanone,
(55) methyl 4-(3,5-dichloro-4-hydroxybenzoyl)-3,4-dihydro-2H-benzo[1,4]oxazine-6-carboxylate,
(56) (3,5-dichloro-4-hydroxyphenyl)-(6-hydroxymethyl-2,3-dihydrobenzo[1,4]oxazin-4-yl)-methanone,
(57) 4-(3,5-dichloro-4-hydroxybenzoyl)-3,4-dihydro-2H-benzo[1,4]oxazine-6-carboxylic acid,
(58) methyl 4-(3,5-dichloro-4-hydroxybenzoyl)-3,4-dihydro-2H-benzo[1,4]oxazine-5-carboxylate,
(59) methyl 4-(3,5-dichloro-4-hydroxybenzoyl)-3,4-dihydro-2H-benzo[1,4]oxazine-7-carboxylate,
(60) 4-(3,5-dichloro-4-hydroxybenzoyl)-3,4-dihydro-2H-benzo[1,4]oxazine-7-carboxylic acid,
(61) methyl 4-(3,5-dichloro-4-hydroxybenzoyl)-3,4-dihydro-2H-benzo[1,4]oxazine-8-carboxylate,
(62) 4-(3,5-dichloro-4-hydroxybenzoyl)-3,4-dihydro-2H-benzo[1,4]oxazine-8-carboxylic acid,
(63) (3,5-dichlorophenyl)-(2,3-dihydrobenzo[l,4]oxazin-4-yl)-methanone,
(64) (3,5-dichloro-4-hydroxyphenyl)-(phenoxazin-10-yl)-methanone,
(65) (3,5-dichloro-4-hydroxyphenyl)-(6-phenyl-2,3-dihydrobenzo[1,4]oxazin-4-yl)-methanone,
(66) (3,5-dichloro-4-hydroxyphenyl)-(6,8-dimethyl-2,3-dihydrobenzo[1,4]oxazin-4-yl)-methanone,
(67) (3,5-dichloro-4-hydroxyphenyl)-(6-nitro-2,3-dihydrobenzo[1,4]oxazin-4-yl)-methanone,
(68) (6-amino-2,3-dihydrobenzo[1,4]oxazin-4-yl)-(3,5-dichloro-4-hydroxyphenyl)-methanone,
(69) (3,5-dibromo-4-hydroxyphenyl)-(6-nitro-2,3-dihydrobenzo[1,4]oxazin-4-yl)-methanone,
(70) (3,5-dichloro-4-hydroxyphenyl)-(7-nitro-2,3-dihydrobenzo[1,4]oxazin-4-yl)-methanone,
(71) (7-amino-2,3-dihydrobenzo[1,4]oxazin-4-yl)-(3,5-dichloro-4-hydroxyphenyl)-methanone,
(72) N-[4-(3,5-dichloro-4-hydroxybenzoyl)-3,4-dihydro-2H-benzo[1,4]oxazin-7-yl]-methanesulfonamide,
(73) 1-[4-(3,5-dichloro-4-hydroxybenzoyl)-3,4-dihydro-2H-quinoxalin-1-yl]-ethanone,
(74) (3,5-dichloro-4-hydroxyphenyl)-(4-methyl-3,4-dihydro-2H-quinoxalin-1-yl)-methanone,
(75) (2,3-dihydrobenzo[1,4]oxazin-4-yl)-(4-hydroxy-3-nitrophenyl)-methanone,
(76) (3,5-dichloro-4-hydroxyphenyl)-(2-methyl-2,3-dihydroindol-1-yl)-methanone,
(77) (3,5-dichloro-4-hydroxyphenyl)-(2,3-dihydroindol-1-yl)-methanone,
(78) (5-amino-2,3-dihydroindol-1-yl)-(3,5-dichloro-4-hydroxyphenyl)-methanone,
(79) (3,5-dibromo-4-hydroxyphenyl)-(6-fluoro-2,3-dihydrobenzo[1,4]oxazin-4-yl)-methanone,
(80) (3,5-dibromo-4-hydroxyphenyl)-(2,3-dihydronaphtho[2,1-b][1,4]oxazin-1-yl)-methanone,
(81) (3,5-dibromo-4-hydroxyphenyl)-(6-methyl-2,3-dihydrobenzo[1,4]oxazin-4-yl)-methanone,
(82) (6-chloro-2,3-dihydrobenzo[1,4]oxazin-4-yl)-(3,5-dibromo-4-hydroxyphenyl)-methanone,
(83) (3,5-dichloro-4-hydroxyphenyl)-(4-methanesulfonyl-3,4-dihydro-2H-quinoxalin-1-yl)-methanone,
(84) (3,5-dichloro-4-hydroxyphenyl)-(6-ethanesulfonyl-2,3-dihydrobenzo[1,4]oxazin-4-yl)-methanone,
(85) (3,5-dichloro-4-hydroxyphenyl)-(6-trifluoromethyl-2,3-dihydrobenzo[1,4]oxazin-4-yl)-methanone,
(86) (3,5-dichloro-4-methoxyphenyl)-(2,3-dihydrobenzo[1,4]oxazin-4-yl)-methanone,
(87) 2,6-dichloro-4-(2,3-dihydrobenzo[1,4]oxazine-4-carbonyl)phenyl acetate,
(88) (2,3-dihydrobenzo[1,4]oxazin-4-yl)-(4-hydroxyphenyl)-methanone,
(89) (3,5-dichloro-4-hydroxyphenyl)-(5-hydroxy-2,3-dihydrobenzo[1,4]oxazin-4-yl)-methanone,
(90) (3,5-dichloro-4-hydroxyphenyl)-(8-hydroxy-2,3-dihydrobenzo[1,4]oxazin-4-yl)-methanone,
(91) ethyl [2,6-dichloro-4-(2,3-dihydrobenzo[1,4]oxazine-4-carbonyl)phenoxy]acetate,
(92) [2,6-dichloro-4-(2,3-dihydrobenzo[1,4]oxazine-4-carbonyl)phenoxy]acetic acid
(93) (3,5-dichloro-4-hydroxyphenyl)-(3-methyl-2,3-dihydrobenzo[1,4]oxazin-4-yl)-methanone,
(94) N-[2,6-dichloro-4-(2,3-dihydrobenzo[1,4]oxazine-4-carbonyl)phenyl]methanesulfonamide, and
(95) (3,5-dichloro-4-hydroxyphenyl)-(7,8-dihydro-6H-5-oxa-9-azabenzocyclohepten-9-yl)-methanone,
or a pharmaceutically acceptable salt thereof.

### <32>

A pharmaceutical composition comprising a compound of any of the above-mentioned <5> to <31> or a pharmaceutically acceptable salt thereof.

### <33>

A URAT1 activity inhibitor comprising a compound of any of the above-mentioned <5> to <31> or a pharmaceutically acceptable salt thereof.

### <34>

An agent for lowering a blood uric acid level comprising the substance in any of the above-mentioned <1> to <3>.

### <35>

An agent for lowering a blood uric acid level comprising the compound in the above-mentioned <4> or a pharmaceutically acceptable salt thereof.

### <36>

An agent for lowering a blood uric acid level comprising a compound of any of the above-mentioned <5> to <31> or a pharmaceutically acceptable salt thereof.

### <37>

An agent for the prophylaxis or treatment of pathology showing involvement of uric acid, comprising the substance in any of the above-mentioned <1> to <3>.

### <38>

An agent for the prophylaxis or treatment of pathology showing involvement of uric acid, which comprises the compound in the above-mentioned <4> or a pharmaceutically acceptable salt thereof.

### <39>

An agent for the prophylaxis or treatment of pathology showing involvement of uric acid, which comprises a compound of any of the above-mentioned <5> to <31> or a pharmaceutically acceptable salt thereof.

### <40>

The agent of any of the above-mentioned <37> to <39>, wherein the pathology showing involvement of uric acid is hyperuricemia, gouty tophus, acute gouty arthritis, chronic gouty arthritis, gouty kidney, urolithiasis, renal function disorder, coronary artery disease or ischemic heart disease.

### <41>

A method for the prophylaxis or treatment of pathology showing involvement of uric acid, which comprises administering a pharmaceutically effective amount of the substance in any of the above-mentioned <1> to <3>.

### <42>

A method for the prophylaxis or treatment of pathology showing involvement of uric acid, which comprises administering a pharmaceutically effective amount of the compound in the above-mentioned <4> or a pharmaceutically acceptable salt thereof.

### <43>

A method for the prophylaxis or treatment of pathology showing involvement of uric acid, which comprises administering a pharmaceutically effective amount of a compound of any of the above-mentioned <5> to <31> or a pharmaceutically acceptable salt thereof.

### <44>

The method of any of the above-mentioned <41> to <43>, wherein the pathology showing involvement of uric acid is hyperuricemia, gouty tophus, acute gouty arthritis, chronic gouty arthritis, gouty kidney, urolithiasis, renal function disorder, coronary artery disease or ischemic heart disease.

### <45>

A method for inhibiting URAT1 activity, which comprises administering a pharmaceutically effective amount of the substance in any of the above-mentioned <1> to <3>.

### <46>

A method for inhibiting URAT1 activity, which comprises administering a pharmaceutically effective amount of the compound in the above-mentioned <4> or a pharmaceutically acceptable salt thereof.

### <47>

A method for inhibiting URAT1 activity, which comprises administering a pharmaceutically effective amount of a compound of any of the above-mentioned <5> to <31> or a pharmaceutically acceptable salt thereof.

### <48>

A method of lowering a blood uric acid level, which comprises administering a pharmaceutically effective amount of the substance in any of the above-mentioned <1> to <3>.

### <49>

A method of lowering a blood uric acid level, which comprises administering a pharmaceutically effective amount of the compound in the above-mentioned <4> or a pharmaceutically acceptable salt thereof.

### <50>

A method of lowering a blood uric acid level, which comprises administering a pharmaceutically effective amount of a compound of any of the above-mentioned <5> to <31> or a pharmaceutically acceptable salt thereof.

### <51>

Use of the substance in any of the above-mentioned <1> to <3> for the production of an agent for the prophylaxis or treatment of pathology showing involvement of uric acid.

### <52>

Use of the compound in the above-mentioned <4> or a pharmaceutically acceptable salt thereof for the production of an agent for the prophylaxis or treatment of pathology showing involvement of uric acid.

### <53>

Use of a compound of any of the above-mentioned <5> to <31> or a pharmaceutically acceptable salt thereof for the production of an agent for the prophylaxis or treatment of pathology showing involvement of uric acid.

### <54>

Use of any of the above-mentioned <51> to <53>, wherein the pathology showing involvement of uric acid is hyperuricemia, gouty tophus, acute gouty arthritis, chronic gouty arthritis, gouty kidney, urolithiasis, renal function disorder, coronary artery disease or ischemic heart disease.

### <55>

Use of the substance in any of the above-mentioned <1> to <3> for the production of a URAT1 activity inhibitor.

### <56>

Use of the compound in the above-mentioned <4> or a pharmaceutically acceptable salt thereof for the production of a URAT1 activity inhibitor.

### <57>

Use of a compound of any of the above-mentioned <5> to <31> or a pharmaceutically acceptable salt thereof for the production of a URAT1 activity inhibitor.

### <58>

Use of the substance in any of the above-mentioned <1> to <3> for the production of an agent for lowering a blood uric acid level.

### <59>

Use of the compound in the above-mentioned <4> or a pharmaceutically acceptable salt thereof for the production of an agent for lowering a blood uric acid level.

### <60>

Use of a compound of any of the above-mentioned <5> to <31> or a pharmaceutically acceptable salt thereof for the production of an agent for lowering a blood uric acid level.

### <61>

A pharmaceutical composition for the prophylaxis or treatment of pathology showing involvement of uric acid, which comprises the substance in any of the above-mentioned <1> to <3>.

### <62>

A pharmaceutical composition for the prophylaxis or treatment of pathology showing involvement of uric acid, which comprises the compound in the above-mentioned <4> or a pharmaceutically acceptable salt thereof.

### <63>

A pharmaceutical composition for the prophylaxis or treatment of pathology showing involvement of uric acid, which comprises a compound of any of the above-mentioned <5> to <31> or a pharmaceutically acceptable salt thereof.

### <64>

The pharmaceutical composition of any of the above-mentioned <61> to <63>, wherein the pathology showing involvement of uric acid is hyperuricemia, gouty tophus, acute gouty arthritis, chronic gouty arthritis, gouty kidney, urolithiasis, renal function disorder, coronary artery disease or ischemic heart disease.

### <65>

A commercial package comprising the pharmaceutical composition of any of the above-mentioned <61> to <64>, and a written matter associated therewith, the written matter stating that the pharmaceutical composition can or should be used for the prophylaxis or treatment of a disease selected from hyperuricemia, gouty tophus, acute gouty arthritis, chronic gouty arthritis, gouty kidney, urolithiasis, renal function disorder, coronary artery disease and ischemic heart disease.

### <66>

The pharmaceutical composition of the above-mentioned <32>, which is used in combination with 1 to 3 agents selected from groups (1) to (11) :
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease that causes decreased uric acid excretion secondary hyperuricemia,
(10) an agent for the prophylaxis and/or treatment of kidney failure, cardiovascular disorder or cerebrovascular disorder caused by hyperuricemia, and
(11) a nucleic acid metabolic antagonist.

### <67>

The inhibitor of any of the above-mentioned <1> to <4> and <33>, which is used in combination with 1 to 3 agents selected from groups (1) to (11):
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease that causes decreased uric acid excretion secondary hyperuricemia,
(10) an agent for the prophylaxis and/or treatment of kidney failure, cardiovascular disorder or cerebrovascular disorder caused by hyperuricemia, and
(11) a nucleic acid metabolic antagonist.

### <68>

The agent of any of the above-mentioned <34> to <36>, which is used in combination with 1 to 3 agents selected from groups (1) to (11) :
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease that causes decreased uric acid excretion secondary hyperuricemia,
(10) an agent for the prophylaxis and/or treatment of kidney failure, cardiovascular disorder or cerebrovascular disorder caused by hyperuricemia, and
(11) a nucleic acid metabolic antagonist.

### <69>

The agent of any of the above-mentioned <37> to <40>, which is used in combination with 1 to 3 agents selected from groups (1) to (11):
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease that causes decreased uric acid excretion secondary hyperuricemia,
(10) an agent for the prophylaxis and/or treatment of kidney failure, cardiovascular disorder or cerebrovascular disorder caused by hyperuricemia, and
(11) a nucleic acid metabolic antagonist.

### <70>

The method of any of the above-mentioned <41> to <44>, which further comprises administering 1 to 3 agents selected from groups (1) to (11):
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease that causes decreased uric acid excretion secondary hyperuricemia,
(10) an agent for the prophylaxis and/or treatment of kidney failure, cardiovascular disorder or cerebrovascular disorder caused by hyperuricemia, and
(11) a nucleic acid metabolic antagonist.

### <71>

The method of any of the above-mentioned <45> to <47>, which further comprises administering 1 to 3 agents selected from groups (1) to (11):
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease that causes decreased uric acid excretion secondary hyperuricemia,
(10) an agent for the prophylaxis and/or treatment of kidney failure, cardiovascular disorder or cerebrovascular disorder caused by hyperuricemia, and
(11) a nucleic acid metabolic antagonist.

### <72>

The method of any of the above-mentioned <48> to <50>, which further comprises administering 1 to 3 agents selected from groups (1) to (11):
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease that causes decreased uric acid excretion secondary hyperuricemia,
(10) an agent for the prophylaxis and/or treatment of kidney failure, cardiovascular disorder or cerebrovascular disorder caused by hyperuricemia, and
(11) a nucleic acid metabolic antagonist.

### <73>

The use of any of the above-mentioned <51> to <54>, wherein the agent is used in combination with 1 to 3 agents selected from groups (1) to (11):
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease that causes decreased uric acid excretion secondary hyperuricemia,
(10) an agent for the prophylaxis and/or treatment of kidney failure, cardiovascular disorder or cerebrovascular disorder caused by hyperuricemia, and
(11) a nucleic acid metabolic antagonist.

### <74>

The use of any of the above-mentioned <55> to <57>, wherein the agent is used in combination with 1 to 3 agents selected from groups (1) to (11):
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease that causes decreased uric acid excretion secondary hyperuricemia,
(10) an agent for the prophylaxis and/or treatment of kidney failure, cardiovascular disorder or cerebrovascular disorder caused by hyperuricemia, and
(11) a nucleic acid metabolic antagonist.

### <75>

The use of any of the above-mentioned <58> to <60>, wherein the agent is used in combination with 1 to 3 agents selected from groups (1) to (11):
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease that causes decreased uric acid excretion secondary hyperuricemia,
(10) an agent for the prophylaxis and/or treatment of kidney failure, cardiovascular disorder or cerebrovascular disorder caused by hyperuricemia, and
(11) a nucleic acid metabolic antagonist.

### <76>

The pharmaceutical composition of the above-mentioned <32>, which is used for suppressing an increase in the blood uric acid level in combination with a pharmaceutical agent that increases the blood uric acid level.

### <77>

The inhibitor of any of the above-mentioned <1> to <4> and <33>, which is used for suppressing an increase in the blood uric acid level in combination with a pharmaceutical agent that increases the blood uric acid level.

### <78>

The method of any of the above-mentioned <45> to <47>, which further comprises administering a pharmaceutical agent that increases the blood uric acid level.

### <79>

The use of any of the above-mentioned <55> to <57> for the production of a URAT1 activity inhibitor, which is used for suppressing an increase in the blood uric acid level in combination with a pharmaceutical agent that increases the blood uric acid level.

### <80>

A method for suppressing a blood uric acid level, which comprises administering the inhibitor of any of the above-mentioned <1> to <4> and <33> in combination with a pharmaceutical agent that increases the blood uric acid level.

The present inventors have also found a crystal of the above-mentioned compound. Accordingly, the present invention encompasses the following crystal and a pharmaceutical composition comprising the crystal.

### <81>

A crystal of the nitrogen-containing fused ring compound which is selected from the group consisting of
(1) (3-chloro-4-hydroxyphenyl)-(2,3-dihydrobenzo[1,4]oxazin-4-yl)-methanone having characteristic diffraction peaks at 12.94°, 17.36°, 23.50°, 26.10° and 26.94° of diffraction angle (2θ) as measured by powder X-ray crystal diffraction (Example 1),
(2) (3-bromo-4-hydroxyphenyl)-(2,3-dihydrobenzo[1,4]oxazin-4-yl)-methanone having characteristic diffraction peaks at 16.96°, 17.54°, 21.66°, 25.68° and 26.62° of diffraction angle (2θ) as measured by powder X-ray crystal diffraction (Example 2),
(3) (3,5-dichloro-4-hydroxyphenyl)-(2,3-dihydrobenzo[1,4]oxazin-4-yl)-methanone having characteristic diffraction peaks at 7.40°, 14.90°, 22.68°, 22.92° and 26.46° of diffraction angle (2θ) as measured by powder X-ray crystal diffraction (Example 3),
(4) (3,5-dibromo-4-hydroxyphenyl)-(2,3-dihydrobenzo[1,4]oxazin-4-yl)-methanone having characteristic diffraction peaks at 7.40_{°}, 14.92°, 16.64°, 22.68° and 26.12° of diffraction angle (2θ) as measured by powder X-ray crystal diffraction (Example 4),
(5) (2,3-dihydrobenzo[1,4]oxazin-4-yl)-(4-hydroxy-3,5-diiodophenyl)-methanone having characteristic diffraction peaks at 7.28°, 15.84°, 23.10°, 29.54° and 37.16° of diffraction angle (2θ) as measured by powder X-ray crystal diffraction (Example 5),
(6) (3,5-difluoro-4-hydroxyphenyl)-(2,3-dihydrobenzo[1,4]oxazin-4-yl)-methanone having characteristic diffraction peaks at 15.58°, 17.92°, 18.48°, 19.86° and 25.90° of diffraction angle (2θ) as measured by powder X-ray crystal diffraction (Example 6),
(7) (2,3-dihydrobenzo[1,4]oxazin-4-yl)-(4-hydroxy-3,5-dimethylphenyl)-methanone having characteristic diffraction peaks at 12.46°, 23.38°, 23.98°, 24.32° and 25.24° of diffraction angle (2θ) as measured by powder X-ray crystal diffraction (Example 7) ,
(8) (3,5-dichloro-4-hydroxyphenyl)-(1,1-dioxo-2,3-dihydro-1H-1λ⁶-berizo[1,4]thiazin-4-yl)-methanone having characteristic diffraction peaks at 16.62°, 18.64°, 19.20°, 21.60° and 23.14° of diffraction angle (2θ) as measured by powder X-ray crystal diffraction (Example 10),
(9) (3,5-dichloro-4-hydroxyphenyl)-(6-methyl-2,3-dihydrobenzo[1,4]oxazin-4-yl)-methanone having characteristic diffraction peaks at 11.58°, 14.36°, 22.18°, 22.48° and 23.36° of diffraction angle (2θ) as measured by powder X-ray crystal diffraction (Example 12),
(10) (3,5-dichloro-4-hydroxyphenyl)-(5-methyl-2,3-dihydrobenzo[1,4]oxazin-4-yl)-methanone having characteristic diffraction peaks at 11.82°, 14.88°, 22.62°, 25.56° and 26.62° of diffraction angle (2θ) as measured by powder X-ray crystal diffraction (Example 14),
(11) (3,5-dichloro-4-hydroxyphenyl)-(8-methyl-2,3-dihydrobenzo[1,4]oxazin-4-yl)-methanone having characteristic diffraction peaks at 7.02°, 14.14°, 21.30°, 21.80° and 26.56° of diffraction angle (2θ) as measured by powder X-ray crystal diffraction (Example 15),
(12) (3,5-dichloro-4-hydroxyphenyl)-(2,3-dihydronaphtho[2,1-b][1,4]oxazin-1-yl)-methanone having characteristic diffraction peaks at 12.40°, 18.36°, 21.34°, 23.66° and 24.24° of diffraction angle (2θ) as measured by powder X-ray crystal diffraction (Example 16),
(13) (3,5-dichloro-4-hydroxyphenyl)-(6-methoxy-2,3-dihydrobenzo[1,4]oxazin-4-yl)-methanone having characteristic diffraction peaks at 10.54°, 11.24°, 21.24°, 21.60° and 24.38° of diffraction angle (2θ) as measured by powder X-ray crystal diffraction (Example 17),
(14) (3,5-dichloro-4-hydroxyphenyl)-(7-methoxy-2,3-dihydrobenzo[1,4]oxazin-4-yl)-methanone having characteristic diffraction peaks at 12.32°, 13.52°, 22.70°, 24.88° and 26.10° of diffraction angle (2θ) as measured by powder X-ray crystal diffraction (Example 18),
(15) (3,5-dichloro-4-hydroxyphenyl)-(6-hydroxy-2,3-dihydrobenzo[1,4]oxazin-4-yl)-methanone having characteristic diffraction peaks at 11.16°, 14.58°, 21.38°, 22.54° and 22.76° of diffraction angle (2θ) as measured by powder X-ray crystal diffraction (Example 19),
(16) (3,5-dichloro-4-hydroxyphenyl)-(7-hydroxy-2,3-dihydrobenzo[1,4]oxazin-4-yl)-methanone having characteristic diffraction peaks at 15.46°, 20.90°, 22.92°, 24.68° and 25.24° of diffraction angle (2θ) as measured by powder X-ray crystal diffraction (Example 20),
(17) 4-(3,5-dichloro-4-hydroxybenzoyl)-3,4-dihydro-2H-benzo[1,4]oxazine-6-sulfonic acid diethylamide having characteristic diffraction peaks at 13.48°, 15.52°, 19.18°, 20.58° and 21.80° of diffraction angle (2θ) as measured by powder X-ray crystal diffraction (Example 21),
(18) (3,5-dichloro-4-hydroxyphenyl)-(3,4-dihydro-2H-quinolin-1-yl)-methanone having characteristic diffraction peaks at 11.66°, 22.20°, 22.48°, 24.68° and 25.52° of diffraction angle (2θ) as measured by powder X-ray crystal diffraction (Example 26),
(19) (3,5-dichloro-4-hydroxyphenyl)-(2,3,4,5-tetrahydrobenzo[b]azepin-1-yl)-methanone having characteristic diffraction peaks at 17.60°, 21.78°, 22.78°, 24.96° and 32.98° of diffraction angle (2θ) as measured by powder X-ray crystal diffraction (Example 27),
(20) (3-chloro-4-hydroxy-5-nitrophenyl)-(2,3-dihydrobenzo[1,4]oxazin-4-yl)-methanone having characteristic diffraction peaks at 12.16°, 14.90°, 22.16°, 23.46° and 24.52° of diffraction angle (2θ) as measured by powder X-ray crystal diffraction (Example 31),
(21) (3,5-dichloro-4-hydroxyphenyl)-(2,3-dihydropyrido[3,2-b][1,4]oxazin-4-yl)-methanone having characteristic diffraction peaks at 11.58°, 14.78°, 18.80°, 23.66° and 25.52° of diffraction angle (2θ) as measured by powder X-ray crystal diffraction (Example 36),
(22) (3,5-dichloro-2-hydroxyphenyl)-(2,3-dihydrobenzo[1,4]oxazin-4-yl)-methanone having characteristic diffraction peaks at 19.94°, 21.86°, 22.52°, 23.84° and 26.14° of diffraction angle (2θ) as measured by powder X-ray crystal diffraction (Example 38),
(23) (2,3-dihydrobenzo[1,4]oxazin-4-yl)-(4-hydroxy-3-trifluoromethylphenyl)-methanone having characteristic diffraction peaks at 7.58°, 12.10°, 15.24°, 22.30° and 24.40° of diffraction angle (2θ) as measured by powder X-ray crystal diffraction (Example 39),
(24) (3-chloro-4-hydroxy-5-methoxyphenyl)-(2,3-dihydrobenzo[1,4]oxazin-4-yl)-methanone having characteristic diffraction peaks at 10.06°, 17.10°, 17.48°, 21.78° and 22.26° of diffraction angle (2θ) as measured by powder X-ray crystal diffraction (Example 40) ,
(25) (4-chloro-3-hydroxyphenyl)-(2,3-dihydrobenzo[1,4]oxazin-4-yl)-methanone having characteristic diffraction peaks at 19.06°, 20.76°, 22.30°, 26.58° and 27.60° of diffraction angle (2θ) as measured by powder X-ray crystal diffraction (Example 41),
(26) (3,5-dichloro-4-hydroxyphenyl)-(6-fluoro-2,3-dihydrobenzo[1,4]oxazin-4-yl)-methanone having characteristic diffraction peaks at 11.70°, 22.44°, 22.74°, 23.58° and 23.90° of diffraction angle (2θ) as measured by powder X-ray crystal diffraction (Example 44),
(27) (3,5-dichloro-2,4-dihydroxyphenyl)-(2,3-dihydrobenzo[1,4]oxazin-4-yl)-methanone having characteristic diffraction peaks at 7.26°, 22.62°, 25.12°, 25.70° and 27.92° of diffraction angle (2θ) as measured by powder X-ray crystal diffraction (Example 50),
(28) (6-chloro-2,3-dihydrobenzo[1,4]oxazin-4-yl)-(3,5-dichloro-4-hydroxyphenyl)-methanone having characteristic diffraction peaks at 11.72°, 21.78°, 22.54°, 22.82° and 23.68° of diffraction angle (2θ) as measured by powder X-ray crystal diffraction (Example 51),
(29) (3,5-dichloro-4-hydroxyphenyl)-(6-hydroxymethyl-2,3-dihydrobenzo[1,4]oxazin-4-yl)-methanone having characteristic diffraction peaks at 21.04°, 22.22°, 23.74°, 24.72° and 27.38° of diffraction angle (2θ) as measured by powder X-ray crystal diffraction (Example 56),
(30) (3,5-dichloro-4-hydroxyphenyl)-(6,8-dimethyl-2,3-dihydrobenzo[1,4]oxazin-4-yl)-methanone having characteristic diffraction peaks at 14.80°, 16.44°, 22.28°, 22.76° and 24.14° of diffraction angle (2θ) as measured by powder X-ray crystal diffraction (Example 66),
(31) (3,5-dichloro-4-hydroxyphenyl)-(6-nitro-2,3-dihydrobenzo[1,4]oxazin-4-yl)-methanone having characteristic diffraction peaks at 14.52°, 21.52°, 21.88°, 27.84° and 30.92° of diffraction angle (2θ) as measured by powder X-ray crystal diffraction (Example 67),
(32) (3,5-dibromo-4-hydroxyphenyl)-(6-nitro-2,3-dihydrobenzo[1,4]oxazin-4-yl)-methanone having characteristic diffraction peaks at 7.16°, 14.40°, 20.96°, 27.58° and 34.62° of diffraction angle (2θ) as measured by powder X-ray crystal diffraction (Example 69),
(33) 1-[4-(3,5-dichloro-4-hydroxybenzoyl)-3,4-dihydro-2H-quinoxalin-1-yl]-ethanone having characteristic diffraction peaks at 15.48°, 19.24°, 22.48°, 25.54° and 30.30° of diffraction angle (2θ) as measured by powder X-ray crystal diffraction (Example 73),
(34) (3,5-dichloro-4-hydroxyphenyl)-(2-methyl-2,3-dihydroindol-1-yl)-methanone having characteristic diffraction peaks at 10.48°, 11.32°, 20.18°, 22.84° and 25.76° of diffraction angle (2θ) as measured by powder X-ray crystal diffraction (Example 76),
(35) (3,5-dichloro-4-hydroxyphenyl)-(2,3-dihydroindol-1-yl)-methanone having characteristic diffraction peaks at 12.10°, 22.48°, 23.40°, 24.42° and 25.98° of diffraction angle (2θ) as measured by powder X-ray crystal diffraction (Example 77),
(36) (3,5-dibromo-4-hydroxyphenyl)-(6-fluoro-2,3-dihydrobenzo[1,4]oxazin-4-yl)-methanone having characteristic diffraction peaks at 9.30°, 14.52°, 18.64°, 23.96° and 27.12° of diffraction angle (2θ) as measured by powder X-ray crystal diffraction (Example 79),
(37) (3,5-dibromo-4-hydroxyphenyl)-(2,3-dihydronaphtho[2,1-b][1,4]oxazin-1-yl)-methanone having characteristic diffraction peaks at 8.92°, 17.98°, 18.26°, 21.10° and 23.40° of diffraction angle (2θ) as measured by powder X-ray crystal diffraction (Example 80),
(38) (3,5-dibromo-4-hydroxyphenyl)-(6-methyl-2,3-dihydrobenzo[1,4]oxazin-4-yl)-methanone having characteristic diffraction peaks at 7.12°, 14.32°, 21.62°, 21.92° and 22.80° of diffraction angle (2θ) as measured by powder X-ray crystal diffraction (Example 81),
(39) (6-chloro-2,3-dihydrobenzo [1,4] oxazin-4-yl) - (3,5-dibromo-4-hydroxyphenyl)-methanone having characteristic diffraction peaks at 7.22°, 14.54°, 22.34°, 29.08° and 33.22° of diffraction angle (2θ) as measured by powder X-ray crystal diffraction (Example 82) ,
(40) (3,5-dichloro-4-hydroxyphenyl)-(6-trifluoromethyl-2,3-dihydrobenzo[1,4]oxazin-4-yl)-methanone having characteristic diffraction peaks at 17.58°, 19.00°, 21.04°, 21.52° and 23.24° of diffraction angle (2θ) as measured by powder X-ray crystal diffraction (Example 85), and
(41) 2,6-dichloro-4-(2,3-dihydrobenzo[1,4]oxazine-4-carbonyl)phenyl acetate having characteristic diffraction peaks at 15.76°, 16.38°, 24.12°, 25.88° and 27.62° of diffraction angle (2θ) as measured by powder X-ray crystal diffraction (Example 87) ,
or a pharmaceutically acceptable salt thereof.

### <82>

A pharmaceutical composition comprising a crystal of the above-mentioned [81].

The nitrogen-containing fused ring compound of the present invention effectively inhibits the activity of URAT1. Therefore, it is effective as an agent for the prophylaxis or treatment of pathology showing involvement of uric acid, such as hyperuricemia, gouty tophus, acute gouty arthritis, chronic gouty arthritis, gouty kidney, urolithiasis, renal function disorder, coronary artery disease, ischemic heart disease and the like. Different from conventional agents for the prophylaxis or treatment of hyperuricemia, the compound of the present invention substantially does not inhibit CYP. Therefore, the possibility of inducing a pharmacokinetic drug interaction is extremely slim, which leads to the expectation of the effect of reducing the side effects.

### Brief Description of the Drawings

Fig. 1 is a powder X-ray diffraction pattern of (3-chloro-4-hydroxyphenyl)-(2,3-dihydrobenzo[1,4]oxazin-4-yl)-methanone (Example 1).
Fig. 2 is a powder X-ray diffraction pattern of (3-bromo-4-hydroxyphenyl)-(2,3-dihydrobenzo[1,4]oxazin-4-yl)-methanone (Example 2).
Fig. 3 is a powder X-ray diffraction pattern of (3,5-dichloro-4-hydroxyphenyl)-(2,3-dihydrobenzo[1,4]oxazin-4-yl)-methanone (Example 3).
Fig. 4 is a powder X-ray diffraction pattern of (3,5-dibromo-4-hydroxyphenyl)-(2,3-dihydrobenzo[1,4]oxazin-4-yl)-methanone (Example 4).
Fig. 5 is a powder X-ray diffraction pattern of (2,3-dihydrobenzo[1,4]oxazin-4-yl)-(4-hydroxy-3,5-diiodophenyl)-methanone (Example 5).
Fig. 6 is a powder X-ray diffraction pattern of (3,5-difluoro-4-hydroxyphenyl)-(2,3-dihydrobenzo[1,4]oxazin-4-yl)-methanone (Example 6) .
Fig. 7 is a powder X-ray diffraction pattern of (2,3-dihydrobenzo[1,4]oxazin-4-yl)-(4-hydroxy-3,5-dimethylphenyl)-methanone (Example 7).
Fig. 8 is a powder X-ray diffraction pattern of (3,5-dichloro-4-hydroxyphenyl)-(1,1-dioxo-2,3-dihydro-1H-1λ⁶-benzo[1,4]thiazin-4-yl)-methanone (Example 10).
Fig. 9 is a powder X-ray diffraction pattern of (3,5-dichloro-4-hydroxyphenyl)-(6-methyl-2,3-dihydrobenzo[1,4]oxazin-4-yl)-methanone (Example 12).
Fig. 10 is a powder X-ray diffraction pattern of (3,5-dichloro-4-hydroxyphenyl)-(5-methyl-2,3-dihydrobenzo[1,4]oxazin-4-yl)-methanone (Example 14).
Fig. 11 is a powder X-ray diffraction pattern of (3,5-dichloro-4-hydroxyphenyl)-(8-methyl-2,3-dihydrobenzo[1,4]oxazin-4-yl)-methanone (Example 15).
Fig. 12 is a powder X-ray diffraction pattern of (3,5-dichloro-4-hydroxyphenyl)-(2,3-dihydronaphtho[2,1-b][1,4]oxazin-1-yl)-methanone (Example 16).
Fig. 13 is a powder X-ray diffraction pattern of (3,5-dichloro-4-hydroxyphenyl)-(6-methoxy-2,3-dihydrobenzo[1,4]oxazin-4-yl)-methanone (Example 17).
Fig. 14 is a powder X-ray diffraction pattern of (3,5-dichloro-4-hydroxyphenyl)-(7-methoxy-2,3-dihydrobenzo[1,4]oxazin-4-yl)-methanone (Example 18).
Fig. 15 is a powder X-ray diffraction pattern of (3,5-dichloro-4-hydroxyphenyl)-(6-hydroxy-2,3-dihydrobenzo[1,4]oxazin-4-yl)-methanone (Example 19).
Fig. 16 is a powder X-ray diffraction pattern of (3,5-dichloro-4-hydroxyphenyl)-(7-hydroxy-2,3-dihydrobenzo[1,4]oxazin-4-yl)-methanone (Example 20).
Fig. 17 is a powder X-ray diffraction pattern of 4-(3,5-dichloro-4-hydroxybenzoyl)-3,4-dihydro-2H-benzo[1,4]oxazine-6-sulfonic acid diethylamide (Example 21).
Fig. 18 is a powder X-ray diffraction pattern of (3,5-dichloro-4-hydroxyphenyl)-(3,4-dihydro-2H-quinolin-1-yl)-methanone (Example 26).
Fig. 19 is a powder X-ray diffraction pattern of (3,5-dichloro-4-hydroxyphenyl)-(2,3,4,5-tetrahydrobenzo[b]azepin-1-yl)-methanone (Example 27).
Fig. 20 is a powder X-ray diffraction pattern of (3-chloro-4-hydroxy-5-nitrophenyl)-(2,3-dihydrobenzo[1,4]oxazin-4-yl)-methanone (Example 31).
Fig. 21 is a powder X-ray diffraction pattern of (3,5-dichloro-4-hydroxyphenyl)-(2,3-dihydropyrido[3,2-b][1,4]oxazin-4-yl)-methanone (Example 36).
Fig. 22 is a powder X-ray diffraction pattern of (3,5-dichloro-2-hydroxyphenyl)-(2,3-dihydrobenzo[1,4]oxazin-4-yl)-methanone (Example 38) .
Fig. 23 is a powder X-ray diffraction pattern of (2,3-dihydrobenzo[1,4]oxazin-4-yl)-(4-hydroxy-3-trifluoromethylphenyl)-methanone (Example 39).
Fig. 24 is a powder X-ray diffraction pattern of (3-chloro-4-hydroxy-5-methoxyphenyl)-(2,3-dihydrobenzo[1,4]oxazin-4-yl)-methanone (Example 40).
Fig. 25 is a powder X-ray diffraction pattern of (4-chloro-3-hydroxyphenyl)-(2,3-dihydrobenzo[1,4]oxazin-4-yl)-methanone (Example 41).
Fig. 26 is a powder X-ray diffraction pattern of (3,5-dichloro-4-hydroxyphenyl)-(6-fluoro-2,3-dihydrobenzo[1,4]oxazin-4-yl)-methanone (Example 44).
Fig. 27 is a powder X-ray diffraction pattern of (3,5-dichloro-2,4-dihydroxyphenyl)-(2,3-dihydrobenzo[1,4]oxazin-4-yl)-methanone (Example 50).
Fig. 28 is a powder X-ray diffraction pattern of (6-chloro-2,3-dihydrobenzo[1,4]oxazin-4-yl)-(3,5-dichloro-4-hydroxyphenyl)-methanone (Example 51).
Fig. 29 is a powder X-ray diffraction pattern of (3,5-dichloro-4-hydroxyphenyl)-(6-hydroxymethyl-2,3-dihydrobenzo[1,4]oxazin-4-yl)-methanone (Example 56).
Fig. 30 is a powder X-ray diffraction pattern of (3,5-dichloro-4-hydroxyphenyl)-(6,8-dimethyl-2,3-dihydrobenzo[1,4]oxazin-4-yl)-methanone (Example 66).
Fig. 31 is a powder X-ray diffraction pattern of (3,5-dichloro-4-hydroxyphenyl)-(6-nitro-2,3-dihydrobenzo[1,4]oxazin-4-yl)-methanone (Example 67).
Fig. 32 is a powder X-ray diffraction pattern of (3,5-dibromo-4-hydroxyphenyl)-(6-nitro-2,3-dihydrobenzo[1,4]oxazin-4-yl)-methanone (Example 69).
Fig. 33 is a powder X-ray diffraction pattern of 1-[4-(3,5-dichloro-4-hydroxybenzoyl)-3,4-dihydro-2H-quinoxalin-1-yl]-ethanone (Example 73).
Fig. 34 is a powder X-ray diffraction pattern of (3,5-dichloro-4-hydroxyphenyl)-(2-methyl-2,3-dihydroindol-1-yl)-methanone (Example 76).
Fig. 35 is a powder X-ray diffraction pattern of (3,5-dichloro-4-hydroxyphenyl)-(2,3-dihydroindol-1-yl)-methanone (Example 77).
Fig. 36 is a powder-X-ray diffraction pattern of (3,5-dibromo-4-hydroxyphenyl)-(6-fluoro-2,3-dihydrobenzo[1,4]oxazin-4-yl)-methanone (Example 79).
Fig. 37 is a powder X-ray diffraction pattern of (3,5-dibromo-4-hydroxyphenyl)-(2,3-dihydronaphtho[2,1-b][1,4]oxazin-1-yl)-methanone (Example 80).
Fig. 38 is a powder X-ray diffraction pattern of (3,5-dibromo-4-hydroxyphenyl)-(6-methyl-2,3-dihydrobenzo[1,4]oxazin-4-yl)-methanone (Example 81).
Fig. 39 is a powder X-ray diffraction pattern of (6-chloro-2,3-dihydrobenzo[1,4]oxazin-4-yl)-(3,5-dibromo-4-hydroxyphenyl)-methanone (Example 82).
Fig. 40 is a powder X-ray diffraction pattern of (3,5-dichloro-4-hydroxyphenyl)-(6-trifluoromethyl-2,3-dihydrobenzo[1,4]oxazin-4-yl)-methanone (Example 85).
Fig. 41 is a powder X-ray diffraction pattern of 2,6-dichloro-4-(2,3-dihydrobenzo[1,4]oxazine-4-carbonyl)phenyl acetate (Example 87).

### Best Mode for Carrying Out the Invention

Each substituent used in the present specification is defined in the following.

The "C₁₋₆ alkyl group" is a straight chain or branched chain alkyl group having 1 to 6 carbon atoms and, for example, methyl group, ethyl group, propyl group, isopropyl group, butyl group, isobutyl group, sec-butyl group, tert-butyl group, pentyl group, isopentyl group, neopentyl group, tert-pentyl group, hexyl group and the like can be mentioned. Preferred is a C₁₋₄ alkyl group and particularly preferred are methyl group, ethyl group, isopropyl group and tert-butyl group.

The "C₁₋₄ alkyl group" is a straight chain or branched chain alkyl group having 1 to 4 carbon atoms and, for example, methyl group, ethyl group, propyl group, isopropyl group, butyl group, isobutyl group, sec-butyl group, tert-butyl group and the like can be mentioned. Preferred are methyl group, ethyl group, isopropyl group and tert-butyl group.

The "C₂₋₆ alkenyl group" is a straight chain or branched chain alkenyl group having 2 to 6 carbon atoms and, for example, vinyl group, n-propenyl group, isopropenyl group, n-butenyl group, isobutenyl group, sec-butenyl group, n-pentenyl group, isopentenyl group, 1-methylpropenyl group, n-hexenyl group, isohexenyl group, 1,1-dimethylbutenyl group, 2,2-dimethylbutenyl group, 3,3-dimethylbutenyl group, 3,3-dimethylpropenyl group, 2-ethylbutenyl group and the like can be mentioned. Preferred is a straight chain or branched chain alkenyl group having 2 to 4 carbon atoms and particularly preferred are vinyl group, n-propenyl group and isopropenyl group.

The "halogen atom" is fluorine atom, chlorine atom, bromine atom or iodine atom.

The "C₁₋₆ alkoxy group" is an alkoxy group wherein the alkyl moiety is the "C₁₋₆ alkyl group" defined above and, for example, methoxy group, ethoxy group, propoxy group, isopropoxy group, butoxy group, isobutoxy group, sec-butoxy group, tert-butoxy group, pentyloxy group, hexyloxy group and the like can be mentioned. Preferred is an alkoxy group wherein the alkyl moiety is the "C₁₋₄ alkyl group" defined above and particularly preferred are methoxy group and ethoxy group.

The "C₁₋₄ alkoxy group" is an alkoxy group wherein the alkyl moiety is the "C₁₋₄ alkyl group" defined above and, for example, methoxy group, ethoxy group, propoxy group, isopropoxy group, butoxy group, isobutoxy group, sec-butoxy group, tert-butoxy group and the like can be mentioned. Particularly preferred are methoxy group and ethoxy group.

The "saturated or unsaturated carbon ring group having 3 to 14 carbon atoms" is a saturated or unsaturated cyclic hydrocarbon group having 3 to 14 carbon atoms, which is specifically an aryl group, a cycloalkyl group, a cycloalkenyl group, a group derived from a fused carbon ring, wherein two or more of rings constituting them are condensed, and the like. The "aryl group" is an aromatic hydrocarbon group having 6 to 14 carbon atoms and, for example, phenyl group, naphthyl group, biphenyl group, anthryl group, azulenyl group, phenanthryl group, pentalenyl group and the like can be mentioned. Preferred is phenyl group.

The "cycloalkyl group" is a cycloalkyl group having 3 to 8 carbon atoms and, for example, cyclopropyl group, cyclobutyl group, cyclopentyl group, cyclohexyl group, cycloheptyl group, cyclooctyl group and the like can be mentioned. Preferred is a cycloalkyl group having 3 to 6 carbon atoms, which is specifically cyclopropyl group, cyclobutyl group, cyclopentyl group and cyclohexyl group. Particularly preferred are cyclopropyl group and cyclohexyl group.

The "cycloalkenyl group" is a cycloalkenyl group having 3 to 8 carbon atoms, and contains at least one, preferably 1 or 2 double bonds. For example, cyclopropenyl group, cyclobutenyl group, cyclopentenyl group, cyclopentadienyl group, cyclohexenyl group, cyclohexadienyl group (2,4-cyclohexadien-1-yl group, 2,5-cyclohexadien-1-yl group etc.), cycloheptenyl group, cyclooctenyl group and the like can be mentioned. Preferred is a cycloalkenyl group having 3 to 6 carbon atoms, and particularly preferred is cyclohexenyl group.

As the group derived from a fused carbon ring, wherein two or more rings constituting these "aryl group", "cycloalkyl group" and "cycloalkenyl group" are condensed, for example, indenyl group, indanyl group, fluorenyl group, 1,4-dihydronaphthyl group, 1,2,3,4-tetrahydronaphthyl group (1,2,3,4-tetrahydro-2-naphthyl group, 5,6,7,8-tetrahydro-2-naphthyl group etc.), perhydronaphthyl group and the like can be mentioned.

The "saturated or unsaturated carbon ring having 3 to 14 carbon atoms" is a ring constituting the "saturated or unsaturated carbon ring group having 3 to 14 carbon atoms" defined above.

The "aralkyl group" is an arylalkyl group wherein the aryl moiety is the -aryl group" defined above and the alkyl moiety is the "C₁₋₆ alkyl group" defined above and, for example, benzyl group, phenethyl group, 3-phenylpropyl group, 4-phenylbutyl group, 6-phenylhexyl group and the like can be mentioned. Preferred is an aralkyl group having 7 to 14 carbon atoms and particularly Preferred is benzyl group.

The "aralkoxy group" is an arylalkoxy group wherein the aryl moiety is the "aryl group" defined above and the alkoxy moiety is the "C₁₋₆ alkoxy group" defined above and, for example, benzyloxy group, 3-phenylpropyloxy group, 4-phenylbutyloxy group, 6-phenylhexyloxy group and the like can be mentioned. Preferred is an aralkoxy group having 7 to 14 carbon atoms and particularly Preferred is benzyloxy group.

The "cycloalkylalkoxy group" is a cycloalkylalkoxy group wherein the cycloalkyl moiety is the "cycloalkyl group" defined above and the alkoxy moiety is the "C₁₋₆ alkoxy group" defined above and, for example, cyclopropylmethoxy group, cyclobutylmethoxy group, cyclopentylmethoxy group, cyclohexylmethoxy group and the like can be mentioned. Preferred is a cycloalkylalkoxy group having 4 to 8 carbon atoms and particularly Preferred are cyclopropylmethoxy group and cyclohexylmethoxy group.

The "aryloxy group" is an aryloxy group wherein the aryl moiety is the "aryl group" defined above and, for example, phenoxy group, naphthyloxy group, biphenyloxy group and the like can be mentioned. Preferred is phenoxy group.

The "saturated or unsaturated heterocyclic group containing at least one heteroatom selected from a nitrogen atom, an oxygen atom and a sulfur atom" is a saturated or unsaturated (including partially unsaturated and completely unsaturated) monocyclic 5-membered or 6-membered heterocyclic group, containing, besides carbon atoms, at least one, preferably 1 to 4, heteroatoms selected from a nitrogen atom, an oxygen atom and a sulfur atom; a fused ring group wherein two or more of these heterocycles are condensed; or a fused ring group wherein one of the heterocycles and a carbon ring selected from benzene, cyclopentane and cyclohexane are condensed.

As the "saturated monocyclic 5-membered or 6-membered heterocyclic group", for example, pyrrolidinyl group, tetrahydrofuryl group, tetrahydrothienyl group, imidazolidinyl group, pyrazolidinyl group, 1,3-dioxolanyl group, 1,3-oxathiolanyl group, oxazolidinyl group, thiazolidinyl group, piperidinyl group, piperazinyl group, tetrahydropyranyl group, tetrahydrothiopyranyl group, dioxanyl group, morpholinyl group, thiomorpholinyl group, 2-oxopyrrolidinyl group, 2-oxopiperidinyl group, 4-oxopiperidinyl group, 2,6-dioxopiperidinyl group and the like can be mentioned.

As the "unsaturated monocyclic 5-membered or 6-membered heterocyclic group", for example, pyrrolyl group, furyl group, thienyl group, imidazolyl group, 1,2-dihydro-2-oxoimidazolyl group, pyrazolyl group, oxazolyl group, isoxazolyl group, thiazolyl group, isothiazolyl group, triazolyl group (e.g., 1,2,4-triazolyl group, 1,2,3-triazolyl group etc.), tetrazolyl group, 1,3,4-oxadiazolyl group, 1,2,4-oxadiazolyl group, 1,3,4-thiadiazolyl group, 1,2,4-thiadiazolyl group, furazanyl group, pyridyl group, pyrimidinyl group, 3,4-dihydro-4-oxopyrimidinyl group, pyridazinyl group, pyrazinyl group, 1,3,5-triazinyl group, imidazolinyl group, pyrazolinyl group, oxazolinyl group (e.g., 2-oxazolinyl group, 3-oxazolinyl group, 4-oxazolinyl group etc.), isoxazolinyl group, thiazolinyl group, isothiazolinyl group, pyranyl group, 2-oxopyranyl group, 2-oxo-2,5-dihydrofuranyl group, 1,1-dioxo-1H-isothiazolyl group and the like can be mentioned.

As the "fused heterocyclic group", for example, indolyl group (e.g., 4-indolyl group, 7-indolyl group etc.), isoindolyl group, 1,3-dihydro-1,3-dioxoisoindolyl group, benzofuranyl group (e.g., 4-benzofuranyl group, 7-benzofuranyl group etc.), indazolyl group, isobenzofuranyl group, benzothiophenyl group (e.g., 4-benzothiophenyl group, 7-benzothiophenyl group etc.), benzoxazolyl group (e.g., 4-benzoxazolyl group, 7-benzoxazolyl group etc.), benzimidazolyl group (e.g., 4-benzimidazolyl group, 7-benzimidazolyl group etc.), benzothiazolyl group (e.g., 4-benzothiazolyl group, 7-benzothiazolyl group etc.), indolizinyl group, quinolyl group, isoquinolyl group, 1,2-dihydro-2-oxoquinolyl group, quinazolinyl group, quinoxalinyl group, cinnolinyl group, phthalazinyl group, quinolizinyl group, purinyl group, pteridinyl group, indolinyl group, isoindolinyl group, 5,6,7,8-tetrahydroquinolyl group, 1,2,3,4-tetrahydroquinolyl group, 2-oxo-1,2,3,4-tetrahydroquinolyl group, benzo[1,3]dioxolyl group, 3,4-methylenedioxypyridyl group, 4,5-ethylenedioxypyrimidinyl group, chromenyl group, chromanyl group, isochromanyl group and the like can be mentioned.

The "monocyclic nitrogen-containing saturated heterocycle" formed together with the adjacent nitrogen atom is a saturated 5-membered or 6-membered monocyclic heterocycle containing at least one nitrogen atom, such as piperidine, morpholine, piperazine, pyrrolidine and the like.

Being "optionally substituted by one or more, the same or different substituents" means being unsubstituted or being substituted by at least one to the acceptable maximum number of substituents. In the case of a methyl group, for example, it means being optionally substituted by 1 to 3 substituents, and in the case of an ethyl group, it means being optionally substituted by 1 to 5 substituents. When substituted by 2 or more substituents, the substituents may be the same or different and the position of the substituents may be any, without any particular limitation. Preferred is being "optionally substituted by the same or different, 1 to 3 substituents".

Preferable examples of each group are as follows.
R¹, R² and R³ are preferably the same or different and each is
1) a hydrogen atom,
2) a halogen atom,
3) -OR¹³ (R¹³ is as defined above) ,
4) a C₁₋₆ alkyl group optionally substituted by one or more, the same or different substituents selected from the aforementioned group B
5) -COR¹⁴ (R¹⁴ is as defined above),
6) -NR¹⁵R¹⁶ (R¹⁵ and R¹⁶ are as defined above) ,
7) -NR¹⁷S(=O)₂R¹⁴ (R¹⁴ and R¹⁷ are as defined above) ,
8) -S(=O)₂R¹⁴ (R¹⁴ is as defined above) ,
9) -S(=O)₂NR¹⁵R¹⁶ (R¹⁵ and R¹⁶ are as defined above) ,
10) a saturated or unsaturated carbon ring group having 3 to 14 carbon atoms optionally substituted by one or more, the same or different substituents selected from (a) and (b):
   (a) a substituent selected from the aforementioned group B,
   (b) a C₁₋₆ alkyl group optionally substituted by one or more, the same or different substituents selected from the aforementioned group B, or
11) a nitro group, or
12) R¹ and R², or R² and R³ may form, together with the carbon atoms they are bonded to, a saturated or unsaturated carbon ring having 3 to 14 carbon atoms optionally substituted by one or more, preferably 2 or 3, the same or different substituents selected from the aforementioned group A.

R¹, R² and R³ are more preferably the same or different and each is
1) a hydrogen atom,
2) a halogen atom (preferably fluorine atom, chlorine atom),
3) a hydroxyl group,
4) a C₁₋₆ alkoxy group (preferably a C₁₋₄ alkoxy group, more preferably methoxy group),
5) a C₁₋₆ alkyl group optionally substituted by one or more, the same or different substituents selected from a halogen atom (preferably fluorine atom) and a hydroxyl group
   (preferably a C₁₋₄ alkyl group (preferably methyl group,
   isopropyl group, tert-butyl group), trifluoromethyl group, hydroxymethyl group),
6) -COOH,
7) -CO-C₁₋₆ alkoxy group (preferably -CO-C₁₋₄ alkoxy group, more preferably -CO-methoxy group),
8) an amino group,
9) -NHS(=O)₂-C₁₋₆ alkyl group (preferably -NHS(=O)₂-C₁₋₄ alkyl group, more preferably -NHS(=O)₂-methyl group),
10) -S(=O)₂-C₁₋₆ alkyl group (preferably -S(=O)₂-C₁₋₄ alkyl group, more preferably -S(=O)₂-ethyl group),
11) -S(=O)₂-NR¹⁵R¹⁶ (R¹⁵ and R¹⁶ are the same or different and each is
   (a) a hydrogen atom, or
   (b) a C₁₋₆ alkyl group (preferably a C₁₋₄ alkyl group, more preferably methyl group, ethyl group), or
   (c) R¹⁵ and R¹⁶ may form, together with the nitrogen atom they are bonded to, a monocyclic nitrogen-containing saturated heterocycle (for example, pyrrolidine, piperidine, imidazolidine, piperazine and the like, preferably pyrrolidine)),
12) a saturated or unsaturated carbon ring group having 3 to 14 carbon atoms (preferably an aryl group, more preferably phenyl group), or
13) a nitro group.

For each of R¹, R² and R³, the following are more preferable.

R¹ is more preferably
1) a hydrogen atom,
2) a hydroxyl group,
3) a C₁₋₆ alkyl group (preferably a C₁₋₄ alkyl group, more preferably methyl group, isopropyl group),
4) -COOH, or
5) -CO-C₁₋₆ alkoxy group (preferably -CO-C₁₋₄ alkoxy group, more preferably -CO-methoxy group).

R² is more preferably
1) a hydrogen atom,
2) a halogen atom (preferably chlorine atom),
3) a hydroxyl group,
4) a C₁₋₆ alkoxy group (preferably a C₁₋₄ alkoxy group, more preferably methoxy group),
5) a C₁₋₆ alkyl group (preferably a C₁₋₄ alkyl group, more preferably methyl group),
6) -COOH,
7) -CO-C₁₋₆ alkoxy group (preferably -CO-C₁₋₄ alkoxy group, more preferably -CO-methoxy group),
8) an amino group,
9) -NHS (=O)₂-C₁₋₆ alkyl group (preferably -NHS (=O) ₂-C₁₋₄ alkyl group, more preferably -NHS (=O)₂-methyl group), or
10) a nitro group.

R³ is more preferably
1) a hydrogen atom,
2) a halogen atom (preferably fluorine atom, chlorine atom),
3) a hydroxyl group,
4) a C₁₋₆ alkoxy group (preferably a C₁₋₄ alkoxy group, more preferably methoxy group),
5) a C₁₋₆ alkyl group optionally substituted by one or more, the same or different substituents selected from a halogen atom (preferably fluorine atom) and a hydroxyl group
   (preferably a C₁₋₄ alkyl group (preferably methyl group, tert-butyl group), trifluoromethyl group, hydroxymethyl group),
6) -COOH,
7) -CO-C₁₋₆ alkoxy group (preferably -CO-C₁₋₄ alkoxy group, more preferably -CO-methoxy group),
8) an amino group,
9) -S(=O)₂-C₁₋₆ alkyl group (preferably -S(=O)₂-C₁₋₄ alkyl group, more preferably -S(=O)₂-ethyl group),
10) -S(=O)₂-NR¹⁵R¹⁶ (R¹⁵ and R¹⁶ are the same or different and each is
   (a) a hydrogen atom, or
   (b) a C₁₋₆ alkyl group (preferably a C₁₋₄ alkyl group, more preferably methyl group, ethyl group), or
   (c) R¹⁵ and R¹⁶ may form, together with the nitrogen atom they are bonded to, a monocyclic nitrogen-containing saturated heterocycle (for example, pyrrolidine, piperidine, imidazolidine, piperazine and the like, preferably pyrrolidine)),
11) a saturated or unsaturated carbon ring group having 3 to 14 carbon atoms (preferably an aryl group, more preferably phenyl group), or
12) a nitro group.

R¹, R² and R³ are particularly preferably the same or different and each is
1) a hydrogen atom,
2) a halogen atom (preferably fluorine atom, chlorine atom),
3) a hydroxyl group,
4) a C₁₋₆ alkoxy group (preferably a C₁₋₄ alkoxy group, more preferably methoxy group),
5) a C₁₋₆ alkyl group optionally substituted by one or more, the same or different substituents selected from a halogen atom (preferably fluorine atom) and a hydroxyl group
   (preferably a C₁₋₄ alkyl group (preferably methyl group,
   isopropyl group, tert-butyl group), trifluoromethyl group,
   hydroxymethyl group),
6) -CO-C₁₋₆ alkoxy group (preferably -CO-C₁₋₄ alkoxy group, more preferably -CO-methoxy group),
7) -S (=O) ₂-NR¹⁵R¹⁶ (R¹⁵ and R¹⁶ are the same or different and each is
   (a) a C₁₋₆ alkyl group (preferably a C₁₋₄ alkyl group, more preferably ethyl group), or
   (b) R¹⁵ and R¹⁶ may form, together with the nitrogen atom they are bonded to, a monocyclic nitrogen-containing saturated heterocycle (preferably pyrrolidine)), or
8) a nitro group.

For each of R¹, R² and R³, the following are particularly preferable.

R¹ is particularly preferably
1) a hydrogen atom,
2) a hydroxyl group,
3) a C₁₋₆ alkyl group (preferably a C₁₋₄ alkyl group, more preferably methyl group, isopropyl group), or
4) -CO-C₁₋₆ alkoxy group (preferably -CO-C₁₋₄ alkoxy group, more preferably -CO-methoxy group).

R² is particularly preferably
1) a hydrogen atom,
2) a halogen atom (preferably chlorine atom),
3) a hydroxyl group,
4) a C₁₋₆ alkoxy group (preferably a C₁₋₄ alkoxy group, more preferably methoxy group),
5) a C₁₋₆ alkyl group (preferably a C₁₋₄ alkyl group, more preferably methyl group),
6) -CO-C₁₋₆ alkoxy group (preferably -CO-C₁₋₄ alkoxy group, more preferably -CO-methoxy group), or
7) a nitro group.

R³ is particularly preferably
1) a hydrogen atom,
2) a halogen atom (preferably fluorine atom, chlorine atom),
3) a hydroxyl group,
4) a C₁₋₆ alkoxy group (preferably a C₁₋₄ alkoxy group, more preferably methoxy group),
5) a C₁₋₆ alkyl group optionally substituted by one or more, the same or different substituents selected from a halogen atom (preferably fluorine atom) and a hydroxyl group
   (preferably a C₁₋₄ alkyl group (preferably methyl group, tert-butyl group), trifluoromethyl group, hydroxymethyl group),
6) -CO-C₁₋₆ alkoxy group (preferably -CO-C₁₋₄ alkoxy group, more preferably -CO-methoxy group),
7) -S (=O) ₂-NR¹⁵R¹⁶ (R¹⁵ and R¹⁶ are the same or different and each is,
   (a) a C₁₋₆ alkyl group (preferably a C₁₋₄ alkyl group, more preferably ethyl group), or
   (b) R¹⁵ and R¹⁶ may form, together with the nitrogen atom they are bonded to, a monocyclic nitrogen-containing saturated heterocycle (preferably pyrrolidine)), or
8) a nitro group.

Y is preferably -CO- or -CS-.

X¹ is preferably
1) a nitrogen atom, or
2) CR⁴ wherein R⁴ is
   (a) a hydrogen atom,
   (b) a hydroxyl group,
   (c) a C₁₋₆ alkyl group (preferably a C₁₋₄ alkyl group, more preferably methyl group), or
   (d) -CO-C₁₋₆ alkoxy group (preferably -CO-C₁₋₄ alkoxy group, more preferably -CO-methoxy group), or
   (e) R³ and R⁴ (R³ is as defined above) may form, together with the carbon atoms they are bonded to, a saturated or unsaturated carbon ring having 3 to 14 carbon atoms (preferably an aromatic hydrocarbon, more preferably benzene ring).
      X¹ is more preferably
   CR⁴ wherein R⁴ is

(a) a hydrogen atom,
(b) a hydroxyl group,
(c) a C₁₋₆ alkyl group (preferably a C₁₋₄ alkyl group, more preferably methyl group), or
(d) -CO-C₁₋₆ alkoxy group (preferably -CO-C₁₋₄ alkoxy group, more preferably -CO-methoxy group), or
(e) R³ and R⁴ (R³ is as defined above) may form, together with the carbon atoms they are bonded to, a saturated or unsaturated carbon ring having 3 to 14 carbon atoms (preferably an aromatic hydrocarbon, more preferably benzene ring).
X² is preferably
1) an oxygen atom,
2) -N(R⁵) - wherein R⁵ is
   (a) a hydrogen atom, or
   (b) a C₁₋₆ alkyl group (preferably a C₁₋₄ alkyl group, more preferably methyl group),
3) -N (COR⁶) - wherein R⁶ is
   (a) a C₁₋₆ alkyl group (preferably a C₁₋₄ alkyl group, more preferably methyl group), or
   (b) a saturated or unsaturated carbon ring group having 3 to 14 carbon atoms (preferably an aryl group, more preferably phenyl group) optionally substituted by one or more, the same or different substituents selected from a halogen atom (preferably chlorine atom) and a hydroxyl group) ,
4) -N(S(=O)₂-C₁₋₆ alkyl group) - (preferably -N(-S(=O)₂-C₁₋₄ alkyl group) -, more preferably -N(-S(=0)₂-methyl group) -) ,
5) a sulfur atom,
6) -S(=O)-,
7) -S (=O)₂-, or
8) -CH₂-.

X²' is preferably
1) an oxygen atom,
2) -N(R⁵)- wherein R⁵ is
   (a) a hydrogen atom, or
   (b) a C₁₋₆ alkyl group (preferably a C₁₋₄ alkyl group, more preferably methyl group),
3) -N (COR⁶) - wherein R⁶ is
   (a) a C₁₋₆ alkyl group (preferably a C₁₋₄ alkyl group, more preferably methyl group), or
   (b) a saturated or unsaturated carbon ring group having 3 to 14 carbon atoms (preferably an aryl group, more preferably phenyl group) optionally substituted by one or more, the same or different substituents selected from a halogen atom (preferably chlorine atom) and a hydroxyl group) ,
4) -N (S (=O)₂-C₁₋₆ alkyl group) - (preferably -N (-S (=O)₂-C₁₋₄ alkyl group)-, more preferably -N(-S(=O)₂-methyl group)-),
5) a sulfur atom,
6) -S(=O)-,
7) -S(=O)₂-, or
8) -CH₂-.

X²' is more preferably an oxygen atom.

-X³-X⁴- is preferably
-(CR¹¹R¹²)n- wherein n is an integer of 1 to 3, and R¹¹ and R¹² each in the number of n are the same or different and each is
(a) a hydrogen atom, or
(b) R¹¹ and R¹² bonded to a single carbon atom may in combination form an oxo group, or
(c) two of R¹¹ and R¹² each in the number of n, which are bonded to a single carbon atom or two adjacent carbon atoms, may form, together with the carbon atom(s), a saturated or unsaturated carbon ring having 3 to 14 carbon atoms (preferably an aromatic hydrocarbon, more preferably benzene ring) ,
"n" of - (CR¹¹R¹²) n- for -X³-X⁴- is preferably 2 or 3, more preferably 2.

As-(CR¹¹R¹²)n-,
1) -CH₂-,
2) -CH(CH₃)
3) -CH(CH₂CH₃)
4) -CH(CH(CH₃)₂
5) -CH₂-CH₂-,
6) -CH (CH₃)-CH₂-,
7) -CH (CH₂CH₃) -CH₂-,
8) -CH (CH (CH₃) ₂) -CH₂-,
9) -CH₂-CH (CH₃)-,
10) -CH₂-CH (CH₂CH₃)-,
11) -CH₂-CH ( CH (CH₃) ₂) -,
12) -CH₂-CH₂-CH₂₋
13) -CH₂-CH (CH₃) -CH₂-,
14) -CH₂-CH₂-CH(CH₃)-,
15) -CO-,
16) -CO-CH₂-,
17) -CH₂-CO-,
18) -CO-CH₂-,
19) -CO-CH₂-CH₂-,
20) -CH₂-CO- CH₂-,
21) -CH₂-CH₂-CO-,
22) 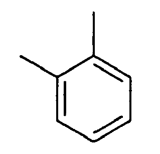
and the like can be specifically mentioned.
-X³-X⁴- is more preferably -CH₂-CH₂-.

Ring A is preferably an unsaturated carbon ring group having 3 to 14 carbon atoms optionally substituted by one or more, preferably 2 or 3, the same or different substituents selected from the aforementioned group A, or an unsaturated heterocyclic group containing at least one heteroatom selected from a nitrogen atom, an oxygen atom and a sulfur atom, optionally substituted by one or more, preferably 2 or 3, the same or different substituents selected from the aforementioned group A.

Ring A is more preferably 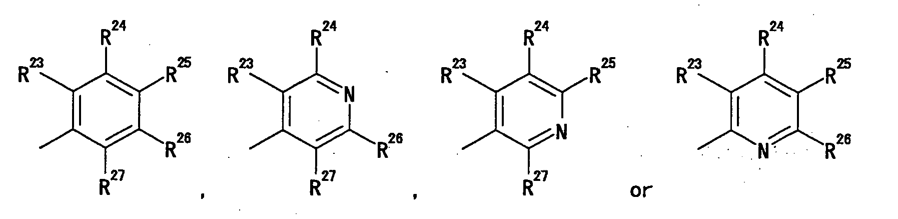 wherein R²³ to R²⁷ are the same or different and each is
1) a hydrogen atom, or
2) a group selected from the aforementioned group A.

R²³ to R²⁷ on ring A are preferably are the same or different and each is
1) a hydrogen atom,
2) a halogen atom,
3) -OR¹³ (R¹³ is as defined above) ,
4) a C₁₋₆ alkyl group optionally substituted by one or more, the same or different substituents selected from the aforementioned group B,
5) -COR¹⁴ (R¹⁴ is as defined above) ,
6) -NR¹⁵R¹⁶ (R¹⁵ and R¹⁶ are as defined above) ,
7) -NR¹⁷S(=O)₂R¹⁴ (R¹⁴ and R¹⁷ are as defined above) , or
8) a nitro group.

R²³ to R²⁷ on ring A are more preferably the same or different and each is
1) a hydrogen atom,
2) a halogen atom,
3) a hydroxyl group,
4) a C₁₋₆ alkoxy group optionally substituted by one or more, the same or different substituents selected from -COOH and -CO-C₁₋₆ alkoxy group (preferably -CO-C₁₋₄ alkoxy group, more preferably - CO-ethoxy group)
   (preferably a C₁₋₄ alkoxy group (preferably methoxy group), carboxymethoxy group, (ethoxycarbonyl)methoxy group),
5) -O-CO-C₁₋₆ alkyl group (preferably -O-CO-C₁₋₄ alkyl group, more preferably -O-CO-methyl group),
6) a C₁₋₆ alkyl group optionally substituted by one or more, the same or different halogen atoms (preferably fluorine atom) (preferably a C₁₋₄ alkyl group (preferably methyl group) , trifluoromethyl group),
7) -COOH,
8) -CO-C₁₋₆ alkoxy group (preferably -CO-C₁₋₄ alkoxy group, more preferably -CO-methoxy group),
9) an amino group,
10) -NHS(=O)₂-C₁₋₆ alkyl group (preferably -NHS(=O)₂-C₁₋₄ alkyl group, more preferably -NHS(=O)₂-methyl group), or
11) a nitro group.

For each of R²³ to R²⁷ on ring A, the following are more preferable.

R²³ on ring A is more preferably
1) a hydrogen atom,
2) a hydroxyl group, or
3) -COOH.

R²⁴ on ring A is more preferably
1) a hydrogen atom,
2) a halogen atom,
3) a hydroxyl group,
4) a C₁₋₆ alkoxy group (preferably a C₁₋₆ alkoxy group, more preferably methoxy group),
5) a C₁_₆ alkyl group optionally substituted by one or more, the same or different halogen atoms (preferably fluorine atom) (preferably a C₁₋₄ alkyl group (preferably methyl group), trifluoromethyl group),
6) -COOH,
7) -CO-C₁₋₆ alkoxy group (preferably -CO-C₁₋₄ alkoxy group, more preferably -CO-methoxy group), or
8) nitro group.

R²⁵ on ring A is more preferably
1) a hydrogen atom,
2) a halogen atom,
3) a hydroxyl group,
4) a C₁₋₆ alkoxy group optionally substituted by one or more, the same or different substituents selected from -COOH and -CO-C₁₋₆ alkoxy group (preferably -CO-C₁₋₄ alkoxy group, more preferably - CO-ethoxy group)
   (preferably a C₁₋₄ alkoxy group (preferably methoxy group) , carboxymethoxy group, (ethoxycarbonyl)methoxy group),
5) -O-CO-C₁₋₆ alkyl group (preferably -O-CO-C₁₋₄ alkyl group, more preferably -O-CO-methyl group),
6) -COOH,
7) -CO-C₁₋₆ alkoxy group (preferably -CO-C₁₋₄ alkoxy group, more preferably -CO-methoxy group),
8) an amino group,
9) -NHS(=O)₂-C₁₋₆ alkyl group (preferably -NHS (=O) ₂-C₁₋₄ alkyl group, more preferably -NHS(=O)₂-methyl group), or
10) a nitro group.

R²⁶ on ring A is more preferably
1) a hydrogen atom,
2) a halogen atom,
3) a hydroxyl group,
4) a C₁₋₆ alkoxy group (preferably a C₁₋₆ alkoxy group, more preferably methoxy group),
5) a C₁₋₆ alkyl group optionally substituted by one or more, the same or different halogen atoms (preferably fluorine atom) (preferably a C₁₋₄ alkyl group (preferably methyl group), trifluoromethyl group),
6) -COOH,
7) -CO-C₁₋₆ alkoxy group (preferably -CO-C₁₋₄ alkoxy group, more preferably -CO-methoxy group), or
8) nitro group.

R²⁷ on ring A is more preferably
1) a hydrogen atom,
2) a hydroxyl group, or
3) -COOH.

As such ring A, 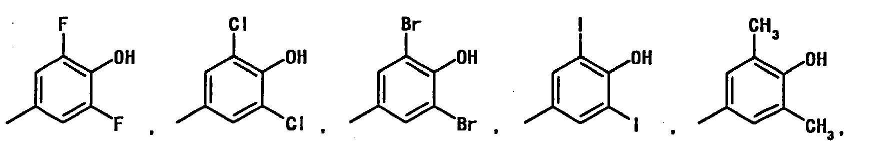 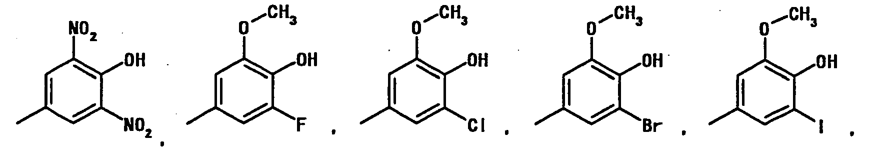 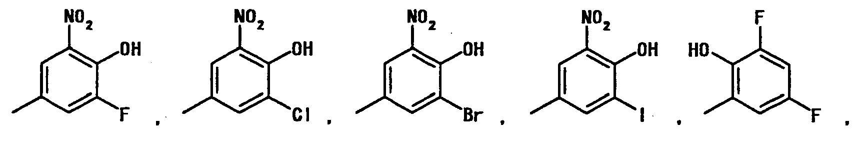 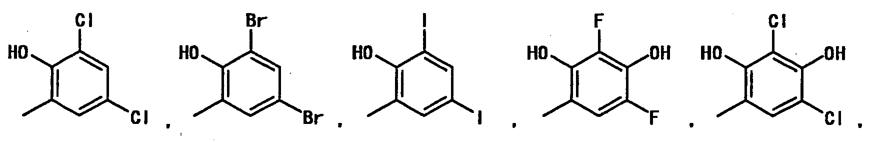 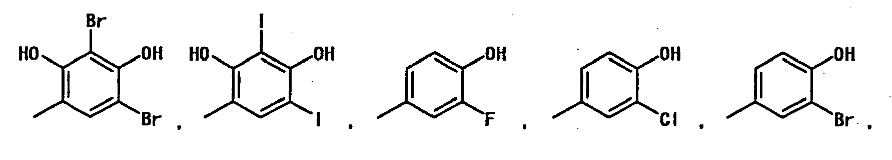 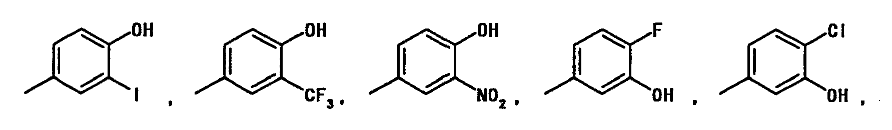 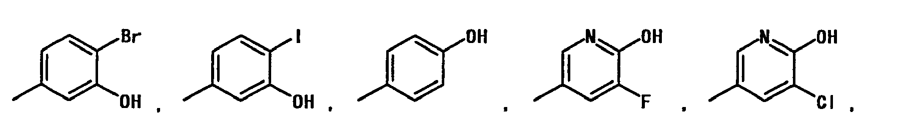 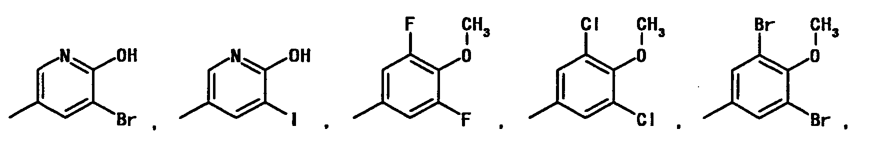 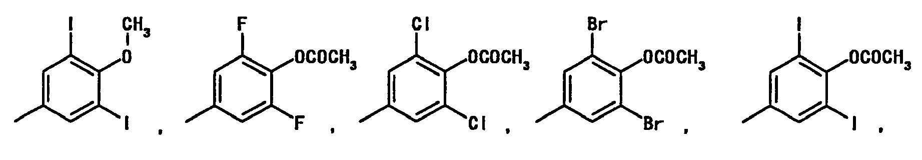 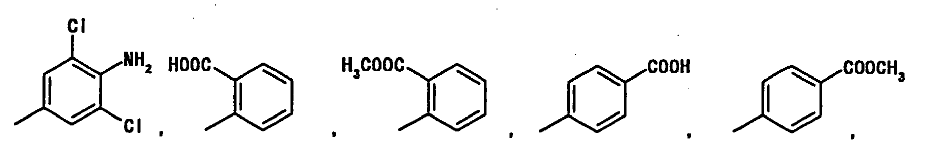 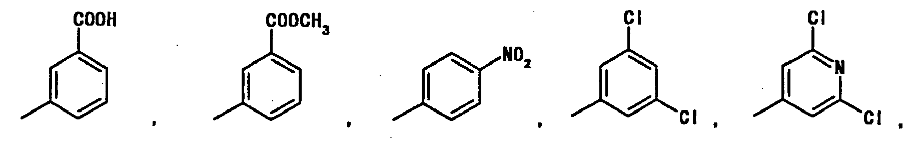 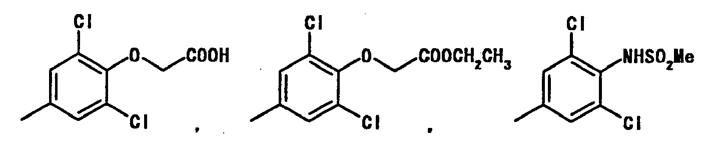 and the like can be mentioned.

R²³ to R²⁷ on ring A are particularly preferably the same or different and each is
1) a hydrogen atom,
2) a halogen atom,
3) a hydroxyl group,
4) a C₁₋₆ alkyl group optionally substituted by one or more, the same or different halogen atoms (preferably fluorine atom) (preferably a C₁₋₄ alkyl group (preferably methyl group) , trifluoromethyl group), or
5) a nitro group.

For each of R²³ to R²⁷ on ring A, the following are particularly preferable.

R²³ on ring A is particularly preferably
1) a hydrogen atom, or
2) a hydroxyl group.

R²⁴ on ring A is particularly preferably
1) a hydrogen atom,
2) a halogen atom,
3) a hydroxyl group,
4) a C₁₋₆ alkyl group optionally substituted by one or more, the same or different halogen atoms (preferably fluorine atom) (preferably a C₁₋₄ alkyl group (preferably methyl group), trifluoromethyl group), or
5) a nitro group.

R²⁵ on ring A is particularly preferably
1) a hydrogen atom,
2) a halogen atom,
3) a hydroxyl group, or
4) nitro group.

R²⁶ on ring A is particularly preferably
1) a hydrogen atom,
2) a halogen atom,
3) a hydroxyl group,
4) a C₁₋₆ alkyl group optionally substituted by one or more, the same or different halogen atoms (preferably fluorine atom) (preferably a C₁₋₄ alkyl group (preferably methyl group), trifluoromethyl group), or
5) a nitro group.

R²⁷ on ring A is particularly preferably
1) a hydrogen atom, or
2) a hydroxyl group.

Ring A' is preferably an unsaturated carbon ring group having 3 to 14 carbon atoms optionally substituted by one or more, preferably 2 or 3, the same or different substituents selected from the aforementioned group C, or an unsaturated heterocyclic group containing at least one heteroatom selected from a nitrogen atom, an oxygen atom and a sulfur atom, optionally substituted by one or more, preferably 2 or 3, the same or different substituents selected from the aforementioned group C. Ring A' is substituted by at least one -OR¹³' wherein R¹³' is as defined above.

Ring A' is preferably 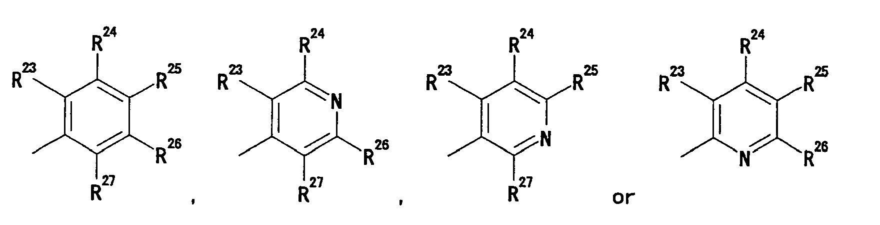 wherein
R²³ to R²⁷ are the same or different and each is
1) a hydrogen atom, or
2) a group selected from the aforementioned group C, and at least one of R²³ to R²⁷ is -OR¹³, wherein R¹³' is as defined above.

R²³ to R²⁷ on ring A' are preferably are the same or different and each is
1) a hydrogen atom,
2) a halogen atom,
3) -OR¹³'(R¹³' is as defined above),
4) a C₁₋₆ alkyl group optionally substituted by one or more, the same or different substituents selected from the aforementioned group B,
5) -COR¹⁴, (R¹⁴' is as defined above) ,
6) -NR¹⁵'R¹⁶' (R^{15,} and R^{16,} are as defined above),
7) -NR¹⁷, S(=O)₂R^{14,} (R^{14,} and R^{17,} are as defined above) , or
8) a nitro group, and
   at least one of R²³ to R²⁷ is -OR¹³' wherein R¹³' is as defined above.

R²³ to R²⁷ on ring A' are more preferably the same or different and each is
1) a hydrogen atom,
2) a halogen atom,
3) a hydroxyl group,
4) a C₁₋₆ alkoxy group optionally substituted by one or more, the same or different substituents selected from -COOH and -CO-C₁₋₆ alkoxy group (preferably -CO-C₁₋₄ alkoxy group, more preferably - CO-ethoxy group)
   (preferably a C₁₋₄ alkoxy group (preferably methoxy group), carboxymethoxy group, (ethoxycarbonyl)methoxy group),
5) -O-CO-C₁₋₆ alkyl group (preferably -O-CO-C₁₋₄ alkyl group, more preferably -O-CO-methyl group),
6) a C₁₋₆ alkyl group optionally substituted by one or more, the same or different halogen atoms (preferably fluorine atom) (preferably a C₁₋₄ alkyl group (preferably methyl group), trifluoromethyl group),
7) -COOH,
8) -CO-C₁₋₆ alkoxy group (preferably -CO-C₁₋₄ alkoxy group, more preferably -CO-methoxy group),
9) an amino group,
10) -NHS (=O)₂-C₁₋₆ alkyl group (preferably -NHS (=O)₂-C₁₋₄ alkyl group, more preferably -NHS(=O)₂-methyl group), or
11) a nitro group, and
   at least one of R²³ to R²⁷ is a group selected from a hydroxyl group, a C₁₋₆ alkoxy group (preferably a C₁₋₄ alkoxy group, more preferably methoxy group) optionally substituted by one or more, the same or different substituents selected from -COOH and -CO-C₁₋₆ alkoxy group (preferably -CO-C₁₋₄ alkoxy group, more preferably -CO-ethoxy group), and -O-CO-C₁₋₆ alkyl group (preferably -O-CO-C₁₋₄ alkyl group, more preferably -O-CO-methyl group).

For each of R²³ to R²⁷ on ring A', the following are more preferable.

R²³ on ring A' is more preferably
1) a hydrogen atom,
2) a hydroxyl group, or
3) -COOH.

R²⁴ on ring A' is more preferably
1) a hydrogen atom,
2) a halogen atom,
3) a hydroxyl group,
4) a C₁₋₆ alkoxy group (preferably a C₁₋₆ alkoxy group, more preferably methoxy group),
5) a C₁₋₆ alkyl group optionally substituted by one or more, the same or different halogen atoms (preferably fluorine atom) (preferably a C₁₋₄ alkyl group (preferably methyl group) , trifluoromethyl group),
6) -COOH,
7) -CO-C₁₋₆ alkoxy group (preferably -CO-C₁₋₄ alkoxy group, more preferably -CO-methoxy group), or
8) nitro group.

R²⁵ on ring A' is more preferably
1) a hydrogen atom,
2) a halogen atom,
3) a hydroxyl group,
4) a C₁₋₆ alkoxy group optionally substituted by one or more, the same or different substituents selected from -COOH and -CO-C₁₋₆ alkoxy group (preferably -CO-C₁₋₄ alkoxy group, more preferably - CO-ethoxy group)
   (preferably a C₁₋₄ alkoxy group (preferably methoxy group), carboxymethoxy group, (ethoxycarbonyl)methoxy group),
5) -O-CO-C₁₋₆ alkyl group (preferably -O-CO-C₁₋₄ alkyl group, more preferably -O-CO-methyl group),
6) -COOH,
7) -CO-C₁₋₆ alkoxy group (preferably -CO-C₁₋₄ alkoxy group, more preferably -CO-methoxy group),
8) an amino group,
9) -NHS(=O)₂-C₁₋₆ alkyl group (preferably -NHS(=O)₂-C₁₋₄ alkyl group, more preferably -NHS(=O)₂-methyl group), or
10) a nitro group.

R²⁶ on ring A' is more preferably
1) a hydrogen atom,
2) a halogen atom,
3) a hydroxyl group,
4) a C₁₋₆ alkoxy group (preferably a C₁₋₆ alkoxy group, more preferably methoxy group),
5) a C₁₋₆ alkyl group optionally substituted by one or more, the same or different halogen atoms (preferably fluorine atom), (preferably a C₁₋₄ alkyl group (preferably methyl group) , trifluoromethyl group),
6) -COOH,
7) -CO-C₁₋₆ alkoxy group (preferably -CO-C₁₋₄ alkoxy group, more preferably -CO-methoxy group), or
8) nitro group.

R²⁷ on ring A' is more preferably
1) a hydrogen atom,
2) a hydroxyl group, or
3) -COOH.

As such ring A', 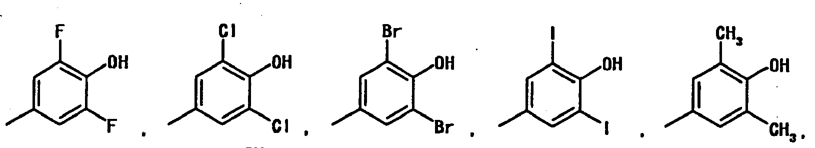 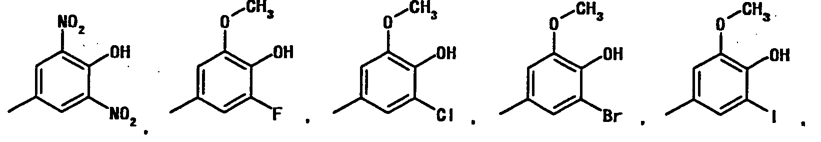 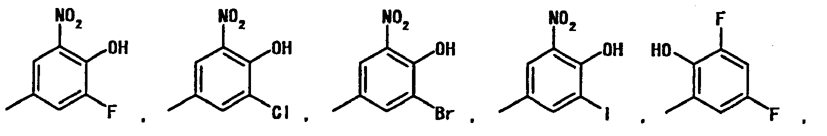 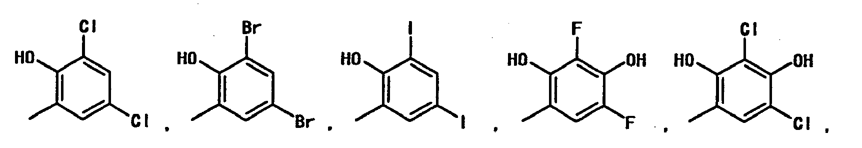 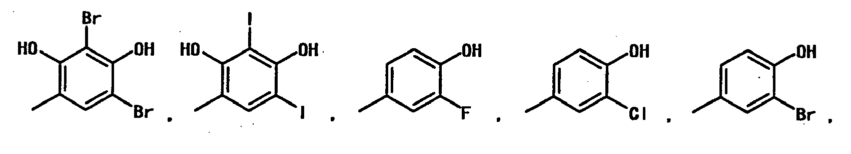 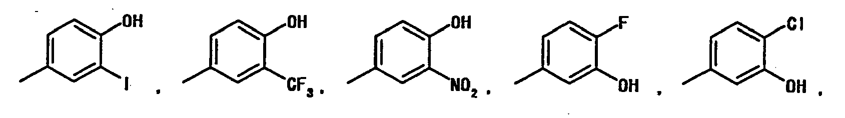 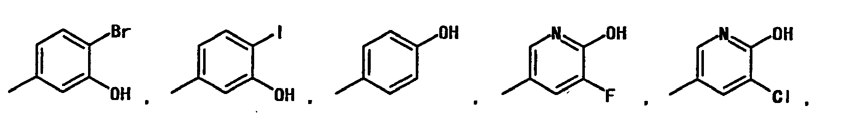 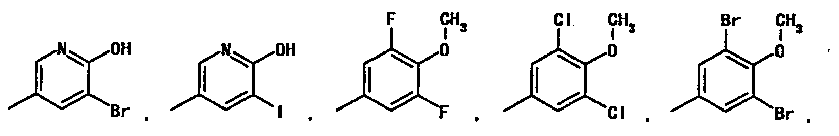 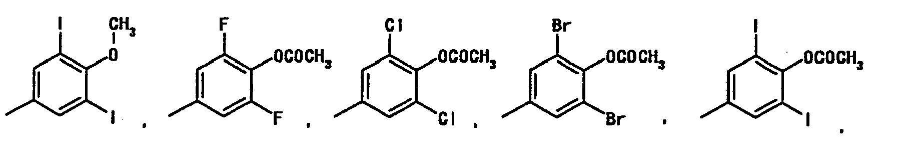 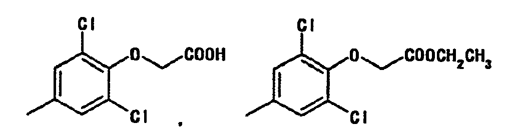 and the like can be mentioned.

R²³ to R²⁷ on ring A' are particularly preferably the same or different and each is
1) a hydrogen atom,
2) a halogen atom,
3) a hydroxyl group,
4) a C₁₋₆ alkyl group optionally substituted by one or more, the same or different halogen atoms (preferably fluorine atom) (preferably a C₁₋₄ alkyl group (preferably methyl group) , trifluoromethyl group), or
5) a nitro group, and at least one of R²³ to R²⁷ is a hydroxyl group.

For each of R²³ to R²⁷ on ring A', the following are particularly preferable.

R²³ on ring A' is particularly preferably
1) a hydrogen atom, or
2) a hydroxyl group.

R²⁴ on ring A' is particularly preferably
1) a hydrogen atom,
2) a halogen atom,
3) a hydroxyl group,
4) a C₁₋₆ alkyl group optionally substituted by one or more, the same or different halogen atoms (preferably fluorine atom) (preferably a C₁₋₄ alkyl group (preferably methyl group) , trifluoromethyl group), or
5) a nitro group.

R²⁵ on ring A' is particularly preferably
1) a hydrogen atom,
2) a halogen atom,
3) a hydroxyl group, or
4) nitro group.

R²⁶ on ring A' is particularly preferably
1) a hydrogen atom,
2) a halogen atom,
3) a hydroxyl group,
4) a C₁₋₆ alkyl group optionally substituted by one or more, the same or different halogen atoms (preferably fluorine atom) (preferably a C₁₋₄ alkyl group (preferably methyl group) , trifluoromethyl group), or
5) a nitro group.

R²⁷ on ring A' is particularly preferably
1) a hydrogen atom, or
2) a hydroxyl group.

Of compounds [1], compound [2] is preferable.
Particularly, a compound wherein
R¹, R² and R³ are the same or different and each is
1) a hydrogen atom,
2) a halogen atom (preferably fluorine atom, chlorine atom),
3) a hydroxyl group,
4) a C₁₋₆ alkoxy group (preferably a C₁₋₄ alkoxy group, more preferably methoxy group),
5) a C₁₋₆ alkyl group optionally substituted by one or more, the same or different substituents selected from a halogen atom (preferably fluorine atom) and a hydroxyl group (preferably a C₁₋₄ alkyl group (preferably methyl group, isopropyl group, tert-butyl group), trifluoromethyl group, hydroxymethyl group),
6) -COOH,
7) -CO-C₁₋₆ alkoxy group (preferably -CO-C₁₋₄ alkoxy group, more preferably -CO-methoxy group),
8) an amino group,
9) -NHS(=O)₂-C₁₋₆ alkyl group (preferably -NHS(=O)₂-C₁₋₄ alkyl group, more preferably -NHS(=O)₂-methyl group),
10) -S (=O)₂-C₁₋₆ alkyl group (preferably -S (=O)₂-C₁₋₄ alkyl group, more preferably -S(=O)₂-ethyl group),
11) -S(=O)₂-NR¹⁵R¹⁶ (R¹⁵ and R¹⁶ are the same or different and each is
   (a) a hydrogen atom, or
   (b) a C₁₋₆ alkyl group (preferably a C₁₋₄ alkyl group, more preferably methyl group, ethyl group), or
   (c) R¹⁵ and R¹⁶ may form, together with the nitrogen atom they are bonded to, a monocyclic nitrogen-containing saturated heterocycle (for example, pyrrolidine, piperidine, imidazolidine, piperazine and the like, preferably pyrrolidine)),
12) a saturated or unsaturated carbon ring group having 3 to 14 carbon atoms (preferably an aryl group, more preferably phenyl group), or
13) a nitro group;
Y is
1) -CO-,
2) -CS-, or
3) -S(=O)₂-;
X¹ is
1) a nitrogen atom, or
2) CR⁴ wherein R⁴ is
   (a) a hydrogen atom,
   (b) a hydroxyl group,
   (c) a C₁₋₆ alkyl group (preferably a C₁₋₄ alkyl group, more preferably methyl group), or
   (d) -CO-C₁₋₆ alkoxy group (preferably -CO-C₁₋₄ alkoxy group, more preferably -CO-methoxy group), or
   (e) R³ and R⁴ (R³ is as defined above) may form, together with the carbon atoms they are bonded to, a saturated or unsaturated carbon ring having 3 to 14 carbon atoms (preferably an aromatic hydrocarbon, more preferably benzene ring) ;
X²' is
1) an oxygen atom,
2) -N (R⁵) - wherein R⁵ is
   (a) a hydrogen atom, or
   (b) a C₁₋₆ alkyl group (preferably a C₁₋₄ alkyl group, more preferably methyl group),
3) -N(COR⁶)-wherein R⁶ is
   (a) a C₁₋₆ alkyl group (preferably a C₁₋₄ alkyl group, more preferably methyl group), or
   (b) a saturated or unsaturated carbon ring group having 3 to 14 carbon atoms (preferably an aryl group, more preferably phenyl group) optionally substituted by one or more, the same or different substituents selected from a halogen atom (preferably chlorine atom) and a hydroxyl group) ,
4) -N(S(=O)₂-C₁₋₆ alkyl group) - (preferably -N(-S(=O)₂-C₁₋₄ alkyl group)-, more preferably -N(-S(=O)₂-methyl group)-),
5) a sulfur atom,
6) -S(=O)-,
7) -S(=O)₂-, or
8) -CH₂-;
-X³-X⁴- is
-(CR¹¹R¹²)n- wherein n is an integer of 1 to 3, and R¹¹ and R¹² each in the number of n are the same or different and each is
(a) a hydrogen atom, or
(b) R¹¹ and R¹² bonded to a single carbon atom may in combination form an oxo group, or
(c) two of R¹¹ and R¹² each in the number of n, which are bonded to a single carbon atom or two adjacent carbon atoms, may form, together with the carbon atom(s), a saturated or unsaturated carbon ring having 3 to 14 carbon atoms (preferably an aromatic hydrocarbon, more preferably benzene ring); and
ring A' is 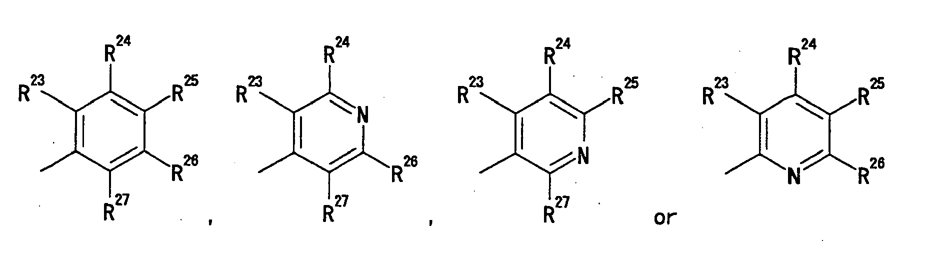 wherein
R²³ to R²⁷ are the same or different and each is
1) a hydrogen atom,
2) a halogen atom,
3) a hydroxyl group,
4) a C₁₋₆ alkoxy group optionally substituted by one or more, the same or different substituents selected from - COOH and -CO-C₁₋₆ alkoxy group (preferably -CO-C₁₋₄ alkoxy group, more preferably -CO-ethoxy group)
   (preferably a C₁₋₄ alkoxy group (preferably methoxy group), carboxymethoxy group, (ethoxycarbonyl)methoxy group) ,
5) -O-CO-C₁₋₆ alkyl group (preferably -O-CO-C₁₋₄ alkyl group, more preferably -O-CO-methyl group),
6) a C₁₋₆ alkyl group optionally substituted by one or more, the same or different halogen atoms (preferably fluorine atom)
   (preferably a C₁₋₄ alkyl group (preferably methyl group) , trifluoromethyl group),
7) -COOH,
8) -CO-C₁₋₆ alkoxy group (preferably -CO-C₁₋₄ alkoxy group, more preferably -CO-methoxy group),
9) an amino group,
10) -NHS (=O)₂-C₁₋₆ alkyl group (preferably -NHS (=O)₂-C₁₋₄ alkyl group, more preferably -NHS(=O)₂-methyl group), or
11) a nitro group, and
at least one of R²³ to R²⁷ is a group selected from a hydroxyl group, a C₁₋₆ alkoxy group (preferably a C₁₋₄ alkoxy group, more preferably methoxy group) optionally substituted by one or more, the same or different substituents selected from - COOH and -CO-C₁₋₆ alkoxy group (preferably -CO-C₁₋₄ alkoxy group, more preferably -CO-ethoxy group), and -O-CO-C₁₋₆ alkyl group (preferably -O-CO-C₁₋₄ alkyl group, more preferably -O-CO-methyl group);
(provided that when X²' is -CH₂-,
then -X³-X⁴- should be
- (CR¹¹R¹²)n- wherein n is an integer of 1 to 3, and R¹¹ and R¹² each in the number of n are the same or different and each is
(a) a hydrogen atom, or
(b) R¹¹ and R¹² bonded to a single carbon atom may in combination form an oxo group, or
(c) two of R¹¹ and R¹² each in the number of n, which are bonded to a single carbon atom or two adjacent carbon atoms, may form, together with the carbon atom(s), a saturated or unsaturated carbon ring having 3 to 14 carbon atoms (preferably an aromatic hydrocarbon, more preferably benzene ring);
R¹³' should be a hydrogen atom; and
ring A' should be further substituted by at least one a halogen atom;
provided that when both R¹¹ and R¹² are hydrogen atoms, and n is 2, then all of each R¹, R² and R³ should be hydrogen atoms) ,
is preferable.

Particularly, a compound wherein
R¹, R² and R³ are the same or different and each is
1) a hydrogen atom,
2) a halogen atom (preferably fluorine atom, chlorine atom),
3) a hydroxyl group,
4) a C₁₋₆ alkoxy group (preferably a C₁₋₄ alkoxy group, more preferably methoxy group)
5) a C₁₋₆ alkyl group optionally substituted by one or more, the same or different substituents selected from a halogen atom (preferably fluorine atom) and a hydroxyl group (preferably a C₁₋₄ alkyl group (preferably methyl group, isopropyl group, tert-butyl group), trifluoromethyl group, hydroxymethyl group),
6) -CO-C₁₋₆ alkoxy group (preferably -CO-C₁₋₄ alkoxy group, more preferably -CO-methoxy group),
7) -S(=O)₂-NR¹⁵R¹⁶ (R¹⁵ and R¹⁶ are the same or different and each is
   (a) a C₁₋₆ alkyl group (preferably a C₁₋₄ alkyl group, more preferably ethyl group), or
   (b) R¹⁵ and R¹⁶ may form, together with the nitrogen atom they are bonded to, a monocyclic nitrogen-containing saturated heterocycle (preferably pyrrolidine)), or
8) a nitro group;
Y is
1) -CO-, or
2) -CS-;
   X¹ is
CR⁴ wherein R⁴ is
(a) a hydrogen atom,
(b) a hydroxyl group,
(c) a C₁₋₆ alkyl group (preferably a C₁₋₄ alkyl group, more preferably methyl group), or
(d) -CO-C₁₋₆ alkoxy group (preferably -CO-C₁₋₄ alkoxy group, more preferably -CO-methoxy group), or
(e) R³ and R⁴ (R³ is as defined above) may form, together with the carbon atoms they are bonded to, a saturated or unsaturated carbon ring having 3 to 14 carbon atoms (preferably an aromatic hydrocarbon, more preferably benzene ring) ;
X²' is an oxygen atom;
-X³-X⁴- is -CH₂-CH₂- ; and
ring A' is 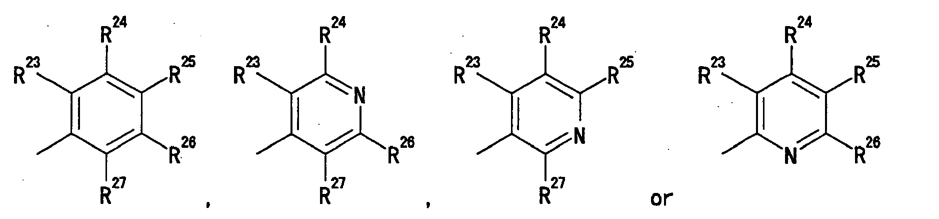 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms (preferably fluorine atom)
   (preferably a C₁₋₄ alkyl group (preferably methyl group) , trifluoromethyl group), or
5) a nitro group, and
   at least one of R²³ to R²⁷ is a hydroxyl group;
is more preferable.

The compound selected from the following group or a pharmaceutically acceptable salt thereof is particularly preferable.
(1) (3-chloro-4-hydroxyphenyl)-(2,3-dihydrobenzo[1,4]oxazin-4-yl)-methanone ,
(2) (3-bromo-4-hydroxyphenyl)-(2,3-dihydrobenzo[1,4]oxazin-4-yl)-methanone,
(3) (3,5-dichloro-4-hydroxyphenyl)-(2,3-dihydrobenzo[1,4]oxazin-4-yl)-methanone,
(4) (3,5-dibromo-4-hydroxyphenyl)-(2,3-dihydrobenzo[1,4]oxazin-4-yl)-methanone,
(5) (2,3-dihydrobenzo[1,4]oxazin-4-yl)-(4-hydroxy-3,5-diiodophenyl)-methanone,
(6) (3,5-difluoro-4-hydroxyphenyl)-(2,3-dihydrobenzo[1,4]oxazin-4-yl)-methanone,
(7) (2,3-dihydrobenzo[1,4]oxazin-4-yl)-(4-hydroxy-3,5-dimethylphenyl)-methanone,
(8) (3,5-dichloro-4-hydroxyphenyl)-(2,3-dihydrobenzo[1,4]thiazin-4-yl)-methanone,
(9) (3,5-dichloro-4-hydroxyphenyl)-(1-oxo-2,3-dihydro-1H-1λ⁴-benzo[1,4]thiazin-4-yl)-methanone,
(10) (3,5-dichloro-4-hydroxyphenyl)-(1,1-dioxo-2,3-dihydro-1H-1λ⁶-benzo [1,4]thiazin-4-yl)-methanone,
(11) (3,5-dichloro-4-hydroxyphenyl)-(2,3-dihydrobenzo[1,4]oxazin-4-yl)-methanethione,
(12) (3,5-dichloro-4-hydroxyphenyl)-(6-methyl-2,3-dihydrobenzo[1,4]oxazin-4-yl)-methanone,
(13) (3,5-dichloro-4-hydroxyphenyl)-(7-methyl-2,3-dihydrobenzo[1,4]oxazin-4-yl)-methanone,
(14) (3,5-dichloro-4-hydroxyphenyl)-(5-methyl-2,3-dihydrobenzo[1,4]oxazin-4-yl)-methanone,
(15) (3,5-dichloro-4-hydroxyphenyl)-(8-methyl-2,3-dihydrobenzo[1,4]oxazin-4-yl)-methanone,
(16) (3,5-dichloro-4-hydroxyphenyl)-(2,3-dihydronaphtho[2,1-b][1,4]oxazin-1-yl)-methanone,
(17) (3,5-dichloro-4-hydroxyphenyl)-(6-methoxy-2,3-dihydrobenzo[1,4]oxazin-4-yl)-methanone,
(18) (3,5-dichloro-4-hydroxyphenyl)-(7-methoxy-2,3-dihydrobenzo[1,4]oxazin-4-yl)-methanone,
(19) (3,5-dichloro-4-hydroxyphenyl)-(6-hydroxy-2,3-dihydrobenzo[1,4]oxazin-4-yl)-methanone,
(20) (3,5-dichloro-4-hydroxyphenyl)-(7-hydroxy-2,3-dihydrobenzo[1,4]oxazin-4-yl)-methanone,
(21) 4-(3,5-dichloro-4-hydroxybenzoyl)-3,4-dihydro-2H-benzo[1,4]oxazine-6-sulfonic acid diethylamide,
(22) 2,6-dichloro-4-(2,3-dihydrobenzo[1,4]oxazine-4-sulfonyl)phenol,
(23) (6-tert-butyl-2,3-dihydrobenzo[1,4]oxazin-4-yl)-(3,5-dichloro-4-hydroxyphenyl)-methanone,
(24) 4-(3,5-dichloro-4-hydroxybenzoyl)-4H-benzo[1,4]oxazin-3-one
(25) 4-(3,5-dichloro-4-hydroxybenzoyl)-3,4-dihydro-2H-benzo[1,4]oxazine-,6-sulfonamide,
(26) (3,5-dichloro-4-hydroxyphenyl)-(3,4-dihydro-2H-quinolin-1-yl)-methanone,
(27) (3,5-dichloro-4-hydroxyphenyl)-(2,3,4,5-tetrahydrobenzo[b]azepin-1-yl)-methanone,
(28) (4-amino-3,5-dichlorophenyl)-(2,3-dihydrobenzo[1,4]oxazin-4-yl)-methanone,
(29) (5-chloro-6-hydroxypyridin-3-yl)-(2,3-dihydrobenzo[1,4]oxazin-4-yl)-methanone,
(30) (2,3-dihydrobenzo[1,4]oxazin-4-yl)-(4-hydroxy-3,5-dinitrophenyl)-methanone,
(31) (3-chloro-4-hydroxy-5-nitrophenyl)-(2,3-dihydrobenzo[1,4]oxazin-4-yl)-methanone,
(32) (3,5-dichloro-4-hydroxyphenyl)-(2,8-diisopropyl-2,3-dihydrobenzo[1,4]oxazin-4-yl)-methanone,
(33) (3,5-dichloro-4-hydroxyphenyl)-[6-(pyrrolidine-1-sulfonyl)-2,3-dihydrobenzo[1,4]oxazin-4-yl]-methanone,
(34) 4-(3,5-dichloro-4-hydroxybenzoyl)-3,4-dihydro-2H-benzo[1,4]oxazine-6-sulfonic acid ethylamide,
(35) 4-(3,5-dichloro-4-hydroxybenzoyl)-3,4-dihydro-2H-benzo[1,4]oxazine-6-sulfonic acid dimethylamide,
(36) (3,5-dichloro-4-hydroxyphenyl)-(2,3-dihydropyrido[3,2-b][1,4]oxazin-4-yl)-methanone,
(37) 5-(3,5-dichloro-4-hydroxybenzoyl)-1,3,4,5-tetrahydrobenzo[b][1,4]diazepin-2-one,
(38) (3,5-dichloro-2-hydroxyphenyl)-(2,3-dihydrobenzo[1,4]oxazin-4-yl)-methanone,
(39) (2,3-dihydrobenzo[1,4]oxazin-4-yl)-(4-hydroxy-3-trifluoromethylphenyl)-methanone,
(40) (3-chloro-4-hydroxy-5-methoxyphenyl)-(2,3-dihydrobenzo[1,4]oxazin-4-yl)-methanone,
(41) (4-chloro-3-hydroxyphenyl)-(2,3-dihydrobenzo[1,4]oxazin-4-yl)-methanone,
(42) (2,6-dichloropyridin-4-yl)-(2,3-dihydrobenzo[1,4]oxazin-4-yl)-methanone,
(43) (2,3-dihydrobenzo[1,4]oxazin-4-yl)-(4-nitrophenyl)-methanone,
(44) (3,5-dichloro-4-hydroxyphenyl)-(6-fluoro-2,3-dihydrobenzo[1,4]oxazin-4-yl)-methanone,
(45) 2-(2,3-dihydrobenzo[1,4]oxazine-4-carbonyl)benzoic acid,
(46) methyl 4-(2,3-dihydrobenzo[1,4]oxazine-4-carbonyl)benzoate,
(47) 4-(2,3-dihydrobenzo[1,4]oxazine-4-carbonyl)benzoic acid,
(48) methyl 3-(2,3-dihydrobenzo[1,4]oxazine-4-carbonyl)benzoate,
(49) 3-(2,3-dihydrobenzo[1,4]oxazine-4-carbonyl)benzoic acid,
(50) (3,5-dichloro-2,4-dihydroxyphenyl)-(2,3-dihydrobenzo[1,4]oxazin-4-yl)-methanone,
(51) (6-chloro-2,3-dihydrobenzo[1,4]oxazin-4-yl)-(3,5-dichloro-4-hydroxyphenyl)-methanone,
(52) (7-chloro-2,3-dihydrobenzo[1,4]oxazin-4-yl)-(3,5-dichloro-4-hydroxyphenyl)-methanone,
(53) [4-(3,5-dichloro-4-hydroxybenzoyl)-3,4-dihydro-2H-quinoxalin-1-yl]-(3,5-dichloro-4-hydroxyphenyl)-methanone,
(54) (3,5-dichloro-4-hydroxyphenyl)-(3,4-dihydro-2H-quinoxalin-1-yl)-methanone,
(55) methyl 4-(3,5-dichloro-4-hydroxybenzoyl)-3,4-dihydro-2H-benzo[1,4]oxazine-6-carboxylate,
(56) (3,5-dichloro-4-hydroxyphenyl)-(6-hydroxymethyl-2,3-dihydrobenzo[1,4]oxazin-4-yl)-methanone,
(57) 4-(3,5-dichloro-4-hydroxybenzoyl)-3,4-dihydro-2H-benzo[1,4]oxazine-6-carboxylic acid,
(58) methyl 4-(3,5-dichloro-4-hydroxybenzoyl)-3,4-dihydro-2H-benzo[1,4]oxazine-5-carboxylate,
(59) methyl 4-(3,5-dichloro-4-hydroxybenzoyl)-3,4-dihydro-2H-benzo[1,4]oxazine-7-carboxylate,
(60) 4-(3,5-dichloro-4-hydroxybenzoyl)-3,4-dihydro-2H-benzo[1,4]oxazine-7-carboxylic acid,
(61) methyl 4-(3,5-dichloro-4-hydroxybenzoyl)-3,4-dihydro-2H-benzo[1,4]oxazine-8-carboxylate,
(62) 4-(3,5-dichloro-4-hydroxybenzoyl)-3,4-dihydro-2H-benzo[1,4]oxazine-8-carboxylic acid,
(63) (3,5-dichlorophenyl)-(2,3-dihydrobenzo[1,4]oxazin-4-yl)-methanone,
(64) (3,5-dichloro-4-hydroxyphenyl)-(phenoxazin-10-yl)-methanone
(65) (3,5-dichloro-4-hydroxyphenyl)-(6-phenyl-2,3-dihydrobenzo[1,4]oxazin-4-yl)-methanone,
(66) (3,5-dichloro-4-hydroxyphenyl)-(6,8-dimethyl-2,3-dihydrobenzo[1,4]oxazin-4-yl)-methanone,
(67) (3,5-dichloro-4-hydroxyphenyl)-(6-nitro-2,3-dihydrobenzo[1,4]oxazin-4-yl)-methanone,
(68) (6-amino-2,3-dihydrobenzo[1,4]oxazin-4-yl)-(3,5-dichloro-4-hydroxyphenyl)-methanone,
(69) (3,5-dibromo-4-hydroxyphenyl)-(6-nitro-2,3-dihydrobenzo[1,4]oxazin-4-yl)-methanone,
(70) (3,5-dichloro-4-hydroxyphenyl)-(7-nitro-2,3-dihydrobenzo[1,4]oxazin-4-yl)-methanone,
(71) (7-amino-2,3-dihydrobenzo[1,4]oxazin-4-yl)-(3,5-dichloro-4-hydroxyphenyl)-methanone,
(72) N-[4-(3,5-dichloro-4-hydroxybenzoyl)-3,4-dihydro-2H-benzo[1,4]oxazin-7-yl]-methanesulfonamide,
(73) 1-[4-(3,5-dichloro-4-hydroxybenzoyl)-3,4-dihydro-2H-quinoxalin-1-yl]-ethanone,
(74) (3,5-dichloro-4-hydroxyphenyl)-(4-methyl-3,4-dihydro-2H-quinoxalin-1-yl)-methanone,
(75) (2,3-dihydrobenzo[1,4]oxazin-4-yl)-(4-hydroxy-3-nitrophenyl)-methanone,
(76) (3,5-dichloro-4-hydroxyphenyl)-(2-methyl-2,3-dihydroindol-1-yl)-methanone,
(77) (3,5-dichloro-4-hydroxyphenyl)-(2,3-dihydroindol-1-yl)-methanone,
(78) (5-amino-2,3-dihydroindol-1-yl)-(3,5-dichloro-4-hydroxyphenyl)-methanone,
(79) (3,5-dibromo-4-hydroxyphenyl)-(6-fluoro-2,3-dihydrobenzo[1,4]oxazin-4-yl)-methanone,
(80) (3,5-dibromo-4-hydroxyphenyl)-(2,3-dihydronaphtho[2,1-b][1,4]oxazin-1-yl)-methanone,
(81) (3,5-dibromo-4-hydroxyphenyl)-(6-methyl-2,3-dihydrobenzo[1,4]oxazin-4-yl)-methanone,
(82) (6-chloro-2,3-dihydrobenzo[1,4]oxazin-4-yl)-(3,5-dibromo-4-hydroxyphenyl)-methanone,
(83) (3,5-dichloro-4-hydroxyphenyl)-(4-methanesulfonyl-3,4-dihydro-2H-quinoxalin-1-yl)-methanone,
(84) (3,5-dichloro-4-hydroxyphenyl)-(6-ethanesulfonyl-2,3-dihydrobenzo[1,4]oxazin-4-yl)-methanone,
(85) (3,5-dichloro-4-hydroxyphenyl)-(6-trifluoromethyl-2,3-dihydrobenzo[1,4]oxazin-4-yl)-methanone,
(86) (3,5-dichloro-4-methoxyphenyl)-(2,3-dihydrobenzo[1,4]oxazin-4-yl)-methanone,
(87) 2,6-dichloro-4-(2,3-dihydrobenzo[1,4]oxazine-4-carbonyl)phenyl acetate,
(88) (2,3-dihydrobenzo[1,4]oxazin-4-yl)-(4-hydroxyphenyl)-methanone,
(89) (3,5-dichloro-4-hydroxyphenyl)-(5-hydroxy-2,3-dihydrobenzo[1,4]oxazin-4-yl)-methanone,
(90) (3,5-dichloro-4-hydroxyphenyl)-(8-hydroxy-2,3-dihydrobenzo[1,4]oxazin-4-yl)-methanone,
(91) ethyl [2,6-dichloro-4-(2,3-dihydrobenzo[1,4]oxazine-4-carbonyl)phenoxy]acetate,
(92) [2,6-dichloro-4-(2,3-dihydrobenzo[1,4]oxazine-4-carbonyl)phenoxy]acetic acid
(93) (3,5-dichloro-4-hydroxyphenyl)-(3-methyl-2,3-dihydrobenzo[1,4]oxazin-4-yl)-methanone,
(94) N-[2,6-dichloro-4-(2,3-dihydrobenzo[1,4]oxazine-4-carbonyl)phenyl]methanesulfonamide, and
(95) (3,5-dichloro-4-hydroxyphenyl)-(7,8-dihydro-6H-5-oxa-9-azabenzocyclohepten-9-yl)-methanone.

The nitrogen-containing fused ring compounds of the present invention are divided into in the following 6 types (type A - type F) depending on the kind of X², Preferable compounds for each are shown in the following.

### type A

Of compounds [2], type A is a compound wherein
X²_{,} is
1) -N(R⁵) - wherein R⁵ is
   (a) a hydrogen atom, or
   (b) a C₁₋₆ alkyl group optionally substituted by one or more, the same or different substituents selected from the aforementioned group B,
2) -N (COR⁶) - wherein R⁶ is
   (a) a hydroxyl group,
   (b) a C₁₋₆ alkyl group optionally substituted by one or more, the same or different substituents selected from the aforementioned group B,
   (c) a C₁₋₆ alkoxy group optionally substituted by one or more, the same or different substituents selected from (i) and (ii) :
      (i) a substituent selected from the aforementioned group B,
      (ii) a C₁₋₆ alkyl group optionally substituted by one or more, the same or different substituents selected from the aforementioned group B,
   (d) a saturated or unsaturated carbon ring group having 3 to 14 carbon atoms optionally substituted by one or more, the same or different substituents selected from the aforementioned group A,
   (e) a cycloalkylalkoxy group optionally substituted by one or more, the same or different substituents selected from the aforementioned group A,
   (f) an aralkyl group optionally substituted by one or more, the same or different substituents selected from the aforementioned group A, or
   (g) an aralkoxy group optionally substituted by one or more, the same or different substituents selected from the aforementioned group A,
3) -N(S(=O)₂R⁶) - wherein R⁶ is as defined above, or
4) -N(CONR⁷R⁸)- wherein R⁷ and R⁸ are the same or different and each is
   (a) a hydrogen atom, or
   (b) a C₁₋₆ alkyl group optionally substituted by one or more, the same or different substituents selected from the aforementioned group B, or
   (c) R⁷ and R⁸ may form, together with the nitrogen atom they are bonded to, a monocyclic nitrogen-containing saturated heterocycle optionally substituted by one or more, the same or different substituents selected from the aforementioned group A;
-X³-X⁴- is -CH₂-CH₂- ; and
R¹³' is a hydrogen atom.

Of these, a compound wherein
ring A' is 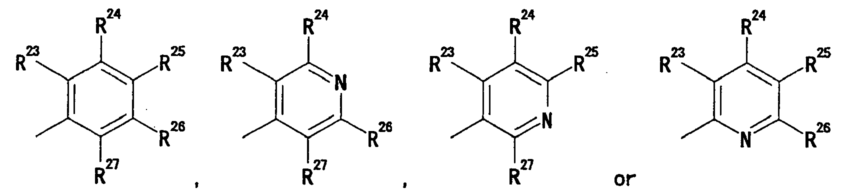 wherein
R²³ to R²⁷ are the same or different and each is
1) a hydrogen atom, or
2) a group selected from the aforementioned group C, and at least one of R²³ to R²⁷ is a hydroxyl group;
   is preferable.

Of these, a compound wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -COOH,
7) -CO-C₁₋₆ alkoxy group,
8) an amino group,
9) -NHS(=O)₂-C₁₋₆ alkyl group,
10) -S (=O)₂-C₁₋₆ alkyl group,
11) -S(=O)₂-NR¹⁵R¹⁶ wherein R¹⁵ and R¹⁶ are the same or different and each is
   (a) a hydrogen atom, or
   (b) a C₁₋₆ alkyl group, or
   (c) R¹⁵ and R¹⁶ may form, together with the nitrogen atom they are bonded to, a monocyclic nitrogen-containing saturated heterocycle,
12) a saturated or unsaturated carbon ring group having 3 to 14 carbon atoms, or
13) a nitro group;
Y is
1) -CO-,
2) -CS-, or
3) -S(=O)₂-;
X¹ is
1) a nitrogen atom, or
2) CR⁴ wherein R⁴ is
   (a) a hydrogen atom,
   (b) a hydroxyl group,
   (c) a C₁₋₆ alkyl group, or
   (d) -CO-C₁₋₆ alkoxy group, or
   (e) R³ and R⁴ may form, together with the carbon atoms they are bonded to, a saturated or unsaturated carbon ring having 3 to 14 carbon atoms;
X², is
1) -N(R⁵)-wherein R⁵ is
   (a) a hydrogen atom, or
   (b) a C₁₋₆ alkyl group,
2) -N(COR⁶)- wherein R⁶ is
   (a) a C₁₋₆ alkyl group, or
   (b) a saturated or unsaturated carbon ring group having 3 to 14 carbon atoms optionally substituted by one or more, the same or different substituents selected from a halogen atom and a hydroxyl group, or
3) -N (S (=O) ₂-C₁₋₆ alkyl group)-;
-X³-X⁴- is -CH₂-CH₂-; and
ring A' is 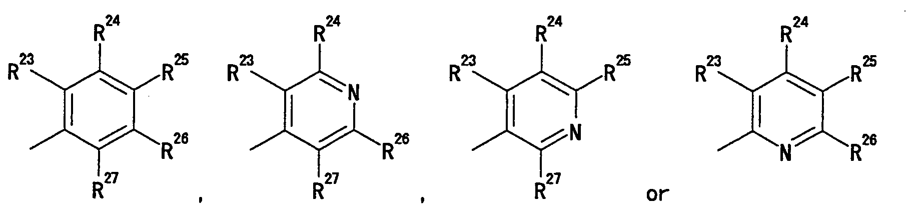 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms,
5) -COOH,
6) -CO-C₁₋₆ alkoxy group,
7) an amino group,
8) -NHS(=O)₂-C₁₋₆ alkyl group, or
9) a nitro group, and
at least one of R²³ to R²⁷ is a hydroxyl group;
is more preferable.

Particularly, a compound wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -CO-C₁₋₆ alkoxy group,
7) -S(=O)₂-NR¹⁵R¹⁶ wherein R¹⁵ and R¹⁶ are the same or different,
   (a) a C₁₋₆ alkyl group, or
   (b) R¹⁵ and R¹⁶ may form, together with the nitrogen atom they are bonded to, a monocyclic nitrogen-containing saturated heterocycle, or
8) a nitro group;
Y is
1) -CO-, or
2) -CS-;
X¹ is
CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X²' is
1) -N (R⁵)- wherein R⁵ is
   (a) a hydrogen atom, or
   (b) a C₁₋₆ alkyl group,
2) -N(COR⁶)- wherein R⁶ is
   (a) a C₁₋₆ alkyl group, or
   (b) a saturated or unsaturated carbon ring group having 3 to 14 carbon atoms optionally substituted by one or more, the same or different substituents selected from a halogen atom and a hydroxyl group, or
3) -N(S(=O)₂-C₁₋₆ alkyl group) -;
-X³-X⁴- is -CH₂-CH₂-; and
ring A' is 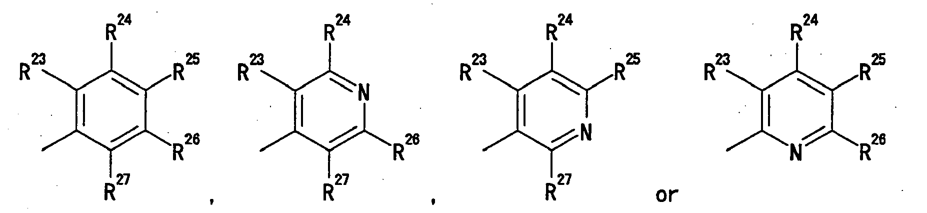 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms, or
5) a nitro group, and
at least one of R²³ to R²⁷ is a hydroxyl group;
is particularly preferable.

### type B

Of compounds [2], type B is a compound wherein
X², is
1) -S(=O)-, or
2) -S(=O)₂-; and
-X³-X⁴- is -CH₂-CH₂-.

Of these, a compound wherein
ring A' is 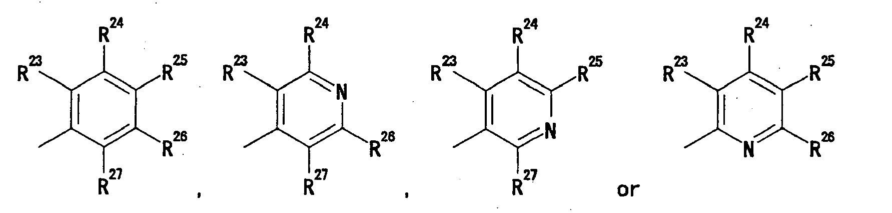 wherein
R²³ to R²⁷ are the same or different and each is
1) a hydrogen atom, or
2) a group selected from the aforementioned group C, and at least one of R²³ to R²⁷ is -OR¹³' wherein R¹³' is as defined above;
is preferable.

Of these, a compound wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -COOH,
7) -CO-C₁₋₆ alkoxy group,
8) an amino group,
9) -NHS(=O)₂-C₁₋₆ alkyl group,
10) -S (=O)₂-C₁₋₆ alkyl group,
11) -S(=O)₂-NR¹⁵R¹⁶ wherein R¹⁵ and R¹⁶ are the same or different and each is
   (a) a hydrogen atom, or
   (b) a C₁₋₆ alkyl group, or
   (c) R¹⁵ and R¹⁶ may form, together with the nitrogen atom they are bonded to, a monocyclic nitrogen-containing saturated heterocycle,
12) a saturated or unsaturated carbon ring group having 3 to 14 carbon atoms, or
13) a nitro group;
Y is
1) -CO-,
2) -CS-, or
3) -S(=O)₂-;
X¹ is
1) a nitrogen atom, or
2) CR⁴ wherein R⁴ is
   (a) a hydrogen atom,
   (b) a hydroxyl group,
   (c) a C₁₋₆ alkyl group, or
   (d) -CO-C₁₋₆ alkoxy group, or
   (e) R³ and R⁴ may form, together with the carbon atoms they are bonded to, a saturated or unsaturated carbon ring having 3 to 14 carbon atoms;
X²' is
1) -S(=O)-, or
2) -S(=O)₂-;
-X³-X⁴- is -CH₂-CH₂-; and
ring A' is 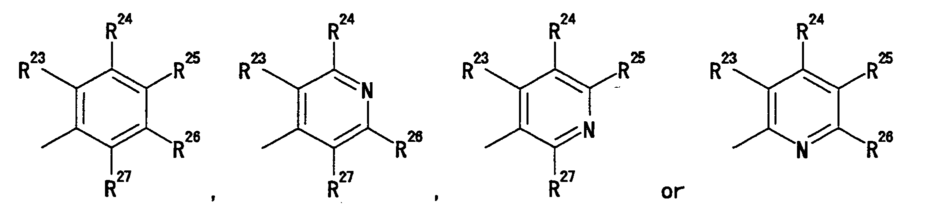 wherein
R²³ to R²⁷ are the same or different and each is
1) a hydrogen atom,
2) a halogen atom,
3) a hydroxyl group,
4) a C₁₋₆ alkoxy group optionally substituted by one or more, the same or different substituents selected from - COOH and -CO-C₁₋₆ alkoxy group,
5) -O-CO-C₁₋₆ alkyl group,
6) a C₁₋₆ alkyl group optionally substituted by one or more, the same or different halogen atoms,
7) -COOH,
8) -CO-C₁₋₆ alkoxy group,
9) an amino group,
10) -NHS (=O)₂-C₁₋₆ alkyl group, or
11) a nitro group, and
at least one of R²³ to R²⁷ is a group selected from a hydroxyl group, a C₁₋₆ alkoxy group optionally substituted by one or more, the same or different substituents selected from -COOH and -CO-C₁₋₆ alkoxy group, and -O-CO-C₁₋₆ alkyl group;
is more preferable.

Particularly, a compound wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -CO-C₁₋₆ alkoxy group,
7) -S(=O)₂-NR¹⁵R¹⁶ wherein R¹⁵ and R¹⁶ are the same or different,
   (a) a C₁₋₆ alkyl group, or
   (b) R¹⁵ and R¹⁶ may form, together with the nitrogen atom they are bonded to, a monocyclic nitrogen-containing saturated heterocycle, or
8) a nitro group;
Y is
1) -CO-, or
2) -CS-;
X¹ is
CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
x², is
1) -S(=O)-, or
2) -S(=O)₂-;
-X³-X⁴- is -CH₂-CH₂-; and
ring A' is 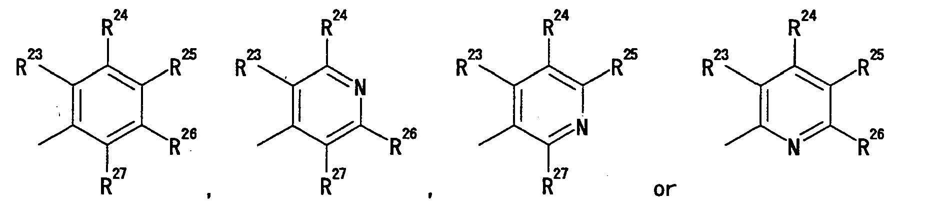 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms, or
5) a nitro group, and
at least one of R²³ to R²⁷ is a hydroxyl group;
is particularly preferable .

### type C

Of compounds [2], type C is a compound wherein
Y is
1) -CO-, or
2) -CS-,
x², is a sulfur atom;
-X³-X⁴- is -CH₂-CH₂-; and
R¹³, is a hydrogen atom.

Of these, a compound wherein
ring A' is 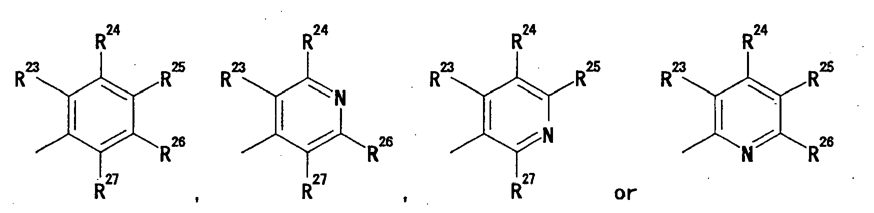 wherein
R²³ to R²⁷ are the same or different and each is
1) a hydrogen atom, or
2) a group selected from the aforementioned group C, and at least one of R²³ to R²⁷ is a hydroxyl group;
   is preferable.

Of these, a compound wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -COOH,
7) -CO-C₁₋₆ alkoxy group,
8) an amino group,
9) -NHS(=O)₂-C₁₋₆ alkyl group,
10) -S(=O)₂-C₁₋₆ alkyl group,
11) -S(=O)₂-NR¹⁵R¹⁶ wherein R¹⁵ and R¹⁶ are the same or different and each is
   (a) a hydrogen atom, or
   (b) a C₁₋₆ alkyl group, or
   (c) R¹⁵ and R¹⁶ may form, together with the nitrogen atom they are bonded to, a monocyclic nitrogen-containing saturated heterocycle,
12) a saturated or unsaturated carbon ring group having 3 to 14 carbon atoms, or
13) a nitro group;
Y is
1) -CO-, or
2) -CS-,
X¹ is
1) a nitrogen atom, or
2) CR⁴ wherein R⁴ is
   (a) a hydrogen atom,
   (b) a hydroxyl group,
   (c) a C₁₋₆ alkyl group, or
   (d) -CO-C₁₋₆ alkoxy group, or
   (e) R³ and R⁴ may form, together with the carbon atoms they are bonded to, a saturated or unsaturated carbon ring having 3 to 14 carbon atoms;
X²' is a sulfur atom;
-X³-X⁴- is -CH₂-CH₂-; and
ring A' is 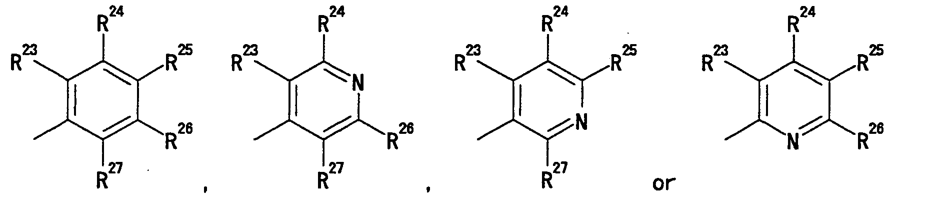 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms,
5) -COOH,
6) -CO-C₁₋₆ alkoxy group,
7) an amino group,
8) -NHS (=O) ₂-C₁₋₆ alkyl group, or
9) a nitro group, and
at least one of R²³ to R²⁷ is a hydroxyl group;
is more preferable.

Particularly, a compound wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -CO-C₁₋₆ alkoxy group,
7) -S (=O)₂-NR¹⁵R¹⁶ wherein R¹⁵ and R¹⁶ are the same or different,
   (a) a C₁₋₆ alkyl group, or
   (b) R¹⁵ and R¹⁶ may form, together with the nitrogen atom they are bonded to, a monocyclic nitrogen-containing saturated heterocycle, or
8) a nitro group;
Y is
1) -CO-, or
2) -CS-;
x¹ is
CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X²' is a sulfur atom;
-X³-X⁴- is -CH₂-CH₂- ; and
ring A' is 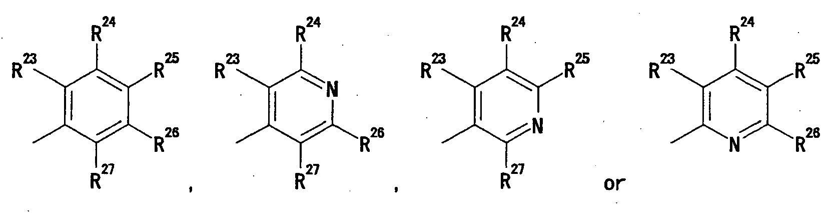 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms, or
5) a nitro group, and
at least one of R²³ to R²⁷ is a hydroxyl group;
is particularly preferable.

### type D

Of compounds [2], type D is a compound wherein
R¹, R² and R³ are the same or different and each is
1) a hydrogen atom, or
2) a group selected from the aforementioned group D, or
3) R¹ and R² may form, together with the carbon atoms they are bonded to, a saturated or unsaturated carbon ring having 3 to 14 carbon atoms optionally substituted by one or more, the same or different substituents selected from the aforementioned group D, or
4) R² and R³ may form, together with the carbon atoms they are bonded to, a saturated or unsaturated carbon ring having 3 to 14 carbon atoms optionally substituted by one or more, the same or different substituents selected from the aforementioned group D; and
Y is
1) -CO-, or
2) -CS-;
X²' is an oxygen atom; and
-X³-X⁴- is -CH₂-CH₂-.

Of these, a compound wherein
ring A' is 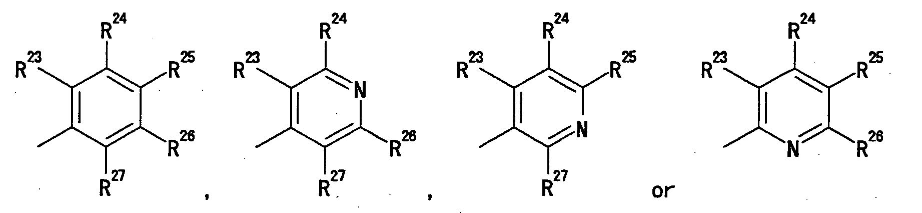 wherein
R²³ to R²⁷ are the same or different and each is
1) a hydrogen atom, or
2) a group selected from the aforementioned group C, and at least one of R²³ to R²⁷ is -OR¹³' wherein R¹³' is as defined above;
is preferable.

Of these, a compound wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -COOH,
7) -CO-C₁₋₆ alkoxy group,
8) an amino group,
9) -NHS(=O)₂-C₁₋₆ alkyl group,
10) *-*S(=O)₂-C₁₋₆ alkyl group,
11) -S(=O)₂-NR¹⁵''¹⁶''wherein R¹⁵" and R¹⁶" are the same or different and each is
   (a) a hydrogen atom, or
   (b) a C₁₋₆ alkyl group, or
   (c) R¹⁵" and R¹⁶" may form, together with the nitrogen atom they are bonded to, a monocyclic nitrogen-containing saturated heterocycle,
12) a saturated or unsaturated carbon ring group having 3 to 14 carbon atoms, or
13) a nitro group;
Y is
1) -CO-, or
2) -CS-;
X¹ is
1) a nitrogen atom, or
2) CR⁴ wherein R⁴ is
   (a) a hydrogen atom,
   (b) a hydroxyl group,
   (c) a C₁₋₆ alkyl group, or
   (d) -CO-C₁₋₆ alkoxy group, or
   (e) R³ and R⁴ may form, together with the carbon atoms they are bonded to, a saturated or unsaturated carbon ring having 3 to 14 carbon atoms;
X²' is an oxygen atom;
-X³-X⁴- is -CH₂-CH₂-; and
ring A' is 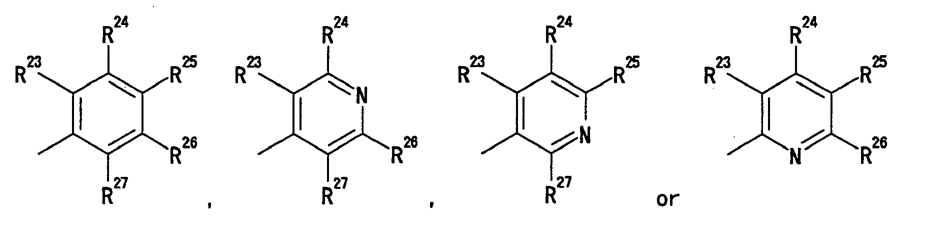 wherein
R²³ to R²⁷ are the same or different and each is
1) a hydrogen atom,
2) a halogen atom,
3) a hydroxyl group,
4) a C₁₋₆ alkoxy group optionally substituted by one or more, the same or different substituents selected from - COOH and -CO-C₁₋₆ alkoxy group,
5) -O-CO-C₁₋₆ alkyl group,
6) a C₁₋₆ alkyl group optionally substituted by one or more, the same or different halogen atoms,
7) -COOH,
8) -CO-C₁₋₆ alkoxy group,
9) an amino group,
10) -NHS(=O)₂-C₁₋₆ alkyl group, or
11) a nitro group, and
at least one of R²³ to R²⁷ is a group selected from a hydroxyl group, a C₁₋₆ alkoxy group optionally substituted by one or more, the same or different substituents selected from -COOH and -CO-C₁₋₆ alkoxy group, and -O-CO-C₁₋₆ alkyl group;
is more preferable.

Particularly, a compound wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -CO-C₁₋₆ alkoxy group,
7) -S(=O)₂-NR¹⁵''¹⁶'' wherein R¹⁵'' and R¹⁶'' are the same or different,
   (a) a C₁₋₆ alkyl group, or
   (b) R¹⁵" and R¹⁶" may form, together with the nitrogen atom they are bonded to, a monocyclic nitrogen-containing saturated heterocycle, or
8) a nitro group;
Y is
1) -CO-, or
2) -CS-;
X¹ is
CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X²' is an oxygen atom;
-X³-X⁴- is -CH₂-CH₂- ; and
ring A' is 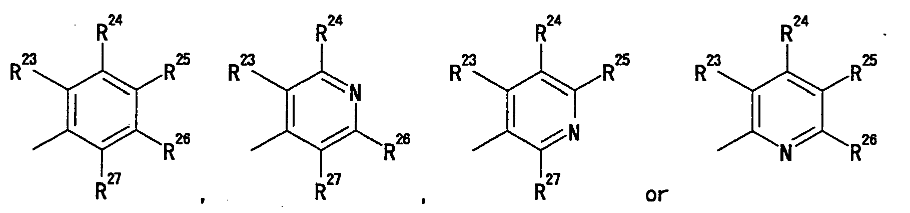 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms, or
5) a nitro group, and
at least one of R²³ to R²⁷ is a hydroxyl group;
is particularly preferable.

### type E

Of compounds [2], type E is a compound wherein
Y is -S(=O)₂-,
X^{2'} is
1) an oxygen atom, or
2) a sulfur atom;
-X³-X⁴- is -CH₂-CH₂-; and
R¹³' is a hydrogen atom.

Of these, a compound wherein
ring A' is 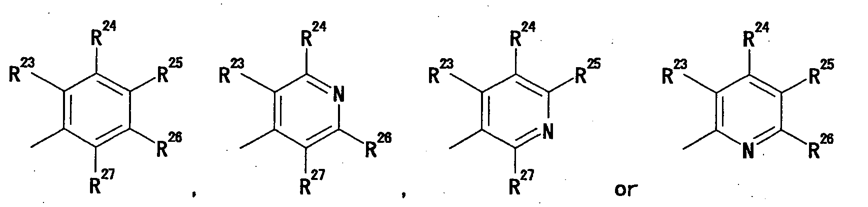 wherein
R²³ to R²⁷ are the same or different and each is
1) a hydrogen atom, or
2) a group selected from the aforementioned group C, and at least one of R²³ to R²⁷ is a hydroxyl group;
   is preferable.

Of these, a compound wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -COOH,
7) -CO-C₁₋₆ alkoxy group,
8) an amino group,
9) -NHS(=O)₂-C₁₋₆ alkyl group,
10) -S (=O)₂-C₁₋₆ alkyl group,
11) -S(=O)₂-NR¹⁵R¹⁶ wherein R¹⁵ and R¹⁶ are the same or different and each is
   (a) a hydrogen atom, or
   (b) a C₁₋₆ alkyl group, or
   (c) R¹⁵ and R¹⁶ may form, together with the nitrogen atom they are bonded to, a monocyclic nitrogen-containing saturated heterocycle,
12) a saturated or unsaturated carbon ring group having 3 to 14 carbon atoms, or
13) a nitro group;
Y is -S(=O)₂-.
X¹ is
1) a nitrogen atom, or
2) CR⁴ wherein R⁴ is
   (a) a hydrogen atom,
   (b) a hydroxyl group,
   (c) a C₁₋₆ alkyl group, or
   (d) -CO-C₁₋₆ alkoxy group, or
   (e) R³ and R⁴ may form, together with the carbon atoms they are bonded to, a saturated or unsaturated carbon ring having 3 to 14 carbon atoms;
X²' is
1) an oxygen atom, or
2) a sulfur atom;
-X³-X⁴- is -CH₂-CH₂-; and
ring A' is 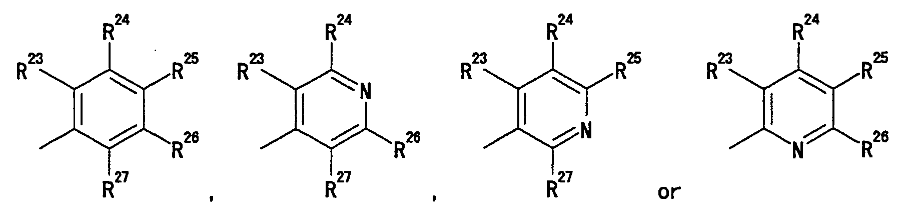 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms,
5) -COOH,
6) -CO-C₁₋₆ alkoxy group,
7) an amino group,
8) -NHS(=O)₂-C₁₋₆ alkyl group, or
9) a nitro group, and
at least one of R²³ to R²⁷ is a hydroxyl group;
is more preferable.

Particularly, a compound wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -CO-C₁₋₆ alkoxy group,
7) -S(=O)₂-NR¹⁵R¹⁶ wherein R¹⁵ and R¹⁶ are the same or different,
   (a) a C₁₋₆ alkyl group, or
   (b) R¹⁵ and R¹⁶ may form, together with the nitrogen atom they are bonded to, a monocyclic nitrogen-containing saturated heterocycle, or
8) a nitro group;
Y is -S(=O)₂-,
X¹ is
CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X²' is
1) an oxygen atom, or
2) a sulfur atom;
-X³-X⁴- is -CH₂-CH₂-; and
ring A' is 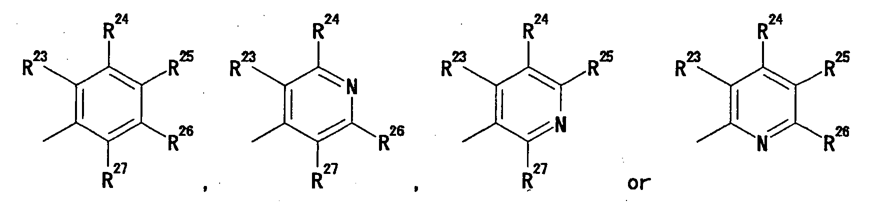 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms, or
5) a nitro group, and
at least one of R²³ to R²⁷ is a hydroxyl group;
is particularly preferable

### type F

Of compounds [2], type F is a compound wherein
X²' is -CH₂-;
-X³-X⁴- is
- (CR¹¹R¹²)n- wherein n is an integer of 1 to 3, and R¹¹ and R¹² each in the number of n are the same or different and each is
(a) a hydrogen atom, or
(b) R¹¹ and R¹² bonded to a single carbon atom may in combination form an oxo group, or
(c) two of R¹¹ and R¹² each in the number of n, which are bonded to a single carbon atom or two adjacent carbon atoms, may form, together with the carbon atom(s), a saturated or unsaturated carbon ring having 3 to 14 carbon atoms optionally substituted by one or more, the same or different substituents selected from the aforementioned group A;
R¹³' is a hydrogen atom; and ring A' is further substituted by at least one a halogen atom; provided that when both R¹¹ and R¹² are hydrogen atoms, and n is 2, then all of R¹, R² and R³ should be hydrogen atoms.

Of these, a compound wherein
ring A' is 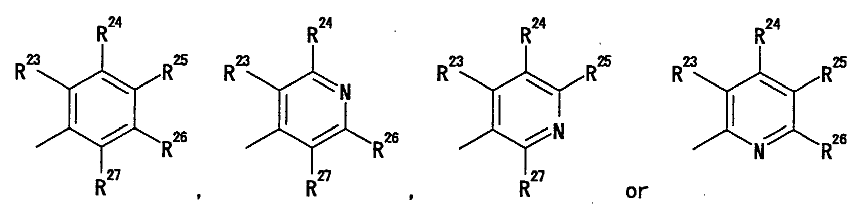 wherein
R²³ to R²⁷ are the same or different and each is
1) a hydrogen atom, or
2) a group selected from the aforementioned group C, and at least one of R²³ to R²⁷ is a hydroxyl group, and at least one of R²³ to R²⁷ is a halogen atom;
is preferable.

Of these, a compound wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -COOH,
7) -CO-C₁₋₆ alkoxy group,
8) an amino group,
9) -NHS (=O)₂-C₁₋₆ alkyl group,
10) -S (=O)₂-C₁₋₆ alkyl group,
11) -S(=O)₂-NR¹⁵R¹⁶ wherein R¹⁵ and R¹⁶ are the same or different and each is
   (a) a hydrogen atom, or
   (b) a C₁₋₆ alkyl group, or
   (c) R¹⁵ and R¹⁶ may form, together with the nitrogen atom they are bonded to, a monocyclic nitrogen-containing saturated heterocycle,
12) a saturated or unsaturated carbon ring group having 3 to 14 carbon atoms, or
13) a nitro group;
Y is
1) -CO-,
2) -CS-, or
3) -S(=O)₂-;
X¹ is
1) a nitrogen atom, or
2) CR⁴ wherein R⁴ is
   (a) a hydrogen atom,
   (b) a hydroxyl group,
   (c) a C₁₋₆ alkyl group, or
   (d) -CO-C₁₋₆ alkoxy group, or
   (e) R³ and R⁴ may form, together with the carbon atoms they are bonded to, a saturated or unsaturated carbon ring having 3 to 14 carbon atoms;
X²' is -CH₂-;
-X³-X⁴- is
-(CR¹¹R¹²)n- wherein n is an integer of 1 to 3, and R¹¹ and R¹² each in the number of n are the same or different and each is
(a) a hydrogen atom, or
(b) R¹¹ and R¹² bonded to a single carbon atom may in combination form an oxo group, or
(c) two of R¹¹ and R¹² each in the number of n, which are bonded to a single carbon atom or two adjacent carbon atoms, may form, together with the carbon atom(s), a saturated or unsaturated carbon ring having 3 to 14 carbon atoms optionally substituted by one or more, the same or different substituents selected from the aforementioned group A; and
ring A' is 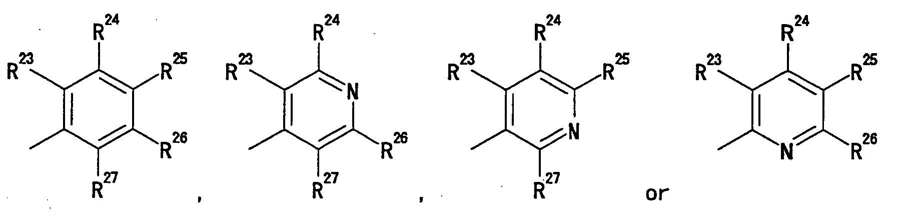 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms,
5) -COOH,
6) -CO-C₁₋₆ alkoxy group,
7) an amino group,
8) -NHS(=O)₂-C₁₋₆ alkyl group, or
9) a nitro group, and at least one of R²³ to R²⁷ is a hydroxyl group, and at least one of R²³ to R²⁷ is a halogen atom;
provided that when both R¹¹ and R¹² are hydrogen atoms, and n is 2, then all of R¹, R² and R³ should be hydrogen atoms; is more preferable.

Particularly, a compound wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -CO-C₁₋₆ alkoxy group,
7) -S(=O)₂-NR¹⁵R¹⁶ wherein R¹⁵ and R¹⁶ are the same or different,
   (a) a C₁₋₆ alkyl group, or
   (b) R¹⁵ and R¹⁶ may form, together with the nitrogen atom they are bonded to, a monocyclic nitrogen-containing saturated heterocycle, or
8) a nitro group;
Y is
1) -CO-, or
2) -CS-;
X¹ is
CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
x^{2,} is -CH₂-;
-X³-X⁴- is
- (CR¹¹R¹²)n- wherein n is an integer of 1 to 3, and R¹¹ and R¹² each in the number of n are the same or different and each
is
(a) a hydrogen atom, or
(b) R¹¹ and R¹² bonded to a single carbon atom may in combination form an oxo group, or
(c) two of R¹¹ and R¹² each in the number of n, which are bonded to a single carbon atom or two adjacent carbon atoms, may form, together with the carbon atom(s), a saturated or unsaturated carbon ring having 3 to 14 carbon atoms optionally substituted by one or more, tHe same or different substituents selected from the aforementioned group A; and
ring A' is 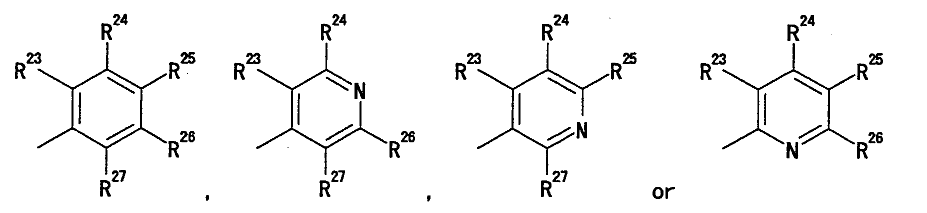 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms, or
5) a nitro group, and at least one of R²³ to R²⁷ is a hydroxyl group, and at least one of R²³ to R²⁷ is a halogen atom;
provided that when both R¹¹ and R¹² are hydrogen atoms, and n is 2, then all of R¹, R² and R³ should be hydrogen atoms; is particularly preferable.

The nitrogen-containing fused ring compound of the present invention can also be obtained as a crystal. For example,
(1) (3-chloro-4-hydroxyphenyl)-(2,3-dihydrobenzo[1,4]oxazin-4-yl)-methanone having characteristic diffraction peaks at 12.94°, 17.36°, 23.50°, 26.10° and 26.94° of diffraction angle (2θ) as measured by powder X-ray crystal diffraction (Example 1),
(2) (3-bromo-4-hydroxyphenyl)-(2,3-dihydrobenzo[1,4]oxazin-4-yl)-methanone having characteristic diffraction peaks at 16.96°, 17.54°, 21.66°, 25.68° and 26.62° of diffraction angle (2θ) as measured by powder X-ray crystal diffraction (Example 2),
(3) (3,5-dichloro-4-hydroxyphenyl)-(2,3-dihydrobenzo[1,4]oxazin-4-yl)-methanone having characteristic diffraction peaks at 7.40°, 14.90°, 22.68°, 22.92° and 26.46° of diffraction angle (2θ) as measured by powder X-ray crystal diffraction (Example 3),
(4) (3,5-dibromo-4-hydroxyphenyl)-(2,3-dihydrobenzo[1,4]oxazin-4-yl)-methanone having characteristic diffraction peaks at 7.40°, 14.92°, 16.64°, 22.68° and 26.12° of diffraction angle (2θ) as measured by powder X-ray crystal diffraction (Example 4),
(5) (2,3-dihydrobenzo[1,4]oxazin-4-yl)-(4-hydroxy-3,5-diiodophenyl)-methanone having characteristic diffraction peaks at 7.28°, 15.84°, 23.10°, 29.54° and 37.16° of diffraction angle (2θ) as measured by powder X-ray crystal diffraction (Example 5),
(6) (3,5-difluoro-4-hydroxyphenyl)-(2,3-dihydrobenzo[1,4]oxazin-4-yl)-methanone having characteristic diffraction peaks at 15.58°, 17.92°, 18.48°, 19.86° and 25.90° of diffraction angle (2θ) as measured by powder X-ray crystal diffraction (Example 6),
(7) (2,3-dihydrobenzo[1,4]oxazin-4-yl)-(4-hydroxy-3,5-dimethylphenyl)-methanone having characteristic diffraction peaks at 12.46°, 23.38°, 23.98°, 24.32° and 25.24° of diffraction angle (2θ) as measured by powder X-ray crystal diffraction (Example 7),
(8) (3,5-dichloro-4-hydroxyphenyl)-(1,1-dioxo-2,3-dihydro-1H-1λ⁶-benzo[1,4]thiazin-4-yl)-methanone having characteristic diffraction peaks at 16.62°, 18.64°, 19.20°, 21.60° and 23.14° of diffraction angle (2θ) as measured by powder X-ray crystal diffraction (Example 10),
(9) (3,5-dichloro-4-hydroxyphenyl)-(6-methyl-2,3-dihydrobenzo[1,4]oxazin-4-yl)-methanone having characteristic diffraction peaks at 11.58°, 14.36°, 22.18°, 22.48° and 23.36° of diffraction angle (2θ) as measured by powder X-ray crystal diffraction (Example 12),
(10) (3,5-dichloro-4-hydroxyphenyl) - (5-methyl-2 , 3-dihydrobenzo[1,4]oxazin-4-yl)-methanone having characteristic diffraction peaks at 11.82°, 14.88°, 22.62°, 25.56° and 26.62° of diffraction angle (2θ) as measured by powder X-ray crystal diffraction (Example 14),
(11) (3,5-dichloro-4-hydroxyphenyl)-(8-methyl-2,3-dihydrobenzo[1,4]oxazin-4-yl)-methanone having characteristic diffraction peaks at 7.02°, 14.14°, 21.30°, 21.80° and 26.56° of diffraction angle (2θ) as measured by powder X-ray crystal diffraction (Example 15),
(12) (3,5-dichloro-4-hydroxyphenyl)-(2,3-dihydronaphtho[2,1-b][1,4]oxazin-1-yl)-methanone having characteristic diffraction peaks at 12.40°, 18.36°, 21.34°, 23.66° and 24.24° of diffraction angle (2θ) as measured by powder X-ray crystal diffraction (Example 16),
(13) (3,5-dichloro-4-hydroxyphenyl)-(6-methoxy-2,3-dihydrobenzo[1,4]oxazin-4-yl)-methanone having characteristic diffraction peaks at 10.54°, 11.24°, 21.24°, 21.60° and 24.38° of diffraction angle (2θ) as measured by powder X-ray crystal diffraction (Example 17),
(14) (3,5-dichloro-4-hydroxyphenyl)-(7-methoxy-2,3-dihydrobenzo[1,4]oxazin-4-yl)-methanone having characteristic diffraction peaks at 12.32°, 13.52°, 22.70°, 24.88° and 26.10° of diffraction angle (2θ) as measured by powder X-ray crystal diffraction (Example 18),
(15) (3,5-dichloro-4-hydroxyphenyl)-(6-hydroxy-2,3-dihydrobenzo [1,4] oxazin-4-yl)-methanone having characteristic diffraction peaks at 11.16°, 14.58°, 21.38°, 22.54° and 22.76° of diffraction angle (2θ) as measured by powder X-ray crystal diffraction (Example 19),
(16) (3,5-dichloro-4-hydroxyphenyl)-(7-hydroxy-2,3-dihydrobenzo[1,4]oxazin-4-yl)-methanone having characteristic diffraction peaks at 15.46°, 20.90°, 22.92°, 24.68° and 25.24° of diffraction angle (2θ) as measured by powder X-ray crystal diffraction (Example 20),
(17) 4-(3,5-dichloro-4-hydroxybenzoyl)-3,4-dihydro-2H-benzo[1,4]oxazine-6-sulfonic acid diethylamide having characteristic diffraction peaks at 13.48°, 15.52°, 19.18°, 20.58° and 21.80° of diffraction angle (2θ) as measured by powder X-ray crystal diffraction (Example 21),
(18) (3,5-dichloro-4-hydroxyphenyl)-(3,4-dihydro-2H-quinolin-1-yl)-methanone having characteristic diffraction peaks at 11.66°, 22.20°, 22.48°, 24.68° and 25.52° of diffraction angle (2θ) as measured by powder X-ray crystal diffraction (Example 26),
(19) (3,5-dichloro-4-hydroxyphenyl)-(2,3,4,5-tetrahydrobenzo[b]azepin-1-yl)-methanone having characteristic diffraction peaks at 17.60°, 21.78°, 22.78°, 24.96° and 32.98° of diffraction angle (2θ) as measured by powder X-ray crystal diffraction (Example 27),
(20) (3-chloro-4-hydroxy-5-nitrophenyl)-(2,3-dihydrobenzo[1,4]oxazin-4-yl)-methanone having characteristic diffraction peaks at 12.16°, 14.90°, 22.16°, 23.46° and 24.52° of diffraction angle (2θ) as measured by powder X-ray crystal diffraction (Example 31),
(21) (3,5-dichloro-4-hydroxyphenyl)-(2,3-dihydropyrido[3,2-b][1,4]oxazin-4-yl)-methanone having characteristic diffraction peaks at 11.58°, 14.78°, 18.80°, 23.66° and 25.52° of diffraction angle (2θ) as measured by powder X-ray crystal diffraction (Example 36),
(22) (3,5-dichloro-2-hydroxyphenyl)-(2,3-dihydrobenzo[1,4]oxazin-4-yl)-methanone having characteristic diffraction peaks at 19.94°, 21.86°, 22.52°, 23.84° and 26.14° of diffraction angle (2θ) as measured by powder X-ray crystal diffraction (Example 38),
(23) (2,3-dihydrobenzo[1,4]oxazin-4-yl)-(4-hydroxy-3-trifluoromethylphenyl)-methanone having characteristic diffraction peaks at 7.58°, 12.10°, 15.24°, 22.30° and 24.40° of diffraction angle (2θ) as measured by powder X-ray crystal diffraction (Example 39),
(24) (3-chloro-4-hydroxy-5-methoxyphenyl)-(2,3-dihydrobenzo[1,4]oxazin-4-yl)-methanone having characteristic diffraction peaks at 10.06°, 17.10°, 17.48°, 21.78° and 22.26° of diffraction angle (2θ) as measured by powder X-ray crystal diffraction (Example 40),
(25) (4-chloro-3-hydroxyphenyl)-(2,3-dihydrobenzo[1,4]oxazin-4-yl)-methanone having characteristic diffraction peaks at 19.06°, 20.76°, 22.30°, 26.58° and 27.60° of diffraction angle (2θ) as measured by powder X-ray crystal diffraction (Example 41),
(26) (3,5-dichloro-4-hydroxyphenyl)-(6-fluoro-2,3-dihydrobenzo[1,4]oxazin-4-yl)-methanone having characteristic diffraction peaks at 11.70°, 22.44°, 22.74°, 23.58° and 23.90° of diffraction angle (2θ) as measured by powder X-ray crystal diffraction (Example 44),
(27) (3,5-dichloro-2,4-dihydroxyphenyl)-(2,3-dihydrobenzo[1,4]oxazin-4-yl)-methanone having characteristic diffraction peaks at 7.26°, 22.62°, 25.12°, 25.70° and 27.92° of diffraction angle (2θ) as measured by powder X-ray crystal diffraction (Example 50) ,
(28) (6-chloro-2,3-dihydrobenzo[1,4]oxazin-4-yl)-(3,5-dichloro-4-hydroxyphenyl)-methanone having characteristic diffraction peaks at 11.72°, 21.78°, 22.54°, 22.82° and 23.68° of diffraction angle (2θ) as measured by powder X-ray crystal diffraction (Example 51),
(29) (3,5-dichloro-4-hydroxyphenyl)-(6-hydroxymethyl-2,3-dihydrobenzo[1,4]oxazin-4-yl)-methanone having characteristic diffraction peaks at 21.04°, 22.22°, 23.74°, 24.72° and 27.38° of diffraction angle (2θ) as measured by powder X-ray crystal diffraction (Example 56),
(30) (3,5-dichloro-4-hydroxyphenyl)-(6,8-dimethyl-2,3-dihydrobenzo[1,4]oxazin-4-yl)-methanone having characteristic diffraction peaks at 14.80°, 16.44°, 22.28°, 22.76° and 24.14° of diffraction angle (2θ) as measured by powder X-ray crystal diffraction (Example 66),
(31) (3,5-dichloro-4-hydroxyphenyl)-(6-nitro-2,3-dihydrobenzo[1,4]oxazin-4-yl)-methanone having characteristic diffraction peaks at 14.52°, 21.52°, 21.88°, 27.84° and 30.92° of diffraction angle (2θ) as measured by powder X-ray crystal diffraction (Example 67),
(32) (3,5-dibromo-4-hydroxyphenyl)-(6-nitro-2,3-dihydrobenzo [1,4] oxazin-4-yl)-methanone having characteristic diffraction peaks at 7.16°, 14.40°, 20.96°, 27.58° and 34.62° of diffraction angle (2θ) as measured by powder X-ray crystal diffraction (Example 69),
(33) 1-[4-(3,5-dichloro-4-hydroxybenzoyl)-3,4-dihydro-2H-quinoxalin-1-yl]-ethanone having characteristic diffraction peaks at 15.48°, 19.24°, 22.48°, 25.54° and 30.30° of diffraction angle (2θ) as measured by powder X-ray crystal diffraction (Example 73),
(34) (3,5-dichloro-4-hydroxyphenyl)-(2-methyl-2,3-dihydroindol-1-yl)-methanone having characteristic diffraction peaks at 10.48°, 11.32°, 20.18°, 22.84° and 25.76° of diffraction angle (2θ) as measured by powder X-ray crystal diffraction (Example 76),
(35) (3,5-dichloro-4-hydroxyphenyl)-(2,3-dihydroindol-1-yl)-methanone having characteristic diffraction peaks at 12.10°, 22.48°, 23.40°, 24.42° and 25.98° of diffraction angle (2θ) as measured by powder X-ray crystal diffraction (Example 77),
(36) (3,5-dibromo-4-hydroxyphenyl)-(6-fluoro-2,3-dihydrobenzo[1,4]oxazin-4-yl)-methanone having characteristic diffraction peaks at 9.30°, 14.52°, 18.64°, 23.96° and 27.12° of diffraction angle (2θ) as measured by powder X-ray crystal diffraction (Example 79),
(37) (3,5-dibromo-4-hydroxyphenyl)-(2,3-dihydronaphtho[2,1-b][1,4]oxazin-1-yl)-methanone having characteristic diffraction peaks at 8.92°, 17.98°, 18.26°, 21.10° and 23.40° of diffraction angle (2θ) as measured by powder X-ray crystal diffraction (Example 80),
(38) (3,5-dibromo-4-hydroxyphenyl)-(6-methyl-2,3-dihydrobenzo[1,4]oxazin-4-yl)-methanone having characteristic diffraction peaks at 7.12°, 14.32°, 21.62°, 21.92° and 22.80° of diffraction angle (2θ) as measured by powder X-ray crystal diffraction (Example 81),
(39) (6-chloro-2,3-dihydrobenzo[1,4]oxazin-4-yl)-(3,5-dibromo-4-hydroxyphenyl)-methanone having characteristic diffraction peaks at 7.22°, 14.54°, 22.34°, 29.08° and 33.22° of diffraction angle (2θ) as measured by powder X-ray crystal diffraction (Example 82) ,
(40) (3,5-dichloro-4-hydroxyphenyl)-(6-trifluoromethyl-2,3-dihydrobenzo[1,4]oxazin-4-yl)-methanone having characteristic diffraction peaks at 17.58°, 19.00°, 21.04°, 21.52° and 23.24° of diffraction angle (2θ) as measured by powder X-ray crystal diffraction (Example 85),
(41) 2,6-dichloro-4-(2,3-dihydrobenzo[1,4]oxazine-4-carbonyl)phenyl acetate having characteristic diffraction peaks at 15.76°, 16.38°, 24.12°, 25.88° and 27.62° of diffraction angle (2θ) as measured by powder X-ray crystal diffraction (Example 87)
   and the like can be mentioned.

The "pharmaceutically acceptable salt thereof" may be any as long as it forms nontoxic salts with compounds [1] and [2] (hereinafter they are also to be collectively referred to as the compound of the present invention) and, for example, salts with inorganic acids such as hydrochloric acid, sulfuric acid, phosphoric acid, hydrobromic acid and the like; salts with organic acids such as oxalic acid, malonic acid, citric acid, fumaric acid, lactic acid, malic acid, succinic acid, tartaric acid, acetic acid, trifluoroacetic acid, gluconic acid, ascorbic acid, methanesulfonic acid, benzenesulfonic acid and the like; salts with inorganic bases such as sodium hydroxide, potassium hydroxide, calcium hydroxide, magnesium hydroxide, ammonium hydroxide and the like; salts with organic bases such as methylamine, diethylamine, triethylamine, triethanolamine, ethylenediamine, tris(hydroxymethyl)methylamine, guanidine, choline, cinchonine and the like; salts with amino acids such as lysine, arginine, alanine and the like, and the like can be mentioned.

The present invention also encompasses water-containing products, hydrates and solvates of the compound of the present invention.

In addition, the compound of the present invention has various isomers. For example, E form and Z form can be present as geometric isomers, when an asymmetric carbon atom is present, enantiomer and diastereomer are present as stereoisomers based thereon, and tautomers can also be present. Therefore, the present invention encompasses all of these isomers and mixtures thereof. Moreover, the present invention encompasses, besides the compound of the present invention, prodrug compounds and metabolite compounds of these compounds as equivalent compounds.

As used herein, the "prodrug" is a derivative having a chemically or metabolically decomposable group, which shows a pharmaceutical activity upon decomposition by hydrolysis or solvolysis, or under physiological conditions. A prodrug is used for, for example, improving absorption by oral administration or targeting the object site. Inasmuch as what the chemically or metabolically decomposable group is, and how to introduced the group into a compound have been sufficiently established in the field of pharmaceutical agents, such known techniques can be employed in the present invention. As the moiety to be modified for producing a prodrug, for example, highly reactive functional groups such as a hydroxyl group, a carboxyl group, an amino group, a thiol group and the like in the compound of the present invention can be mentioned. For example, the compound of the present invention represented by the formula [2] wherein -OR¹³ or -OR^{13'} is a hydroxyl group and the like can be mentioned.

For example, a derivative wherein a substituent such as - CO-C₁₋₆ alkyl group, -CO₂-C₁₋₆ alkyl group, -CONH-C₁₋₆ alkyl group, -CO-C₂₋₆ alkenyl group, -CO₂-C₂₋₆ alkenyl group, -CONH-C₂₋₆ alkenyl group, -CO-aryl group, -CO₂-aryl group, -CONH-aryl group, -CO-heterocyclic group, -CO₂-heterocyclic group, -CONH-heterocyclic group (the C₁₋₆ alkyl group, C₂₋₆ alkenyl group, aryl group and heterocyclic group are each optionally substituted by halogen atom, C₁₋₆ alkyl group, hydroxyl group, C₁₋₆ alkoxy group, carboxy group, amino group, amino acid residue, -PO₃H₂, -SO₃H, -CO-polyethylene glycol residue, -CO₂-polyethylene glycol residue, - CO-polyethylene glycol monoalkyl ether residue, -CO₂-polyethylene glycol monoalkyl ether residue and the like) and the like has been introduced into a hydroxyl group can be mentioned.

In addition, a derivative wherein a substituent such as - CO-C₁₋₆ alkyl group, -CO₂-C₁₋₆ alkyl group, -CO-C₂₋₆ alkenyl group, -CO₂-C₂₋₆ alkenyl group, -CO-aryl group, -CO₂-aryl group, -CO-heterocyclic group, -CO₂-heterocyclic group (the C₁₋₆ alkyl group, C₂₋₆ alkenyl group, aryl group and heterocyclic group are each optionally substituted by halogen atom, C₁₋₆ alkyl group, hydroxyl group, C₁₋₆ alkoxy group, carboxy group, amino group, amino acid residue, -PO₃H₂, -SO₃H, -CO-polyethylene glycol residue, -CO₂-polyethylene glycol residue, -CO-polyethylene glycol monoalkyl ether residue, -CO₂-polyethylene glycol monoalkyl ether residue, -PO₃H₂ and the like) and the like has been introduced into an amino group can be mentioned.

Furthermore, a derivative wherein a substituent such as C₁_ 6 alkoxy group, aryloxy group (the C₁_₆ alkoxy group and aryloxy group are each optionally substituted by halogen atom, C₁₋₆ alkyl group, hydroxyl group, C₁₋₆ alkoxy group, carboxy group, amino group, amino acid residue, -PO₃H₂, -SO₃H, polyethylene glycol residue, polyethylene glycol monoalkyl ether residue and the like) and the like has been introduced into a carboxyl group can be mentioned.

The "pharmaceutical composition" includes, besides what is called a "composition" comprising an active ingredient as a pharmaceutical agent and a pharmaceutically acceptable carrier and the like, a combination agent with other pharmaceutical agents, and the like. It is needless to say that the pharmaceutical composition of the present invention can be concurrently used with any other pharmaceutical agent within the range acceptable in clinical situations. Therefore, the present pharmaceutical composition can also be considered a pharmaceutical composition to be combined with other pharmaceutical agents.

Moreover, the pharmaceutical composition of the present invention can be administered to not only human but also other mammals (mouse, rat, hamster, rabbit, cat, dog, bovine, sheep, monkey etc.). Therefore, the pharmaceutical composition of the present invention is also useful as a pharmaceutical product for animals, not to mention human.

To not "substantially inhibit CYP" means that the function of the drug metabolizing enzyme, cytochrome P450 (CYP), preferably CYP2D6, CYP2C8, CYP2C9, CYP2C19 or CYP3A4, more preferably CYP2C9, is not substantially inhibited and that, for example, the concentration of a substance that dose not substantially inhibit CYP necessary for inhibiting CYP by 50% is not less than 1 µM, preferably not less than 3 µM, more preferably not less than 10 µM, still more preferably not less than 25 µM, particularly preferably not less than 50 µM, based on the conditions of the below-mentioned Experimental Example 2, preferably under the conditions of Experimental Example 2.

To "inhibit URAT1 activity" means to specifically inhibit the function of URAT1 as a uric acid transporter to eliminate or attenuate the activity and means, for example, to specifically inhibit the function of URAT1 based on the conditions of the below-mentioned Experimental Example 1 and preferably means that the concentration necessary for inhibiting URAT1 by 50% is less than 3 µM, more preferably less than 1 µM, more preferably less than 0.3 µM, still more preferably less than 0.1 µM, yet more preferably less than 0.03 µM, under the conditions of the below-mentioned Experimental Example 1. The URAT1 activity inhibitor does not include biological substrates of URAT1, such as uric acid and the like.

To "decrease the blood uric acid level" means to decrease uric acid (including uric acid salt) in blood (including in serum and plasma), preferably to decrease high blood uric acid level, more preferably serum uric acid level, to less than 8 mg/dL (preferably less than 7 mg/dl, more preferably less than 6 mg/dL, as serum uric acid level).

The "pathology showing involvement of uric acid" means pathology in which uric acid (including uric acid salt) in the body such as blood (including in serum and plasma) or urine is involved. Specifically, pathology caused by high blood (including in serum and plasma) uric acid level or urine uric acid level such as hyperuricemia, gouty tophus, acute gouty arthritis, chronic gouty arthritis, gouty kidney, urolithiasis, renal function disorder, coronary artery disease, ischemic heart disease and the like can be mentioned.

For example, the "high blood uric acid level" means a serum uric acid level of not less than 6 mg/dL, preferably not less than 7 mg/dL, more preferably not less than 8 mg/dL. The "pathology caused by high blood uric acid level" is pathology caused by high blood uric acid level, or to which high blood uric acid level contributes. For example, according to "Guideline for the management of hyperuricemia and gout (1st Edit.)" (Gout and Nucleic Acid Metabolism, vol. 26, suppl. 1 (2002), Japanese Society of Gout and Nucleic Acid Metabolism), 7 mg/dL, which is a dissolution concentration of uric acid in plasma, is the normal upper limit, irrespective of sex·age, and any value exceeding this level is defined to be hyperuricemia, and concludes that the serum uric acid level should be desirably controlled to not more than 6 mg/dL for the treatment of hyperuricemia or gout and for the prevention of the onset of gout arthritis.

As the "pharmaceutically acceptable carrier", various organic or inorganic carrier substances conventionally used as preparation materials are employed, which are added as excipient, lubricant, binder, disintegrant, solvent, dissolution aids, suspending agent, isotonicity agent, buffer, soothing agent and the like. Where necessary, preparation additives such as preservative, antioxidant, sweetening agent, coloring agent and the like can also be used. As preferable examples of the above-mentioned excipient, lactose, sucrose, D-mannitol, starch, crystalline cellulose, light silicic anhydride and the like can be mentioned. As preferable examples of the above-mentioned lubricant, magnesium stearate, calcium stearate, talc, colloidal silica and the like can be mentioned. As preferable examples of the above-mentioned binder, polymer compound such as crystalline cellulose, sucrose, D-mannitol, dextrin, hydroxypropylcellulose, hydroxypropylmethylcellulose, polyvinylpyrrolidone and the like can be mentioned. As preferable examples of the above-mentioned disintegrant, starch, carboxymethylcellulose, carboxymethylcellulose calcium, croscarmellose sodium, sodium carboxymethyl starch and the like can be mentioned. As preferable examples of the above-mentioned solvent, water for injection, alcohol, propylene glycol, macrogol, sesame oil, corn oil, propylene glycol esters of fatty acids and the like can be mentioned. As preferable examples of the above-mentioned dissolution aids, polyethylene glycol, propylene glycol, D-mannitol, benzyl benzoate, ethanol, trisaminomethane, cholesterol, triethanolamine, sodium carbonate, sodium citrate and the like can be mentioned. As preferable examples of the above-mentioned suspending agent, surfactants such as stearyl triethanolamine, sodium lauryl sulfate, lauryl aminopropionic acid, lecithin, benzalkonium chloride, benzethonium chloride, glyceryl monostearate and the like; polyvinyl alcohol, polyvinylpyrrolidone, carboxymethylcellulose sodium, methylcellulose, hydroxymethylcellulose and the like can be mentioned. As preferable examples of the above-mentioned isotonicity agent, sodium chloride, glycerol, D-mannitol and the like can be mentioned. As preferable examples of the above-mentioned buffer, buffers such as phosphate, acetate, carbonate, citrate and the like, and the like can be mentioned. As preferable examples of the above-mentioned soothing agent, benzyl alcohol and the like can be mentioned. As preferable examples of the above-mentioned preservative, paraoxybenzoates, chlorobutanol, benzyl alcohol, phenethyl alcohol, dehydroacetic acid, sorbic acid and the like can be mentioned. As preferable examples of the above-mentioned antioxidant, sulfite, ascorbic acid and the like can be mentioned. As preferable examples of the above-mentioned sweetening agent, aspartam, saccharin sodium, stevia and the like can be mentioned. As preferable examples of the above-mentioned coloring agent, foodcolors such as Food Color Yellow No. 5, Food Color Red No. 2 and Food Color Blue No. 2 and the like, food lake colors, iron oxide and the like can be mentioned.

The compound of the present invention can be contained as an active ingredient of a pharmaceutical composition, a URAT1 activity inhibitor, an agent for lowering a blood uric acid level, or an agent for the prophylaxis or treatment of pathology showing involvement of uric acid, along with a pharmaceutically acceptable carrier.

When the compound of the present invention is used as the pharmaceutical composition, the URAT1 activity inhibitor, the agent for lowering a blood uric acid level, or the drug for the prophylaxis or treatment of pathology showing involvement of uric acid of the present invention, it can be systemically or topical, and orally or parenterally administered. While the dose varies depending on the age, body weight, condition, treatment effect and the like, for example, it can be generally administered within the range of 0.1 mg to 1 g per administration to an adult once to several times a day. The compound of the present invention can also be used as a drug for the treatment or prophylaxis of the aforementioned diseases in, not to mention human, animals other than human, particularly mammals. The same applies when the compound of the present invention is used as a drug for the prophylaxis or treatment of hyperlipidemia, diabetes, obesity or cardiovascular disease, such as hypertension, coronary artery disease, vascular endothelial disorder, ischemic heart disease and the like.

To produce a preparation of the compound of the present invention such as a solid composition or liquid composition for oral administration, or an injection for parenteral administration and the like, the compound can be admixed additives such as a suitable diluent, a dispersing agent, an adsorbent, a solubilizer and the like. In addition, the pharmaceutical composition of the present invention may have known form such as tablet, pill, powder, granule, suppository, injection, eye drop, liquid, capsule, troche, aerosol, elixir, suspension, emulsion, syrup and the like.

When the pharmaceutical composition, the URAT1 activity inhibitor, the agent for lowering a blood uric acid level, or the agent for the prophylaxis or treatment of pathology showing involvement of uric acid of the present invention is, for example, a solid preparation such as tablet, pill, powder, granule etc., the additive includes, for example, lactose, mannitol, glucose, hydroxypropylcellulose, microcrystalline cellulose, starch, polyvinylpyrrolidone , magnesium aluminosilicate, silicic anhydride powder and the like. When a tablet or pill is to be prepared, sucrose, gelatin, hydroxypropylcellulose, or a film coating of gastrosoluble or enteric substance such as hydroxymethylcellulose phthalate etc. may be applied as necessary, or a multi-layer tablet having two or more layers may be produced.

As the pharmaceutical composition, the URAT1 activity inhibitor, the agent for lowering a blood uric acid level, or the agent for the prophylaxis or treatment of pathology showing involvement of uric acid of the present invention, capsules obtained by dissolving the compound of the present invention in a solvent, adding an additive to give a liquid, semi-solid or solid content and packing the content therein can also be mentioned. As the aforementioned solvent, for example, purified water, ethanol or vegetable oil and the like can be mentioned. Of these, ethanol or a mixture of purified water and ethanol is preferably used. As the additive, additives generally used for the production of capsules can be used without any particularly limitation. As the aforementioned additive, for example, propylene glycol esters of fatty acids; low molecular weight polyethylene glycol such as polyethylene glycol 200-600, low molecular weight polyethylene glycol such as polyethylene glycol 200-600 etc., esters of fatty acids, middle chain fatty acid triglyceride; alcohol/polyhydric alcohols such as stearyl alcohol, cetanol, polyethylene glycol etc., and esters thereof; fats and oils such as sesame oil, soy bean oil, peanut oil, corn oil, hydrogenated oil, paraffin oil, white beeswax etc.; organic carboxylic acid or fatty acid such as triethyl citrate, triacetine, stearic acid, palmitic acid, myristic acid etc. and derivatives thereof and the like can be mentioned, which are suitable for the production of liquid or semi-solid contents. For capsules of the present invention, propylene glycol esters of fatty acids are preferably used as additive. As propylene glycol esters of fatty acids, for example, propylene glycol monocaprylate (Capmul PG-8 (trade name), Sefol218 (trade name), Capryol90 (trade name)), propylene glycol monolaurate (Lauroglycol FCC (trade name)), propylene glycol monooleate (Myverol P-O6 (trade name)), propylene glycol myristate, propylene glycol monostearate, propylene glycol ricinoleate (Propymuls (trade name)), propylene glycol dicaprylate/dicaprate (Captex (trademark) 200 (trade name)), propylene glycol dilaurate, propylene glycol distearate, and propylene glycol dioctanoate (Captex (trademark) 800 (trade name)) can be mentioned. While the material constituting the capsule of the present invention is not particularly limited, natural polysacchalides such as agar, alginate, starch, xanthan, dextran etc.; protein such as gelatin, casein etc.; chemically processed products such as hydroxystarch, pullulan, hydroxypropylcellulose, hydroxypropylmethylcellulose, polyvinyl alcohol and derivatives thereof, polyacryl derivative, polyvinylpyrrolidone and derivatives thereof, polyethylene glycol and the like can be mentioned.

When the pharmaceutical composition, the URAT1 activity inhibitor, the agent for lowering a blood uric acid level, or the agent for the prophylaxis or treatment of pathology showing involvement of uric acid of the present invention is, for example, a liquid preparation for oral administration of a pharmaceutically acceptable suspending agent, a solubilizer, a suspension, a syrup, an elixir etc., as the diluent to be used, for example, purified water, ethanol, vegetable oil, emulsifier and the like can be mentioned. This liquid preparation may contain, besides the diluent, an auxiliary agent such as an infiltration, a suspension, a sweetening agent, a flavor, an aromatic, a preservative and the like.

When the pharmaceutical composition, the URAT1 activity inhibitor, the agent for lowering a blood uric acid level, or the agent for the prophylaxis or treatment of pathology showing involvement of uric acid of the present invention is a parenteral preparation such as injection etc., sterile aqueous or non-aqueous solution, solubilizer, suspension, emulsifier and the like are used as additives. As the aqueous solution, solubilizer and suspension, for example, distilled water for injection; saline, cyclodextrin and derivatives thereof; organic amines such as triethanolamine, diethanolamine, monoethanolamine, triethylamine and the like, inorganic alkali solution and the like can be mentioned. When a water-soluble solution is used, for example, propylene glycol, polyethylene glycol or vegetable oil such as olive oil, alcohols such as ethanol, and the like may be further added. As the solubilizer, for example, surfactants such as polyoxyethylene hydrogenated castor oil, sucrose esters of fatty acids and the like (for forming mixed micelle), lecithin or hydrogenated lecithin (for forming liposome) and the like can also be used. In addition, the parenteral preparation of the present invention can be produced as an emulsion preparation containing water-insoluble solubilizer such as vegetable oil etc., lecithin, polyoxyethylene hydrogenated castor oil, polyoxyethylenepolyoxypropylene glycol and the like.

The compound, pharmaceutical composition, URAT1 activity inhibitor, agent for lowering a blood uric acid level, or agent for the prophylaxis or treatment of pathology showing involvement of uric acid of the present invention can be used in combination with other pharmaceutical composition or pharmaceutical agent (hereinafter to be also referred to as a concomitant drug).

The "use in combination" means use of multiple pharmaceutical agents in combination as active ingredients, and use as a combination drug, use as a kit, use in a combination characterized by independent administration of each by the same or different administration routes and the like can be mentioned.

The administration time of the compound, the pharmaceutical composition, the URAT1 activity inhibitor, the agent for lowering a blood uric acid level, or the agent for the prophylaxis or treatment of pathology showing involvement of uric acid of the present invention and a concomitant drug is not limited, and these may be administered simultaneously to the subject of administration or administered in a staggered manner. The dose of the concomitant drug can be determined according to the dose employed clinically, and appropriately determined depending on the subject of administration, the age and body weight of the subject of administration, condition, administration time, dosage form, administration method, combination and the like. The administration mode of the concomitant drug is not particularly limited, and it is only necessary to combine the compound of the present invention, a pharmaceutical composition, a URAT1 activity inhibitor, an agent for lowering a blood uric acid level, or an agent for the prophylaxis or treatment of pathology showing involvement of uric acid and a concomitant drug at the time of administration.

As the concomitant drug,
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease causing decreased uric acid excretion secondary hyperuricemia,
(10) an agent for the prophylaxis and/or treatment of kidney failure, cardiovascular disorder, cerebrovascular disorder caused by hyperuricemia, and
(11) a nucleic acid metabolic antagonist can be mentioned. One to three of these agents and the compound of the present invention can be used in combination.

As the "agent for the prophylaxis and/or treatment of hyperuricemia", for example, uric acid production suppressants such as xanthine oxidase inhibitor etc., uricosuric agents and the like can be mentioned, and allopurinol, probenecid, bucolome, febuxostat, benzbromarone, oxipurinol and the like can be specifically mentioned.

As the "agent for the prophylaxis and/or treatment of gout arthritis", for example, NSAIDs such as indomethacin, naproxen, fenbufen, pranoprofen, oxaprozin and the like, colchicine, corticosteroid and the like can be mentioned.

As the "agent for the prophylaxis and/or treatment of gouty kidney", for example, uric acid production suppressants such as xanthine oxidase inhibitor etc., uricosuric agents, alkalinizing urine agents such as citric acid preparation, sodium bicarbonate etc., and the like can be mentioned, and allopurinol, probenecid, bucolome, febuxostat, benzbromarone and oxipurinol can be specifically mentioned.

As the "agent for the prophylaxis and/or treatment of urolithiasis", for example, alkalinizing urine agents such as citric acid preparation, sodium bicarbonate etc., and the like can be mentioned.

As the "agent for the prophylaxis and/or treatment of hypertension or hypertensive complications", for example, loop diuretics, angiotensin-convertase inhibitors, angiotensin II receptor antagonists, Ca antagonists, β blockers, α,β blockers, α blockers and the like can be mentioned. More specifically, for example, sustained-release furosemide preparation, captopril, sustained-release captopril preparation, enalapril maleate, alacepril, delapril hydrochloride, cilazapril, lisinopril, benazepril hydrochloride, imidapril hydrochloride, temocapril hydrochloride, quinapril hydrochloride, trandolapril, perindopril erbumine, losartan potassium, candesartan cilexetil, nicardipine hydrochloride, sustained-release nicardipine hydrochloride preparation, nilvadipine, nifedipine, sustained-release nifedipine preparation, benidipine hydrochloride, diltiazem hydrochloride, sustained-release diltiazem hydrochloride preparation, nisoldipine, nitrendipine, manidipine hydrochloride, barnidipine hydrochloride, efonidipine hydrochloride, amlodipine besylate, felodipine, cilnidipine, aranidipine, propranolol hydrochloride, sustained-release propranolol hydrochloride preparation, pindolol, sustained-release pindolol preparation, indenolol hydrochloride, carteolol hydrochloride, sustained-release carteolol hydrochloride preparation, bunitrolol hydrochloride, sustained-release bunitrolol hydrochloride preparation, atenolol, acebutolol hydrochloride, metoprolol tartrate, sustained-release metoprolol tartrate preparation, nipradilol, penbutolol sulfate, tilisolol hydrochloride, carvedilol, bisoprolol fumarate, betaxolol hydrochloride, celiprolol hydrochloride, bopindolol malonate, bevantolol hydrochloride, labetalol hydrochloride, arotinolol hydrochloride, amosulalol hydrochloride, prazosin hydrochloride, terazosin hydrochloride, doxazosin mesilate, bunazosin hydrochloride, sustained-release bunazosin hydrochloride preparation, urapidil, phentolamine mesylate and the like can be mentioned.

As the "agent for the prophylaxis and/or treatment of hyperlipidemia or hyperlipidemic complications", for example, statin pharmaceutical agents, anion exchange resins, probucol, nicotinic acid preparations, fibrate pharmaceutical agents, eicosapentaenoic acid preparations and the like can be mentioned. More specifically, for example, lovastatin, simvastatin, pravastatin, fluvastatin, atorvastatin, cerivastatin, colestimide, colestyramine, niceritrol, nicomol, fenofibrate, bezafibrate, clinofibrate, clofibrate, ethyl icosapentate and the like can be mentioned.

As the "agent for the prophylaxis and/or treatment of diabetes or diabetic complications", for example, insulin preparations, sulfonylureas, insulin secretagogues, sulfonamides, biguanides, α glucosidase inhibitors, insulin sensitizers, angiotensin-convertase inhibitors, aldose reductase inhibitors, antiarrhythmic drugs and the like can be mentioned. More specifically, for example, insulin, chlorpropamide, glibenclamide, glipizide, tolbutamide, glyclopyramide, acetohexamide, glimepiride, tolazamide, gliclazide, nateglinide, glybuzole, metformin hydrochloride, buformin hydrochloride, voglibose, acarbose, pioglitazone hydrochloride, mexiletine and the like can be mentioned.

As the "agent for the prophylaxis and/or treatment of obesity or obesity complications", for example, mazindol, acarbose, voglibose, orlistat and the like can be mentioned.

As the "agent for the prophylaxis and/or treatment of primary disease causing decreased uric acid excretion secondary hyperuricemia", for example, agents for the prophylaxis or treatment of chronic renal disease, polycyctic kidney, toxemia of pregnancy, lead nephropathy, hyperlactacidemia, Down's syndrome, sarcoidosis, glycogenosis I type (via hyperlactacidemia), dehydrating etc., and the like can be mentioned.

As the "agent for the prophylaxis and/or treatment of kidney failure, cardiovascular disorder, cerebrovascular disorder caused by hyperuricemia", for example, loop diuretics (e.g., furosemide), citric acid preparations, sodium bicarbonate, cation exchange resins, aluminum hydroxide, alfacalcidol, β-blockers (e.g., propranolol hydrochloride), ACE inhibitors (e.g., captopril), cardiac stimulants (e.g., digoxin), angina pectoris therapeutic agents (e.g., isosorbide nitrate), Ca antagonists (e.g., diltiazem hydrochloride), uric acid production suppressants (e.g., allopurinol), amino acid preparations, hyperammonemia improvers, therapeutic agents for antiarrhythmic (e.g., mexiletine), therapeutic agents for anemia (e.g., mepitiostane, erythropoietin), other "agents for the prophylaxis and/or treatment of hypertension or hypertensive complications", "agents for the prophylaxis and/or treatment of hyperlipidemia or hyperlipidemic complications", "agents for the prophylaxis and/or treatment of diabetes or diabetic complications", "agents for the prophylaxis and/or treatment of obesity or obesity complications" and the like can be mentioned.

As the "nucleic acid metabolic antagonist", for example, azathiopurine, mizoribine, mycophenolic acid and the like can be mentioned.

Furthermore, the compound of the present invention, a pharmaceutical composition, a URAT1 activity inhibitor, an agent for lowering a blood uric acid level, and an agent for the prophylaxis or treatment of pathology showing involvement of uric acid can be used in combination with a pharmaceutical agent that increases the blood uric acid level, thereby to suppress increase in the blood uric acid level.

As the "pharmaceutical agent that increases the blood uric acid level", nucleic acid metabolic antagonists, hypotensive diuretics, anti-tuberculosis, anti-inflammatory analgesic drugs, hyperlipidemic drugs, therapeutic drugs for asthma, immunosuppressants, salicylic acid, pyrazinamide, ethambutol, nicotinic acid, ethanol, cyclosporine and the like can be mentioned.

The compound of the crystal of the present invention (hereinafter to be sometimes abbreviated as the crystal of the present invention) only needs to be contained in a pharmaceutical composition, and it preferably contains substantially most of the crystal of the present invention.

By "contains substantially most of the crystal of the present invention" is meant that the crystal of the compound of the present invention is contained in a proportion of not less than 80%, preferably not less than 90%, more preferably not less than 95%.

The production methods of the compound of the present invention are specifically explained below. It is needless to say that the present invention is not limited to the production methods below. For production of the compound of the present invention, the order of the reaction can be appropriately changed. The reaction only needs to be carried out from a step or a position that seems to be reasonable.

In addition, a step for appropriately changing substituents (change or further modification of substituents) may be inserted between respective steps. When a reactive functional group is involved, appropriate protection or deprotection may be conducted. To promote progress of the reaction, moreover, reagents other than those exemplified can be appropriately used. The starting material compounds, for which a production method is not described, are commercially available or can be easily produced by combining known synthetic reactions.

The compound obtained in each step can be isolated and purified by conventional methods such as crystallization, recrystallization, column chromatography, preparative HPLC and the like. In some cases, it is possible to proceed to the next step without isolation and purification.

In the following production methods, the "room temperature" means 1-40°C.

### Production method 1

A compound [1] wherein X² is an oxygen atom and Y is C=O can be produced by the following Steps: 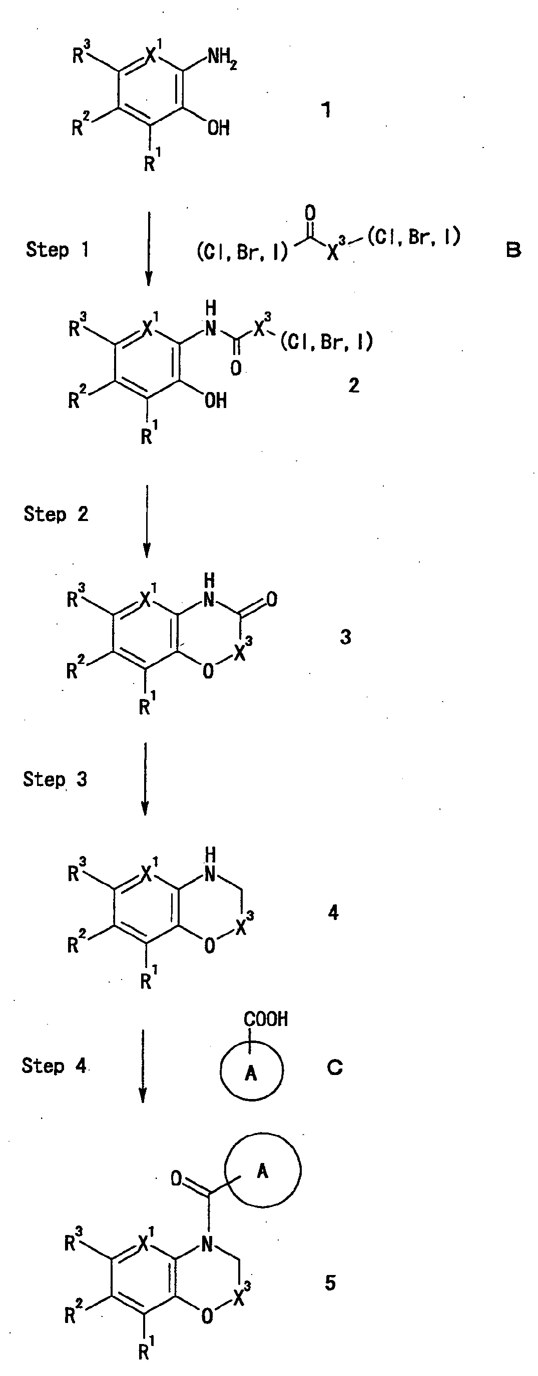 wherein each symbol is as defined above.

### Step 1

Compound 2 can be obtained by amidating compound 1 with acid halide B in a solvent in the presence of a base.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide, dimethyl sulfoxide, water and the like; and the like can be mentioned. These can be used alone or in a mixture of two or more kinds thereof. The preferable solvents for this reaction include methylene chloride, chloroform, toluene, ethyl acetate, water and tetrahydrofuran.

As the base to be used for the reaction, for example, organic bases such as triethylamine, pyridine, 4-dimethylaminopyridine, N-methylmorpholine and the like; alkali metal hydroxides such as lithium hydroxide, sodium hydroxide, potassium hydroxide and the like; alkali metal hydrides such as sodium hydride, potassium hydride and the like; alkali metal carbonates such as sodium carbonate, potassium carbonate and the like; alkali metal hydrogencarbonates such as sodium hydrogencarbonate, potassium hydrogencarbonate and the like; and the like can be mentioned, with preference given to triethylamine, pyridine, sodium hydroxide and sodium hydrogencarbonate.

The reaction temperature is about 0°C to 80°C, preferably about 0°C to room temperature.

The reaction time is about 10 min to 48 hr, preferably about 30 min to 24 hr.

### Step 2

Compound 3 can be obtained by subjecting compound 2 to cyclization in a solvent in the presence of a base, as necessary in the presence of a catalytic amount of sodium iodide, potassium iodide and the like.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include acetone and N,N-dimethylformamide.

As the base to be used for the reaction, for example, organic bases such as triethylamine, pyridine, 4-dimethylaminopyridine, N-methylmorpholine and the like; alkali metal hydroxides such as lithium hydroxide, sodium hydroxide, potassium hydroxide and the like; alkali metal carbonates such as sodium carbonate, potassium carbonate and the like; alkali metal hydrogencarbonates such as sodium hydrogencarbonate, potassium hydrogencarbonate and the like; alkali metal carboxylates such as sodium acetate, potassium acetate and the like; alkali metal hydrides such as sodium hydride, potassium hydride and the like; alkali metal alkoxides such as sodium ethoxide, sodium methoxide, potassium tert-butoxide and the like; alkyllithiums such as n-butyllithium, s-butyllithium and the like; alkali metal amides such as lithium diisopropylamide, sodium amide, lithium bistrimethylsilylamide and the like; and the like can be mentioned, with preference given to potassium carbonate.

The reaction temperature is about 0°C to 150°C, preferably room temperature to 100°C.

The reaction time is about 10 min to 48 hr, preferably about 1 hr to 24 hr.

### Step 3

Compound 4 can be obtained by reducing compound 3 with a reducing agent in a solvent.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; alcohols such as methanol, ethanol, isopropyl alcohol, tert-butanol and the like; and the like can be mentioned, with preference given to toluene, diethyl ether and tetrahydrofuran.

As the reducing agent to be used for the reaction, for example, lithium aluminum hydride, sodium borohydride, diborane, diisobutylaluminum hydride, borane-tetrahydrofuran complex, sodium bis(2-methoxyethoxy)aluminum hydride and the like can be mentioned, with preference given to sodium bis(2-methoxyethoxy)aluminum hydride, lithium aluminum hydride and borane-tetrahydrofuran complex.

The reaction temperature is about 0°C to 150°C, preferably room temperature to 130°C.

The reaction time is about 10 min to 48 hr, preferably about 30 min to 24 hr.

### Step 4

Compound 5 can be obtained by converting carboxylic acid compound C to the acid chloride with oxalyl chloride, thionyl chloride and the like in a solvent, and amidating compound 4 with the acid chloride in a solvent, as necessary in the presence of a base.

As the solvent to be used for the reaction to obtain the acid chloride, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; and the like can be mentioned. These can be used alone or in a mixture of two or more kinds thereof. The preferable solvents for this reaction include 1,2-dimethoxyethane, ethyl acetate, and methylene chloride, chloroform, toluene, 1,2-dimethoxyethane and ethyl acetate each containing a catalytic amount of N,N-dimethylformamide.

The reaction temperature is about -20°C to 120°C, preferably about 0°C to 80°C.

The reaction time is about 10 min to 48 hr, preferably about 30 min to 24 hr.

As the solvent to be used for the amidation reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include methylene chloride, chloroform, toluene, ethyl acetate, N,N-dimethylformamide and tetrahydrofuran.

As the base to be used for the reaction, for example, organic bases such as triethylamine, pyridine, 4-dimethylaminopyridine, N-methylmorpholine and the like; alkali metal hydroxides such as lithium hydroxide, sodium hydroxide, potassium hydroxide and the like; alkali metal hydrides such as sodium hydride, potassium hydride and the like; alkali metal carbonates such as sodium carbonate, potassium carbonate and the like; alkali metal hydrogencarbonates such as sodium hydrogencarbonate, potassium hydrogencarbonate and the like; and the like can be mentioned. This reaction is preferably carried out without a base, or in the presence of triethylamine, pyridine, sodium hydroxide, sodium hydride or sodium hydrogencarbonate.

The reaction temperature is about 0°C to 120°C, preferably about 0°C to 95°C.

The reaction time is about 10 min to 48 hr, preferably about 30 min to 24 hr.

Compound 5 can also be obtained by subjecting compound 4 and carboxylic acid compound C to condensation with, for example, water-soluble carbodiimide (WSC HCl: 1-ethyl-3-(3'-dimethylaminopropyl)carbodiimide hydrochloride), dicyclohexylcarbodiimide (DCC), diphenylphosphoryl azide (DPPA), carbonyldiimidazole (CDI), 1-hydroxy-1H-benzotriazole (HOBT), 4-dimethylaminopyridine (DMAP) and the like; or by converting carboxylic acid compound C to a mixed acid anhydride with a chloroformate such as ethyl chloroformate, isopropyl chloroformate, isobutyl chloroformate and the like in the presence of a base such as triethylamine and the like, and reacting compound 4 with the mixed acid anhydride in the presence of a base.

As regards a compound wherein X⁴ is other than -CH₂-, the corresponding compound can be obtained by a method similar to Step 4 and using known compounds.

Step 1 and Step 2 can be conducted in a single step according to Synthesis, 10, 851-852 (1984) to give compound 3.

### Production method 2

A compound [1] wherein X² is an oxygen atom and Y is C=O can be produced by the following Steps: 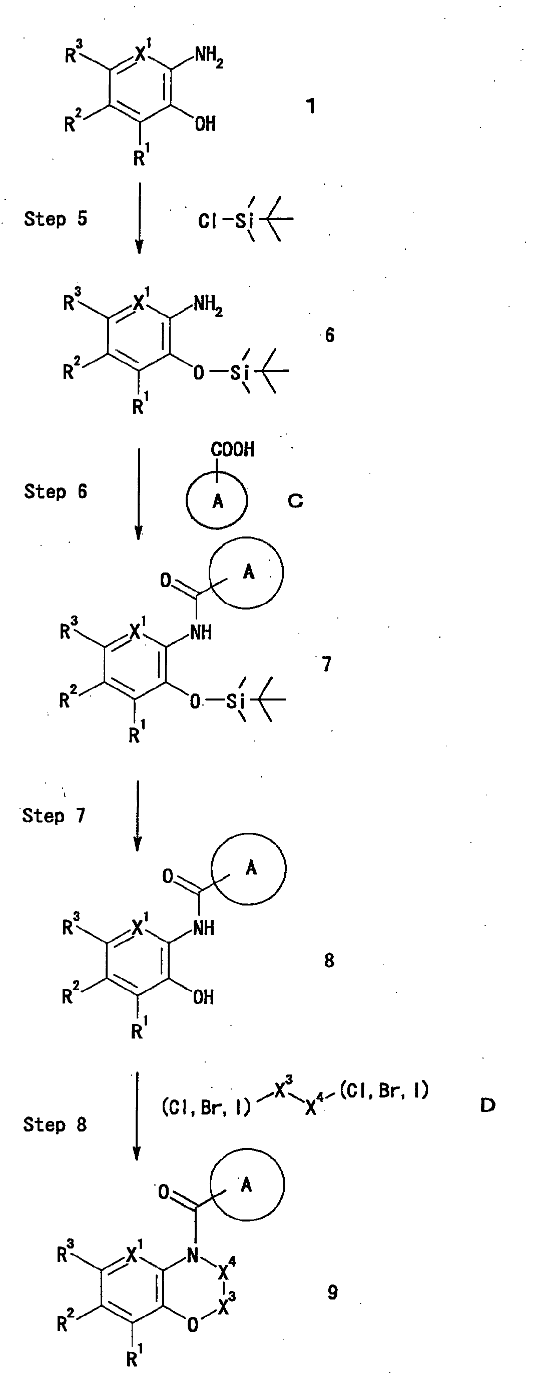 wherein each symbol is as defined above.

### Step 5

Compound 6 can be obtained by subjecting compound 1 to silylation with tert-butylchlorodimethylsilane in a solvent in the presence of a base.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include N,N-dimethylformamide.

As the base to be used for the reaction, for example, organic bases such as triethylamine, pyridine, imidazole, 4-dimethylaminopyridine, N-methylmorpholine and the like; alkali metal hydroxides such as lithium hydroxide, sodium hydroxide, potassium hydroxide and the like; alkali metal hydrides such as sodium hydride, potassium hydride and the like; alkali metal carbonates such as sodium carbonate, potassium carbonate and the like; alkali metal hydrogencarbonates such as sodium hydrogencarbonate, potassium hydrogencarbonate and the like; and the like can be mentioned. The preferable base for this reaction is imidazole.

The reaction temperature is about 0°C to 150°C, preferably about 0°C to room temperature.

The reaction time is about 10 min to 24 hr, preferably about 30 min to 12 hr.

### Step 6

Compound 7 can be obtained by converting carboxylic acid compound C to the acid chloride with oxalyl chloride, thionyl chloride and the like in a solvent, and amidating compound 6 with the acid chloride in a solvent, as necessary in the presence of a base.

As the solvent to be used for the reaction to obtain the acid chloride, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; and the like can be mentioned. These can be used alone or in a mixture of two or more kinds thereof. The preferable solvents for this reaction include 1,2-dimethoxyethane, ethyl acetate, and methylene chloride, chloroform, toluene, 1,2-dimethoxyethane and ethyl acetate each containing a catalytic amount of N,N-dimethylformamide.

The reaction temperature is about -20°C to 120°C, preferably about 0°C to 80°C.

The reaction time is about 10 min to 48 hr, preferably about 30 min to 24 hr.

As the solvent to be used for the amidation reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include methylene chloride, chloroform, toluene, ethyl acetate and tetrahydrofuran.

As the base to be used for the reaction, for example, organic bases such as triethylamine, pyridine, 4-dimethylaminopyridine, N-methylmorpholine and the like; alkali metal hydroxides such as lithium hydroxide, sodium hydroxide, potassium hydroxide and the like; alkali metal hydrides such as sodium hydride, potassium hydride and the like; alkali metal carbonates such as sodium carbonate, potassium carbonate and the like; alkali metal hydrogencarbonates such as sodium hydrogencarbonate, potassium hydrogencarbonate and the like; and the like can be mentioned. This reaction is preferably carried out without a base, or in the presence of triethylamine, pyridine, sodium hydroxide or sodium hydrogencarbonate.

The reaction temperature is about 0°C to 120°C, preferably about 0°C to 95°C.

The reaction time is about 10 min to 48 hr, preferably about 30 min to 24 hr.

Compound 7 can also be obtained by subjecting compound 6 and carboxylic acid compound C to condensation with, for example, water-soluble carbodiimide (WSC HCl: 1-ethyl-3-(3'-dimethylaminopropyl)carbodiimide hydrochloride), dicyclohexylcarbodiimide (DCC), diphenylphosphoryl azide (DPPA), carbonyldiimidazole (CDI), 1-hydroxy-1H-benzotriazole (HOBT), 4-dimethylaminopyridine (DMAP) and the like; or by converting carboxylic acid compound C to a mixed acid anhydride with a chloroformate such as ethyl chloroformate, isopropyl chloroformate, isobutyl chloroformate and the like in the presence of a base such as triethylamine and the like, and reacting compound 6 with the mixed acid anhydride in the presence of a base.

### Step 7

Compound 8 can be obtained by subjecting compound 7 to desilylation in a solvent.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; alcohols such as methanol, ethanol, isopropyl alcohol, tert-butanol and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include N,N-dimethylformamide.

As the reagent to be used for the reaction, for example, potassium carbonate, tetrabutylammonium fluoride and the like can be mentioned, with preference given to potassium carbonate.

The reaction temperature is about 0°C to 150°C, preferably room temperature to 80°C.

The reaction time is about 10 min to 48 hr, preferably about 30 min to 24 hr.

### Step 8

Compound 9 can be obtained by subjecting compound 8 to cyclization with halide D in a solvent in the presence of a base.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include N,N-dimethylformamide.

As the base to be used for the reaction, for example, organic bases such as triethylamine, pyridine, 4-dimethylaminopyridine, N-methylmorpholine and the like; alkali metal hydroxides such as lithium hydroxide, sodium hydroxide, potassium hydroxide and the like; alkali metal hydrides such as sodium hydride, potassium hydride and the like; alkali metal carbonates such as sodium carbonate, potassium carbonate and the like; alkali metal hydrogencarbonates such as sodium hydrogencarbonate, potassium hydrogencarbonate and the like; and the like can be mentioned. The preferable base for this reaction is potassium carbonate.

The reaction temperature is about 0°C to 150°C, preferably room temperature to 100°C.

The reaction time is about 10 min to 48 hr, preferably about 3 hr to 24 hr.

### Production method 3

A compound [1] wherein X² is a carbon atom or a sulfur atom and Y is C=O can be produced by the following Steps: 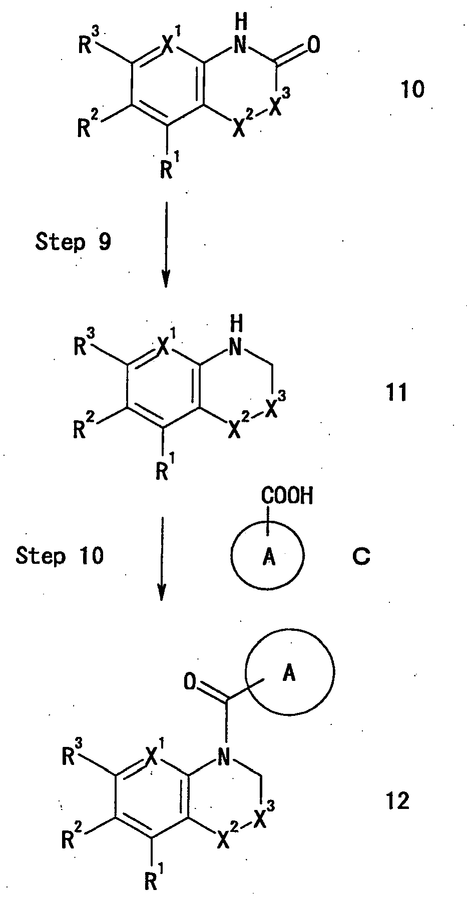 wherein each symbol is as defined above.

### Step 9

Compound 11 can be obtained by reducing known compound 10 with a reducing agent in a solvent.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; alcohols such as methanol, ethanol, isopropyl alcohol, tert-butanol and the like; and the like can be mentioned, with preference given to toluene, diethyl ether and tetrahydrofuran.

As the reducing agent to be used for the reaction, for example, lithium aluminum hydride, sodium borohydride, diborane, diisobutylaluminum hydride, borane-tetrahydrofuran complex, sodium bis(2-methoxyethoxy)aluminum hydride and the like can be mentioned, with preference given to sodium bis(2-methoxyethoxy)aluminum hydride, lithium aluminum hydride and borane-tetrahydrofuran complex.

The reaction temperature is about 0°C to 150°C, preferably room temperature to 130°C.

The reaction time is about 10 min to 48 hr, preferably about 30 min to 24 hr.

### Step 10

Compound 12 can be obtained by converting carboxylic acid compound C to the acid chloride with oxalyl chloride, thionyl chloride and the like in a solvent, and amidating compound 11 with the acid chloride in a solvent, as necessary in the presence of a base.

As the solvent to be used for the reaction to obtain the acid chloride, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; and the like can be mentioned. These can be used alone or in a mixture of two or more kinds thereof. The preferable solvents for this reaction include 1,2-dimethoxyethane, ethyl acetate, and methylene chloride, chloroform, toluene, 1,2-dimethoxyethane and ethyl acetate each containing a catalytic amount of N,N-dimethylformamide.

The reaction temperature is about -20°C to 120°C, preferably about 0°C to 80°C.

The reaction time is about 10 min to 48 hr, preferably about 30 min to 24 hr.

As the solvent to be used for the amidation reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include methylene chloride, chloroform, toluene, ethyl acetate, N,N-dimethylformamide and tetrahydrofuran.

As the base to be used for the reaction, for example, organic bases such as triethylamine, pyridine, dimethylaminopyridine, N-methylmorpholine and the like; alkali metal hydroxides such as lithium hydroxide, sodium hydroxide, potassium hydroxide and the like; alkali metal hydrides such as sodium hydride, potassium hydride and the like; alkali metal carbonates such as sodium carbonate, potassium carbonate and the like; alkali metal hydrogencarbonates such as sodium hydrogencarbonate, potassium hydrogencarbonate and the like; and the like can be mentioned. This reaction is preferably carried out without a base, or in the presence of triethylamine, pyridine, sodium hydroxide, sodium hydride or sodium hydrogencarbonate.

The reaction temperature is about 0°C to 120°C, preferably about 0°C to 95°C.

The reaction time is about 10 min to 48 hr, preferably about 30 min to 24 hr.

The compound 12 can also be obtained by subjecting compound 11 and carboxylic acid compound C to condensation with, for example, water-soluble carbodiimide (WSC HCl: 1-ethyl-3-(3'-dimethylaminopropyl)carbodiimide hydrochloride), dicyclohexylcarbodiimide (DCC), diphenylphosphoryl azide (DPPA), carbonyldiimidazole (CDI), 1-hydroxy-1H-benzotriazole (HOBT), 4-dimethylaminopyridine (DMAP) and the like; or by converting carboxylic acid compound C to a mixed acid anhydride with a chloroformate such as ethyl chloroformate, isopropyl chloroformate, isobutyl chloroformate and the like in the presence of a base such as triethylamine and the like, and reacting compound 11 with the mixed acid anhydride in the presence of a base.

As regards a compound wherein X⁴ is other than -CH₂-, the corresponding compound can be obtained by a method similar to Step 10 and using known compounds.

### Production method 4

A compound [1] wherein X² is S=O or S(=O)₂ and Y is C=O can be produced by the following Step: 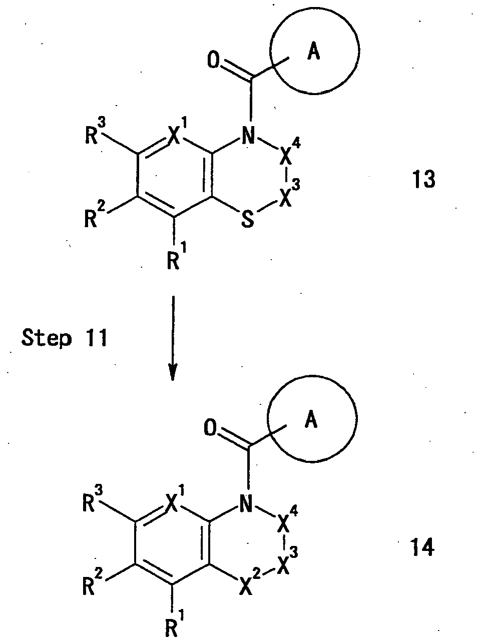 wherein each symbol is as defined above.

### Step 11

Compound 14 can be obtained by oxidizing compound 13 with an oxidant in a solvent.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; alcohols such as methanol, ethanol, isopropyl alcohol, tert-butanol and the like; esters such as ethyl acetate, methyl acetate, butyl acetate and the like; polar solvents such as acetone, N,N-dimethylformamide, dimethyl sulfoxide, water, acetic acid and the like; and the like can be mentioned. These can be used alone or in a mixture of two or more kinds thereof. The preferable solvents for this reaction include methylene chloride and chloroform .

As the oxidant to be used for the reaction, for example, 3-chloroperbenzoic acid, hydrogen peroxide, sodium periodate, tert-butylhydroperoxide and the like can be mentioned, with preference given to 3-chloroperbenzoic acid.

The reaction temperature is about 0°C to 80°C, preferably about 0°C to room temperature.

The reaction time is about 10 min to 48 hr, preferably about 2 hr to 24 hr.

### Production method 5

A compound [1] wherein X² is NR⁵, N(COR⁶), N (S (=O)₂R⁶) or N(CONR⁷R⁸) and Y is C=O can be produced by the following Step: 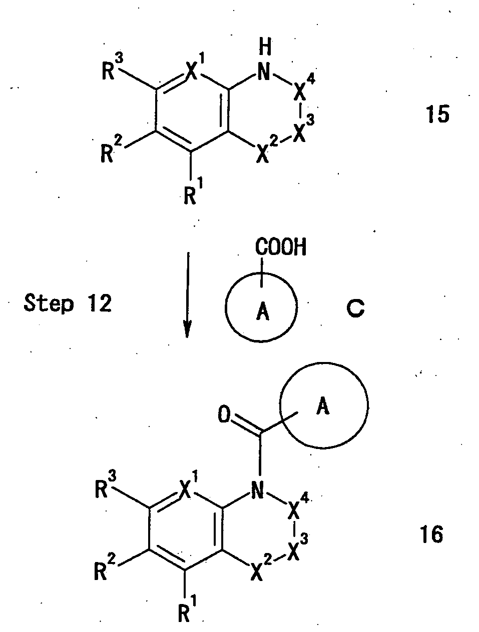 wherein each symbol is as defined above.

### Step 12

Compound 16 can be obtained by converting carboxylic acid compound C to the acid chloride with oxalyl chloride, thionyl chloride and the like in a solvent, and amidating known compound 15 with the acid chloride in a solvent, as necessary in the presence of a base.

As the solvent to be used for the reaction to obtain the acid chloride, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; and the like can be mentioned. These can be used alone or in a mixture of two or more kinds thereof. The preferable solvents for this reaction include 1,2-dimethoxyethane, ethyl acetate, and methylene chloride, chloroform, toluene, 1,2-dimethoxyethane and ethyl acetate each containing a catalytic amount of N,N-dimethylformainide.

The reaction temperature is about -20°C to 120°C, preferably about 0°C to 80°C.

The reaction time is about 10 min to 48 hr, preferably about 30 min to 24 hr.

As the solvent to be used for the amidation reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include methylene chloride, chloroform, toluene, ethyl acetate, N,N-dimethylformamide and tetrahydrofuran.

As the base to be used for the reaction, for example, organic bases such as triethylamine, pyridine, 4-dimethylaminopyridine, N-methylmorpholine and the like; alkali metal hydroxides such as lithium hydroxide, sodium hydroxide, potassium hydroxide and the like; alkali metal hydrides such as sodium hydride, potassium hydride and the like; alkali metal carbonates such as sodium carbonate, potassium carbonate and the like; alkali metal hydrogencarbonates such as sodium hydrogencarbonate, potassium hydrogencarbonate and the like; and the like can be mentioned. This reaction is preferably carried out without a base, or in the presence of triethylamine, pyridine, sodium hydroxide, sodium hydride or sodium hydrogencarbonate.

The reaction temperature is about 0°C to 120°C, preferably about 0°C to 95°C.

The reaction time is about 10 min to 48 hr, preferably about 30 min to 24 hr.

Compound 16 can also be obtained by subjecting known compound 15 and carboxylic acid compound C to condensation with, for example, water-soluble carbodiimide (WSC HC1: 1-ethyl-3-(3'-dimethylaminopropyl)carbodiimide hydrochloride), dicyclohexylcarbodiimide (DCC), diphenylphosphoryl azide (DPPA), carbonyldiimidazole (CDI), 1-hydroxy-1H-benzotriazole (HOBT), 4-dimethylaminopyridine (DMAP) and the like; or by converting carboxylic acid compound C to a mixed acid anhydride with a chloroformate such as ethyl chloroformate, isopropyl chloroformate, isobutyl chloroformate and the like in the presence of a base such as triethylamine and the like, and reacting known compound 15 with the mixed acid anhydride in the presence of a base.

Note that R⁵, COR⁶, S (=O)₂R⁶ and CONR⁷R⁸ can be introduced into the nitrogen atom of X² before and after Step 12, and the reactions can be carried out in a reasonable order.

### Production method 6

A compound [1] wherein Y is C=S can be produced by the following Step: 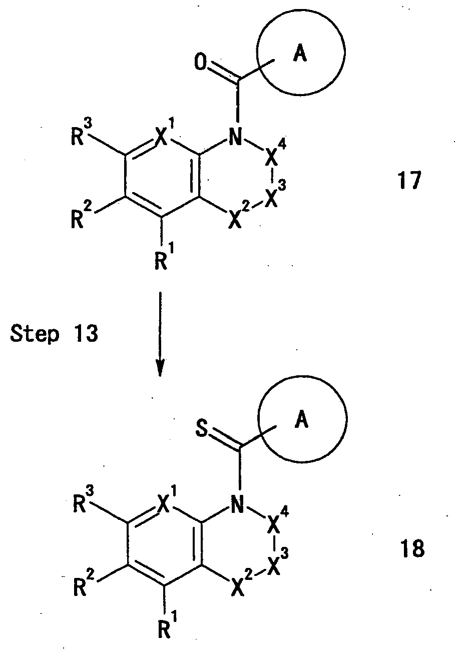 wherein each symbol is as defined above.

### Step 13

Compound 18 can be obtained by reacting compound 17 with Lawesson reagent, diphosphorus pentasulfide and the like in a solvent.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; alcohols such as methanol, ethanol, isopropyl alcohol, tert-butanol and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include tetrahydrofuran and toluene.

The reaction temperature is about 0°C to 150°C, preferably room temperature to 120°C.

The reaction time is about 10 min to 24 hr, preferably about 30 min to 12 hr.

### Production method 7

A compound [1] wherein Y is S(=0)₂ can be produced by the following Step: 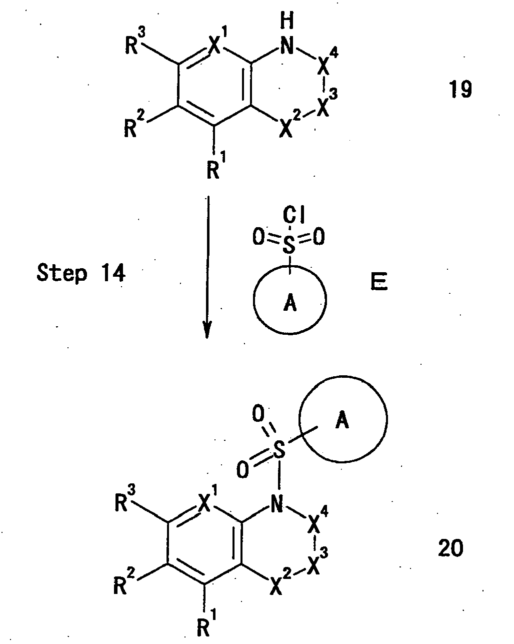 wherein each symbol is as defined above.

### Step 14

Compound 20 can be obtained by amidating compound 19 with known acid chloride E in a solvent, as necessary in the presence of a base.

As the solvent to be used for the amidation reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include methylene chloride, chloroform, toluene, ethyl acetate, N,N-dimethylformamide and tetrahydrofuran.

As the base to be used for the reaction, for example, organic bases such as triethylamine, pyridine, 4-dimethylaminopyridine, N-methylmorpholine and the like; alkali metal hydroxides such as lithium hydroxide, sodium hydroxide, potassium hydroxide and the like; alkali metal hydrides such as sodium hydride, potassium hydride and the like; alkali metal carbonates such as sodium carbonate, potassium carbonate and the like; alkali metal hydrogencarbonates such as sodium hydrogencarbonate, potassium hydrogencarbonate and the like; and the like can be mentioned. Preferably, this reaction is carried out without a base, or in the presence of triethylamine, pyridine, sodium hydroxide, sodium hydride or sodium hydrogencarbonate.

The reaction temperature is about 0°C to 120°C, preferably about 0°C to 95°C.

The reaction time is about 10 min to 48 hr, preferably about 30 min to 24 hr.

### Production method 8

A compound [1] wherein X² is an oxygen atom and Y is C=O can be produced by the following Steps: 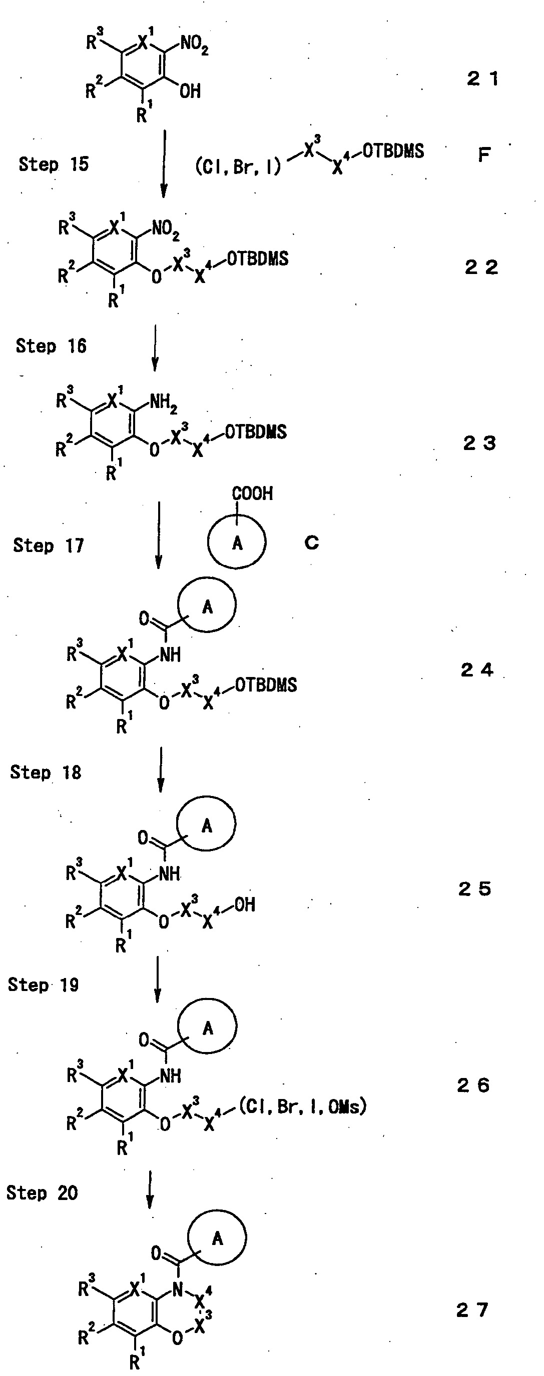 wherein TBDMS is a tert-butyldimethylsilyl group, -OMs is - OS(=O)₂-CH₃, and the other symbols are as defined above.

### Step 15

Compound 22 can be obtained by reacting compound 21 with halide F in a solvent in the presence of a base, as necessary in the presence of sodium iodide, potassium iodide and the like.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include acetone and N,N-dimethylformamide.

As the base to be used for the reaction, for example, organic bases such as triethylamine, pyridine, 4-dimethylaminopyridine, N-methylmorpholine and the like; alkali metal hydroxides such as lithium hydroxide, sodium hydroxide, potassium hydroxide and the like; alkali metal carbonates such as sodium carbonate, potassium carbonate and the like; alkali metal hydrogencarbonates such as sodium hydrogencarbonate, potassium hydrogencarbonate and the like; alkali metal carboxylates such as sodium acetate, potassium acetate and the like; alkali metal hydrides such as sodium hydride, potassium hydride and the like; alkali metal alkoxides such as sodium ethoxide, sodium methoxide, potassium tert-butoxide and the like; alkyllithiums such as n-butyllithium, s-butyllithium and the like; alkali metal amides such as lithium diisopropylamide, sodium amide, lithium bistrimethylsilylamide and the like; and the like can be mentioned, with preference given to sodium hydride and potassium carbonate.

The reaction temperature is about 0°C to 150°C, preferably room temperature to 130°C.

The reaction time is about 10 min to 48 hr, preferably about 1 hr to 24 hr.

### Step 16

Compound 23 can be obtained by reducing compound 22 in a solvent.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; alcohols such as methanol, ethanol, isopropyl alcohol, tert-butanol and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include tetrahydrofuran, ethyl acetate and ethanol.

As the reduction reaction, for example, hydrogenation using a noble metal catalyst (e.g., palladium carbon, palladium-barium sulfate, palladium black, platinum carbon, platinum oxide, rhodium carbon, Raney-nickel etc.) and the like, or reduction reaction using tin dichloride, iron, sodium hydrosulfite and the like, and the like can be mentioned, with preference given to hydrogenation using a noble metal catalyst (palladium carbon).

The reaction temperature is about 0°C to 150°C, preferably room temperature to 100°C.

The reaction time is about 10 min to 48 hr, preferably about 30 min to 24 hr.

### Step 17

Compound 24 can be obtained by converting carboxylic acid compound C to the acid chloride with oxalyl chloride, thionyl chloride and the like in a solvent, and amidating compound 23 with the acid chloride in a solvent, as necessary in the presence of a base.

As the solvent to be used for the reaction to obtain the acid chloride, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; and the like can be mentioned. These can be used alone or in a mixture of two or more kinds thereof. The preferable solvents for this reaction include 1,2-dimethoxyethane, ethyl acetate, and methylene chloride, chloroform, toluene, 1,2-dimethoxyethane and ethyl acetate each containing a catalytic amount of N,N-dimethylformamide.

The reaction temperature is about -20°C to 120°C, preferably about 0°C to 80°C.

The reaction time is about 10 min to 48 hr, preferably about 30 min to 24 hr.

As the solvent to be used for the amidation reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include methylene chloride, chloroform, toluene, ethyl acetate and tetrahydrofuran.

As the base to be used for the reaction, for example, organic bases such as triethylamine, pyridine, 4-dimethylaminopyridine, N-methylmorpholine and the like; alkali metal hydroxides such as lithium hydroxide, sodium hydroxide, potassium hydroxide and the like; alkali metal hydrides such as sodium hydride, potassium hydride and the like; alkali metal carbonates such as sodium carbonate, potassium carbonate and the like; alkali metal hydrogencarbonates such as sodium hydrogencarbonate, potassium hydrogencarbonate and the like; and the like can be mentioned. This reaction is preferably carried out without a base, or in the presence of triethylamine, pyridine, sodium hydroxide or sodium hydrogencarbonate.

The reaction temperature is about 0°C to 120°C, preferably about 0°C to 95°C.

The reaction time is about 10 min to 48 hr, preferably about 30 min to 24 hr.

The compound 24 can also be obtained by subjecting compound 23 and carboxylic acid compound C to condensation with, for example, water-soluble carbodiimide (WSC HCl: 1-ethyl-3-(3'-dimethylaminopropyl)carbodiimide hydrochloride), dicyclohexylcarbodiimide (DCC), diphenylphosphoryl azide (DPPA), carbonyldiimidazole (CDI), 1-hydroxy-1H-benzotriazole (HOBT), 4-dimethylaminopyridine (DMAP) and the like; or by converting carboxylic acid compound C to a mixed acid anhydride with a chloroformate such as ethyl chloroformate, isopropyl chloroformate, isobutyl chloroformate and the like in the presence of a base such as triethylamine and the like, and reacting compound 23 with the mixed acid anhydride in the presence of a base.

### Step 18

Compound 25 can be obtained by subjecting compound 24 to desilylation in a solvent.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; alcohols such as methanol, ethanol, isopropyl alcohol, tert-butanol and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include tetrahydrofuran and N,N-dimethylformamide.

As the reagent to be used for the reaction, for example, potassium carbonate, tetrabutylammonium fluoride and the like can be mentioned, with preference given to tetrabutylammonium fluoride.

The reaction temperature is about 0°C to 150°C, preferably room temperature to 80°C.

The reaction time is about 10 min to 48 hr, preferably about 30 min to 24 hr.

### Step 19

Compound 25 can be converted to compound 26.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. Preferably, this reaction is carried out without a solvent or in the presence of methylene chloride or chloroform.

As the reagent to be used for the reaction, for example, carbon tetrachloride in the presence of triphenylphosphine; N-chlorosuccinimide (NCS) in the presence of triphenylphosphine; thionyl chloride; carbon tetrabromide in the presence of triphenylphosphine; N-bromosuccinimide (NBS) in the presence of triphenylphosphine; phosphorus tribromide; phosphorus bromide; iodine in the presence of triphenylphosphine and imidazole; methanesulfonyl chloride in the presence of a base (pyridine, triethylamine etc.); and the like can be mentioned, with preference given to methanesulfonyl chloride in the presence of a base (pyridine, triethylamine etc.).

The reaction temperature is about 0°C to 150°C, preferably room temperature to 100°C.

The reaction time is about 10 min to 48 hr, preferably about 30 min to 24 hr.

### Step 20

Compound 27 can be obtained by subjecting compound 26 to cyclization in a solvent in the presence of a base, as necessary in the presence of sodium iodide, potassium iodide and the like.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include tetrahydrofuran and N,N-dimethylformamide.

As the base to be used for the reaction, for example, organic bases such as triethylamine, pyridine, 4-dimethylaminopyridine, N-methylmorpholine and the like; alkali metal hydroxides such as lithium hydroxide, sodium hydroxide, potassium hydroxide and the like; alkali metal carbonates such as sodium carbonate, potassium carbonate and the like; alkali metal hydrogencarbonates such as sodium hydrogencarbonate, potassium hydrogencarbonate and the like; alkali metal carboxylates such as sodium acetate, potassium acetate and the like; alkali metal hydrides such as sodium hydride, potassium hydride and the like; alkali metal alkoxides such as sodium ethoxide, sodium methoxide, potassium tert-butoxide and the like; alkyllithiums such as n-butyllithium, s-butyllithium and the like; alkali metal amides such as lithium diisopropylamide, sodium amide, lithium bistrimethylsilylamide and the like; and the like can be mentioned, with preference given to potassium carbonate and sodium hydride.

The reaction temperature is about 0°C to 150°C, preferably room temperature to 100°C.

The reaction time is about 10 min to 48 hr, preferably about 30 min to 24 hr.

The crystal of the present invention can also be produced by applying a crystallization method known per se to the compound of the present invention.

Here, as the crystallization method, for example, crystallization from a solution, crystallization from vapor, crystallization from a molten form and the like can be mentioned.

For the "crystallization from a solution", a method comprising shifting from a non-saturation state to a supersaturation state by changing the factors (solvent composition, pH, temperature, ionic strength, oxidization-reduction state etc.) relating to the solubility of the compound or the amount of solvent is generally employed. Specifically, for example, concentration method, slow cooling method, reaction methods (diffusion method, electrolysis method), hydrothermal growth method, fusing agent method and the like can be mentioned. As the solvent to be used, for example, aromatic hydrocarbons (e.g., benzene, toluene, xylene etc.), halogenated hydrocarbons (e.g., dichloromethane, chloroform etc.), saturated hydrocarbons (e.g., hexane, heptane, cyclohexane etc.), ethers (e.g., diethyl ether, diisopropyl ether, tetrahydrofuran, dioxane etc.), nitriles (e.g., acetonitrile etc.), ketones (e.g., acetone etc.), sulfoxides (e.g., dimethyl sulfoxide etc.), acid amides (e.g., N,N-dimethylformamide etc.), esters (e.g., ethyl acetate etc.), alcohols (e.g., methanol, ethanol, isopropyl alcohol, n-butanol etc.), water and the like can be mentioned. These solvents are used alone or a mixture of two or more thereof at a suitable ratio (e.g., 1:1 to 1:100 (volume ratio)).

For the "crystallization from vapor", for example, gasification methods (sealed tube method, gas stream method), gas phase reaction method, chemical transportation method and the like can be mentioned.

For the "crystallization from a molten form", for example, normal freezing methods (pulling-up method, temperature gradient method, Bridgman method), zone melting methods (zone leveling method, float zone method), special growth methods (VLS method, liquid phase epitaxis method) and the like can be mentioned.

As preferable examples of crystallization, a method wherein the compound of the present invention is dissolved in a suitable solvent (e.g., alcohols such as methanol, ethanol etc. and the like) at a temperature of 20-120°C, the obtained solution is cooled to a temperature not more than that for dissolution (e.g., 0-50°C, preferably 0-20°C) and the like can be mentioned.

The crystal of the present invention thus obtained can be isolated by, for example, filtration and the like.

### Examples

The production of the compound of the present invention is concretely explained by referring to Examples, which are not to be construed as limitative.

In the Examples, the room temperature means 1-40°C.

### Example 1

### Production of (3-chloro-4-hydroxyphenyl)-(2,3-dihydrobenzo[1,4]oxazin-4-yl)-methanone

### Step 1

### Production of 3-chloro-4-methoxybenzoyl chloride

Chloroform (20 mL) was added to 3-chloro-4-methoxybenzoic acid (2.0 g), and oxalyl chloride (1.84 mL) and N,N-dimethylformamide (1 drop) were added under ice-cooling. The mixture was stirred at room temperature for 3 hrs, concentrated and azeotroped with toluene to give the title compound (2.063 g).

### Step 2

### Production of 3,4-dihydro-2H-benzo[1,4]oxazine

Synthesis was performed in reference to Australian journal of chemistry, 9, 397-405 (1956). To be specific, lithium aluminum hydride (3 g) was suspended in tetrahydrofuran (120 mL), and 2H-1,4-benzoxazin-3(4H)-one (6 g) was added by small portions under ice-cooling. After heating under reflux for 10 hrs, water (3 mL), 15% aqueous sodium hydroxide (3 mL) and water (9 mL) were successively added under ice-cooling, and the mixture was stirred at room temperature. The mixture was dried over anhydrous sodium sulfate and concentrated. The obtained residue was purified by silica gel chromatography (n-hexane-ethyl acetate=4:1) to give the title compound (4.9239 g) as an orange oil.

### Step 3

### Production of (3-chloro-4-methoxyphenyl)-(2,3-dihydrobenzo[1,4]oxazin-4-yl)-methanone

3,4-Dihydro-2H-benzo[1,4]oxazine.(525 mg) was dissolved in chloroform (10 mL), and triethylamine (0.65 mL) and 3-chloro-4-methoxybenzoyl chloride (836 mg) were added under ice-cooling. The mixture was stirred at room temperature for 12 hrs, and the reaction mixture was purified by silica gel chromatography (n-hexane-ethyl acetate=4:1) to give the title compound (1.180 g) as a white solid.

### Step 4

### Production of (3-chloro-4-hydroxypheriyl)-(2,3-dihydrobenzo[1,4]oxazin-4-yl)-methanone

(3-Chloro-4-methoxyphenyl)-(2,3-dihydrobenzo[1,4]oxazin-4-yl)-methanone (1.175 g) was dissolved in chloroform (10 mL) . Boron tribromide (1.0 M methylene chloride solution, 7.74 mL) was added dropwise at -45°C, and the mixture was stirred at room temperature for 3 hrs. Water and ethyl acetate were added to the reaction mixture, and the mixture was extracted with chloroform. The obtained chloroform layer was washed with water and saturated aqueous sodium hydrogencarbonate, and dried over anhydrous sodium sulfate. The solvent was evaporated, and the residue was purified by silica gel chromatography (n-hexane-ethyl acetate=65:35) to give the title compound (124 mg) as white crystals.

### Example 2

### Production of (3-bromo-4-hydroxyphenyl)-(2,3-dihydrobenzo[1,4]oxazin-4-yl)-methanone

### Step 1

### Production of 3-bromo-4-hydroxybenzoyl chloride

1,2-Dimethoxyethane (30 mL) was added to 3-bromo-4-hydroxybenzoic acid (3.25 g) to dissolve same by heating the mixture to 80°C. Thionyl chloride (1.6 mL) was added, and the mixture was stirred overnight at 80°C. The reaction mixture was concentrated under reduced pressure, azeotroped with toluene, and dried to give the title compound (3.6181 g) as a white solid.

### Step 2

### Production of (3-bromo-4-hydroxyphenyl)-(2,3-dihydrobenzo[1,4]oxazin-4-yl)-methanone

3,4-Dihydro-2H-benzo[1,4]oxazine (203 mg) obtained in Step 2 of Example 1 and 3-bromo-4-hydroxybenzoyl chloride (353 mg) were dissolved in ethyl acetate (4 mL), and the mixture was stirred overnight at 95°C. The reaction mixture was allowed to cool to room temperature, and the precipitated solid was collected by filtration to give the title compound (236.7 mg) as beige crystals.

### Example 3

### Production of (3,5-dichloro-4-hydroxyphenyl)-(2,3-dihydrobenzo[1,4]oxazin-4-yl)-methanone

### Step 1

### Production of 3,5-dichloro-4-hydroxybenzoyl chloride

1,2-Dimethoxyethane (30 mL) was added to 3,5-dichloro-4-hydroxybenzoic acid (1.242 g) to dissolve same by heating the mixture to 80°C. Thionyl chloride (0.57 mL) was added, and the mixture was stirred overnight at 80°C. The reaction mixture was concentrated under reduced pressure, azeotroped with toluene, and dried to give the title compound (1.358 g) as a white solid.

### Step 2

### Production of (3,5-dichloro-4-hydroxyphenyl)-(2,3-dihydrobenzo[1,4]oxazin-4-yl)-methanone

3,4-Dihydro-2H-benzo[1,4]oxazine (135 mg) obtained in Step 2 of Example 1 and 3,5-dichloro-4-hydroxybenzoyl chloride (225 mg) were dissolved in ethyl acetate (3.2 mL), and the mixture was stirred overnight at 95°C. The reaction mixture was allowed to cool to room temperature, and the precipitated solid was collected by filtration to give the title compound (282 mg) as white crystals.

### Example 4

### Production of (3,5-dibromo-4-hydroxyphenyl)-(2,3-dihydrobenzo[1,4]oxazin-4-yl)-methanone

### Step 1

### Production of 3,5-dibromo-4-hydroxybenzoyl chloride

1,2-Dimethoxyethane (20 mL) was added to 3,5-dibromo-4-hydroxybenzoic acid (2.96 g) to dissolve same by heating the mixture to 80°C. Thionyl chloride (1.1 mL) was added, and the mixture was stirred overnight at 80°C. The reaction mixture was concentrated under reduced pressure, azeotroped with toluene, and dried to give the title compound (3.1562 g) as a white solid.

### Step 2

### Production of (3,5-dibromo-4-hydroxyphenyl)-(2,3-dihydrobenzo[1,4]oxazin-4-yl)-methanone

3,4-Dihydro-2H-benzo[1,4]oxazine (270 mg) obtained in Step 2 of Example 1 and 3,5-dibromo-4-hydroxybenzoyl chloride (629 mg) were dissolved in ethyl acetate (10 mL), and the mixture was stirred overnight at 95°C. The reaction mixture was allowed to cool to room temperature, and the precipitated solid was collected by filtration to give the title compound (700.9 mg) as pale-orange crystals.

### Example 5

### Production of (2,3-dihydrobenzo[1,4]oxazin-4-yl)-(4-hydroxy-3,5-diiodophenyl)-methanone

### Step 1

### Production of 4-hydroxy-3,5-diiodobenzoyl chloride

1,2-Dimethoxyethane (12 mL) was added to 4-hydroxy-3,5-diiodobenzoic acid (2.34 g) to dissolve same by heating the mixture to 80°C. Thionyl chloride (0.66 mL) was added, and the mixture was stirred overnight at 80°C. The reaction mixture was concentrated under reduced pressure, azeotroped with toluene, and dried to give the title compound (2.4922 g) as a cream color solid.

### Step 2

### Production of (2,3-dihydrobenzo[1,4]oxazin-4-yl)-(4-hydroxy-3,5-diiodophenyl)-methanone

3,4-Dihydro-2H-benzo[1,4]oxazine (406 mg) obtained in Step 2 of Example 1 and 4-hydroxy-3,5-diiodobenzoyl chloride (1.26 g) were dissolved in ethyl acetate (15 mL), and the mixture was stirred overnight at 95°C. The reaction mixture was allowed to cool to room temperature, and the precipitated solid was collected by filtration to give the title compound (1.2887 g) as pale-orange crystals.

### Example 6

### Production of (3,5-difluoro-4-hydroxyphenyl)-(2,3-dihydrobenzo[1,4]oxazin-4-yl)-methanone

### Step 1

### Production of 3,5-difluoro-4-methoxybenzoyl chloride

Chloroform (20 mL) was added to 3,5-difluoro-4-methoxybenzoic acid (2 g), and oxalyl chloride (1.87 mL) and N,N-dimethylformamide (1 drop) were added under ice-cooling. After stirring at room temperature for 3 hrs, the reaction mixture was concentrated under reduced pressure, and azeotroped with toluene to give the title compound as an oil.

### Step 2

### Production of (3,5-difluoro-4-methoxyphenyl)-(2,3-dihydrobenzo[1,4]oxazin-4-yl)-methanone

3,4-Dihydro-2H-benzo[1,4]oxazine (300 mg) obtained in Step 2 of Example 1 was dissolved in chloroform (6 mL) and triethylamine(0.371 mL) and 3,5-difluoro-4-methoxybenzoyl chloride (459 mg) were added under ice-cooling. The mixture was stirred at room temperature for 12 hrs, and the reaction mixture was purified by silica gel chromatography (n-hexane-ethyl acetate=4:1) to give the title compound (709 mg) as an oil.

### Step 3

### Production of (3,5-difluoro-4-hydroxyphenyl)-(2,3-dihydrobenzo [1,4] oxazin-4-yl)-methanone

(3,5-Difluoro-4-methoxyphenyl)-(2,3-dihydrobenzo [1,4]oxazin-4-yl)-methanone (672 mg) was dissolved in methylene chloride (7 mL). After cooling to -78°C, boron tribromide (1.0 M methylene chloride solution, 3.3 mL) was added dropwise, and the mixture was stirred at room temperature for 20 hrs. The reaction mixture was poured into water, and the mixture was extracted with chloroform. The extract was washed successively with water and saturated brine, dried over anhydrous sodium sulfate, and concentrated. The obtained residue was purified by silica gel chromatography (n-hexane-ethyl acetate=7:3) to give the title compound (258 mg) as white crystals.

### Example 7

### Production of (2,3-dihydrobenzo[1,4]oxazin-4-yl)-(4-hydroxy-3,5-dimethylphenyl)-methanone

### Step 1

### Production of 4-benzyloxy-3,5-dimethylbenzoyl chloride

Methylene chloride (8 mL) was added to 4-benzyloxy-3,5-dimethylbenzoic acid (256 mg), and oxalyl chloride (0.1 mL) and N,N-dimethylformamide (1 drop) were added under ice-cooling. After stirring overnight at room temperature, the reaction mixture was concentrated under reduced pressure, and azeotroped with toluene to give the title compound (276.7 mg) as a pale-yellow solid.

### Step 2

### Production of (4-benzyloxy-3,5-dimethylphenyl)-(2,3-dihydrobenzo[1,4]oxazin-4-yl)-methanone

3,4-Dihydro-2H-benzo[1,4]oxazine (135 mg) obtained in Step 2 of Example 1 was dissolved in methylene chloride (7 mL), and triethylamine (0.17 mL) and 4-benzyloxy-3,5-dimethylbenzoyl chloride (275 mg) were added under ice-cooling. After stirring overnight at room temperature, the reaction mixture was purified by silica gel chromatography (n-hexane-ethyl acetate=4:1) to give the title compound (378.3 mg) as an oil.

### Step 3

### Production of (2,3-dihydrobenzo[1,4]oxazin-4-yl)-(4-hydroxy-3,5-dimethylphenyl)-methanone

(4-Benzyloxy-3,5-dimethylphenyl)-(2,3-dihydrobenzo[1,4]oxazin-4-yl)-methanone (370.1 mg) was dissolved in tetrahydrofuran (10 mL). 7.5% Palladium-carbon (37 mg) was added to this solution and, under a hydrogen atmosphere, the mixture was stirred at room temperature for 1 hr. The reaction mixture was filtered through celite, and the residue was washed with tetrahydrofuran. The filtrate and washing solution were combined and the mixture was concentrated under reduced pressure. The obtained solid was crystallized from ethyl acetate to give the title compound (220.1 mg) as white crystals.

### Example 8

### Production of (3,5-dichloro-4-hydroxyphenyl)-(2,3-dihydrobenzo[1,4]thiazin-4-yl)-methanone

### Step 1

### Production of ethyl 4-benzyloxy-3,5-dichlorobenzoate

Ethyl 3,5-dichloro-4-hydroxybenzoate (55.64 g) was dissolved in N,N-dimethylformamide (280 mL) and potassium carbonate (42.56 g) was added. Under ice-cooling, benzyl bromide (36 mL) was added dropwise, and the mixture was stirred overnight at 70°C. The solvent was evaporated, and the mixture was partitioned between water and ethyl acetate. The ethyl acetate layer was washed with saturated brine, dried over anhydrous sodium sulfate, and concentrated. The obtained residue was recrystallized from n-hexane to give the title compound (32.45 g). In addition, the filtrate was concentrated to give the title compound (42.19 g).

### Step 2

### Production of 4-benzyloxy-3,5-dichlorobenzoic acid

Ethyl 4-benzyloxy-3,5-dichlorobenzoate (42.19 g) was dissolved in methanol (70 mL) and tetrahydrofuran (140 mL). Under ice-cooling, 2N aqueous lithium hydroxide (130 mL) was added dropwise, and the mixture was stirred overnight at room temperature. A small amount of an insoluble material was removed by filtration. The filtrate was concentrated. Water was added, and the mixture was acidified by dropwise addition of 1N hydrochloric acid under ice-cooling. The precipitated solid was collected by filtration to give the title compound (34.83 g).

### Step 3

### Production of 4-benzyloxy-3,5-dichlorobenzoyl chloride

Chloroform (175 mL) was added to 4-benzyloxy-3,5-dichlorobenzoic acid (34.83 g), and oxalyl chloride (15.3 mL) and N,N-dimethylformamide (2 drops) were added under ice-cooling. The mixture was stirred at room temperature for 4 hrs, an insoluble material was removed by filtration, the filtrate was concentrated and azeotroped with toluene to give the title compound (37.371 g) as a pale-yellow solid.

### Step 4

### Production of 3,4-dihydro-2H-benzo[1,4]thiazine

Lithium aluminum hydride (1 g) was suspended in tetrahydrofuran (40 mL), 4H-benzo[1,4]thiazin-3-one (2 g) was added under ice-cooling by small portions. The mixture was heated under reflux for 8 hrs, and water (1 mL), 15% aqueous sodium hydroxide (1 mL) and water (3 mL) were successively added under ice-cooling, and the mixture was stirred at room temperature. The mixture was dried over anhydrous sodium sulfate, and concentrated to give the title compound (1.9181 g) as a yellow oil.

### Step 5

### Production of (4-benzyloxy-3,5-dichlorophenyl)-(2,3-dihydrobenzo[1,4]thiazin-4-yl)-methanone

3,4-Dihydro-2H-benzo[1,4]thiazine (1 g) was dissolved in chloroform (19 mL), and triethylamine (1.1 mL) and 4-benzyloxy-3,5-dichlorobenzoyl chloride (2.08 g) were added under ice-cooling. After stirring overnight at room temperature, the reaction mixture was purified by silica gel chromatography (n-hexane-ethyl acetate=5:1) to give the title compound (2.3350 g) as an oil.

### Step 6

### Production of (3,5 -dichloro-4-hydroxyphenyl)-(2,3-dihydrobenzo[1,4]thiazin-4-yl)-methanone

(4-Benzyloxy-3,5-dichlorophenyl)-(2,3-dihydrobenzo[1,4]thiazin-4-yl)-methanone (223.7 mg) was dissolved in toluene (2 mL), and trifluoroacetic acid (2 mL) was added at room temperature. After stirring with heating at 80°C for 1.5 hrs, the mixture was concentrated. The obtained solid was crystallized from ethyl acetate to give the title compound (101.6 mg) as white crystals.

### Example 9

### Production of (3,5-dichloro-4-hydroxyphenyl)-(1-oxo-2,3-dihydro-1H-1λ⁴-benzo[1,4]thiazin-4-yl)-methanone

### Step 1

### Production of (4-benzyloxy-3,5-dichlorophenyl)-(1-oxo-2,3-dihydro-1H-1λ⁴-benzo[1,4]thiazin-4-yl)-methanone

(4-Benzyloxy-3,5-dichlorophenyl)-(2,3-dihydrobenzo[1,4]thiazin-4-yl)-methanone (426 mg) obtained in Step 5 of Example 8 was dissolved in chloroform (10 mL), 3-chloroperbenzoic acid (171 mg) was added under ice-cooling, and the mixture was stirred overnight at room temperature. Saturated aqueous sodium hydrogencarbonate was added to the reaction solution, and the mixture was extracted with chloroform. The extract was dried over anhydrous sodium sulfate, and concentrated. The obtained residue was purified by silica gel chromatography (n-hexane-ethyl acetate=1:3) to give the title compound (398.4 mg) as a white amorphous form.

### Step 2

### Production of (3,5-dichloro-4-hydroxyphenyl)-(1-oxo-2,3-dihydro-1H-1λ⁴-benzo[1,4]thiazin-4-yl)-methanone

(4-Benzyloxy-3,5-dichlorophenyl)-(1-oxo-2,3-dihydro-1H-1λ⁴-benzo[1,4]thiazin-4-yl)-methanone (210.9 mg) was dissolved in toluene (2 mL), and trifluoroacetic acid (2 mL) was added at room temperature. After stirring with heating at 80°C for 1.5 hrs, the mixture was concentrated. The obtained solid was crystallized from ethyl acetate to give the title compound (157.5 mg) as pale-blue crystals.

### Example 10

### Production of (3,5-dichloro-4-hydroxyphenyl)-(1,1-dioxo-2,3-dihydro-1H-1λ⁶-benzo[1,4]thiazin-4-yl)-methanone

### Step 1

### Production of (4-benzyloxy-3,5-dichlorophenyl)-(1,1-dioxo-2,3-dihydro-1H-1λ⁶-benzo[1,4]thiazin-4-yl)-methanone

(4-Benzyloxy-3,5-dichlorophenyl)-(2,3-dihydrobenzo[1,4]thiazin-4-yl)-methanone (496.6 mg) obtained in Step 5 of Example 8 was dissolved in chloroform (15 mL), 3-chloroperbenzoic acid (597 mg) was added under ice-cooling, and the mixture was stirred overnight at room temperature. The insoluble material was removed by filtration, saturated aqueous sodium hydrogencarbonate was added to the filtrate, and the mixture was extracted with chloroform. The extract was dried over anhydrous sodium sulfate, and concentrated. The obtained residue was purified by silica gel chromatography (n-hexane-ethyl acetate=2:1) to give the title compound (363.9 mg) as a white solid.

### Step 2

### Production of (3,5-dichloro-4-hydroxyphenyl)-(1,1-dioxo-2,3-dihydro-1H-1λ⁶-benzo[1,4]thiazin-4-yl)-methanone

(4-Benzyloxy-3,5-dichlorophenyl)-(1,1-dioxo-2,3-dihydro-1H-1λ⁶-benzo[1,4]thiazin-4-yl)-methanone (245 mg) was dissolved in toluene (2.5 mL), and trifluoroacetic acid (2.5 mL) was added at room temperature. After stirring with heating at 80°C for 2 hrs, and the mixture was concentrated. The obtained solid was crystallized from ethyl acetate to give the title compound (112 mg) as white crystals.

### Example 11

### Production of (3,5-dichloro-4-hydroxyphenyl)-(2,3-dihydrobenzo[1,4]oxazin-4-yl)-methanethione

### Step 1

### Production of (4-benzyloxy-3,5-dichlorophenyl)-(2,3-dihydrobenzo[1,4]oxazin-4-yl)-methanone

3,4-Dihydro-2H-benzo[1,4]oxazine (0.4 g) obtained in Step 2 of Example 1 was dissolved in chloroform (20 mL), triethylamine (0.5 mL) and 4-benzyloxy-3,5-dichlorobenzoyl chloride (0.947 g) obtained in Step 3 of Example 8 were added under ice-cooling. After stirring overnight at room temperature, the reaction mixture was purified by silica gel chromatography (n-hexane-ethyl acetate=6:1) to give the title compound (1.0635 g) as a white solid.

### Step 2

### Production of (4-benzyloxy-3,5-dichlorophenyl)-(2,3-dihydrobenzo[1,4]oxazin-4-yl)-methanethione

(4-Benzyloxy-3,5-dichlorophenyl)-(2,3-dihydrobenzo[1,4]oxazin-4-yl)-methanone (400 mg) and Lawesson reagent (235 mg) were suspended in tetrahydrofuran (3 mL), and the mixture was stirred with heating at 95°C for 2 hrs. The reaction mixture was purified by silica gel chromatography (n-hexane-ethyl acetate=6:1) to give the title compound (372.4 mg) as an orange amorphous form.

### Step 3

### Production of (3,5-dichloro-4-hydroxyphenyl)-(2,3-dihydrobenzo[1,4]oxazin-4-yl)-methanethione

(4-Benzyloxy-3,5-dichlorophenyl)-(2,3-dihydrobenzo[1,4]oxazin-4-yl)-methanethione (366.8 mg) was dissolved in toluene (4 mL), and trifluoroacetic acid (4 mL) was added at room temperature. After stirring with heating at 80°C for 2 hrs, and the mixture was concentrated. The obtained solid was crystallized from ethyl acetate to give the title compound (84.6 mg) as orange crystals.

### Example 12

### Production of (3,5-dichloro-4-hydroxyphenyl)-(6-methyl-2,3-dihydrobenzo[1,4]oxazin-4-yl)-methanone

### Step 1

### Production of 6-methyl-3,4-dihydro-2H-benzo[1,4]oxazine

Lithium aluminum hydride (0.8 g) was suspended in tetrahydrofuran (50 mL), and 6-methyl-4H-benzo[1,4]oxazin-3-one (1.76 g) was added under ice-cooling by small portions. After heating under reflux for 6 hrs, water (0.8 mL), 15% aqueous sodium hydroxide (0.8 mL) and water (2.4 mL) were successively added under ice-cooling, and the mixture was stirred at room temperature. The mixture was dried over anhydrous sodium sulfate and concentrated. The obtained residue was purified by silica gel chromatography (n-hexane-ethyl acetate=3:1) to give the title compound (1.5719 g) as an orange oil.

### Step 2

### Production of (3,5-dichloro-4-hydroxyphenyl)-(6-methyl-2,3-dihydrobenzo[1,4]oxazin-4-yl)-methanone

6-Methyl-3,4-dihydro-2H-benzo[1,4]oxazine (298 mg) and 3,5-dichloro-4-hydroxybenzoyl chloride (451 mg) obtained in Step 1 of Example 3 were dissolved in ethyl acetate (7 mL), and the mixture was stirred overnight at 95°C. The reaction mixture was allowed to cool to room temperature, and the precipitated solid was collected by filtration to give the title compound (606.4 mg) as pale-orange crystals.

### Example 13

### Production of (3,5-dichloro-4-hydroxyphenyl)-(7-methyl-2,3-dihydrobenzo[1,4]oxazin-4-yl)-methanone

### Step 1

### Production of 7-methyl-4H-benzo[1,4]oxazin-3-one

2-Amino-5-methylphenol (2.463 g) and benzyltriethylammonium chloride (4.56 g) were suspended in chloroform (50 mL), sodium hydrogencarbonate (13.44 g) and chloroacetyl chloride (1.9 mL) were added under ice-cooling and the mixture was stirred under ice-cooling for 1 hr. Thereafter, and the mixture was stirred overnight with heating at 55°C. The reaction mixture was concentrated, water was added and the mixture was extracted with ethyl acetate. The obtained ethyl acetate layer was washed successively with 1N hydrochloric acid, water, saturated aqueous sodium hydrogencarbonate, water and saturated brine, dried over anhydrous sodium sulfate, and concentrated. The obtained solid was crystallized from ethyl acetate to give the title compound (1.7636 g) as a khaki solid.

### Step 2

### Production of 7-methyl-3,4-dihydro-2H-benzo[1,4]oxazine

Lithium aluminum hydride (0.8 g) was suspended in tetrahydrofuran (50 mL), and 7-methyl-4H-benzo[1,4]oxazin-3-one (1.76 g) was added under ice-cooling by small portions. After heating under reflux for 6 hrs, water (0.8 mL), 15% aqueous sodium hydroxide (0.8 mL) and water (2.4 mL) were successively added under ice-cooling, and the mixture was stirred at room temperature. The mixture was dried over anhydrous sodium sulfate and concentrated. The obtained residue was purified by silica gel chromatography (n-hexane-ethyl acetate=3:1) to give the title compound (1.5038 g) as an orange-tan oil.

### Step 3

### Production of (3,5-dichloro-4-hydroxyphenyl)-(7-methyl-2,3-dihydrobenzo[1,4]oxazin-4-yl)-methanone

7-Methyl-3,4-dihydro-2H-benzo[1,4]oxazine (298 mg) and 3,5-dichloro-4-hydroxybenzoyl chloride (451 mg) obtained in Step 1 of Example 3 were dissolved in ethyl acetate (7 mL), and the mixture was stirred overnight at 95°C. The reaction mixture was purified by silica gel chromatography (n-hexane-ethyl acetate=4:1) to give the title compound (609.3 mg) as a white amorphous solid.

### Example 14

### Production of (3,5-dichloro-4-hydroxyphenyl)-(5-methyl-2,3-dihydrobenzo[1,4]oxazin-4-yl)-methanone

### Step 1

### Production of 2-chloro-N-(2-hydroxy-6-methylphenyl)-acetamide

2-Amino-3-methylphenol (737 mg) was dissolved in tetrahydrofuran (20 mL), triethylamine (1 mL) and chloroacetyl chloride (0.5 mL) were added under ice-cooling, and the mixture was stirred overnight at room temperature. The reaction mixture was poured into water, and the mixture was extracted with ethyl acetate. The obtained ethyl acetate layer was washed successively with 1N hydrochloric acid, water and saturated brine, and dried over anhydrous sodium sulfate. The solvent was evaporated to give the title compound (1.2217 g) as a khaki solid.

### Step 2

### Production of 5-methyl-4H-benzo[1,4]oxazin-3-one

2-chloro-N-(2-hydroxy-6-methylphenyl)-acetamide (1.2217 g) was dissolved in N,N-dimethylformamide (7 mL), and potassium carbonate (0.99 g) and sodium iodide (catalytic amount) were added at room temperature. After stirring overnight at 80°C, the mixture was poured into water. The mixture was extracted with ethyl acetate. The obtained ethyl acetate layer was washed successively with 1N hydrochloric acid and water, then successively washed with saturated aqueous sodium hydrogencarbonate, water and saturated brine, and dried over anhydrous sodium sulfate. The solvent was evaporated to give the title compound (813.5 mg) as a pale-yellow solid.

### Step 3

### Production of 5-methyl-3,4-dihydro-2H-benzo[1,4]oxazine

Lithium aluminum hydride (0.4 g) was suspended in tetrahydrofuran (40 mL) and, under ice-cooling, 5-methyl-4H-benzo[1,4]oxazin-3-one (810 mg) was added by small portions. After heating under reflux for 7 hrs, water (0.4 mL), 15% aqueous sodium hydroxide (0.4 mL) and water (1.2 mL) were successively added under ice-cooling, and the mixture was stirred at room temperature. The mixture was dried over anhydrous sodium sulfate and concentrated. The obtained residue was purified by silica gel chromatography (n-hexane-ethyl acetate=3:1) to give the title compound (471.7 mg) as a red brown oil.

### Step 4

### Production of (3,5-dichloro-4-hydroxyphenyl)-(5-methyl-2,3-dihydrobenzo[1,4]oxazin-4-yl)-methanone

5-Methyl-3,4-dihydro-2H-benzo[1,4]oxazine (224 mg) and 3,5-dichloro-4-hydroxybenzoyl chloride (338 mg) obtained in Step 1 of Example 3 were dissolved in ethyl acetate (3.5 mL), and the mixture was stirred overnight at 95°C. The reaction mixture was allowed to cool to room temperature, and the precipitated solid was collected by filtration to give the title compound (331.9 mg) as gray crystals.

### Example 15

### Production of (3,5-dichloro-4-hydroxyphenyl)-(8-methyl-2,3-dihydrobenzo[1,4]oxazin-4-yl)-methanone

### Step 1

### Production of 4-bromo-2-methylphenol

o-Cresol (5 g) was dissolved in acetic acid (50 mL) and 48% aqueous hydrogen bromide (25 mL), dimethyl sulfoxide (25 mL) was added dropwise at room temperature. After stirring at room temperature for 1 hr, the reaction mixture was neutralized with sodium carbonate. Water was added, and the mixture was extracted with ethyl ether. The obtained ethyl ether layer was washed successively with water and saturated brine, and dried over magnesium sulfate. The solvent was evaporated, and the residue was used for the next Step without purification.

### Step 2

### Production of 4-bromo-2-methyl-6-nitrophenol

To a mixture of 4-bromo-2-methylphenol produced in Step 1 and sodium nitrite (10.4 g) were added n-hexane (75 mL), isopropyl ether (35 mL) and water (50 mL), and 4.5N sulfuric acid (110 mL) was added dropwise at room temperature. After stirring at room temperature for 15 hrs, the reaction mixture was washed successively with saturated aqueous sodium hydrogencarbonate, water and saturated brine, and dried over anhydrous sodium sulfate. The solvent was evaporated, and the obtained residue was purified by silica gel chromatography (n-hexane-ethyl acetate=5:1) to give the title compound (7.14 g) as a yellow solid.

### Step 3

### Production of 2-amino-6-methylphenol hydrobromide

4-Bromo-2-methyl-6-nitrophenol (7.1 g) was dissolved in methanol (50 mL). 7.5% Palladium-carbon (1.5 g) was added to this solution, and under a hydrogen atmosphere (2 kgf/cm²), and the mixture was stirred at room temperature for 18 hrs. The reaction mixture was filtered through celite, and the filtrate was concentrated under reduced pressure. The obtained solid was crystallized from ethyl acetate to give the title compound (5.40 g) as a brown solid.

### Step 4

### Production of 2-chloro-N-(2-hydroxy-3-methylphenyl)-acetamide

2-Amino-6-methylphenol hydrobromide (408 mg) was suspended in tetrahydrofuran (10 mL), triethylamine (0.7 mL) and chloroacetyl chloride (0.17 mL) were added under ice-cooling, and the mixture was stirred overnight at room temperature. The reaction mixture was poured into water, and the mixture was extracted with ethyl acetate. The obtained ethyl acetate layer was washed successively with 1N hydrochloric acid, water and saturated brine, and dried over anhydrous sodium sulfate. The solvent was evaporated to give the title compound (384.6 mg) as a dark brown solid.

### Step 5

### Production of 8-methyl-4H-benzo[1,4]oxazin-3-one

2-Chloro-N-(2-hydroxy-3-methylphenyl)-acetamide (380 mg) was dissolved in N,N-dimethylformamide (4 mL), potassium carbonate (315 mg) and sodium iodide (catalytic amount) were added at room temperature. After stirring overnight at 80°C, the mixture was poured into water and the mixture was extracted with ethyl acetate. The obtained ethyl acetate layer washed successively with 1N hydrochloric acid and water, and successively with saturated aqueous sodium hydrogencarbonate, water and saturated brine, and dried over anhydrous sodium sulfate. The solvent was evaporated to give the title compound (284.3 mg) as a red brown solid.

### Step 6

### Production of 8-methyl-3,4-dihydro-2H-benzo[1,4]oxazine

Lithium aluminum hydride (150 mg) was suspended in tetrahydrofuran (25 mL), 8-methyl-4H-benzo[1,4]oxazin-3-one (280 mg) was added under ice-cooling by small portions. After heating under reflux for 7 hrs, water (0.15 mL), 15% aqueous sodium hydroxide (0.15 mL) and water (0.45 mL) were successively added under ice-cooling, and the mixture was stirred at room temperature. The mixture was dried over anhydrous sodium sulfate and concentrated. The obtained residue was purified by silica gel chromatography (n-hexane-ethyl acetate=3:1) to give the title compound (145.2 mg) as a red brown oil.

### Step 7

### Production of (3,5-dichloro-4-hydroxyphenyl)-(8-methyl-2,3-dihydrobenzo[1,4]oxazin-4-yl)-methanone

8-Methyl-3,4-dihydro-2H-benzo[1,4]oxazine (116.3 mg) and 3,5-dichloro-4-hydroxybenzoyl chloride (176 mg) obtained in Step 1 of Example 3 were dissolved in ethyl acetate (2 mL), and the mixture was stirred overnight at 95°C. The reaction mixture was allowed to cool to room temperature, and the precipitated solid was collected by filtration to give the title compound (121.3 mg) as pale-brown crystals.

### Example 16

### Production of (3,5-dichloro-4-hydroxyphenyl)-(2,3-dihydronaphtho[2,1-b][1,4]oxazin-1-yl)-methanone

### Step 1

### Production of 2-chloro-N-(2-hydroxynaphthalen-1-yl)-acetamide

1-Aminonaphthalen-2-ol hydrochloride (1.1739 g) was suspended in tetrahydrofuran (20 mL), triethylamine (2 mL) and chloroacetyl chloride (0.5 mL) were added under ice-cooling, and the mixture was stirred overnight at room temperature. The reaction mixture was poured into water, and the mixture was extracted with ethyl acetate. The obtained ethyl acetate layer was washed successively with 1N hydrochloric acid, water and saturated brine, and dried over anhydrous sodium sulfate. The solvent was evaporated to give the title compound (1.227 g) as a dark brown solid.

### Step 2

### Production of 1H-naphtho[2,1-b][1,4]oxazin-2-one

2-Chloro-N-(2-hydroxynaphthalen-1-yl)-acetamide (1.22 g) was dissolved in N,N-dimethylformamide (10 mL), and potassium carbonate (860 mg) and sodium iodide (catalytic amount) were added at room temperature. After stirring overnight at 80°C, and the mixture was poured into water, and the mixture was extracted with ethyl acetate. The obtained ethyl acetate layer was washed successively with 1N hydrochloric acid and water, subsequently with saturated aqueous sodium hydrogencarbonate; water and saturated brine, and dried over anhydrous sodium sulfate. The solvent was evaporated to give the title compound (1.0553 g) as a dark brown solid.

### Step 3

### Production of 2,3-dihydro-1H-naphtho[2,1-b][1,4]oxazine

Lithium aluminum hydride (400 mg) was suspended in tetrahydrofuran (40 mL), 1H-naphtho[2,1-b][1,4]oxazin-2-one (1.055 g) was added under ice-cooling by small portions. After heating under reflux for 7 hrs, water (0.4 mL), 15% aqueous sodium hydroxide (0.4 mL) and water (1.2 mL) were successively added under ice-cooling, and the mixture was stirred at room temperature. The mixture was dried over anhydrous sodium sulfate and concentrated. The obtained residue was purified by silica gel chromatography (n-hexane-ethyl acetate=3:1) to give the title compound (361.4 mg) as a dark brown oil.

### Step 4

### Production of (3,5-dichloro-4-hydroxyphenyl)-(2,3-dihydronaphtho[2,1-b][1,4]oxazin-1-yl)-methanone.

2,3-Dihydro-1H-naphtho[2,1-b][1,4]oxazine (185 mg) and 3,5-dichloro-4-hydroxybenzoyl chloride (225 mg) obtained in Step 1 of Example 3 were dissolved in ethyl acetate (3 mL), and the mixture was stirred overnight at 95°C. The reaction mixture was allowed to cool to room temperature; and the precipitated solid was collected by filtration to give the title compound (334.5 mg) as gray crystals.

### Example 17

### Production of (3,5-dichloro-4-hydroxyphenyl)-(6-methoxy-2,3-dihydrobenzo[1,4]oxazin-4-yl)-methanone

### Step 1

### Production of 2-amino-4-methoxyphenol

4-Methoxy-2-nitrophenol (3.38 g) was dissolved in tetrahydrofuran (100 mL). 7.5% Palladium-carbon (0.34 g) was added to this solution and, under a hydrogen atmosphere, and the mixture was stirred at room temperature for 3.5 hrs. The reaction mixture was filtered through celite, and the residue was washed with tetrahydrofuran. The filtrate and washing solution were combined and the mixture was concentration under reduced pressure to give the title compound (2.8528 g) as a beige solid.

### Step 2

### Production of 6-methoxy-4H-benzo [1,4] oxazin-3-one

2-Amino-4-methoxyphenol (2.85 g) and benzyltriethylammonium chloride (4.56 g) were suspended in chloroform (50 mL), sodium hydrogencarbonate (6.72 g) and chloroacetyl chloride (1.9 mL) were added under ice-cooling, and the mixture was stirred under ice-cooling for 1 hr. Thereafter, the mixture was stirred overnight with heating at 55°C. The reaction mixture was concentrated, water was added and the mixture was extracted with ethyl acetate. The obtained ethyl acetate layer was washed successively with 1N hydrochloric acid, water, saturated aqueous sodium hydrogencarbonate, water and saturated brine, dried over anhydrous sodium sulfate, and concentrated. The obtained solid was crystallized from ethyl acetate to give the title compound (1.3884 g) as a pale-orange solid. The mother liquor was purified by silica gel chromatography (n-hexane-ethyl acetate=3:1) to give the title compound (151 mg) as a white solid.

### Step 3

### Production of 6-methoxy-3,4-dihydro-2H-benzo[1,4]oxazine

Lithium aluminum hydride (700 mg) was suspended in tetrahydrofuran (50 mL), 6-methoxy-4H-benzo[1,4]oxazin-3-one (1.53 g) was added under ice-cooling by small portions. After heating under reflux for 6 hrs, water (0.7 mL), 15% aqueous sodium hydroxide (0.7 mL) and water (2.1 mL) were successively added under ice-cooling, and the mixture was stirred at room temperature. The mixture was dried over anhydrous sodium sulfate and concentrated. The obtained residue was purified by silica gel chromatography (n-hexane-ethyl acetate=3:1) to give the title compound (1.3294 g) as a pale-yellow solid.

### Step 4

### Production of (3,5-dichloro-4-hydroxyphenyl)-(6-methoxy-2,3-dihydrobenzo[1,4]oxazin-4-yl)-methanone

6-Methoxy-3,4-dihydro-2H-benzo[1,4]oxazine (600 mg) and 3,5-dichloro-4-hydroxybenzoyl chloride (818 mg) obtained in Step 1 of Example 3 were dissolved in ethyl acetate (14 mL), and the mixture was stirred overnight at 95°C. The reaction mixture was allowed to cool to room temperature, and the precipitated solid was collected by filtration to give the title compound (1.059 g) as white crystals.

### Example 18

### Production of (3,5-dichloro-4-hydroxyphenyl)-(7-methoxy-2,3-dihydrobenzo[1,4]oxazin-4-yl)-methanone

### Step 1

### Production of 2-amino-5-methoxyphenol

5-Methoxy-2-nitrophenol (1.059 g) was dissolved in tetrahydrofuran (30 mL). 7.5% Palladium-carbon (0.1 g) was added to this solution and, under a hydrogen atmosphere, the mixture was stirred at room temperature for 3 hrs. The reaction mixture was filtered through celite, and the residue was washed with tetrahydrofuran. The filtrate and washing solution were combined and the mixture was concentrated under reduced pressure to give the title compound (0.9615 g) as a pale-orange solid.

### Step 2

### Production of 2-chloro-N-(2-hydroxy-4-methoxyphenyl)-acetamide

2-Amino-5-methoxyphenol (0.96 g) was dissolved in tetrahydrofuran (35 mL), triethylamine (1.15 mL) and chloroacetyl chloride (0.58 mL) were added under ice-cooling, and the mixture was stirred overnight at room temperature. The reaction mixture was poured into water, and the mixture was extracted with ethyl acetate. The obtained ethyl acetate layer was washed successively with 1N hydrochloric acid, water and saturated brine, and dried over anhydrous sodium sulfate. The solvent was evaporated to give the title compound (1.5106 g) as a bright yellow solid.

### Step 3

### Production of 7-methoxy-4H-benzo[1,4]oxazin-3-one

2-Chloro-N-(2-hydroxy-4-methoxyphenyl)-acetamide (1.51 g) was dissolved in N,N-dimethylformamide (7 mL), and potassium carbonate (1.04 g) and sodium iodide (catalytic amount) were added at room temperature. After stirring overnight at 80°C, and the mixture was poured into water, and the mixture was extracted with ethyl acetate. The obtained ethyl acetate layer was washed successively with 1N hydrochloric acid and water, and sequentially washed with saturated aqueous sodium hydrogencarbonate, water and saturated brine, and dried over anhydrous sodium sulfate. The solvent was evaporated, and the obtained solid was crystallized from ethyl acetate to give the title compound (682.6 mg) as a bright yellow solid.

### Step 4

### Production of 7-methoxy-3,4-dihydro-2H-benzo[1,4]oxazine

Lithium aluminum hydride (300 mg) was suspended in tetrahydrofuran (20 mL), 7-methoxy-4H-benzo[1,4]oxazin-3-one (680 mg) was added under ice-cooling by small portions. After heating under reflux for 6 hrs, water (0.3 mL), 15% aqueous sodium hydroxide (0.3 mL) and water (0.9 mL) were successively added under ice-cooling, and the mixture was stirred at room temperature. The mixture was dried over anhydrous sodium sulfate and concentrated. The obtained residue was purified by silica gel chromatography (n-hexane-ethyl acetate=2:1) to give the title compound (588.1 mg) as a red brown oil.

### Step 5

### Production of (3,5-dichloro-4-hydroxyphenyl)-(7-methoxy-2,3-dihydrobenzo[1,4]oxazin-4-yl)-methanone

7-Methoxy-3,4-dihydro-2H-benzo[1,4]oxazine (367.6 mg) and 3,5-dichloro-4-hydroxybenzoyl chloride (496 mg) obtained in Step 1 of Example 3 were dissolved in ethyl acetate (4 mL), and the mixture was stirred overnight at 95°C. The reaction mixture was allowed to cool to room temperature, and the precipitated solid was collected by filtration to give the title compound (518.2 mg) as light purple crystals.

### Example 19

### Production of (3,5-dichloro-4-hydroxyphenyl)-(6-hydroxy-2,3-dihydrobenzo[1,4]oxazin-4-yl)-methanone

(3,5-Dichloro-4-hydroxyphenyl)-(6-methoxy-2,3-dihydrobenzo[1,4]oxazin-4-yl)-methanone (500 mg) obtained in Step 4 of Example 17 was dissolved in methylene chloride (10 mL). After cooling to -78°C, boron tribromide.(1.0 M methylene chloride solution, 2.1 mL) was added dropwise, and the mixture was stirred overnight at room temperature. The reaction mixture was poured into water, and the mixture was extracted with chloroform, and dried over anhydrous sodium sulfate. The solvent was evaporated, and the obtained residue was purified by silica gel chromatography (chloroform-methanol=10:1) to give the title compound (157.6 mg) as pale-blue crystals.

### Example 20

### Production of (3,5-dichloro-4-hydroxyphenyl)-(7-hydroxy-2,3-dihydrobenzo[1,4]oxazin-4-yl)-methahone

(3,5-Dichloro-4-hydroxyphenyl)-(7-methoxy-2,3-dihydrobenzo[1,4]oxazin-4-yl)-methanone (504 mg) obtained in Step 5 of Example 18 was dissolved in methylene chloride (10 mL). After cooling to -78°C, boron tribromide (1.0 M methylene chloride solution, 4.2 mL) was added dropwise, and the mixture was stirred overnight at room temperature. The reaction mixture was poured into water, and the precipitated solid was collected by filtration. The collected solid was purified by silica gel chromatography (chloroform-methanol=30:1) to give the title compound (355.7 mg) as white crystals.

### Example 21

### Production of 4-(3,5-dichloro-4-hydroxybenzoyl)-3,4-dihydro-2H-benzo[1,4]oxazine-6-sulfonic acid diethylamide

### Step 1

### Production of 3-amino-N,N-diethyl-4-hydroxybenzenesulfonamide -

3-Amino-N,N-diethyl-4-methoxybenzenesulfonamide (5 g) was dissolved in methylene chloride (150 mL). Under ice-cooling, boron tribromide (1.0 M methylene chloride solution, 38.7 mL) was added dropwise, and the mixture was stirred overnight at room temperature. Water (150 mL) was added dropwise to the reaction mixture under ice-cooling, and the aqueous layer was washed with chloroform. The obtained aqueous layer was weak acidified with 4N aqueous sodium hydroxide under ice-cooling. The precipitated solid was collected by filtration to give the title compound (4.0344 g) as a pale-beige solid.

### Step 2

### Production of 3-amino-4-(tert-butyldimethylsilyloxy)-N,N-diethylbenzenesulfonamide

3-Amino-N,N-diethyl-4-hydroxybenzenesulfonamide (1.5 g) was dissolved in N,N-dimethylformamide (8 mL), and imidazole (0.61 g) and tert-butylchlorodimethylsilane (1.18 g) were added under ice-cooling. After stirring at room temperature for 1.5 hrs, the mixture was poured into water, and the mixture was extracted with ethyl ether. The obtained ethyl ether layer was washed with water and saturated brine, dried over anhydrous sodium sulfate and concentrated. The obtained residue was purified by silica gel chromatography (n-hexane-ethyl acetate=7:1) to give the title compound (2.0654 g) as a white solid.

### Step 3

### Production of 4-benzyloxy-3,5-dichloro-N-(5-diethylsulfamoyl-2-hydroxyphenyl)-benzamide

3-Amino-4-(tert-butyldimethylsilyloxy)-N,N-diethylbenzenesulfonamide (1.5 g) was dissolved in methylene chloride (40 mL), pyridine (0.41 mL) and 4-benzyloxy-3,5-dichlorobenzoyl chloride (1.32 g) obtained in Step 3 of Example 8 were added under ice-cooling, and the mixture was stirred overnight at room temperature. The solvent was evaporated and the residue was dissolved in N,N-dimethylformamide (5 mL), and potassium carbonate (2.89 g) was added at room temperature. After stirring with heating at 60°C for 1.5 hrs, the mixture was acidified with 10% aqueous citric acid solution and water under ice-cooling. The precipitated solid was collected by filtration to give the title compound (2.1455 g) as a cream color solid.

### Step 4

### Production of 4-(4-benzyloxy-3,5-dichlorobenzoyl)-3,4-dihydro-2H-benzo[1,4]oxazine-6-sulfonic acid diethylamide

4-Benzyloxy-3,5-dichloro-N-(5-diethylsulfamoyl-2-hydroxyphenyl)-benzamide (2.136 g) was dissolved in N,N-dimethylformamide (40 mL), and potassium carbonate (1.41 g) and 1,2-dibromoethane (0.42 mL) were added at room temperature. After stirring overnight at 70°C, the mixture was poured into water, and the mixture was extracted with ethyl acetate. The obtained ethyl acetate layer was washed with water and saturated brine, and dried over anhydrous sodium sulfate. The solvent was evaporated, and the obtained residue was purified by silica gel chromatography (n-hexane-ethyl acetate=5:2) to give the title compound (1.6181 g) as a white amorphous form.

### Step 5

### Production of 4-(3,5-dichloro-4-hydroxybenzoyl)-3,4-dihydro-2H-benzo[1,4]oxazine-6-sulfonic acid diethylamide

4-(4-Benzyloxy-3,5-dichlorobenzoyl)-3,4-dihydro-2H-benzo[1,4]oxazine-6-sulfonic acid diethylamide (1.6151 g) was dissolved in tetrahydrofuran (70 mL). 7.5% Palladium-carbon (0.16 g) was added to this solution and, under a hydrogen atmosphere, the mixture was stirred at room temperature for 0.5 hr. The reaction mixture was filtered through celite, and the residue was washed with tetrahydrofuran. The filtrate and washing solution were combined and the mixture was concentrated under reduced pressure. The obtained solid was crystallized from ethyl ether to give the title compound (1.2863 g) as white crystals.

### Example 22

### Production of 2,6-dichloro-4-(2,3-dihydrobenzo[1,4]oxazine-4-sulfonyl)phenol

3,4-Dihydro-2H-benzo[1,4]oxazine (246 mg) obtained in Step 2 of Example 1 and 3,5-dichloro-4-hydroxybenzenesulfonyl chloride (475 mg) were dissolved in chloroform (8 mL), and the mixture was stirred overnight at room temperature. The reaction mixture was purified by silica gel chromatography (n-hexane-ethyl acetate=2:1) to give the title compound (146.8 mg) as beige crystals.

### Example 23

### Production of (6-tert-butyl-2,3-dihydrobenzo[1,4]oxazin-4-yl)-(3,5-dichloro-4-hydroxyphenyl)-methanone

The title compound (539.6 mg) was obtained as white crystals by a method similar to Example 14 and using 2-amino-4-tert-butylphenol instead of 2-amino-3-methylphenol.

### Example 24

### Production of 4-(3,5-dichloro-4-hydroxybenzoyl)-4H-benzo[1,4]oxazin-3-one

### Step 1

### Production of 4-(4-benzyloxy-3,5-dichlorobenzoyl)-4H-benzo[1,4]oxazin-3-one

4H-Benzo[1,4]oxazin-3-one (224 mg) was dissolved in tetrahydrofuran (10 mL) , 60% sodium hydride (78 mg) and 4-benzyloxy-3,5-dichlorobenzoyl chloride (473 mg) obtained in Step 3 of Example 8 were successively added with stirring under ice-cooling, and the mixture was stirred overnight at room temperature. Under ice-cooling, water was added to the reaction mixture, and the mixture was extracted with ethyl acetate. The obtained ethyl acetate layer was washed successively with saturated aqueous sodium hydrogencarbonate, water and saturated brine, dried over anhydrous sodium sulfate, and the mixture was concentrated. The obtained residue was purified by silica gel chromatography (n-hexane-ethyl acetate=9:1) to give the title compound (294.3 mg) as a yellow solid.

### Step 2

### Production of 4-(3,5-dichloro-4-hydroxybenzoyl)-4H-benzo[1,4]oxazin-3-one

4-(4-Benzyloxy-3,5-dichlorobenzoyl)-4H-benzo[1,4]oxazin-3-one (290 mg) was dissolved in tetrahydrofuran (12 mL). 7.5% Palladium-carbon (29 mg) was added to this solution and, under a hydrogen atmosphere, the mixture was stirred at room temperature for 0.5 hr. The reaction mixture was filtered through celite, and the residue was washed with tetrahydrofuran. The filtrate and washing solution were combined and the mixture was concentrated under reduced pressure. The obtained solid was crystallized from ethyl acetate to give the title compound (176.8 mg) as yellow crystals.

### Example 25

### Production of 4-(3,5-dichloro-4-hydroxybenzoyl)-3,4-dihydro-2H-benzo[1,4]oxazine-6-sulfonamide

The title compound (160.6 mg) was obtained as white crystals by a method similar to Step 2 to 5 of Example 21 and using 3-amino-4-hydroxybenzenesulfonamide instead of 3-amino-N,N-diethyl-4-hydroxybenzenesulfonamide.

### Example 26

### Production of (3,5-dichloro-4-hydroxyphenyl)-(3,4-dihydro-2H-quinolin-1-yl)-methanone

### Step 1

### Production of (4-benzyloxy-3,5-dichlorophenyl)-(3,4-dihydro-2H-quinolin-1-yl)-methanone

1,2,3,4-Tetrahydroquinoline (147 mg) was dissolved in chloroform (6 mL), and triethylamine (0.18 mL) and 4-benzyloxy-3,5-dichlorobenzoyl chloride (347 mg) obtained in Step 3 of Example 8 were added under ice-cooling. After stirring overnight at room temperature, the reaction mixture was purified by silica gel chromatography (n-hexane-ethyl acetate=6:1) to give the title compound (407.9 mg) as a white amorphous form.

### Step 2

### Production of (3,5-dichloro-4-hydroxyphenyl)-(3,4-dihydro-2H-quinolin-1-yl)-methanone

(4-Benzyloxy-3,5-dichlorophenyl)-(3,4-dihydro-2H-quinolin-1-yl)-methanone (398.7 mg) was dissolved in tetrahydrofuran (12 mL). 7.5% Palladium-carbon (40 mg) was added to this solution and, under a hydrogen atmosphere, the mixture was stirred at room temperature for 0.5 hr. The reaction mixture was filtered through celite, and the residue was washed with tetrahydrofuran. The filtrate and washing solution were combined and the mixture was concentrated under reduced pressure. The obtained solid was crystallized from ethyl acetate to give the title compound (267.4 mg) as white crystals.

### Example 27

### Production of (3,5-dichloro-4-hydroxyphenyl)-(2,3,4,5-tetrahydrobenzo[b]azepin-1-yl)-methanone

### Step 1

### Production of 2,3,4,5-tetrahydro-1H-benzo[b]azepine

Lithium aluminum hydride (100 mg) was suspended in tetrahydrofuran (10 mL), 1,3,4,5-tetrahydrobenzo[b]azepin-2-one (245 mg) was added under ice-cooling by small portions. After heating under reflux for 5.5 hrs, water (0.1 mL), 15% aqueous sodium hydroxide (0.1 mL) and water (0.3 mL) were successively added under ice-cooling, and the mixture was stirred at room temperature. The mixture was dried over anhydrous sodium sulfate, and concentrated to give the title compound (301.6 mg) as a yellow oil.

### Step 2

### Production of (4-benzyloxy-3,5-dichlorophenyl)-(2,3,4,5-tetrahydrobenzo[b]azepin-1-yl)-methanone

2,3,4,5-Tetrahydro-1H-benzo[b]azepine (301 mg) was dissolved in methylene chloride (10 mL), triethylamine (0.25 mL) and 4-benzyloxy-3,5-dichlorobenzoyl chloride (480 mg) obtained in Step 3 of Example 8 were added under ice-cooling. After stirring overnight at room temperature, the reaction mixture was purified by silica gel chromatography (n-hexane-ethyl acetate=6:1) to give the title compound (531.9 mg) as a yellow green oil.

### Step 3

### Production of (3,5-dichloro-4-hydroxyphenyl)-(2,3,4,5-tetrahydrobenzo[b]azepin-1-yl)-methanone

(4-Benzyloxy-3,5-dichlorophenyl)-(2,3,4,5-tetrahydrobenzo[b]azepin-1-yl)-methanone (530 mg) was dissolved in tetrahydrofuran (15 mL). 7.5% Palladium-carbon (53 mg) was added to this solution and, under a hydrogen atmosphere, the mixture was stirred at room temperature for 0.5 hr. The reaction mixture was filtered through celite, and the residue was washed with tetrahydrofuran. The filtrate and washing solution were combined and the mixture was concentrated under reduced pressure. The obtained solid was crystallized from ethyl acetate to give the title compound (259.6 mg) as white crystals.

### Example 28

### Production of (4-amino-3,5-dichlorophenyl)-(2,3-dihydrobenzo[1,4]oxazin-4-yl)-methanone

3,4-Dihydro-2H-benzo[1,4]oxazine (270 mg) obtained in Step 2 of Example 1, 4-amino-3,5-dichlorobenzoic acid (412 mg) and N,N-dimethylaminopyridine (269 mg) were dissolved in chloroform (8 mL), and WSC·HCl (422 mg) was added under ice-cooling. After stirring overnight at room temperature, the reaction mixture was purified by silica gel chromatography (n-hexane-ethyl acetate=5:1) to give the title compound (412.5 mg) as bright yellow crystals.

### Example 29

### Production of (5-chloro-6-hydroxypyridin-3-yl)-(2,3-dihydrobenzo[1,4]oxazin-4-yl)-methanone

3,4-Dihydro-2H-benzo[1,4]oxazine (270 mg) obtained in Step 2 of Example 1, 5-chloro-6-hydroxynicotinoic acid (347 mg) and 4-dimethylaminopyridine (269 mg) were dissolved in chloroform (12 mL), and WSC·HC1 (422 mg) was added under ice-cooling. After stirring overnight at room temperature, the reaction mixture was purified by silica gel chromatography (chloroform-methanol=20:1) to give the title compound (295.7 mg) as cream color crystals.

### Example 30

### Production of (2,3-dihydrobenzo[1,4]oxazin-4-yl)-(4-hydroxy-3,5-dinitrophenyl)-methanone

### Step 1

### Production of 4-hydroxy-3,5-dinitrobenzoyl chloride

1,2-Dimethoxyethane (5 mL) was added to 4-hydroxy-3,5-dinitrobenzoic acid (1 g) to dissolve same by heating the mixture to 70°C. Thionyl chloride (0.415 mL) was added, and the mixture was stirred overnight at 70°C. The reaction mixture was concentrated under reduced pressure, azeotroped with toluene, and dried to give the title compound as a yellow solid.

### Step 2

### Production of (2,3-dihydrobenzo[1,4]oxazin-4-yl)-(4-hydroxy-3,5-dinitrophenyl)-methanone

3,4-Dihydro-2H-benzo[1,4]oxazine (135 mg) obtained in Step 2 of Example 1 and 4-hydroxy-3,5-dinitrobenzoyl chloride (271 mg) ware dissolved in ethyl acetate (4 mL), and the mixture was heated under reflux for 3 hrs. The solvent was evaporated, and the obtained solid was crystallized from methanol to give the title compound (211 mg) as pale-yellow crystals.

### Example 31

### Production of (3-chloro-4-hydroxy-5-nitrophenyl)-(2,3-dihydrobenzo[1,4]oxazin-4-yl)-methanone

### Step 1

### Production of methyl 3-chloro-4-methoxy-5-nitrobenzoate

Concentrated sulfuric acid (200 mL) was added to methyl 3-chloro-4-methoxybenzoate (24.6 g) under ice-cooling and a mixture of fuming nitric acid (10.3 mL) and concentrated sulfuric acid (20 mL) was successively added dropwise under ice-cooling. After stirring under ice-cooling, the reaction mixture was poured into ice water (1 L). The precipitated solid was collected by filtration to give the title compound (28.8 g) as a cream color solid.

### Step 2

### Production of 3-chloro-4-hydroxy-5-nitrobenzoic acid

Methyl 3-chloro-4-methoxy-5-nitrobenzoate (28.8 g) was suspended in dimethyl sulfoxide (130 mL), and 50% aqueous potassium hydroxide (130 mL) was added under ice-cooling. After stirring with heating at 80°C for 1.5 hrs, the mixture was ice-cooled, and 6N hydrochloric acid (200 mL) and water were added. The mixture was extracted with ethyl acetate, and the extract was washed successively with water and saturated brine, and dried over anhydrous sodium sulfate. The solvent was evaporated, and the obtained solid was crystallized from n-hexane to give the title compound (21.3 g) as a lemon solid.

### Step 3

### Production of 3-chloro-4-hydroxy-5-nitrobenzoyl chloride

1,2-Dimethoxyethane (5 mL) was added to 3-chloro-4-hydroxy-5-nitrobenzoic acid (1 g) to dissolve same by heating the mixture to 70°C. Thionyl chloride (0.436 mL) was added, and the mixture was stirred overnight at 70°C. The reaction mixture was concentrated under reduced pressure, azeotroped with toluene, and dried to give the title compound (1.10 g) as a yellow solid.

### Step 4

### Production of (3-chloro-4-hydroxy-5-nitrophenyl)-(2,3-dihydrobenzo[1,4]oxazin-4-yl)-methanone

3,4-Dihydro-2H-benzo[1,4]oxazine (270 mg) obtained in Step 2 of Example 1 and 3-chloro-4-hydroxy-5-nitrobenzoyl chloride (519 mg) were dissolved in ethyl acetate (6 mL), and the mixture was stirred overnight at 95°C. The reaction mixture was allowed to cool to room temperature, and the precipitated solid was collected by filtration to give the title compound (537 mg) as yellow crystals.

### Example 32

### Production of (3,5-dichloro-4-hydroxyphenyl)-(2,8-diisopropyl-2,3-dihydrobenzo[1,4]oxazin-4-yl)-methanone

### Step 1

### Production of 4-bromo-2-isopropylphenol

2-Isopropylphenol (25 g) was dissolved in acetic acid (250 mL) and 48% aqueous hydrogen bromide (125 mL), and dimethyl sulfoxide (125 mL) was added dropwise at room temperature. After stirring at room temperature for 2 hrs, the reaction mixture was neutralized with sodium carbonate (257 g). Water was added, and the mixture was extracted with ethyl ether. The obtained ethyl ether layer was washed successively with water and saturated brine, and dried over anhydrous sodium sulfate. The solvent was evaporated to give the title compound (40.2 g) as a pale-yellow solid.

### Step 2

### Production of 4-bromo-2-isopropyl-6-nitrophenol

To a mixture of 4-bromo-2-isopropylphenol (40.2 g) produced in Step 1 and sodium nitrite (41.5 g) were added n-hexane (300 mL), isopropyl ether (130 mL) and water (200 mL), and 4.5N sulfuric acid (430 mL) was added dropwise at room temperature. After stirring at room temperature for 1 hr, the reaction mixture was washed successively with saturated aqueous sodium hydrogencarbonate, water and saturated brine, and dried over anhydrous sodium sulfate. The solvent was evaporated, and the obtained residue was purified by silica gel chromatography (n-hexane-ethyl acetate=20:1) to give the title compound (41.1-g) as a yellow oil.

### Step 3

### Production of 2-amino-6-isopropylphenol hydrobromide

4-Bromo-2-isopropyl-6-nitrophenol (41.1 g) was dissolved in methanol (300 mL). 7.5% Palladium-carbon (8 g) was added to this solution and, under a hydrogen atmosphere (2 kgf/cm²) , the mixture was stirred overnight at room temperature. The reaction mixture was filtered through celite, and the filtrate was concentrated under reduced pressure. The obtained solid was crystallized from ethyl acetate to give the title compound (27.4 g) as a beige solid.

### Step 4

### Production of 2-bromo-3-methylbutyryl chloride

Chloroform (200 mL) was added to 2-bromo-3-methylbutyric acid (25 g), and oxalyl chloride (14.5 mL) and N,N-dimethylformamide (3 drops) were added under ice-cooling. After stirring overnight at room temperature, the reaction mixture was concentrated under reduced pressure, and azeotroped with toluene to give the title compound as a yellow oil.

### Step 5

### Production of 2-bromo-N-(2-hydroxy-3-isopropylphenyl)-3-methylbutyramide

Ethyl acetate (70 mL) and water (80 mL) were added to 2-amino-6-isopropylphenol hydrobromide (6.14 g) obtained in Step 3 and dissolved therein. Sodium hydrogencarbonate (6.68 g) and 2-bromo-3-methylbutyryl chloride (5.80 g) obtained in Step 4 were added at room temperature, and the mixture was stirred at room temperature for 2 hrs. The reaction mixture was partitioned. The obtained ethyl acetate layer was washed successively with saturated aqueous sodium hydrogencarbonate, water and saturated brine, and dried over anhydrous sodium sulfate. The solvent was evaporated to give the title compound (9.0 g) as a pale-pink solid.

### Step 6

### Production of 2,8-diisopropyl-4H-benzo[1,4]oxazin-3-one

2-Bromo-N-(2-hydroxy-3-isopropylphenyl)-3-methylbutyramide (9.0 g) was dissolved in N,N-dimethylformamide (45 mL), potassium carbonate (4.94 g) was added, and the mixture was stirred overnight at room temperature. The reaction mixture was neutralized with 1N hydrochloric acid, and water was added. The precipitated solid was collected by filtration. The obtained solid was crystallized from n-hexane-isopropyl ether to give the title compound (4.5 g) as a white solid.

### Step 7

### Production of 2,8-diisopropyl-3,4-dihydro-2H-benzo[1,4]oxazine

2,8-Diisopropyl-4H-benzo[1,4]oxazin-3-one (1.0 g) was dissolved in tetrahydrofuran (10 mL), borane-tetrahydrofuran complex (1M tetrahydrofuran solution, 5.14 mL) was added, and the mixture was heated under reflux for 14.5 hrs. 6N Hydrochloric acid (5 mL) was added, and the mixture was stirred with heating again. The mixture was allowed to cool to room temperature, neutralized with sodium hydrogencarbonate, and the mixture was extracted with ethyl acetate. The obtained ethyl acetate layer was washed with water and saturated brine, and dried over anhydrous sodium sulfate. The solvent was evaporated, and the obtained residue was purified by silica gel chromatography (n-hexane-ethyl acetate=6:1) to give the title compound (768 mg) as a colorless oil.

### Step 8

### Production of (4-benzyloxy-3,5-dichlorophenyl)-(2,8-diisopropyl-2,3-dihydrobenzo[1,4]oxazin-4-yl)-methanone

2,8-Diisopropyl-3,4-dihydro-2H-benzo[1,4]oxazine (150 mg) was dissolved in chloroform (5 mL), and pyridine (0.066 mL) and 4-benzyloxy-3,5-dichlorobenzoyl chloride (227 mg) obtained in Step 3 of Example 8 were added under ice-cooling. After stirring overnight at room temperature, water was added to the reaction mixture, and the mixture was extracted with ethyl acetate. The obtained ethyl acetate layer was washed with water and saturated brine, and dried over anhydrous sodium sulfate. The solvent was evaporated, and the obtained residue was purified by silica gel chromatography (n-hexane-ethyl acetate=9:1) to give the title compound (375 mg) as an oil.

### Step 9

### Production of (3,5-dichloro-4-hydroxyphenyl)-(2,8-diisopropyl-2,3-dihydrobenzo[1,4]oxazin-4-yl)-methanone

(4-Benzyloxy-3,5-dichlorophenyl)-(2,8-diisopropyl-2,3-dihydrobenzo[1,4]oxazin-4-yl)-methanone (370 mg) was dissolved in tetrahydrofuran (5 mL). 7.5% Palladium-carbon (70 mg) was added to this solution and, under a hydrogen atmosphere, the mixture was stirred at room temperature for 0.5 hr. The reaction mixture was filtered through celite, and the residue was washed with tetrahydrofuran. The filtrate and washing solution were combined and the mixture was concentrated under reduced pressure. The obtained solid was crystallized from n-hexane to give the title compound (212 mg) as white crystals.

### Example 33

### Production of (3,5-dichloro-4-hydroxyphenyl)-[6-(pyrrolidine-1-sulfonyl)-2,3-dihydrobenzo[1,4]oxazin-4-yl]-methanone

### Step 1

### Production of 4-methoxy-3-nitrobenzenesulfonyl chloride

1-Methoxy-2-nitrobenzene (11 mL) was added dropwise to chlorosulfonic acid (25 mL) under ice-cooling, and the mixture was stirred at room temperature for 1.5 hrs. The reaction mixture was poured into ice water and the mixture was extracted with ethyl ether. The obtained ethyl ether layer was washed with water, and dried over anhydrous sodium sulfate. The solvent was evaporated to give the title compound (15.1527 g) as a red brown oil.

### Step 2

### Production of 1-(4-methoxy-3-nitrobenzenesulfonyl)pyrrolidine

4-Methoxy-3-nitrobenzenesulfonyl chloride (5.1531 g) was dissolved in pyridine (17 mL), pyrrolidine (2.05 g) was added under ice-cooling, and the mixture was stirred overnight at room temperature. The reaction mixture was concentrated under reduced pressure, the residue was poured into water. The mixture was extracted with chloroform, and dried over anhydrous sodium sulfate. The solvent was evaporated, and the obtained residue was purified by silica gel chromatography (n-hexane-ethyl acetate=1:1) to give the title compound (4.158 g) as a yellow solid.

### Step 3

### Production of 2-nitro-4-(pyrrolidine-l-sulfonyl)phenol

1-(4-Methoxy-3-nitrobenzenesulfonyl)pyrrolidine (2.054 g) was dissolved in dimethyl sulfoxide (40 mL), and 50% aqueous potassium hydroxide (40 mL) was added under ice-cooling. After stirring with heating at 80°C for 6 hrs, the mixture was ice-cooled, and 6N hydrochloric acid (80 mL) was added. The mixture was extracted with chloroform. The extract was washed successively with water and saturated brine, and dried over anhydrous sodium sulfate. The solvent was evaporated to give the title compound (2.198 g) as a yellow solid.

### Step 4

### Production of 2-amino-4-(pyrrolidine-1-sulfonyl)phenol

2-Nitro-4-(pyrrolidine-1-sulfonyl)phenol (2.19 g) was dissolved in tetrahydrofuran (50 mL). 7.5% Palladium-carbon (0.2 g) was added to this solution and, under a hydrogen atmosphere, the mixture was stirred at room temperature for 4 hrs. The reaction mixture was filtered through celite, and the residue was washed with tetrahydrofuran. The filtrate and washing solution were combined and the mixture was concentrated under reduced pressure. The obtained residue was purified by silica gel chromatography (n-hexane-ethyl acetate=1:1) to give the title compound (1.5494 g) as a cream color solid.

### Step 5

### Production of 2-(tert-butyldimethylsilyloxy)-5-(pyrrolidine-1-sulfonyl)phenylamine

2-Amino-4-(pyrrolidine-1-sulfonyl)phenol (1.54 g) was dissolved in N,N-dimethylformamide (9 mL), and imidazole (0.649 g) and tert-butylchlorodimethylsilane (1.254 g) were added under ice-cooling. After stirring at room temperature of 1.5 hrs, the mixture was poured into water, and the mixture was extracted with ethyl ether. The obtained ethyl ether layer was washed with water and saturated brine, dried over anhydrous sodium sulfate, and concentrated. The obtained residue was purified by silica gel chromatography (n-hexane-ethyl acetate=7:1) to give the title compound (2.1549 g) as a white solid.

### Step 6

### Production of 4-benzyloxy-3,5-dichloro-N-[2-hydroxy-5-(pyrrolidine-1-sulfonyl)phenyl]benzamide

2-(tert-Butyldimethylsilyloxy)-5-(pyrrolidine-1-sulfonyl)phenylamine (727.8 mg) was dissolved in methylene chloride (17 mL), pyridine (0.2 mL) and 4-benzyloxy-3,5-dichlorobenzoyl chloride (644 mg) obtained in Step 3 of Example 8 were added under ice-cooling, and the mixture was stirred overnight at room temperature. The reaction mixture was concentrated under reduced pressure, and the residue was dissolved in N,N-dimethylformamide- (4 mL). Potassium carbonate (1.41 g) was added at room temperature. After stirring with heating at 60°C for 1.5 hrs, the mixture was acidified with 10% aqueous citric acid solution and water under ice-cooling. The precipitated solid was collected by filtration to give the title compound (1.0619 g) as a cream color solid.

### Step 7

### Production of (4-benzyloxy-3,5-dichlorophenyl)-[6-(pyrrolidine-5 1-sulfonyl)-2,3-dihydrobenzo[1,4]oxazin-4-yl]-methanone

4-Benzyloxy-3,5-dichloro-N-[2-hydroxy-5-(pyrrolidine-1-sulfonyl)phenyl]benzamide (500 mg) was dissolved in N,N-dimethylformamide (5 mL), and potassium carbonate (332 mg) and 1,2-dibromoethane (0.099 mL) were added at room temperature. After stirring overnight at 70°C, the mixture was poured into water. The mixture was extracted with ethyl acetate. The obtained ethyl acetate layer was washed with water and saturated brine, and dried over anhydrous sodium sulfate. The solvent was evaporated, and the obtained residue was purified by silica gel chromatography (n-hexane-ethyl acetate=2:1) to give the title compound (459.5 mg) as a white amorphous form.

### Step 8

### Production of (3,5-dichloro-4-hydroxyphenyl)-[6-(pyrrolidine-1-sulfonyl)-2,3-dihydrobenzo[1,4]oxazin-4-yl]-methanone

(4-Benzyloxy-3,5-dichlorophenyl)-[6-(pyrrolidine-1-sulfonyl)-2,3-dihydrobenzo[1,4]oxazin-4-yl]-methanone (442.5 mg) was dissolved in tetrahydrofuran (20 mL). 7.5% Palladium-carbon (45 mg) was added to this solution and, under a hydrogen atmosphere, the mixture was stirred at room temperature for 0.5 hr. The reaction mixture was filtered through celite, and the residue was washed with tetrahydrofuran. The filtrate and washing solution were combined and the mixture was concentrated under reduced pressure. The obtained residue was purified by silica gel chromatography (n-hexane-ethyl acetate=1:1) to give the title compound (384.5 mg) as a white amorphous solid.

### Example 34

### Production of 4-(3,5-dichloro-4-hydroxybenzoyl)-3,4-dihydro-2H-benzo[1,4]oxazine-6-sulfonic acid ethylamide

The title compound (341.8 mg) was obtained as a white amorphous solid by a method similar to Steps 2 to 8 of Example 33 and using ethylamine instead of pyrrolidine.

### Example 35

### Production of 4-(3,5-dichloro-4-hydroxybenzoyl)-3,4-dihydro-2H-benzo[1,4]oxazine-6-sulfonic acid dimethylamide

The title compound (379.2 mg) was obtained as a white amorphous solid by a method similar to Steps 2 to 8 of Example 33 and using dimethylamine instead of pyrrolidine.

### Example 36

### Production of (3,5-dichloro-4-hydroxyphenyl)-(2,3-dihydropyrido[3,2-b][1,4]oxazin-4-yl)-methanone

### Step 1

### Production of 3,4-dihydro-2H-pyrido[3,2-b][1,4]oxazine

Lithium aluminum hydride (2 g) was suspended in tetrahydrofuran (80 mL), and 4H-pyrido[3,2-b][1,4]oxazin-3-one (3.956 g) was added under ice-cooling by small portions. After heating under reflux for 2 hrs, water (2 mL), 15% aqueous sodium hydroxide (2 mL) and water (6 mL) were successively added under ice-cooling, and the mixture was stirred at room temperature. The mixture was dried over anhydrous sodium sulfate. The solvent was evaporated, and the residue was purified by silica gel chromatography (n-hexane-ethyl acetate=1:9) to give the title compound (3.407 g) as a white solid.

### Step 2

### Production of (4-benzyloxy-3,5-dichlorophenyl)-(2,3-dihydropyrido[3,2-b][1,4]oxazin-4-yl)-methanone

3,4-Dihydro-2H-pyrido[3,2-b][1,4]oxazine (272 mg) was dissolved in chloroform (15 mL), and triethylamine (0.335 mL) and 4-benzyloxy-3,5-dichlorobenzoyl chloride (662 mg) obtained in Step 3 of Example 8 were added under ice-cooling. The mixture was stirred at room temperature for 12 hrs, and the reaction mixture was purified by silica gel chromatography (n-hexane-ethyl acetate=4:1) to give the title compound (646 mg) as a white solid.

### Step 3

### Production of (3,5-dichloro-4-hydroxyphenyl)-(2,3-dihydropyrido[3,2-b][1,4]oxazin-4-yl)-methanone

(4-Benzyloxy-3,5-dichlorophenyl)-(2,3-dihydropyrido[3,2-b][1,4]oxazin-4-yl)-methanone (415 mg) was dissolved in tetrahydrofuran (10 mL). 7.5% Palladium-carbon (40 mg) was added to this solution and, under a hydrogen atmosphere, the mixture was stirred at room temperature for 0.5 hr. The reaction mixture was filtered through celite, and the residue was washed with tetrahydrofuran. The filtrate and washing solution were combined and the mixture was concentrated under reduced pressure. The obtained solid was crystallized from ethyl acetate-tetrahydrofuran to give the title compound (266 mg) as white crystals.

### Example 37

### Production of 5-(3,5-dichloro-4-hydroxybenzoyl)-1,3,4,5-tetrahydrobenzo[b][1,4]diazepin-2-one

### Step 1

### Production of 1,3,4,5-tetrahydrobenzo[b][1,4]diazepin-2-one

Acrylic acid (8.6 g) and benzene-1,2-diamine (5.4 g) were added to polyphosphoric acid (1.5 g), and the mixture was heated under reflux for 3 hrs. Water (100 mL), chloroform (200 mL) and N,N-dimethylformamide (50 mL) were added to the reaction mixture, and the mixture was extracted with chloroform. The obtained chloroform layer was washed successively with water, saturated aqueous sodium hydrogencarbonate and water, and dried over anhydrous sodium sulfate. The solvent was evaporated and the obtained residue was purified by silica gel chromatography (n-hexane-ethyl acetate=1:4) to give the title compound (490 mg).

### Step 2

### Production of 5-(3,5-dichloro-4-hydroxybenzoyl)-1,3,4,5-tetrahydrobenzo[b] [1,4]diazepin-2-one

1,3,4,5-Tetrahydrobenzo[b][1,4]diazepin-2-one (194.4 mg) and 3,5-dichloro-4-hydroxybenzoyl chloride (177.99 mg) obtained in Step 1 of Example 3 were dissolved in ethyl acetate (3 mL), and the mixture was stirred with heating at 95°C for 12 hrs. The reaction mixture was purified by silica gel chromatography to give the title compound (118 mg) as white crystals.

### Example 38

### Production of (3,5-dichloro-2-hydroxyphenyl)-(2,3-dihydrobenzo[1,4]oxazin-4-yl)-methanone

### Step 1

### Production of 3,5-dichloro-2-hydroxybenzoyl chloride

3,5-Dichloro-2-hydroxybenzoic acid (600 mg) was suspended in toluene (6 mL), and thionyl chloride (0.275 mL) and N,N-dimethylformamide (1 drop) were added. After stirring with heating at 70°C for 2 hrs, and the mixture was concentrated and azeotroped with toluene to give the title compound as a pale-yellow solid.

### Step 2

### Production of (3,5-dichloro-2-hydroxyphenyl)-(2,3-dihydrobenzo[1,4]oxazin-4-yl)-methanone

3,4-Dihydro-2H-benzo[1,4]oxazine (391.7 mg) obtained in Step 2 of Example 1 and 3,5-dichloro-2-hydroxybenzoyl chloride obtained in the previous Step were dissolved in ethyl acetate (6 mL), and the mixture was stirred with heating at 90°C for 1 hr. Ethyl acetate (6 mL) was added to the reaction mixture, and the mixture was washed successively with water, 1N hydrochloric acid, water, saturated aqueous sodium hydrogencarbonate, water and saturated brine, and dried over anhydrous sodium sulfate. The solvent was evaporated, and the obtained residue was purified by silica gel chromatography (n-hexane-ethyl acetate=85:15) to give the title compound (457 mg) as pale-yellow crystals.

### Example 39

### Production of (2,3-dihydrobenzo[1,4]oxazin-4-yl)-(4-hydroxy-3-trifluoromethylphenyl)-methanone

### Step 1

### Production of 4-hydroxy-3-trifluoromethylbenzoyl chloride

4-Hydroxy-3-trifluoromethylbenzoic acid (610 mg) was suspended in toluene (6 mL), and thionyl chloride (0.28 mL) and N,N-dimethylformamide (1 drop) were added. After stirring with heating at 70°C for 2 hrs, the mixture was concentrated and azeotroped with toluene to give the title compound.

### Step 2

### Production of (2,3-dihydrobenzo[1,4]oxazin-4-yl)-(4-hydroxy-3-trifluoromethylphenyl)-methanone

3,4-Dihydro-2H-benzo[1,4]oxazine (400 mg) obtained in Step 2 of Example 1 and 4-hydroxy-3-trifluoromethylbenzoyl chloride obtained in the previous Step were dissolved in ethyl acetate (6 mL), and the mixture was stirred with heating at 90°C for 1 hr. Ethyl acetate was added to the reaction mixture, and the mixture was washed successively with water, 1N hydrochloric acid, water, saturated aqueous sodium hydrogencarbonate and saturated brine, and dried over anhydrous sodium sulfate. The solvent was evaporated, and the obtained solid was crystallized from ethyl acetate to give the title compound (359 mg) as crystals.

### Example 40

### Production of (3-chloro-4-hydroxy-5-methoxyphenyl)-(2,3-dihydrobenzo[1,4]oxazin-4-yl)-methanone

### Step 1

### Production of 3-chloro-4-hydroxy-5-methoxybenzoyl chloride

3-Chloro-4-hydroxy-5-methoxybenzoic acid (598 mg) was suspended in toluene (6 mL), and thionyl chloride (0.28 mL) and N,N-dimethylformamide (1 drop) were added. After stirring with heating at 70°C for 2 hrs, the mixture was concentrated and azeotroped with toluene to give the title compound.

### Step 2

### Production of (3-chloro-4-hydroxy-5-methoxyphenyl)-(2,3-dihydrobenzo[1,4]oxazin-4-yl)-methanone

3,4-Dihydro-2H-benzo[1,4]oxazine (400 mg) obtained in Step 2 of Example 1 and 3-chloro-4-hydroxy-5-methoxybenzoyl chloride obtained in the previous Step were dissolved in ethyl acetate (6 mL), and the mixture was stirred with heating at 90°C for 1 hr. Ethyl acetate was added to the reaction mixture, and the mixture was washed successively with water, 1N hydrochloric acid, water, saturated aqueous sodium hydrogencarbonate, water and saturated brine, and dried over anhydrous sodium sulfate. The solvent was evaporated, and the obtained solid was crystallized from ethyl acetate to give the title compound (428 mg) as crystals.

### Example 41

### Production of (4-chloro-3-hydroxyphenyl)-(2,3-dihydrobenzo[1,4]oxazin-4-yl)-methanone

### Step 1

### Production of 4-chloro-3-hydroxybenzoyl chloride

4-Chloro-3-hydroxybenzoic acid (510 mg) was suspended in toluene (6 mL), and thionyl chloride (0.28 mL) and N,N-dimethylformamide (1 drop) were added. After stirring with heating at 70°C for 2 hrs, and the mixture was concentrated and azeotroped with toluene to give the title compound.

### Step 2

### Production of (4-chloro-3-hydroxyphenyl)-(2,3-dihydrobenzo[1,4]oxazin-4-yl)-methanone

3,4-Dihydro-2H-benzo[1,4]oxazine (400 mg) obtained in Step 2 of Example 1 and 4-chloro-3-hydroxybenzoyl chloride obtained in the previous Step were dissolved in ethyl acetate (6 mL), and the mixture was stirred with heating at 90°C for 1 hr. Ethyl acetate was added to the reaction mixture, and the mixture was washed successively with water, 1N hydrochloric acid, water, saturated aqueous sodium hydrogencarbonate, water and saturated brine, and dried over anhydrous sodium sulfate. The solvent was evaporated, and the obtained solid was crystallized from ethyl acetate to give the title compound (583 mg) as crystals.

### Example 42

### Production of (2,6-dichloropyridin-4-yl)-(2,3-dihydrobenzo[1,4]oxazin-4-yl)-methanone

3,4-Dihydro-2H-benzo[1,4]oxazine (270 mg) obtained in Step 2 of Example 1, 2,6-dichloroisonicotinoic acid (384 mg) and 4-dimethylaminopyridine (269 mg) were dissolved in chloroform (12 mL), and WSC·HC1 (422 mg) was added under ice-cooling. After stirring overnight at room temperature, the reaction mixture was purified by silica gel chromatography (n-hexane-ethyl acetate=4:1) to give the title compound (419.8 mg) as pale-yellow crystals.

### Example 43

### Production of (2,3-dihydrobenzo[1,4]oxazin-4-yl)-(4-nitrophenyl)-methanone

3,4-Dihydro-2H-benzo[1,4]oxazine (135 mg) obtained in Step 2 of Example 1 and 4-nitrobenzoyl chloride (185 mg) were dissolved in ethyl acetate (3 mL), and the mixture was stirred overnight with heating at 95°C. The reaction mixture was purified by silica gel chromatography (n-hexane-ethyl acetate=5:1) to give the title compound (288.1 mg) as a yellow solid.

### Example 44

### Production of (3,5-dichloro-4-hydroxyphenyl)-(6-fluoro-2,3-dihydrobenzo[1,4]oxazin-4-yl)-methanone

### Step 1

### Production of 6-fluoro-3,4-dihydro-2H-benzo[1,4]oxazine

6-Fluoro-4H-benzo[1,4]oxazin-3-one (1.5 g) was dissolved in tetrahydrofuran (20 mL), borane-tetrahydrofuran complex (1M tetrahydrofuran solution, 11 mL) was added under ice-cooling, and the mixture was stirred overnight at room temperature. 6N Hydrochloric acid (5 mL) was added, and the mixture was stirred with heating at 70°C. The mixture was allowed to cool to room temperature, weak-alkalified with 4N aqueous sodium hydroxide and saturated aqueous sodium hydrogencarbonate, and the mixture was extracted with ethyl acetate. The obtained ethyl acetate layer was washed successively with water, saturated aqueous sodium hydrogencarbonate, water and saturated brine, and dried over anhydrous sodium sulfate. The solvent was evaporated, and the obtained residue was purified by silica gel chromatography (n-hexane-ethyl acetate=4:1) to give the title compound (954 mg) as an oil.

### Step 2

### Production of (3,5-dichloro-4-hydroxyphenyl)-(6-fluoro-2,3-dihydrobenzo[1,4]oxazin-4-yl)-methanone

6-Fluoro-3,4-dihydro-2H-benzo[1,4]oxazine (230 mg) and 3,5-dichloro-4-hydroxybenzoyl chloride (338 mg) obtained in Step 1 of Example 3 were dissolved in ethyl acetate (3.5 mL), and the mixture was stirred overnight at 95°C. The reaction mixture was allowed to cool to room temperature, and the precipitated solid was collected by filtration to give the title compound (374.9 mg) as pale-beige crystals.

### Example 45

### Production of 2-(2,3-dihydrobenzo[1,4]oxazine-4-carbonyl)benzoic acid

3,4-Dihydro-2H-benzo[1,4]oxazine (300 mg) obtained in Step 2 of Example 1 and phthalic anhydride (329 mg) were dissolved in toluene (3 mL), and the mixture was heated under reflux for 4 hrs. The reaction mixture was allowed to cool, and the precipitated solid was collected by filtration to give the title compound (519 mg) as crystals.

### Example 46.

### Production of methyl 4-(2,3-dihydrobenzo[1,4]oxazine-4-carbonyl)benzoate

3,4-Dihydro-2H-benzo[1,4]oxazine (154 mg) obtained in Step 2 of Example 1 was dissolved in chloroform (6 mL), triethylamine (0.18 mL) and methyl 4-chlorocarbonylbenzoate (226 mg) was added under ice-cooling. After stirring overnight at room temperature, the reaction mixture was purified by silica gel chromatography (n-hexane-ethyl acetate=7:2) to give the title compound (287.4 mg) as a pale-orange solid.

### Example 47

### Production of 4-(2,3-dihydrobenzo[1,4]oxazine-4-carbonyl)benzoic acid

Methyl 4-(2,3-dihydrobenzo[1,4]oxazine-4-carbonyl)benzoate (283 mg) obtained in Example 46 was dissolved in methanol (10 mL) and tetrahydrofuran (5 mL), and 4N aqueous lithium hydroxide (1.5 mL) was added. After stirring overnight at room temperature, the reaction mixture was concentrated under reduced pressure, and the residue was acidified with 1N hydrochloric acid under ice-cooling. The precipitated solid was collected by filtration to give the title compound (207.6 mg) as a white solid.

### Example 48

### Production of methyl 3-(2,3-dihydrobenzo[1,4]oxazine-4-carbonyl)benzoate

### Step 1

### Production of methyl 3-chlorocarbonylbenzoate

Chloroform (5 mL) was added to isophthalic acid monomethyl ester (198 mg), and oxalyl chloride (0.12 mL) and N,N-dimethylformamide (1 drop) were added under ice-cooling. After stirring at room temperature for 7 hrs, the mixture was concentrated and azeotroped with toluene to give the title compound.

### Step 2

### Production of methyl 3-(2,3-dihydrobenzo[1,4]oxazine-4-carbonyl)benzoate

3,4-Dihydro-2H-benzo[1,4]oxazine (154 mg) obtained in Step 2 of Example 1 was dissolved in chloroform (6 mL), and triethylamine (0.18 mL) and methyl 3-chlorocarbonylbenzoate obtained in the previous Step were added under ice-cooling. After stirring overnight at room temperature, the reaction mixture was purified by silica gel chromatography (n-hexane-ethyl acetate=3:1) to give the title compound (323.7 mg) as a white solid.

### Example 49

### Production of 3-(2,3-dihydrobenzo[1,4]oxazine-4-carbonyl)benzoic acid

Methyl 3-(2,3-dihydrobenzo[1,4]oxazine-4-carbonyl)benzoate (293 mg) obtained in Step 2 of Example 48 was dissolved in methanol (10 mL) and tetrahydrofuran (5 mL), and 4N aqueous lithium hydroxide (1.5 mL) was added. After stirring overnight at room temperature, the reaction mixture was concentrated under reduced pressure, and the residue was acidified with 1N hydrochloric acid under ice-cooling. The precipitated solid was collected by filtration, and the obtained solid was purified by silica gel chromatography (chloroform-methanol=10:1) to give the title compound (71.8 mg) as a white amorphous solid.

### Example 50

### Production of (3,5-dichloro-2,4-dihydroxyphenyl)-(2,3-dihydrobenzo[1,4]oxazin-4-yl)-methanone

### Step 1

### Production of 3,5-dichloro-2,4-dihydroxybenzoic acid

2,4-Dihydroxybenzoic acid (25.0 g) was dissolved in ethyl acetate (400 mL), tert-butyl hypochlorite (61.9 g) was added dropwise under ice-cooling, and the mixture was stirred for 2 hrs. The reaction mixture was washed with saturated brine, dried over anhydrous sodium sulfate, and concentrated. The obtained residue was crystallized from ethyl ether-n-hexane to give the title compound (11.88 g) as a solid.

### Step 2

### Production of 3,5-dichloro-2,4-dihydroxybenzoyl chloride

3,5-Dichloro-2,4-dihydroxybenzoic acid (605 mg) was suspended in toluene (6 mL), and thionyl chloride (0.25 mL) and N,N-dimethylformamide (1 drop) were added. After heating under reflux for 1 hr, the mixture was concentrated and azeotroped with toluene to give the title compound.

### Step 3

### Production of (3,5-dichloro-2,4-dihydroxyphenyl)-(2,3-dihydrobenzo[1,4]oxazin-4-yl)-methanone.

3,4-Dihydro-2H-benzo[1,4]oxazine (366 mg) obtained in Step 2 of Example 1 and 3,5-dichloro-2,4-dihydroxybenzoyl chloride obtained in the previous Step were dissolved in ethyl acetate (6 mL), and the mixture was heated under reflux for 1 hr. The reaction mixture was washed successively with water, 1N hydrochloric acid, water, saturated aqueous sodium hydrogencarbonate, water and saturated brine, and dried over anhydrous sodium sulfate. The solvent was evaporated, and the obtained residue was crystallized from ethyl acetate to give the title compound (171 mg) as crystals.

### Example 51

### Production of (6-chloro-2,3-dihydrobenzo[1,4]oxazin-4-yl)-(3,5-dichloro-4-hydroxyphenyl)-methanone

### Step 1

### Production of 2-chloro-N-(5-chloro-2-hydroxyphenyl)-acetamide

2-Amino-4-chlorophenol (1.0061 g) was dissolved in ethyl acetate (10 mL), water (10 mL) and sodium hydrogencarbonate (1.185 g) were added under ice-cooling, and chloroacetyl chloride (0.67 mL) was added dropwise. After stirring at room temperature for 2.5 hrs, water (20 mL) was added, and the mixture was extracted with ethyl acetate. The obtained ethyl acetate layer was washed with saturated brine, and dried over anhydrous sodium sulfate. The solvent was evaporated to give the title compound (1.5455 g) as a yellow solid.

### Step 2

### Production of 6-chloro-4H-benzo[1,4]oxazin-3-one

2-Chloro=N-(5-chloro-2-hydroxyphenyl)-acetamide (1.54 g) was dissolved in N,N-dimethylformamide (15 mL), and potassium carbonate (1.26 g) was added at room temperature. After stirring overnight at room temperature, 1N hydrochloric acid (12 mL) was added under ice-cooling, and the mixture was extracted with ethyl acetate. The obtained ethyl acetate layer was washed with water and saturated brine, and dried over anhydrous sodium sulfate. The solvent was evaporated to give the title compound (1.281 g) as a pale-yellow solid.

### Step 3

### Production of 6-chloro-3,4-dihydro-2H-benzo[1,4]oxazine

6-Chloro-4H-benzo[1,4]oxazin-3-one (764 mg) was dissolved in tetrahydrofuran (8 mL), borane-tetrahydrofuran complex (1M tetrahydrofuran solution, 6.2 mL) was added under ice-cooling, and the mixture was stirred overnight at room temperature. After stirring with heating at 70°C for 1 hr, methanol (3 mL) was added dropwise at the same temperature, and the mixture was further stirred with heating for 1 hr. Then, while maintaining at 70°C, 1N hydrochloric acid (6.2 mL) was added dropwise and, after stirring with heating for 0.5 hr, the mixture was allowed to cool to room temperature. The mixture was extracted with ethyl acetate, and the obtained ethyl acetate layer was washed with water and saturated brine, and dried over anhydrous sodium sulfate. The solvent was evaporated, and the obtained residue was purified by silica gel chromatography (chloroform-methanol=99:1) to give the title compound (640.1 mg) as a solid.

### Step 4

### Production of (6-chloro-2,3-dihydrobenzo[1,4]oxazin-4-yl)-(3,5-dichloro-4-hydroxyphenyl)-methanone

6-Chloro-3,4-dihydro-2H-benzo[1,4]oxazine (168.4 mg) and 3,5-dichloro-4-hydroxybenzoyl chloride (242.7 mg) obtained in Step 1 of Example 3 were dissolved in ethyl acetate (3 mL), and the mixture was heated under reflux overnight. The solvent was evaporated, and the obtained solid was crystallized from methanol to give the title compound (318.4 mg) as white crystals.

### Example 52

### Production of (7-chloro-2,3-dihydrobenzo[1,4]oxazin-4-yl)-(3,5-dichloro-4-hydroxyphenyl)-methanone

The title compound (749.3 mg) was obtained as white crystals by a method similar to Steps 1 to 4 of Example 51 and using 2-amino-5-chlorophenol instead of 2-amino-4-chlorophenol.

### Example 53

### Production of [4-(3,5-dichloro-4-hydroxybenzoyl)-3,4-dihydro-2H-quinoxalin-1-yl]-(3,5-dichloro-4-hydroxyphenyl)-methanone

1,2,3,4-Tetrahydroquinoxaline (68.1 mg) and 3,5-dichloro-4-hydroxybenzoyl chloride (250.5 mg) obtained in Step 1 of Example 3 were dissolved in ethyl acetate (5 mL), and the mixture was heated under reflux overnight. The solvent was evaporated, and the obtained solid was crystallized from methanol to give the title compound (122.0 mg) as pale-gray crystals.

### Example 54

### Production of (3,5-dichloro-4-hydroxyphenyl)-(3,4-dihydro-2H-quinoxalin-1-yl)-methanone

### Step 1

### Production of (4-benzyloxy-3,5-dichlorophenyl)-(3,4-dihydro-2H-quinoxalin-1-yl)-methanone

1,2,3,4-Tetrahydroquinoxaline (805.3 mg) was dissolved in ethyl acetate (30 mL), triethylamine (1.0 mL) and 4-benzyloxy-3,5-dichlorobenzoyl chloride (1.8988 g) obtained in Step 3 of Example 8 were added under ice-cooling, and the mixture was stirred overnight at room temperature. Methanol was added to the reaction mixture, and the mixture was concentrated. The obtained solid was crystallized from ethyl acetate-water to give the title compound (2.042 g) as a pale-yellow solid.

### Step 2

### Production of (3,5-dichloro-4-hydroxyphenyl)-(3,4-dihydro-2H= quinoxalin-1-yl)-methanone

(4-Benzyloxy-3,5-dichlorophenyl)-(3,4-dihydro-2H-quinoxalin-1-yl)-methanone (287.7 mg) was dissolved in tetrahydrofuran (6 mL). 7.5% Palladium-carbon (26 mg) was added to this solution and, under a hydrogen atmosphere, the mixture was stirred at room temperature for 1 hr. The reaction mixture was filtered through celite, and the residue was washed with tetrahydrofuran. The filtrate and washing solution were combined and the mixture was concentrated under reduced pressure. The obtained solid was crystallized from methanol to give the title compound (194.1 mg) as pale-yellow crystals.

### Example 55

### Production of methyl 4-(3,5-dichloro-4-hydroxybenzoyl)-3,4-dihydro-2H-benzo[1,4]oxazine-6-carboxylate

### Step 1

### Production of methyl 4-hydroxy-3-nitrobenzoate

Methanol (50 mL) and concentrated sulfuric acid (0.5 mL) were added to 4-hydroxy-3-nitrobenzoic acid (5.0022 g), and the mixture was stirred overnight at 80°C. The solvent was evaporated, and the residue was partitioned between ethyl acetate and saturated aqueous sodium hydrogencarbonate. The aqueous layer was acidified with 1N hydrochloric acid, and the mixture was extracted with ethyl acetate. The obtained ethyl acetate layer was washed with water and saturated brine, and dried over anhydrous sodium sulfate. The solvent was evaporated to give the title compound (5.3436 g) as a yellow solid.

### Step 2

### Production of methyl 3-amino-4-hydroxybenzoate

Methyl 4-hydroxy-3-nitrobenzoate (5.3436 g) was dissolved in tetrahydrofuran (27 mL) and methanol (27 mL). 7.5% Palladium-carbon (276.6 mg) was added to this solution and, under a hydrogen atmosphere, the mixture was stirred overnight at room temperature. The reaction mixture was filtered through celite, and the residue was washed with tetrahydrofuran. The filtrate and washing solution were combined and the mixture was concentrated under reduced pressure to give the title compound (4.6803 g) as a solid.

### Step 3

### Production of methyl 3-oxo-3,4-dihydro-2H-benzo[1,4]oxazine-6-carboxylate

Methyl 3-amino-4-hydroxybenzoate (4.6803 g) and benzyltriethylammonium chloride (6.1787 g) were suspended in chloroform (50 mL), sodium hydrogencarbonate (9.10 g) and chloroacetyl chloride (2.6 mL) were added under ice-cooling, and the mixture was stirred under ice-cooling for 1 hr. Thereafter, the mixture was stirred with heating at 70°C. The reaction mixture was concentrated, water and ethyl acetate were added, and the precipitated solid was collected by filtration. The mother liquor was extracted with ethyl acetate, and the obtained ethyl acetate layer was washed with water and saturated brine, and dried over anhydrous sodium sulfate. The solvent was evaporated, and the obtained residue and the solid collected earlier by filtration were combined and the mixture was crystallized from methanol to give the title compound (4.8085 g) as a solid.

### Step 4

### Production of methyl 3,4-dihydro-2H-benzo[1,4]oxazine-6-carboxylate

Methyl 3-oxo-3,4-dihydro-2H-benzo[1,4]oxazine-6-carboxylate (1.009 g) was added to borane-tetrahydrofuran complex (1M tetrahydrofuran solution, 11 mL) under ice-cooling, and the mixture was stirred with heating at 70°C for 3.5 hrs. Methanol (3 mL) was added dropwise and the mixture was further stirred with heating for 3 hrs. The mixture was extracted with ethyl acetate, and the obtained ethyl acetate layer was washed with saturated brine, and dried over anhydrous sodium sulfate. The solvent was evaporated, and the obtained residue was purified by silica gel chromatography (chloroform-methanol=98:2) to give the title compound (281.3 mg) as a pink solid and (3,4-dihydro-2H-benzo [1,4]oxazin-6-yl) methanol (413.8 mg) as an oil.

### Step 5

### Production of methyl 4-(3,5-dichloro-4-hydroxybenzoyl)-3,4-dihydro-2H-benzo[1,4]oxazine-6-carboxylate

Methyl 3,4-dihydro-2H-benzo[1,4]oxazine-6-carboxylate (81.2 mg) was dissolved in ethyl acetate (3 mL), 3,5-dichloro-4-hydroxybenzoyl chloride (108.3 mg) obtained in Step 1 of Example 3 was added under ice-cooling, and the mixture was stirred overnight at 80°C. The solvent was evaporated, and the obtained residue was purified by silica gel chromatography (chloroform-methanol=95:5) to give the title compound (167.8 mg) as a white amorphous solid.

### Example 56

### Production of (3,5-dichloro-4-hydroxyphenyl)-(6-hydroxymethyl-2,3-dihydrobenzo[1,4]oxazin-4-yl)-methanone

### Step 1

### Production of 6-(tert-butyldimethylsilyloxymethyl)-3,4-dihydro-2H-benzo[1,4]oxazine

(3,4-Dihydro-2H-benzo[1,4]oxazin-6-yl)methanol (406.1 mg) obtained in Step 4 of Example 55 was dissolved in N,N-dimethylformamide (4 mL), and imidazole(203.8 mg) and tert-butylchlorodimethylsilane (443.4 mg) were added under ice-cooling. After stirring at room temperature for 1 hr, the mixture was poured into water, and the mixture was extracted with ethyl acetate. The obtained ethyl acetate layer was washed with water and saturated brine, dried over anhydrous sodium sulfate, and concentrated. The obtained residue was purified by silica gel chromatography (n-hexane-ethyl acetate=4:1) to give the title compound (659.0 mg) as a pale-yellow oil.

### Step 2

### Production of [6-(tert-butyldimethylsilyloxymethyl)-2,3-dihydrobenzo[1,4]oxazin-4-yl]-(3,5-dichloro-4-hydroxyphenyl)-methanone

6-(tert-Butyldimethylsilyloxymethyl)-3,4-dihydro-2H-benzo[1,4]oxazine (279.1 mg) was dissolved in ethyl acetate (3 mL), triethylamine (0.167 mL) and 3,5-dichloro-4-hydroxybenzoyl chloride (249.4 mg) obtained in Step 1 of Example 3 were added under ice-cooling, and the mixture was stirred overnight at 80°C. The solvent was evaporated, and water and ethyl acetate were added and the mixture was extracted with ethyl acetate. The obtained ethyl acetate layer was washed with water and saturated brine, dried over anhydrous sodium sulfate, and concentrated. The obtained residue was purified by silica gel chromatography (n-hexane-ethyl acetate=4:1) to give the title compound (131.7 mg) as a pale-yellow solid.

### Step 3

### Production of (3,5-dichloro-4-hydroxyphenyl)-(6-hydroxymethyl-2,3-dihydrobenzo[1,4]oxazin-4-yl)-methanone

[6-(tert-Butyldimethylsilyloxymethyl)-2,3-dihydrobenzo[1,4]oxazin-4-yl]-(3,5-dichloro-4-hydroxyphenyl)-methanone (131.7 mg) was dissolved in tetrahydrofuran (1.5 mL), 1M tetrabutylammonium fluoride/tetrahydrofuran solution (0.34 mL) was added under ice-cooling, and the mixture was stirred overnight at room temperature. The solvent was evaporated, and the,obtained residue was purified by silica gel chromatography (chloroform-methanol=97:3) to give the title compound (50.0 mg) as crystals.

### Example 57

### Production of 4-(3,5-dichloro-4-hydroxybenzoyl)-3,4-dihydro-2H-benzo[1,4]oxazine-6-carboxylic acid

Methyl 4-(3,5-dichloro-4-hydroxybenzoyl)-3,4-dihydro-2H-benzo[1,4]oxazine-6-carboxylate (140.8 mg) obtained in Step 5 of Example 55 was dissolved in methanol (1.5 mL) and tetrahydrofuran (1.5 mL), and 2N aqueous sodium hydroxide (0.55 mL) was added. After stirring overnight at room temperature, the solvent was evaporated, and the residue was acidified with 10% aqueous citric acid solution under ice-cooling. The precipitated solid was collected by filtration, and the obtained solid was crystallized from methanol to give the title compound (81.3 mg) as crystals.

### Example 58

### Production of methyl 4-(3,5-dichloro-4-hydroxybenzoyl)-3,4-dihydro-2H-benzo[1,4]oxazine-5-carboxylate

The title compound (246.0 mg) was obtained as a white amorphous solid by a method similar to Steps 1 to 5 of Example 55 and using 3-hydroxy-2-nitrobenzoic acid instead of 4-hydroxy-3-nitrobenzoic acid.

### Example 59

### Production of methyl 4-(3,5-dichloro-4-hydroxybenzoyl)-3,4-dihydro-2H-benzo[1,4]oxazine-7-carboxylate

The title compound (317.9 mg) was obtained as white crystals by a method similar to Steps 1 to 5 of Example 55 and using 3-hydroxy-4-nitrobenzoic acid instead of 4-hydroxy-3-nitrobenzoic acid.

### Example 60

### Production of 4-(3,5-dichloro-4-hydroxybenzoyl)-3,4-dihydro-2H-benzo [1,4]oxazine-7-carboxylic acid

Methyl 4-(3,5-dichloro-4-hydroxybenzoyl)-3,4-dihydro-2H-benzo[1,4]oxazine-7-carboxylate (257.4 mg) obtained in Example 59 was dissolved in methanol (2.5 mL) and tetrahydrofuran (2.5 mL), and 2N aqueous sodium hydroxide (1.0 mL) was added. After stirring with heating at 60°C for 2.5 hrs, the solvent was evaporated, and the residue was acidified with 10% aqueous citric acid solution under ice-cooling. The precipitated solid was collected by filtration, and the obtained solid was crystallized from methanol to give the title compound (76.6 mg) as crystals.

### Example 61

### Production of methyl 4-(3,5-dichloro-4-hydroxybenzoyl)-3,4-dihydro-2H-benzo[1,4]oxazine-8-carboxylate

The title compound (313.8 mg) was obtained as white crystals by a method similar to Steps 1 to 5 of Example 55 and using 2-hydroxy-3-nitrobenzoic acid instead of 4-hydroxy-3-nitrobenzoic acid.

### Example 62

### Production of 4-(3,5-dichloro-4-hydroxybenzoyl)-3,4-dihydro-2H-benzo[1,4]oxazine-8-carboxylic acid

Methyl 4-(3,5-dichloro-4-hydroxybenzoyl)-3,4-dihydro-2H-benzo [1,4] oxazine-8-carboxylate (244.2 mg) obtained in Example 61 was dissolved in methanol (2.5 mL) and tetrahydrofuran (2.5 mL), and 2N aqueous sodium hydroxide (0.96 mL) was added. After stirring overnight at room temperature, the solvent was evaporated, and the residue was acidified with 10% aqueous citric acid solution under ice-cooling. The precipitated solid was collected by filtration, and the obtained solid was crystallized from ethyl acetate to give the title compound (186.6 mg) as crystals.

### Example 63

### Production of (3,5-dichlorophenyl)-(2,3-dihydrobenzo[1,4]oxazin-4-yl)-methanone

3,4-Dihydro-2H-benzo[1,4]oxazine (250.1 mg) obtained in Step 2 of Example 1 and 3,5-dichlorobenzoyl chloride (426.9 mg) were dissolved in ethyl acetate (5 mL), and the mixture was stirred overnight with heating at 80°C. The solvent was evaporated, and the obtained residue was crystallized from methanol to give the title compound (469.6 mg) as pale-beige crystals.

### Example 64

### Production of (3,5-dichloro-4-hydroxyphenyl)-phenoxazin-10-ylmethanone

Phenoxazine (276.1 mg) and 3,5-dichloro-4-hydroxybenzoyl chloride (370.8 mg) obtained in Step 1 of Example 3 were dissolved in ethyl acetate (3 mL), and the mixture was stirred overnight with heating at 80°C. The solvent was evaporated, and the obtained residue was purified by silica gel chromatography (n-hexane-ethyl acetate=4:1) to give the title compound (397.6 mg) as a yellow green solid.

### Example 65

### Production of (3,5-dichloro-4-hydroxyphenyl)-(6-phenyl-2,3-dihydrobenzo[1,4]oxazin-4-yl)-methanone

The title compound (160.0 mg) was obtained as white crystals by a method similar to Steps 2 to 5 of Example 21 and using 2-amino-4-phenylphenol instead of 3-amino-N,N-diethyl-4-hydroxybenzenesulfonamide.

### Example 66

### Production of (3,5-dichloro-4-hydroxyphenyl)-(6,8-dimethyl-2,3-dihydrobenzo[1,4]oxazin-4-yl)-methanone

The title compound (152.7 mg) was obtained as crystals by a method similar to Steps 1 to 4 of Example 51 and using 2-amino-4,6-dimethylphenol instead of 2-amino-4-chlorophenol.

### Example 67

### Production of (3,5-dichloro-4-hydroxyphenyl)-(6-nitro-2,3-dihydrobenzo[1,4]oxazin-4-yl)-methanone

### Step 1

### Production of 6-nitro-4H-benzo[1,4]oxazin-3-one

2-Amino-4-nitrophenol (4.6283 g) and benzyltriethylammonium chloride (6.8772 g) were suspended in chloroform (46 mL), sodium hydrogencarbonate (10.10 g) and chloroacetyl chloride (4.0707 g) were added under ice-cooling, and the mixture was stirred for 1 hr under ice-cooling. Thereafter, the mixture was stirred with heating at 70°C. The reaction mixture was concentrated, water and ethyl acetate were added, and the precipitated solid was collected by filtration. The mother liquor was extracted with ethyl acetate, and the obtained ethyl acetate layer was washed with water and saturated brine, and dried over anhydrous sodium sulfate. The solvent was evaporated, the obtained residue and the solid collected earlier by filtration were combined and the mixture was crystallized from ethanol to give the title compound (5.4344 g) as a solid.

### Step 2

### Production of 6-nitro-3,4-dihydro-2H-benzo[1,4]oxazine

To borane-tetrahydrofuran complex (1M tetrahydrofuran solution, 33.5 mL) was added 6-nitro-4H-benzo[1,4]oxazin-3-one (3.0084 g) under ice-cooling, and the mixture was stirred with heating at 70°C for 5 hrs. Methanol (5 mL) was added dropwise, and the mixture was further stirred with heating for 2.5 hrs. Concentrated hydrochloric acid (5 mL) was added dropwise, and the mixture was stirred with heating for 1.5 hrs. The mixture was extracted with ethyl acetate, and the obtained ethyl acetate layer was washed with water and saturated brine, and dried over anhydrous sodium sulfate. The solvent was evaporated, and the obtained residue was crystallized from n-hexane-ethyl acetate to give the title compound (2.2092 g) as a solid.

### Step 3

### Production of (3,5-dichloro-4-hydroxyphenyl)-(6-nitro-2,3-dihydrobenzo[1,4]oxazin-4-yl)-methanone

6-Nitro-3,4-dihydro-2H-benzo[1,4]oxazine (376.1 mg) was dissolved in ethyl acetate (3 mL), 3,5-dichloro-4-hydroxybenzoyl chloride (270.7 mg) obtained in Step 1 of Example 3 was added under ice-cooling, and the mixture was stirred overnight at 80°C. The reaction mixture was allowed to cool to room temperature, and the precipitated solid was collected by filtration to give the title compound (538.0 mg) as pale-yellow crystals.

### Example 68

### Production of (6-amino-2,3-dihydrobenzo[1,4]oxazin-4-yl)-(3,5-dichloro-4-hydroxyphenyl)-methanone

(3,5-Dichloro-4-hydroxyphenyl)-(6-nitro-2,3-dihydrobenzo[1,4]oxazin-4-yl)-methanone (200.0 mg) obtained in Step 3 of Example 67 was dissolved in tetrahydrofuran (12 mL). 7.5% Palladium-carbon (19.4 mg) was added to this solution and, under a hydrogen atmosphere, the mixture was stirred overnight at room temperature. The reaction mixture was filtered through celite, and the residue was washed with tetrahydrofuran. The filtrate and washing solution were combined and the mixture was concentrated under reduced pressure. The obtained residue was purified by silica gel chromatography (chloroform-methanol=95:5) to give the title compound (6.4 mg) as crystals.

### Example 69

### Production of (3,5-dibromo-4-hydroxyphenyl)-(6-nitro-2,3-dihydrobenzo[1,4]oxazin-4-yl)-methanone

6-Nitro-3,4-dihydro-2H-benzo[1,4]oxazine (179.8 mg) obtained in Step 2 of Example 67 was dissolved in ethyl acetate (5 mL), 3,5-dibromo-4-hydroxybenzoyl chloride (346.6 mg) obtained in Step 1 of Example 4 was added under ice-cooling, and the mixture was stirred overnight at 80°C. The reaction mixture was allowed to cool to room temperature, and the precipitated solid was collected by filtration to give the title compound (437.0 mg) as yellow crystals.

### Example 70

### Production of (3,5-dichloro-4-hydroxyphenyl)-(7-nitro-2,3-dihydrobenzo[1,4]oxazin-4-yl)-methanone

The title compound (485.2 mg) was obtained as yellow crystals by a method similar to Steps 1 to 3 of Example 67 and using 2-amino-5-nitrophenol instead of 2-amino-4-nitrophenol.

### Example 71

### Production of (7-amino-2,3-dihydrobenzo[1,4]oxazin-4-yl)-(3,5-dichloro-4-hydroxyphenyl)-methanone

(3,5-Dichloro-4-hydroxyphenyl)-(7-nitro-2,3-dihydrobenzo[1,4]oxazin-4-yl)-methanone (303.1 mg) obtained in Example 70 was dissolved in tetrahydrofuran (6 mL) and methanol (3 mL). 7.5% Palladium-carbon (30 mg) was added to this solution and, under a hydrogen atmosphere, and the mixture was stirred at room temperature for 1.5 hrs. The reaction mixture was filtered through celite, and the residue was washed with tetrahydrofuran. The filtrate and washing solution were combined and the mixture was concentrated under reduced pressure. The obtained residue was crystallized from ethanol to give the title compound (257.9 mg) as yellow crystals.

### Example 72

### Production of N-[4-(3,5-dichloro-4-hydroxybenzoyl)-3,4-dihydro-2H-benzo[1,4]oxazin-7-yl]-methanesulfonamide

(7-Amino-2,3-dihydrobenzo[1,4]oxazin-4-yl)-(3,5-dichloro-4-hydroxyphenyl)-methanone (251.7 mg) obtained in Example 71 was suspended in methylene chloride (5 mL), pyridine (0.0776 mL) and methanesulfonyl chloride (0.0689 mL) were added under ice-cooling, and the mixture was stirred overnight at room temperature. Methanol was added to the reaction mixture, and the mixture was concentrated under reduced pressure. The obtained residue was purified by silica gel chromatography (chloroform-methanol=95:5) to give the title compound (173.6 mg) as a pale-orange amorphous solid.

### Example 73

### Production of 1-[4-(3,5-dichloro-4-hydroxybenzoyl)-3,4-dihydro-2H-quinoxalin-1-yl]-ethanone

### Step 1

### Production of 1-[4-(4-benzyloxy-3,5-dichlorobenzoyl)-3,4-dihydro-2H-quinoxalin-1-yl]-ethanone

(4-Benzyloxy-3,5-dichlorophenyl)-(3,4-dihydro-2H-quinoxalin-1-yl)-methanone (400 mg) obtained in Step 1 of Example 54 was suspended in methylene chloride (8 mL), triethylamine (0.162 mL) and acetyl chloride (0.082 mL) were added under ice-cooling, and the mixture was stirred overnight at room temperature. Methanol was added to the reaction mixture, and the mixture was concentrated under reduced pressure. Water was added and the mixture was extracted with ethyl acetate. The obtained ethyl acetate layer was washed with water and saturated brine, and dried over anhydrous sodium sulfate. The solvent was evaporated, and the obtained residue was purified by silica gel chromatography (chloroform-methanol=99:1) to give the title compound (295.9 mg) as a pale-yellow amorphous form.

### Step 2

### Production of 1-[4-(3,5-dichloro-4-hydroxybenzoyl)-3,4-dihydro-2H-quinoxalin-1-yl]-ethanone

1-[4-(4-Benzyloxy-3,5-dichlorobenzoyl)-3,4-dihydro-2H-quinoxalin-1-yl]-ethanone (288.6 mg) was dissolved in tetrahydrofuran (6 mL). 7.5% Palladium-carbon (27.7 mg) was added to this solution and, under a hydrogen atmosphere, the mixture was stirred at room temperature for 1 hr. The reaction mixture was filtered through celite, and the residue was washed with tetrahydrofuran. The filtrate and washing solution were combined and the mixture was concentrated under reduced pressure. The obtained residue was purified by silica gel chromatography (chloroform-methanol=97:3) and crystallized from n-hexane-ethyl acetate to give the title compound (108.1 mg) as white crystals.

### Example 74

### Production of (3,5-dichloro-4-hydroxyphenyl)-(4-methyl-3,4-dihydro-2H-quinoxalin-1-yl)-methanone

### Step 1

### Production of (4-benzyloxy-3,5-diclilorophenyl)-(4-methyl-3,4-dihydro-2H-quinoxalin-1-yl)-methanone

(4-Benzyloxy-3,5-dichlorophenyl)-(3,4-dihydro-2H-quinoxalin-1-yl)-methanone (401.6 mg) obtained in Step 1 of Example 54 was dissolved in N,N-dimethylformamide (8 mL), potassium carbonate (335.7 mg) and iodomethane (0.076 mL) were added, and the mixture was stirred with heating at 50°C. The mixture was poured into water, and the mixture was extracted with ethyl acetate. The obtained ethyl acetate layer was washed with water and saturated brine, dried over anhydrous sodium sulfate, and concentrated under reduced pressure. The obtained residue was purified by silica gel chromatography (chloroform-methanol=99:1) to give the title compound (114.7 mg) as an amorphous form.

### Step 2

### Production of (3,5-dichloro-4-hydroxyphenyl)-(4-methyl-3,4-dihydro-2H-quinoxalin-1-yl)-methanone

(4-Benzyloxy-3,5-dichlorophenyl)-(4-methyl-3,4-dihydro-2H-quinoxalin-1-yl)-methanone (114.7 mg) was dissolved in tetrahydrofuran (5 mL). 7.5% Palladium-carbon (10.0 mg) was added to this solution and, under a hydrogen atmosphere, the mixture was stirred at room temperature for 1 hr. The reaction mixture was filtered through celite, and the residue was washed with tetrahydrofuran. The filtrate and washing solution were combined and the mixture was concentrated under reduced pressure. The obtained residue was crystallized from ethyl acetate to give the title compound (56.3 mg) as pale-yellow crystals.

### Example 75

### Production of (2,3-dihydrobenzo[1,4]oxazin-4-yl)-(4-hydroxy-3-nitrophenyl)-methanone

### Step 1

### Production of 4-hydroxy-3-nitrobenzoyl chloride

1,2-Dimethoxyethane (20 mL) was added to 4-hydroxy-3-nitrobenzoic acid (1.83 g) to dissolve same by heating the mixture to 80°C. Thionyl chloride (1.1 mL) was added, and the mixture was stirred overnight at 80°C. The reaction mixture was concentrated under reduced pressure, and azeotroped with toluene to give the title compound (2.0551 g) as a yellow oil.

### Step 2

### Production of (2,3-dihydrobenzo [1,4] oxazin-4-yl)-(4-hydroxy-3-nitrophenyl)-methanone

3,4-Dihydro-2H-benzo[1,4]oxazine (203 mg) obtained in Step 2 of Example 1 and 4-hydroxy-3-nitrobenzoyl chloride (302 mg) were dissolved in ethyl acetate (2 mL), and the mixture was heated under reflux overnight. The reaction mixture was purified by silica gel chromatography (n-hexane-ethyl acetate=4:1) to give the title compound (460.7 mg) as a yellow amorphous solid.

### Example 76

### Production of (3,5-dichloro-4-hydroXyphenyl)-(2-methyl-2,3-dihydroindol-1-yl)-methanone

### Step 1

### Production of (4-benzyloxy-3,5-dichlorophenyl)-(2-methyl-2,3-dihydroindol-1-yl)-methanone

2-Methyl-2,3-dihydro-1H-indole (139.3 mg) was dissolved in methylene chloride (2.5 mL), pyridine (0.1 mL) and 4-benzyloxy-3,5-dichlorobenzoyl chloride (396.6 mg) obtained in Step 3 of Example 8 were added, and the mixture was stirred overnight at room temperature. The reaction mixture was concentrated, water was added, and the mixture was extracted with ethyl acetate. The obtained ethyl acetate layer was washed with water and saturated brine, and dried over anhydrous sodium sulfate. The solvent was evaporated, and the obtained residue was purified by silica gel chromatography (n-hexane-ethyl acetate=6:1) to give the title compound (449.4 mg) as a yellow oil.

### Step 2,

### Production of (3,5-dichloro-4-hydroxyphenyl)-(2-methyl-2,3-dihydroindol-1-yl)-methanone

(4-Benzyloxy-3,5-dichlorophenyl)-(2-methyl-2,3-dihydroindol-1-yl)-methanone (437.1 mg) was dissolved in toluene (4 mL), and trifluoroacetic acid (2.5 mL) was added at room temperature. After stirring with heating at 80°C, the mixture was concentrated. The obtained solid was crystallized from n-hexane-ethyl acetate to give the title compound (300.3 mg) as white crystals.

### Example 77

### Production of (3,5-dichloro-4-hydroxyphenyl)-(2,3-dihydroindol-1-yl)-methanone

The title compound (230.6 mg) was obtained as white crystals by a method similar to Steps 1 and 2 of Example 76 and using 2,3-dihydro-1H-indole instead of 2-methyl-2,3-dihydro-1H-indole.

### Example 78

### Production of (5-amino-2,3-dihydroindol-1-yl)-(3,5-dichloro-4-hydroxyphenyl)-methanone

### Step 1

### Production of (4-benzyloxy-3,5-dichlorophenyl)-(5-nitro-2,3-dihydroindol-1-yl)-methanone

5-Nitro-2,3-dihydro-1H-indole (329.5 mg) was dissolved in methylene chloride (5 mL), pyridine (0.194 mL) and 4-benzyloxy-3,5-dichlorobenzoyl chloride (762.1 mg) obtained in Step 3 of Example 6 were added, and the mixture was stirred overnight at room temperature. The reaction mixture was concentrated, water was added, and the mixture was extracted with ethyl acetate. The obtained ethyl acetate layer was washed with water and saturated brine, and dried over anhydrous sodium sulfate. The solvent was evaporated, and the obtained residue was purified by silica gel chromatography (n-hexane-ethyl acetate=4:1) to give the title compound (539.7 mg) as a yellow solid.

### Step 2

### Production of (5-amino-2,3-dihydroindol-1-yl)-(4-benzyloxy-3,5-dichlorophenyl)-methanone

(4-Benzyloxy-3,5-dichlorophenyl)-(5-nitro-2,3-dihydroindol-1-yl)-methanone (539.7 mg) was dissolved in tetrahydrofuran (10 mL). Platinum oxide (IV) (14 mg) was added to this solution and, under a hydrogen atmosphere, the mixture was stirred overnight at room temperature. The reaction mixture was filtered through celite, and the residue was washed with tetrahydrofuran. The filtrate and washing solution were combined and the mixture was concentrated under reduced pressure. Water was added, and the mixture was extracted with chloroform. The obtained chloroform layer was washed with saturated brine, and dried over anhydrous sodium sulfate. The solvent was evaporated to give a mixture (489.5 mg) of the title compound and (5-amino-2,3-dihydroindol-1-yl)-(3,5-dichloro-4-hydroxyphenyl)-methanone as a yellow amorphous form.

### Step 3

### Production of (5-amino-2,3-dihydroindol-1-yl)-(3,5-dichloro-4-hydroxyphenyl)-methanone

A mixture (489.5 mg) of (5-amino-2,3-dihydroindol-1-yl)-(4-benzyloxy-3,5-dichlorophenyl)-methanone and (5-amino-2,3-dihydroindol-1-yl)-(3,5-dichloro-4-hydroxyphenyl)-methanone was dissolved in toluene (5 mL), and trifluoroacetic acid (2.75 mL) was added at room temperature. After stirring with heating at 80°C for 2.5 hrs, the mixture was concentrated. The obtained solid was crystallized from ethyl acetate to give the title compound (282.4 mg) as pale-gray crystals.

### Example 79

### Production of (3,5-dibromo-4-hydroxyphenyl)-(6-fluoro-2,3-dihydrobenzo[1,4]oxazin-4-yl)-methanone

6-Fluoro-3,4-dihydro-2H-benzo[1,4]oxazine (230 mg) obtained in Step 1 of Example 44 and 3,5-dibromo-4-hydroxybenzoyl chloride (472 mg) obtained in Step 1 of Example 4 were dissolved in ethyl acetate (3.5 mL), and the mixture was stirred overnight at 95°C. The reaction mixture was allowed to cool to room temperature, and the precipitated solid was collected by filtration to give the title compound (533.7 mg) as white crystals.

### Example 80

### Production of (3,5-dibromo-4-hydroxyphenyl)-(2,3-dihydronaphtho[2,1-b][1,4]oxazin-1-yl)-methanone

2,3-Dihydro-1H-naphtho[2,1-b][1,4]oxazine (140.9 mg) obtained in Step 3 of Example 16 and 3,5-dibromo-4-hydroxybenzoyl chloride (239 mg) obtained in Step 1 of Example 4 were dissolved in ethyl acetate (2.3 mL), and the mixture was stirred overnight at 95°C. The reaction mixture was allowed to cool to room temperature, and the precipitated solid was collected by filtration to give the title compound (298.8 mg) as black blue crystals.

### Example 81

### Production of (3,5-dibromo-4-hydroxyphenyl)-(6-methyl-2,3-dihydrobenzo[1,4]oxazin-4-yl)-methanone

6-Methyl-3,4-dihydro-2H-benzo [1,4] oxazine (149 mg) obtained in Step 1 of Example 12 and 3,5-dibromo-4-hydroxybenzoyl chloride (314 mg) obtained in Step 1 of Example 4 were dissolved in ethyl acetate (3.5 mL), and the mixture was stirred overnight at 95°C. The reaction mixture was allowed to cool to room temperature, and the precipitated solid was collected by filtration to give the title compound (368.2 mg) as beige crystals.

### Example 82

### Production of (6-chloro-2,3-dihydrobenzo[1,4]oxazin-4-yl)-(3,5-dibromo-4-hydroxyphenyl)-methanone

6-Chloro-3,4-dihydro-2H-benzo[1,4]oxazine (170.6 mg) obtained in Step 3 of Example 51 and 3,5-dibromo-4-hydroxybenzoyl chloride (349.3 mg) obtained in Step 1 of Example 4 were dissolved in ethyl acetate (5 mL), and the mixture was heated under reflux overnight. The solvent was evaporated, and the obtained solid was crystallized from methanol to give the title compound (441.0 mg) as crystals.

### Example 83

### Production of (3,5-dichloro-4-hydroxyphenyl)-(4-methanesulfonyl-3,4-dihydro-2H-quinoxalin-1-yl)-methanone

### Step 1

### Production of (4-benzyloxy-3,5-dichlorophenyl)-(4-methanesulfonyl-3,4-dihydro-2H-quinoxalin-1-yl)-methanone

(4-Benzyloxy-3,5-dichlorophenyl)-(3,4-dihydro-2H-quinoxalin-1-yl)-methanone (399.7 mg) obtained in Step 1 of Example 54 was dissolved in methylene chloride (8 mL), triethylamine (0.162 mL) and methanesulfonyl chloride (0.094 mL) were added under ice-cooling, and the mixture was stirred overnight at room temperature. The reaction mixture was concentrated, water was added, and the mixture was extracted with ethyl acetate. The obtained ethyl acetate layer was washed with water and saturated brine, and dried over anhydrous sodium sulfate. The solvent was evaporated, and the obtained residue was purified by silica gel chromatography (chloroform-methanol=98:2) to give the title compound (451.5 mg) as a pale-yellow amorphous form.

### Step 2

### Production of (3,5-dichloro-4-hydroxyphenyl)-(4-methanesulfonyl-3,4-dihydro-2H-quinoxalin-l-yl)-methanone

(4-Benzyloxy-3,5-dichlorophenyl)-(4-methanesulfonyl-3,4-dihydro-2H-quinoxalin-1-yl)-methanone (451.5 mg) was dissolved in tetrahydrofuran (10 mL). 7.5% Palladium-carbon (43.1 mg) was added to this solution and, under a hydrogen atmosphere, the mixture was stirred at room temperature for 1 hr. The reaction mixture was filtered through celite, and the residue was washed with tetrahydrofuran. The filtrate and washing solution were combined and the mixture was concentrated under reduced pressure. The obtained residue was purified by silica gel chromatography (chloroform-methanol=95:5) to give the title compound (335.9 mg) as a pale-yellow amorphous solid.

### Example 84

### Production of (3,5-dichloro-4-hydroxyphenyl)-(6-ethanesulfonyl-2,3-dihydrobenzo[1,4]oxazin-4-yl)-methanone

### Step 1

### Production of 2-chloro-N-(5-ethanesulfonyl-2-hydroxyphenyl)acetamide

2-Amino-4-ethanesulfonylphenol (1.5063 g) was dissolved in ethyl acetate (15 mL), water (15 mL) and sodium hydrogencarbonate (1.2588 g) were added under ice-cooling, and chloroacetyl chloride (0.715 mL) was added dropwise. After stirring at room temperature for 5 hrs, water (20 mL) was added, and the mixture was extracted with ethyl acetate. The obtained ethyl acetate layer was washed with saturated brine, and dried over anhydrous sodium sulfate. The solvent was evaporated to give the title compound (1.9375 g) as a brown solid.

### Step 2

### Production of 6-ethanesulfonyl-4H-benzo[1,4]oxazin-3-one

2-Chloro-N-(5-ethanesulfonyl-2-hydroxyphenyl)acetamide (1.9375 g) was dissolved in N,N-dimethylformamide (20 mL), and potassium carbonate (1.2520 g) was added at room temperature. After stirring overnight at room temperature, water (20 mL) was added, and the mixture was extracted with ethyl acetate. The obtained ethyl acetate layer was washed with water and saturated brine, and dried over anhydrous sodium sulfate. The solvent was evaporated, and the obtained solid was crystallized from methanol to give the title compound (1.3358 g) as a solid.

### Step 3

### Production of 6-ethanesulfonyl-3,4-dihydro-2H-benzo[1,4]oxazine

6-Ethanesulfonyl-4H-benzo[1,4]oxazin-3-one (802.3 mg) was dissolved in tetrahydrofuran (4 mL), and borane-tetrahydrofuran complex (1M tetrahydrofuran solution, 7 mL) was added under ice-cooling. After stirring with heating at 70°C for 9 hrs, methanol (5 mL) was added dropwise at the same temperature, and the mixture was further stirred with heating for 1 hr. 1N Hydrochloric acid (5 mL) was added dropwise while heating at 70°C and, after stirring with heating for 1 hr, the mixture was allowed to cool to room temperature. The mixture was extracted with ethyl acetate, and the obtained ethyl acetate layer was washed with water and saturated brine, and dried over anhydrous sodium sulfate. The solvent was evaporated, and the obtained residue was purified by silica gel chromatography (chloroform-methanol=95:5) to give the title compound (748.7 mg) as an orange oil.

### Step 4

### Production of (3,5-dichloro-4-hydroxyphenyl)-(6-ethanesulfonyl-2,3-dihydrobenzo [1,4]oxazin-4-yl)-methanone

6-Ethanesulfonyl-3,4-dihydro-2H-benzo [1,4] oxazine (233.5 mg) and 3,5-dichloro-4-hydroxybenzoyl chloride (255.4 mg) obtained Step 1 of Example 3 were dissolved in ethyl acetate (5 mL), and the mixture was heated under reflux overnight. The solvent was evaporated, and the obtained residue was purified by silica gel chromatography (chloroform-methanol=95:5) to give the title compound (446.9 mg) as a white amorphous solid.

### Example 85

### Production of (3,5-dichloro-4-hydroxyphenyl)-(6-trifluoromethyl-2,3-dihydrobenzo[1,4]oxazin-4-yl)-methanone

### Step 1

### Production of 2-amino-4-trifluoromethylphenol

2-Nitro-4-trifluoromethylphenol (3.0951 g) was dissolved in tetrahydrofuran (15 mL). 7.5% Palladium-carbon (299.8 mg) was added to this solution and, under a hydrogen atmosphere, the mixture was stirred overnight at room temperature. The reaction mixture was filtered through celite, and the residue was washed with tetrahydrofuran. The filtrate and washing solution were combined and the mixture was concentrated under reduced pressure to give the title compound (2.6346 g) as a gray solid.

### Step 2

### Production of 2-chloro-N-(2-hydroxy-5-trifluoromethylphenyl)acetamide

2-Amino-4-trifluoromethylphenol (1.0075 g) was dissolved in ethyl acetate (10 mL), water (10 mL) and sodium hydrogencarbonate (0.9531 g) were added under ice-cooling, and chloroacetyl chloride (0.55 mL) was added dropwise. The mixture was stirred overnight at room temperature, and extracted with ethyl acetate. The obtained ethyl acetate layer was washed with saturated brine, and dried over anhydrous sodium sulfate. The solvent was evaporated to give the title compound (1.4341 g) as a pale-brown solid.

### Step 3

### Production of 6-trifluoromethyl-4H-benzo[1,4]oxazin-3-one

2-Chloro-N-(2-hydroxy-5-trifluoromethylphenyl)acetamide (1.4244 g) was dissolved in N,N-dimethylformamide (14 mL), and potassium carbonate (1.0072 g) was added at room temperature. After stirring at room temperature for 2 hrs, water (20 mL) was added, and the mixture was extracted with ethyl acetate. The obtained ethyl acetate layer was washed with water and saturated brine, and dried over anhydrous sodium sulfate. The solvent was evaporated to give the title compound (1.2084 g) as a pale-gray solid.

### Step 4

### Production of 6-trifluoromethyl-3,4-dihydro-2H-benzo[1,4]oxazine

6-Trifluoromethyl-4H-benzo[1,4]oxazin-3-one (810.8 mg) was dissolved in tetrahydrofuran (4 mL), borane-tetrahydrofuran complex (1M tetrahydrofuran solution, 7.4 mL) was added under ice-cooling. After stirring with heating at 70°C for 6 hrs, methanol (5 mL) was added dropwise at the same temperature, and the mixture was further stirred with heating for 1 hr. 1N Hydrochloric acid (5 mL) was added dropwise while heating at 70°C, and the mixture was stirred with heating for 30 min. The mixture was allowed to cool to room temperature and extracted with ethyl acetate. The obtained ethyl acetate layer was washed with water and saturated brine, and dried over anhydrous sodium sulfate. The solvent was evaporated, and the obtained residue was purified by silica gel chromatography (chloroform-methanol=99:1) to give the title compound (718.3 mg) as a white solid.

### Step 5

### Production of (3,5-dichloro-4-hydroxyphenyl)-(6-trifluoromethyl-2,3-dihydrobenzo[1,4]oxazin-4-yl)-methanone

6-Trifluoromethyl-3,4-dihydro-2H-benzo[1,4]oxazine (205.3 mg) and 3,5-dichloro-4-hydroxybenzoyl chloride (252.5 mg) obtained in Step 1 of Example 3 were dissolved in ethyl acetate (5 mL), and the mixture was heated under reflux overnight. The solvent was evaporated, and the obtained residue was purified by silica gel chromatography (chloroform-methanol=95:5) to give the title compound (286.5 mg) as white crystals.

### Example 86

### Production of (3,5-dichloro-4-methoxyphenyl)-(2,3-dihydrobenzo[1,4]oxazin-4-yl)-methanone

(3,5-Dichloro-4-hydroxyphenyl)-(2,3-dihydrobenzo[1,4]oxazin-4-yl)-methanone (100 mg) obtained in Step 2 of Example 3 was dissolved in acetone (5 mL), potassium carbonate (47 mg) and iodomethane(0.096 mL) were added, and the mixture was stirred with heating at 45°C overnight. The reaction mixture was purified by silica gel chromatography (n-hexane-ethyl acetate=5:1) to give the title compound (109.2 mg) as a white solid.

### Example 87

### Production of 2,6-dichloro-4-(2,3-dihydrobenzo[1,4]oxazine-4-carbonyl)-phenyl acetate

(3,5-Dichloro-4-hydroxyphenyl) - (2,3-dihydrobenzo[1,4]oxazin-4-yl)-methanone (100 mg) obtained in Step 2 of Example 3 was dissolved in chloroform (5 mL), triethylamine (0.064 mL) and acetyl chloride (0.026 mL) were added under ice-cooling, and the mixture was stirred overnight at room temperature. The reaction mixture was purified by silica gel chromatography (n-hexane-ethyl acetate=5:1) to give the title compound (123.1 mg) as white crystals.

### Example 88

### Production of (2,3-dihydrobenzo[1,4]oxazin-4-yl)-(4-hydroxyphenyl)-methanone

### Step 1

### Production of (4-benzyloxyphenyl)-(2,3-dihydrobenzo[1,4]oxazin-4-yl)-methanone

3,4-Dihydro-2H-benzo[1,4]oxazine (270 mg) obtained in Step 2 of Example 1, 4-benzyloxybenzoic acid (457 mg) and 4-dimethylaminopyridine (269 mg) were dissolved in chloroform (7 mL), and WSC·HC1 (422 mg) was added under ice-cooling. After stirring overnight at room temperature, the reaction mixture was purified by silica gel chromatography (n-hexane-ethyl acetate=3:1) to give the title compound (636.2 mg) as an orange oil.

### Step 2

### Production of (2,3-dihydrobenzo [1,4] oxazin-4-yl)-(4-hydroxyphenyl)-methanone

(4-Benzyloxyphenyl)-(2,3-dihydrobenzo [1,4] oxazin-4-yl)-methanone (630 mg) was dissolved in tetrahydrofuran (20 mL). 7.5% Palladium-carbon (70 mg) was added to this solution and, under a hydrogen atmosphere, the mixture was stirred at room temperature for 6 hrs. The reaction mixture was filtered through celite, and the residue was washed with tetrahydrofuran. The filtrate and washing solution were combined and the mixture was concentrated under reduced pressure. The obtained residue was crystallized from ethyl acetate to give the title compound (149.1 mg) as white crystals.

### Example 89

### Production of (3,5-dichloro-4-hydroxyphenyl)-(5-hydroxy-2,3-dihydrobenzo[1,4]oxazin-4-yl)-methanone

### Step 1

### Production of 2-chloro-N-(2,6-dihydroxyphenyl)-acetamide

2-Aminobenzene-1,3-diol hydrochloride (2.00 g) was dissolved in ethyl acetate (20 mL), water (20 mL) and sodium hydrogencarbonate (2.50 g) were added, chloroacetyl chloride (1.02 mL) was added dropwise under ice-cooling. After stirring at room temperature for 12 hrs, and the mixture was extracted with ethyl acetate. The obtained ethyl acetate layer was washed successively with water and saturated brine, and dried over anhydrous sodium sulfate. The solvent was evaporated and the obtained residue was purified by silica gel chromatography (n-hexane-ethyl acetate=65:35) to give the title compound (1.508 g) as a pale-orange solid.

### Step 2

### Production of 5-hydroxy-4H-benzo[1,4]oxazin-3-one

2-Chloro-N-(2,6-dihydroxyphenyl)-acetamide (1.274 g) was dissolved in N,N-dimethylformamide (10 mL), potassium carbonate (873 mg) was added at room temperature. After stirring at room temperature for 2.5 hrs, water (30 mL) was added. The mixture was stirred under ice-cooling for 0.5 hr, and the precipitated solid was collected by filtration to give the title compound (915 mg) as a pale-orange solid.

### Step 3

### Production of 5-benzyloxy-4H-benzo[1,4]oxazin-3-one

5-Hydroxy-4H-benzo[1,4]oxazin-3-one (200 mg) was dissolved in N,N-dimethylformamide (2 mL), potassium carbonate (167 mg) and benzyl bromide (158 mL) were added at room temperature. The mixture was stirred at room temperature for 3 hrs and stirred with heating at 60°C for 1 hr, and water was added. The precipitated solid was collected by filtration to give the title compound (283 mg) as a pale-yellow solid.

### Step 4

### Production of 5-benzyloxy-3,4-dihydro-2H-benzo[1,4]oxazine

Lithium aluminum hydride (50 mg) was suspended in tetrahydrofuran (2 mL), and 5-benzyloxy-4H-benzo[1,4]oxazin-3-one (276 mg) was added by small portions under ice-cooling. The mixture was stirred at room temperature for 1 hr and stirred with heating at 60°C for 1 hr. Water (0.05 mL), 15% aqueous sodium hydroxide (0.05 mL) and water (0.15 mL) were successively added under ice-cooling, and the mixture was stirred at room temperature. The mixture was dried over anhydrous sodium sulfate and concentrated. The obtained residue was purified by silica gel chromatography (n-hexane-ethyl acetate=9:1) to give the title compound (54 mg) as an oil.

### Step 5

### Production of (4-benzyloxy-3,5-dichlorophenyl)-(5-benzyloxy-2,3-dihydrobenzo[1,4]oxazin-4-yl)-methanone

5-Benzyloxy-3,4-dihydro-2H-benzo[1,4]oxazine (47 mg) was dissolved in chloroform (2 mL), triethylamine (0.0716 mL) and 4-benzyloxy-3,5-dichlorobenzoyl chloride (689.3 mg) obtained in Step 3 of Example 8 were added. The mixture was stirred at room temperature for 25 hrs, water was added to the reaction mixture, and the mixture was extracted with ethyl acetate. The mixture was washed successively with water, saturated aqueous sodium hydrogencarbonate and saturated brine, dried over anhydrous sodium sulfate, and concentrated. The obtained residue was purified by silica gel chromatography (n-hexane-ethyl acetate=85:15) to give the title compound (54.7 mg) as a white solid.

### Step 6

### Production of (3,5-dichloro-4-hydroxyphenyl)-(5-hydroxy-2,3-dihydrobenzo[1,4]oxazin-4-yl)-methanone

(4-Benzyloxy-3,5-dichlorophenyl)-(5-benzyloxy-2,3-dihydrobenzo[1,4]oxazin-4-yl)-methanone (52 mg) was dissolved in tetrahydrofuran (2 mL). 7.5% Palladium-carbon (10 mg) was added to this solution and, under a hydrogen atmosphere, the mixture was stirred at room temperature for 1 hr. The reaction mixture was filtered through celite, and the residue was washed with tetrahydrofuran. The solvent was evaporated and the obtained solid was crystallized from ethyl ether to give the title compound (14.8 mg) as a pale-yellow solid.

### Example 90

### Production of (3,5-dichloro-4-hydroxyphenyl)-(8-hydroxy-2,3-dihydrobenzo[1,4]oxazin-4-yl)-methanone

### Step 1

### Production of 2-methoxy-6-nitrophenol

1,2-Dimethoxyethane (100 mL) was added to 2-methoxyphenol (6.21 g), and the mixture was cooled to -50°C. Nitronium tetrafluoroborate (6.77 g) was added, and the mixture was stirred at -50°C. After the completion of reaction, the reaction mixture was poured into ice water, and ethyl acetate and ethyl ether were added. The insoluble material was removed by filtration, and the filtrate was extracted with ethyl acetate. The extract was washed successively with water and saturated brine, dried over anhydrous sodium sulfate, and concentrated. The obtained residue was purified by silica gel chromatography (n-hexane-ethyl acetate=4:1-3:1) to give the title compound (2.46 g) as a yellow solid.

### Step 2

### Production of 2-amino-6-methoxyphenol hydrochloride

2-Methoxy-6-nitrophenol (2.46 g) was dissolved in tetrahydrofuran (20 mL). 7.5% Palladium-carbon (400 mg) was added to this solution and, under a hydrogen atmosphere, and the mixture was stirred at room temperature for 7 hrs. The reaction mixture was filtered through celite, and the residue was washed with ethyl acetate. Under ice-cooling, 4N hydrogen chloride-ethyl acetate (10 mL) was added dropwise and the mixture was stirred for 30 min. The precipitated solid was collected by filtration to give the title compound (2.41 g) as a white solid.

### Step 3

### Production of 2-chloro-N-(2-hydroxy-3-methoxyphenyl)-acetamide

2-Amino-6-methoxyphenol hydrochloride (2.40 g) was dissolved in ethyl acetate (25 mL), water (30 mL) and sodium hydrogencarbonate (2.76 g) were added under ice-cooling, and chloroacetyl chloride (1.2 mL) was added dropwise. The mixture was stirred at room temperature for 0.5 hr, and extracted with ethyl acetate. The obtained ethyl acetate layer was washed successively with water and saturated brine, and dried over anhydrous sodium sulfate. The solvent was evaporated to give the title compound (2.95 g) as a pale-orange solid.

### Step 4

### Production of 8-methoxy-4H-benzo[1,4]oxazin-3-one

2-Chloro-N-(2-hydroxy-3-methoxyphenyl)-acetamide (2.95 g) was dissolved in N,N-dimethylformamide (20 mL), and potassium carbonate (2.46 g) was added under ice-cooling. The mixture was stirred at room temperature for 4 hrs, and water was added. The mixture was stirred at room temperature for 0.5 hr, and the precipitated solid was collected by filtration to give the title compound (2.06 g) as a pink solid.

### Step 5

### Production of 8-hydroxy-4H-benzo[1,4]oxazin-3-one

8-Methoxy-4H-benzo[1,4]oxazin-3-one (950 mg) was dissolved in methylene chloride (90 mL). After cooling to -78°C, boron tribromide (1.0M methylene chloride solution, 13.3 mL) was added dropwise, and the mixture was stirred at room temperature for 2 hrs. Under ice-cooling, the reaction mixture was poured into water, and the mixture was extracted with ethyl acetate. The extract was washed with saturated brine, and dried over anhydrous sodium sulfate. The solvent was evaporated to give the title compound (864 mg) as a khaki solid.

### Step 6

### Production of 8-(tert-butyldimethylsilyloxy)-4H-benzo[1,4]oxazin-3-one

8-Hydroxy-4H-benzo[1,4]oxazin-3-one (900 mg) was dissolved in N,N-dimethylformamide (10 mL), and imidazole (482 mg) and tert-butylchlorodimethylsilane (986 mg) were added. The mixture was stirred at room temperature for 1 hr, and water and 10% aqueous citric acid solution were added. The mixture was extracted with ethyl acetate, washed successively with water and saturated brine, dried over anhydrous sodium sulfate, and concentrated. The obtained residue was purified by silica gel chromatography (n-hexane-ethyl acetate=7:3) to give the title compound (1.41 g) as a white solid.

### Step 7

### Production of 8-(tert-butyldimethylsilyloxy)-3,4-dihydro-2H-benzo[1,4]oxazine

8-(tert-Butyldimethylsilyloxy)-4H-benzo[1,4]oxazin-3-one (700 mg) was dissolved in tetrahydrofuran (5 mL), borane-tetrahydrofuran complex (1M tetrahydrofuran solution, 3.76 mL) was added, and the mixture was heated under reflux for 2.5 hrs. The mixture was allowed to cool to room temperature, weak-alkalified with saturated aqueous sodium hydrogencarbonate and water, and extracted with ethyl acetate. The obtained ethyl acetate layer was washed successively with water and saturated brine, and dried over anhydrous sodium sulfate. The solvent was evaporated to give the title compound (675 mg) as an oil.

### Step 8

### Production of (4-benzyloxy-3,5-dichlorophenyl)-[8-(tert-butyldimethylsilyloxy)-2,3-dihydrobenzo[1,4]oxazin-4-yl]-methanone

8-(tert-Butyldimethylsilyloxy)-3,4-dihydro-2H-benzo[1,4]oxazine (792 mg) was dissolved in chloroform (10 mL), and pyridine (0.242 mL) and 4-benzyloxy-3,5-dichlorobenzoyl chloride (792 mg) obtained in Step 3 of Example 8 were added. The mixture was stirred at room temperature for 1 hr, 10% aqueous citric acid solution was added, and the mixture was extracted with chloroform. The extract was washed successively with water and saturated brine, dried over anhydrous sodium sulfate, and concentrated. The obtained residue was purified by silica gel chromatography (n-hexane-ethyl acetate=8:1) to give the title compound (1.28 g) as a white solid.

### Step 9

### Production of (3,5-dichloro-4-hydroxyphenyl)-(8-hydroxy-2,3-dihydrobenzo[1,4]oxazin-4-yl)-methanone

(4-Benzyloxy-3,5-dichlorophenyl)-[8-(tert-butyldimethylsilyloxy)-2,3-dihydrobenzo[1,4]oxazin-4-yl]-methanone (545 mg) was dissolved in toluene (2 mL), and trifluoroacetic acid (4 mL) was added at room temperature. After stirring with heating at 85°C for 7.5 hrs, and the mixture was concentrated. The obtained residue was dissolved in tetrahydrofuran, and the solution was treated with activated carbon. The solvent was evaporated, and the residue was purified by silica gel chromatography (n-hexane-ethyl acetate=1:1) and crystallized from ethyl ether was to give the title compound (220 mg) as a yellow solid.

### Example 91

### Production of ethyl [2,6-dichloro-4-(2,3-dihydrobenzo[1,4]oxazine-4-carbonyl)phenoxy]acetate

(3,5-Dichloro-4-hydroxyphenyl)-(2,3-dihydrobenzo[1,4]oxazin-4-yl)-methanone (1.0067 g) obtained in Example 3 was dissolved in N,N-dimethylformamide (10 mL), and potassium carbonate (0.6553 g) and ethyl bromoacetate (0.52 mL) were added at room temperature. After stirring overnight with heating at 70°C, ethyl acetate (20 mL) and 10% aqueous citric acid solution (20mL) were added under ice-cooling, and the mixture was extracted with ethyl acetate. The extract was washed successively with water and saturated brine, dried over anhydrous sodium sulfate, and concentrated. The obtained residue was purified by silica gel chromatography (n-hexane-ethyl acetate=3:1) to give the title compound (1.2891 g) as a pale-yellow oil.

### Example 92

### Production of [2,6-dichloro-4-(2,3-dihydrobenzo[1,4]oxazine-4-carbonyl)phenoxy]acetic acid

Ethyl [2,6-dichloro-4-(2,3-dihydrobenzo[1,4]oxazine-4-carbonyl)phenoxy]acetate (1.2753 g) obtained in Example 91 was dissolved in methanol (6.5 mL), 2N aqueous sodium hydroxide (3.1 mL) was added, and the mixture was stirred at room temperature for 0.5 hr. Water was added to the reaction mixture, the mixture was washed with ethyl ether, and acidified with 10% aqueous citric acid solution. The mixture was extracted with ethyl acetate. The extract was washed with saturated brine, dried over anhydrous sodium sulfate, and concentrated. The obtained residue was crystallized from methanol to give the title compound (156.4 mg) as white crystals.

### Example 93

### Production of (3,5-dichloro-4-hydroxyphenyl)-(3-methyl-2,3-dihydrobenzo[1,4]oxazin-4-yl)-methanone

### Step 1

### Production of 1-(2-nitrophenoxy)-propan-2-one

2-Nitrophenol (2.78 g) was dissolved in N,N-dimethylformamide (20 mL), potassium carbonate (3.34 g) and bromoacetone (1.85 mL) were added at room temperature; and the mixture was stirred overnight at room temperature. Water was added to the reaction mixture, and the mixture was extracted with ethyl ether. The extract was washed successively with water and saturated brine, dried over anhydrous sodium sulfate, and concentrated. The obtained residue was purified by silica gel chromatography (n-hexane-ethyl acetate=3:1) to give the title compound (3.2101 g) as a bright yellow solid.

### Step 2

### Production of 3-methyl-3,4-dihydro-2H-benzo[1,4]oxazine

1-(2-Nitrophenoxy)propan-2-one (501.1 mg) was dissolved in tetrahydrofuran (10 mL). 7.5% Palladium-carbon (49.1 mg) was added to this solution and, under a hydrogen atmosphere, the mixture was stirred at room temperature for 3 hrs. The reaction mixture was filtered through celite, and the residue was washed with tetrahydrofuran. The solvent was evaporated, and the obtained residue was purified by silica gel chromatography (n-hexane-ethyl acetate=3:1) to give the title compound (248.1 mg) as a yellow oil.

### Step 3

### Production of (3,5-dichloro-4-hydroxyphenyl)-(3-methyl-2,3-dihydrobenzo[1;4]oxazin-4-yl)-methanone

3-Methyl-3,4-dihydro-2H-benzo[1,4]oxazine (244.9 mg) and 3,5-dichloro-4-hydroxybenzoyl chloride (370.1 mg) obtained in Step 1 of Example 3 were dissolved in ethyl acetate (7 mL), and the mixture was stirred with heating at 80°C for 3 hrs. Ethyl acetate (15 mL) and water (20 mL) were added to partition the reaction mixture into layers, and the aqueous layer extracted with ethyl acetate. The extract was washed successively with water and saturated brine, dried over anhydrous sodium sulfate, and concentrated. The obtained solid was crystallized from methanol to give the title compound (320.2 mg) as white crystals.

### Example 94

### Production of N-[2,6-dichloro-4-(2,3-dihydrobenzo[1,4]oxazine-4-carbonyl)phenyl]methanesulfonamide

### Step 1

### Production of N-methanesulfonyl-N-[2,6-dichloro-4-(2,3-dihydrobenzo[1,4]oxazine-4-carbonyl)phenyl]methanesulfonamide

(4-Amino-3,5-dichlorophenyl)-(2,3-dihydrobenzo[1,4]oxazin-4-yl)-methanone (323 mg) obtained in Example 28 was dissolved in N,N-dimethylformamide (5 mL), 60% sodium hydride (52 mg) was added, and the mixture was stirred at room temperature for 0.5 hr. Methanesulfonyl chloride (0.0851 mL) was added, and the mixture was stirred at room temperature. 10% Aqueous citric acid solution was added to the reaction mixture, and the mixture was extracted with ethyl acetate and tetrahydrofuran. The extract was washed successively with water and saturated brine, dried over anhydrous sodium sulfate, and concentrated. The obtained residue was purified by silica gel chromatography (n-hexane-ethyl acetate=4:1), and crystallized from isopropyl ether to give the title compound (78 mg) as a white solid.

### Step 2

### Production of N-[2,6-dichloro-4-(2,3-dihydrobenzo[1,4]oxazine-4-carbonyl)phenyl]methanesulfonamide

N-Methanesulfonyl-N-[2,6-dichloro-4-(2,3-dihydrobenzo[1,4]oxazine-4-carbonyl)phenyl]methanesulfonamide (73 mg) was dissolved in tetrahydrofuran (3 mL), tetrabutylammonium fluoride (1M tetrahydrofuran solution, 0.168 mL) was added, and the mixture was stirred overnight at room temperature. 10% Aqueous citric acid solution and water added to the reaction mixture, and the mixture was extracted with ethyl acetate. The extract was washed successively with water and saturated brine, dried over anhydrous sodium sulfate, and concentrated. The obtained residue was crystallized from ethyl acetate to give the title compound (48 mg) as a white solid.

### Example 95

### Production of (3,5-dichloro-4-hydroxyphenyl)-(7,8-dihydro-6H-5-oxa-9-azabenzocyclohepten-9-yl)-methanone

### Step 1

### Production of tert-butyldimethyl[3-(2-nitrophenoxy)propoxy]silane

2-Nitrophenol (3 g) was dissolved in N,N-dimethylformamide (20 mL), and sodium hydride (1.04 g) was added under ice-cooling. The mixture was stirred at room temperature for 0.5 hr, (3-bromopropoxy)-tert-butyldimethylsilane (5.49 mL) was added under ice-cooling, and the mixture was stirred with heating at 90°C overnight. Potassium carbonate (1 g) and (3-bromopropoxy)-tert-butyldimethylsilane (2.5 mL) were added, and the mixture was stirred with heating at 90°C for 1.5 hrs. 10% Aqueous citric acid solution and water were added to the reaction mixture, and the mixture was extracted with ethyl acetate. The extract was washed successively with water and saturated brine, dried over anhydrous sodium sulfate, and concentrated. The obtained residue was purified by silica gel chromatography (n-hexane-ethyl acetate=9:1) to give the title compound (6.71 g) as a yellow oil.

### Step 2

### Production of 2-[3-(tert-butyldimethylsilyloxy)propoxy]phenylamine

tert-Butyldimethyl[3-(2-nitrophenoxy)propoxy]silane (6.71 g) was dissolved in tetrahydrofuran (50 mL). 7.5% Palladium-carbon (1 g) was added to this solution and, under a hydrogen atmosphere, the mixture was stirred overnight at room temperature. The reaction mixture was filtered through celite, and the residue was washed with tetrahydrofuran. The solvent was evaporated, and the obtained residue was purified by silica gel chromatography (n-hexane-ethyl acetate=9:1) to give the title compound (5.88 g) as a pale-orange oil.

### Step 3

### Production of 4-benzyloxy-N-{2-[3-(tert-butyldimethylsilyloxy)propoxy]phenyl}-3,5-dichlorobenzamide

2-[3-(tert-Butyldimethylsilyloxy)propoxy]phenylamine (844 mg) was dissolved in chloroform (10 mL), and pyridine (0.314 mL) and 4-benzyloxy-3,5-dichlorobenzoyl chloride (947 mg) obtained in Step 3 of Example 8 were added. The mixture was stirred at room temperature for 1 hr. The reaction mixture was concentrated, and the obtained residue was purified by silica gel chromatography (n-hexane-ethyl acetate=9:1) to give the title compound (1.63 g) as a pale-yellow oil.

### Step 4

### Production of 4-benzyloxy-3,5-dichloro-N-[2-(3-hydroxypropoxy)phenyl]benzamide

4-Benzyloxy-N-{2-[3-(tert-butyldimethylsilyloxy)propoxy]phenyl}-3,5-dichlorobenzamide (1.62 g) was dissolved in tetrahydrofuran (10 mL), tetrabutylammonium fluoride (1M tetrahydrofuran solution, 4.33 mL) was added, and the mixture was stirred at room temperature for 1 hr. 10% Aqueous citric acid solution was added to the reaction mixture, and extracted with ethyl acetate and tetrahydrofuran. The extract was washed successively with water and saturated brine, dried over anhydrous sodium sulfate, and concentrated. The obtained residue was crystallized from isopropyl ether to give the title compound (1.19 g) as a white solid.

### Step 5

### Production of 4-benzyloxy-3,5-dichloro-N-[2-(3-chloropropoxy)phenyl]benzamide

4-Benzyloxy-3,5-dichloro-N-[2-(3-hydroxypropoxy)phenyl]benzamide (1.00 g) was dissolved in pyridine (10 m-L), methanesulfonyl chloride (0.225 mL) was added, and the mixture was stirred with heating at 70°C for 2 hrs. Water was added to the reaction mixture, and the mixture was extracted with ethyl acetate. The extract was washed successively with 10% aqueous citric acid solution, water and saturated brine, and dried over anhydrous sodium sulfate. The solvent was evaporated to give the title compound (713 mg) as a beige solid.

### Step 6

### Production of (4-benzyloxy-3,5-dichlorophenyl)-(7,8-dihydro-6H-5-oxa-9-azabenzocyclohepten-9-yl)-methanone

4-Benzyloxy-3,5-dichloro-N-[2-(3-chloropropoxy)phenyl]benzamide (200 mg) was dissolved in N,N-dimethylformamide (3 mL), 60% sodium hydride (21 mg) and sodium iodide (64 mg) were added, and the mixture was stirred with heating at 60°C. 10% Aqueous citric acid solution was added to the reaction mixture, and the mixture was extracted with ethyl acetate. The extract was washed successively with water and saturated brine, dried over anhydrous sodium sulfate, and concentrated. The obtained residue was purified by silica gel chromatography (n-hexane-ethyl acetate=9:1) to give the title compound (121 mg) as an oil.

### Step 7

### Production of (3,5-dichloro-4-hydroxyphenyl)-(7,8-dihydro-6H-5-oxa-9-azabenzocyclohepten-9-yl)-methanone

(4-Benzyloxy-3,5-dichlorophenyl)-(7,8-dihydro-6H-5-oxa-9-azabenzocyclohepten-9-yl)-methanone (115 mg) was dissolved in tetrahydrofuran (5 mL). 7.5% Palladium-carbon (15 mg) was added to this solution and, under a hydrogen atmosphere, the mixture was stirred at room temperature for 0.5 hr. The reaction mixture was filtered through celite, and the residue was washed with tetrahydrofuran. The solvent was evaporated and the obtained residue was crystallized from isopropyl ether to give the title compound (69 mg) as a white solid.

The ¹H-NMR spectrum data of the compounds of Examples 1-95 are shown in Table 1-Table 18.

The ¹H-NMR spectrum was measured in CDC1₃ or DMSO-d₆, with tetramethylsilane as an inner standard, and the total δ value are shown in ppm.

The symbols in the tables mean the following.
s: singlet
d: doublet
t: triplet
dd: double doublet
ddd: double double doublet
brs: broad singlet
m: multiplet
J: coupling constant

**Table 1**

| Ex. No. | Structural Formula | NMR |
|---|---|---|
| 1 | 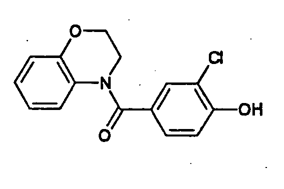 | (400 MHz, DMSO-d6) 3.87 (t, J=4.50Hz, 2H), 4.30 (t, J=4.50Hz, 2H), 6.74 (dd, J=7.80, 7.80Hz, 1H), 6.90 (d, J=8.30Hz, 1H), 6.95 - 7.05 (m, 2H), 7.10 - 7.20 (m, 1H), 7.34 (dd, J=8.40, 2.20Hz, 1H), 7.54 (d, J=2.0Hz, 1H),10. 82 (s, 1H) |
| 2 | 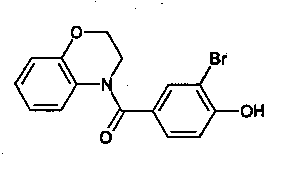 | (400 MHz, DMSO-d6) 3.87 (t, J=4.63Hz, 2H), 4.30 (t, J=4.63Hz, 2H), 6.74 (ddd, J=8.34, 7.06, 1.39Hz, 1H), 6.89 (dd, J=8.34, 1,62Hz, 1H) , 6.95-7.01 (m, 2H), 7.15 (d, J=7.87Hz, 1H), 7.38 (dd, J=8.35, 2.07Hz, 1H), 7.66 (d, J=1.76Hz, 1H) , 10.89 (s, 1H) |
| 3 | 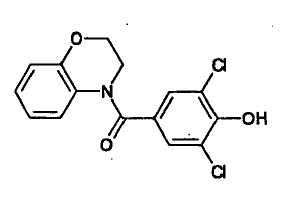 | (300 MHz, DMSO-d6) 3.87 (t, J=4.58Hz, 2H), 4.31 (t, J=4.58Hz, 2H), 6.76 (ddd, J=7.20, 6.90, 1.50Hz, 1H), 6.90 (dd, J=7.80, 1.50Hz, 1H), 7.01 (ddd, J=7.80, 6.90, 1.50Hz, 1H), 7.18 - 7.25 (m, 1H), 7.54 (s, 2H), 10.77 (s, 1H) |
| 4 | 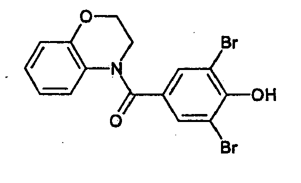 | (400 MHz, DMSO-d6) 3.87 (t, J=4.55Hz, 2H), 4.31 (t, J=4.55Hz, 2H), 6.77 (t, J=8.33Hz, 1H), 6.90 (d, J=8.10Hz, 1H), 7.01 (t, J=7.18Hz, 1H), 7.21 (brs, 1H), 7.70 (s, 2H), 10.54 (s, 1H) |
| 5 | 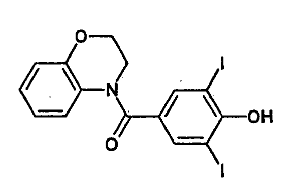 | (400 MHz, DMSO-d6) 3.85 (t, J=4.40Hz, 2H), 4.30 (t, J=4.40Hz, 2H), 6.75 (dd, J=7.76, 7.76Hz, 1H), 6.90 (dd, J=8.10, 1.50Hz, 1H), 7.01 (ddd, J=8.10, 7.76, 1.50Hz, 1H), 7.19 (brs, 1H), 7.86 (s, 2H), 10.05 (brs, 1H) |

**Table 2**

| Ex. No. | Structural Formula | NMR |
|---|---|---|
| 6 | 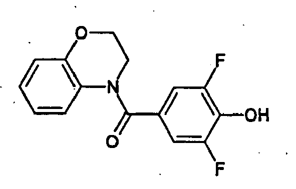 | (400 MHz, DMSO-d6) 3.87 (t, J=4.65Hz, 2H), 4.30 (t, J=4.65Hz, 2H), 6.76 (dd, J=7.65, 7.65Hz, 1H), 6.90 (d, J=8.10Hz, 1H), 7.01 (dd, J=7.80, 7.80Hz, 1H), 7.10 - 7.35 (m, 3H), 10.85 (s, 1H) |
| 7 | 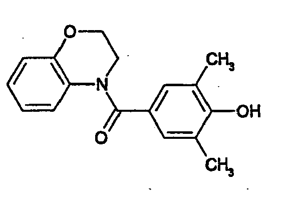 | (400 MHz, DMSO-d6) 2.12 (s, 6H), 3.82 (t, J=4.50Hz, 2H), 4.20 (t, J=4.50Hz, 2H), 6.70 (ddd, J=7.00, 7.00, 1.60Hz, 1H), 6.84 (dd, J=8.10, 1.40Hz, 1H), 6.93 (ddd, J=7.00, 6.70, 1.60Hz, 1H), 7.10 (s, 2H), 7.19 (d, J=8.10Hz, 1H), 8.85 (brs, 1H) |
| 8 | 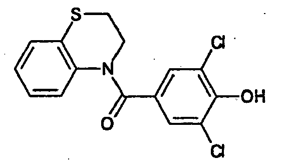 | (400 MHz, DMSO-d6) 3.30 - 3.35 (m, 2H), 3.99 - 4.05 (m, 2H), 6.75 (d, J=8.10Hz, 1H), 6.89 (dd, J=8.10, 7.41Hz, 1H), 7.07 (dd, J=7.87, 7.41Hz, 1H), 7.20 (s, 2H), 7.29 (d, J=7.87Hz, 1H), 10.77 (s, 1H). |
| 9 | 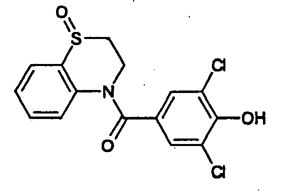 | (400 MHz, DMSO-d6) 3.28 - 3.34 (m, 1H), 3.45 - 3.53 (m, 1H), 3.88 - 3.95 (m, 1H), 4.24 - 4.31 (m, 1H), 6.98 - 7.00 (m, 1H), 7.31 - 6.36 (m, 2H), 7.34 (s, 2H), 7.75 - 7.78 (m, 1H), 10.76 (s, 1H) |
| 10 | 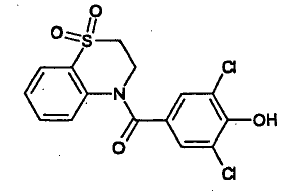 | (400 MHz, DMSO-d6) 3.92 (t, J=6.25Hz, 2H), 4.34 (t, J=6.25Hz, 2H), 7.10 (d, J=7.87Hz, 1H), 7.37 - 7.47 (m, 2H), 7.38 (s, 2H), 7.85 (dd, J=7.64, 1.62Hz, 1H), 10.90 (s, 1H) |
| 11 | 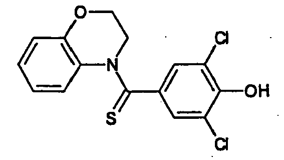 | (400 MHz, DMSO-d6) 4.51 - 4.58 (m, 4H), 6.61 - 6.67 (m, 2H), 6.95 (d, J=8.10Hz, 1H), 7.03 - 7.08 (m, 1H), 7.44 (s, 2H), 10.74 (s, 1H) |

**Table 3**

| Ex. No. | Structural Formula | NMR |
|---|---|---|
| 12 | 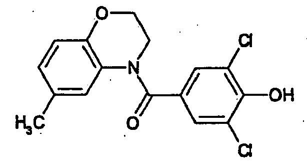 | (400 MHz, DMSO-d6) 2.12 (s, 3H), 3.86 (t, J=3.60Hz, 2H), 4.25 (t, J=3.60Hz, 2H), 6.78 - 6.85 (m, 2H), 7.15 (brs, 1H), 7.54 (s, 2H), 10.77 (brs, 1H) |
| 13 | 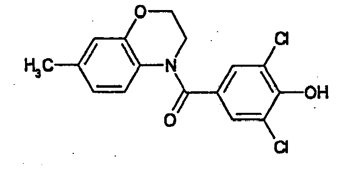 | (400 MHz, DMSO-d6) 2.20 (s, 3H), 3.84 (t, J=4.50Hz, 2H), 4.29 (t, J=4.50Hz, 2H), 6.58 (d, J=8.00Hz, 1H), 6.72 (s, 1H), 7.09 (brs, 1H) , 7.52 (s, 2H), 10.76 (brs, 1H) |
| 14 | 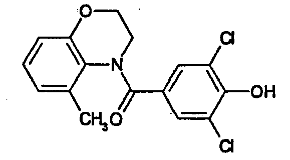 | (400 MHz, DMSO-d6) 1.99 (brs, 3H), 3.40 - 4.40 (m, 4H), 6.75 (d, J=7.87Hz, 2H), 7.04 (t, J=7.87Hz, 1H), 7.64 (brs, 2H), 10.89 (brs, 1H) |
| 15 | 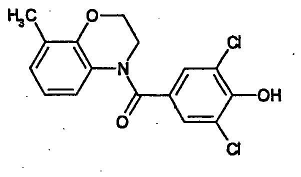 | (400 MHz, DMSO-d6) 2.16 (s, 3H), 3.86 (t, J=4.63Hz, 2H), 4.37 (t, J=4.63Hz, 2H), 6.63 (t, J=7.87Hz, 1H), 6.89 (d, J=7.18Hz, 1H), 6.94 (brs, 1H), 7.51 (s, 2H), 10.76 (brs, 1H) |
| 16 | 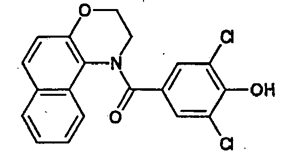 | (400 MHz, DMSO-d6) 3.40 - 4.45 (m, 4H), 7.05 - 7.40 (m, 5H), 7. 65 - 7.80 (m, 3H), 10.88 (brs, 1H) |
| 17 | 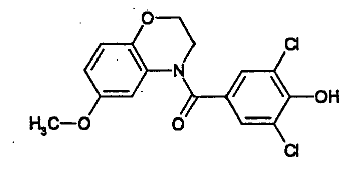 | (400 MHz, DMSO-d6) 3.53 (s, 3H), 3.84 (t, J=4.40Hz, 2H), 4.24 (t, J=4.40Hz, 2H), 6.64 (dd, J=8.80, 3.10Hz, 1H), 6.83 (d, J=8.80Hz, 1H), 6.88 (brs, 1H), 7.56 (s, 2H), 10.81 (brs, 1H) |
| 18 | 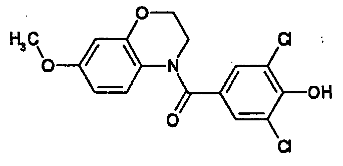 | (400 MHz, DMSO-d6) 3.69 (s, 3H), 3.84 (t, J=4.40Hz, 2H), 4.29 (t, J=4.40Hz, 2H), 6.39 (d, J=9.03Hz, 1H), 6.47 (d, J=2.78Hz, 1H), 7. 14 (brs, 1H), 7.52 (s, 2H), 10.72 (brs, 1H) |

**Table 4**

| Ex. No. | Structural Formula | NMR |
|---|---|---|
| 19 | 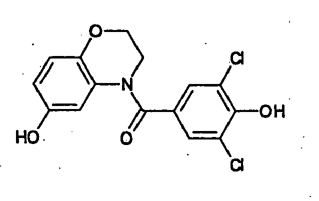 | (400 MHz, DMSO-d6) 3.80 (t, J=4.52Hz, 2H), 4.20 (t, J=4.52Hz, 2H), 6.44 (dd, J=8.70, 2.55Hz, 1H), 6.70 (d, J=8.70Hz, 1H), 6.76 (brs, 1H), 7.54 (s, 2H), 8.91 (s, 1H), 10.77 (brs, 1H) |
| 20 | 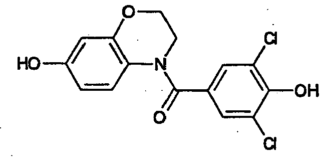 | (400 MHz, DMSO-d6) 3.81 (t, J=4.51Hz, 2H), 4.25 (t, J=4.51Hz, 2H), 6.17 - 6.22 (m, 1H), 6.28 (d, J=2.78Hz, 1H), 7.00 (brs, 1H), 7.49 (s, 2H), 9.35 (brs, 1H), 10.71 (brs, 1H) |
| 21 | 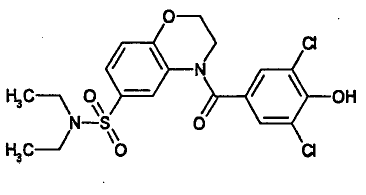 | (400 MHz, DMSO-d6) 1.00 (t, J=7.06Hz, 6H), 2.90 (q, J=7.06Hz, 4H), 3.91 (t, J=4.52Hz, 2H), 4.44 (t, J=4.52Hz, 2H), 7.09 (d, J=8.69Hz, 1H), 7.41 (dd, J=8.69, 2.32Hz, 1H), 7.60 (s, 3H), 10.89 (s, 1H) |
| 22 | 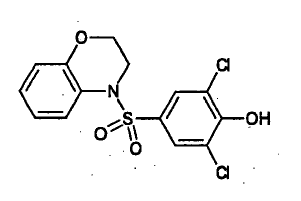 | (400 MHz, DMSO-d6) 3.78 (t, J=4.46Hz, 2H), 3.88 (t, J=4.46Hz, 2H), 6.81 (d, J=8.10Hz, 1H), 6.92 (dd, J=8.88, 7.41Hz, 1H), 7.07 (dd, J=8.10 , 7. 41Hz, 1H), 7.55 (s, 2H), 7.62 (d, J=8.33Hz, 1H), 11.65 (brs, 1H) |
| 23 | 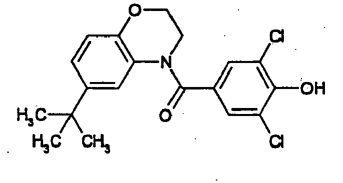 | (400 MHz, DMSO-d6) 1.02 (s, 3H), 3.90 (t, J=4.17Hz, 2H), 4.35 (t, J=4.17Hz, 2H), 6.81 (d, J=8.56Hz, 1H), 6.62 (brs, 1H), 7.00 (dd, J=8.56, 2.32Hz, 1H), 7.47 (s, 2H), 10.73 (brs, 1H) |
| 24 | 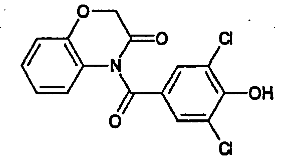 | (400 MHz, DMSO-d6) 4.83 (s, 2H), 6.90 (d, J=8.33Hz, 1H), 7.00 (dd, J=6.71, 6.71Hz, 1H), 7.09 - 7.16 (m, 2H), 8.00 (s, 2H) |

**Table 5**

| Ex. No. | Structural Formula | NMR |
|---|---|---|
| 25 | 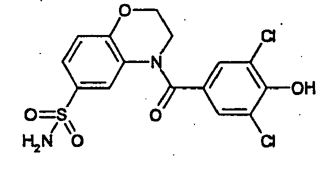 | (400 MHz, DMSO-d6) 3.88 (t, J=4.46Hz, 2H), 4.31 (t, J=4.46Hz, 2H), 7.08 (d, J=8.68Hz, 1H), 7.24 (s, 2H), 7.48 (dd, J=8.68, 2.08Hz, 1H), 7.62 (s, 2H), 8.13 (s, 1H), 10.87 (s, 1H) |
| 26 | 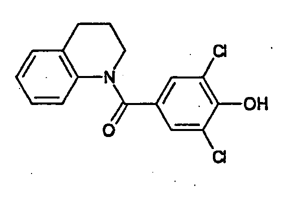 | (300 MHz, DMSO-d6) 1.94 (quintet, J=6.59Hz, 2H), 2.81 (t, J=6.59Hz, 2H), 3.73 (t, J=6.59Hz, 2H), 6.78 (d, J=7.87Hz, 1H), 6.95 (dd, J=7.87, 7.50Hz, 1H), 7.03 (dd, J=7.87, 7.87Hz, 1H), 7.21 (d, J=7.50, 1H) , 7.27 (s, 2H), 10.67 (s, 1H) |
| 27 | 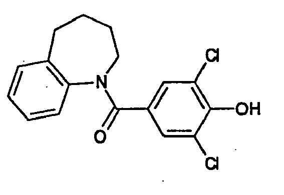 | (400 MHz, DMSO-d6) 1.30 - 1.45 (m, 1H), 1.75 - 1.90 (m, 2H) , 1.95 - 2.05 (m, 1H), 2.60 - 2.70 (m, 1H), 2.80 - 3.05 (m, 2H), 4.70 - 4.85 (m, 1H), 6.76 - 6.81 (m, 1H), 6.98 - 7.06 (m, 1H), 7.04 (s, 2H), 7.15 (t, J=7.64Hz, 1H), 7.32 (d, J=7.64Hz, 1H), 10.54 (brs, 1H) |
| 28 | 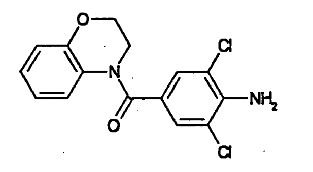 | (400 MHz, DMSO-d6) 3.88 (t, J=4.52Hz, 2H), 4.30 (t, J=4.52, 2H), 6.08 (s, 2H), 6.75 (ddd, J=8.34, 8.34, 6.94Hz, 1H), 6.89 (dd, J=8.10, 1.39Hz, 1H), 6.99 (ddd, J=10.98, 7.03, 1.39Hz, 1H), 7.15 (d, J=7.30Hz, 1H), 7.42 (s, 2H) |

**Table 6**

| Ex No. | Structural Formula | NMR |
|---|---|---|
| 29 | 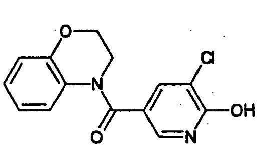 | (400 MHz, DMSO-d6) 3.90 (t, J=4.51Hz, 2H), 4.32 (t, J=4.51Hz, 2H), 6.79 (ddd, J=8.34, 7.06, 1.39Hz, 1H), 6.90 (dd, J=8.10, 1.39Hz, 1H), 7.01 (ddd, J=8.28, 7.12, 1.62Hz, 1H), 7.23 (d, J=8.10Hz, 1H), 7.72 (d, J=2.32Hz, 1H), 7.80 (d, J=2.32Hz, 1H), 12.51 (brs, 1H) |
| 30 | 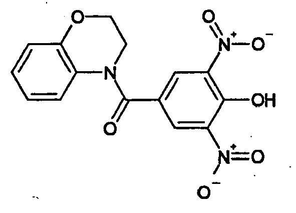 | (300 MHz, DMSO-d6) 3.90 (t, J=4.58Hz, 2H), 4.32 (t, J=4.58Hz, 2H), 5.00 (brs, 1H), 6.74 - 6.80 (m, 1H), 6.89 - 7.04 (m, 2H), 7.24 (dd, J=8.80, 1.50Hz, 1H), 8.20 (s, 2H). |
| 31 | 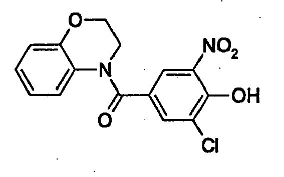 | (300 MHz, DMSO-d6) 3.87 - 3.90 (m, 2H), 4.32 - 4.35 (m, 2H), 6.74 - 6.79 (m, 1H) , 6.90 - 6.93 (m, 1H) , 7.00 - 7.05 (m, 1H), 7.24 - 7.27 (m, 1H), 7.91 (d, J=2.20Hz, 1H), 8.06 (d, J=2.20Hz, 1H) |
| 32 | 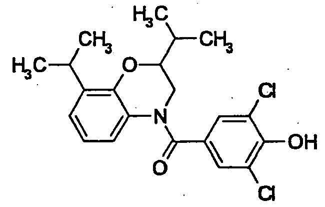 | (300 MHz, DMSO-d6) 0.95 (d, J=7.00Hz, 3H), 1.03 (d, J=6.60Hz, 3H), 1.18 - 1.22 (m, 6H), 1. 86 - 1.99 (m, 1H), 3.20 - 3.39 (m, 2H), 4.08 - 4.21 (m, 2H), 6.67 (dd, J=8.10, 7.70Hz, 1H), 6.86 - 6.89 (m, 1H), 6.95 (dd, J=7.70, 1.30Hz, 1H), 7.47 (s, 2H), 10.76 (brs, 1H) |

**Table 7**

| Ex. No. | Structural Formula | NMR |
|---|---|---|
| 33 | 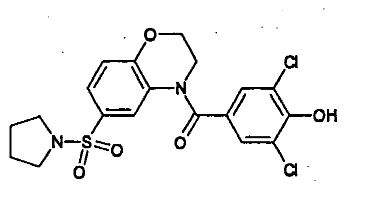 | (400 MHz, DMSO-d6) 1.62 - 1.66 (m, 4H), 2.90 - 2.95 (m, 4H), 3.91 (t, J=4.40Hz, 2H), 4.44 (t, J=4.40Hz, 2H), 7.12 (d, J=8.57Hz, 1H), 7.43 (dd, J=8.57, 2.08Hz, 1H), 7.61 (s, 2H), 7.68 (brs, 1H), 10.86 (brs, 1H) |
| 34 | 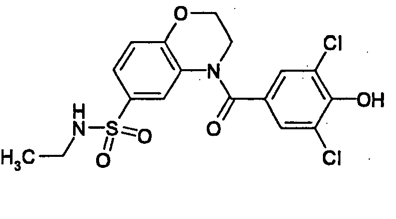 | (400 MHz, DMSO-d6) 0.95 (t, J=7.18Hz, 3H), 2.51 - 2.59 (m, 2H), 3.91 (t, J=4.40Hz, 2H), 4.40 (t, J=4.40Hz, 2H), 7.11 (d, J=8.80Hz, 1H), 7.36 (t, J=5.79Hz, 1H), 7.41 (dd, J=8.80, 2.31Hz, 1H), 7.60 (s, 2H), 7.73 (brs, 1H), 10.84 (s, 1H) |
| 35 | 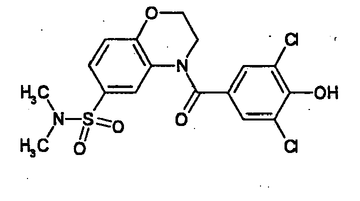 | (400 MHz, DMSO-d6) 2.47 (s, 6H), 3.92 (t, J=3.94Hz, 2H), 4.42 (t, J=3.94Hz, 2H), 7. 14 (d, J=8. 57Hz, 1H), 7.38 (dd, J=8.57, 2.08Hz, 1H), 7.62 (s, 2H), 7.68 (brs, 1H), 10.85 (brs, 1H) |
| 36 | 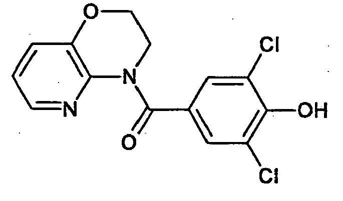 | (400 MHz, DMSO-d6) 3.96 (t, J=4.65Hz, 2H), 4.49 (t, J=4.65Hz, 2H), 7.03 (dd, J=7.90, 4.60Hz, 1H), 7.33 (dd, J=8.00, 1.50Hz, 1H), 7.58 (dd, J=4.60, 1.40Hz, 1H), 10.72 (s, 1H) |
| 37 | 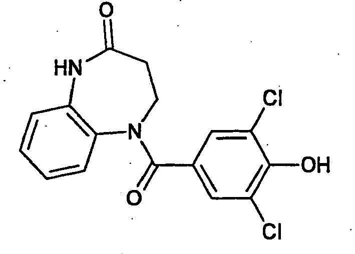 | (400 MHz, DMSO-d6) 2.20 - 2.80 (m, 2H), 3.60 - 3.85 (m, 1H), 4.40 - 4.70 (m, 1H), 6.85 - 7.05 (m, 4H), 7.14 (d, J=7.90Hz, 1H), 7.28 (ddd, J=7.40, 7.40, 1.80Hz, 1H), 10.04 (s, 1H), 10.66(s, 1H) |

**Table 8**

| Ex No. | Structural Formula | NMR |
|---|---|---|
| 38 | 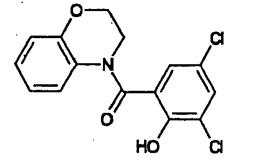 | (400 MHz, DMSO-d6) 3.50 - 3.90 (m, 2H), 4.20 - 4.40 (m, 2H) , 6.70 - 7.10 (m, 3H), 7.39 (s, 1H), 7.62 (s, 1H), 10.31(s, 1H) |
| 39 | 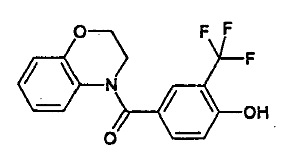 | (400 MHz, DMSO-d6) 3.88 (t, J=4.50Hz, 2H), 4.31 (t, J=4.50Hz, 2H), 6.74 (dd, J=7.65, 7.65Hz, 1H), 6.90 (d, J=8.30Hz, 1H), 6.95 - 7.25 (m, 3H), 7.64 (d, J= 8.60Hz, 1H), 7.77(s, 1H), 11.20 (s, 1H) |
| 40 | 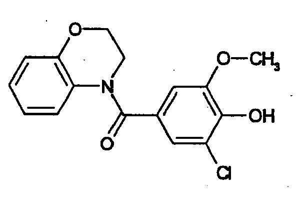 | (400 MHz, DMSO-d6) 3.79 (s, 3H), 3.88 (t, J=4.60Hz, 2H), 4.30 (t, J=4.60Hz, 2H), 6.76 (dd, J=7.70, 7.70Hz, 1H), 6.90 (d, J=8. 20Hz, 1H), 6.99 (dd, J=7.70, 7.70Hz, 1H), 7.09 (s, 1H) , 7. 14 (s, 1H) , 7.15 - 7.30 (m, 1H), 10.01 (s, 1H) |
| 41 | 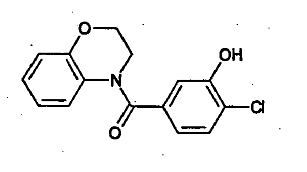 | (400 MHz, DMSO-d6) 3.85 (t, J=4.55Hz, 2H), 4.30 (t, J=4.55Hz, 2H), 6.75 (dd, J=7.80, 7.80Hz, 1H), 6.90 (d, J=8.20Hz, 1H), 6.94 (d, J=8.2Hz, 1H), 7.00 (dd, J=8.30, 8.30Hz, 1H), 7.10 (s, 1H), 7.15 - 7.30 (m, 1H), 7.39 (d, J=8. 30Hz, 1H), 10.50 (s, 1H) |
| 42 | 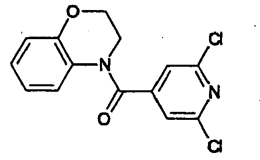 | (400 MHz, DMSO-d6) 3.81 (brs, 2H), 4.32 (brs, 2H), 6.84 (brs, 1H), 6.93 (dd, J=8.10, 1.39Hz, 1H), 7.08 (t, J=8.10, 1H), 7.77 (s, 2H), 8.00 (brs, 1H) |

**Table 9**

| Ex. No. | Structural Formula | NMR |
|---|---|---|
| 43 | 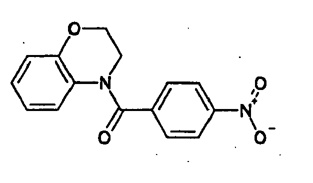 | (400 MHz, DMSO-d6) 3.83 - 3.89 (m, 2H), 4.33 - 4.39 (m, 2H), 6.74 (brs, 1H), 6.92 (dd, J=8.10, 1.39Hz, 1H), 7.03 (ddd, J=7.64, 7.64, 1.39Hz, 1H), 7.20 (brs, 1H), 7.80 (d, J=8.80Hz, 2H), 8.28 (d, J=8.80Hz, 2H) |
| 44 | 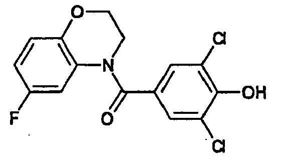 | (400 MHz, DMSO-d6) 3.84 (t, J=4.40Hz, 2H), 4.25 (t, J=4.40Hz, 2H) , 6.87 - 6.95 (m, 2H), 7.34 (d, J=10.40Hz, 1H), 7.59 (s, 2H), 10.83 (brs, 1H) |
| 45 | 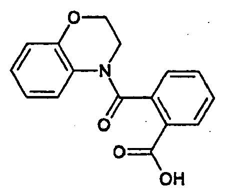 | (400 MHz, DMSO-d6) 3.20 - 3.60 (m, 2H), 4.10 - 4.60 (m, 2H), 6.10 - 6.50 (m, 1H), 6.70 - 7.30 (m, 3H), 7.40 - 8.40 (m, 5H), 13.33 (s, 1H) |
| 46 | 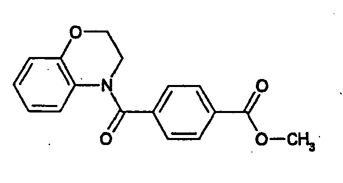 | (300 MHz, CDCl3) 3.93 (s, 3H), 4.02 (t, J=4.80Hz, 2H), 4.40 (t, J=4.80Hz, 2H), 6.63 (dd, J=7.50, 7.50Hz, 1H), 6.82 (brs, 1H), 6.91 (dd, J=8.10, 1.50Hz, 1H), 7.00 (ddd, J=8.10, 7.50, 1.50Hz, 1H), 7.56 (d, J=8.10Hz, 2H), 8.04 (d, J=8.10Hz, 2H) |
| 47 | 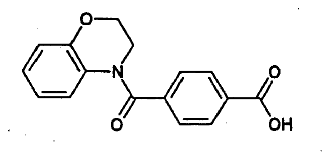 | (300 MHz, DMSO-d6) 3.86 (t, J=4.50Hz, 2H), 4.33 (t, J=4.50Hz, 2H), 6.73 (dd, J=7.20, 7.20Hz, 1H), 6.91 (dd, J=8.10, 1.50Hz, 1H), 7.01 (ddd, J=8.10, 7.20, 1.50Hz, 1H), 7.22 (brs, 1H), 7.61 (d, J=8.10Hz, 2H), 7.97 (d, J=8.10Hz, 2H) |

**Table 10**

| Ex. No. | Structural Formula | NMR |
|---|---|---|
| 48 | 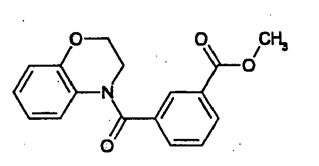 | (300 MHz, CDCl3) 3.92 (s, 3H), 4.02 (t, J=4.80Hz, 2H), 4.40 (t, J=4.80Hz, 2H), 6.65 (dd, J=7.20, 7.20Hz, 1H), 6.92 (dd, J=8.40, 1.20Hz, 1H), 7.00 (ddd, J=8.40, 7.20, 1.20Hz, 1H), 7.45 (dd, J=7.80, 7.80Hz, 1H), 7.66 (d, J=7.80Hz, 1H), 8.11 (d, J=7.80Hz, 1H), 8.20 (s, 1H) |
| 49 | 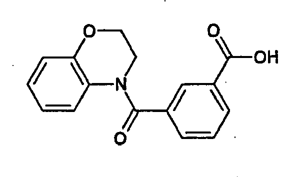 | (400 MHz, DMSO-d6) 3.87 (t, J=4.50Hz, 2H), 4.33 (t, J=4.50Hz, 2H), 6.72 (dd, J=7.80, 7.80Hz, 1H), 6.91 (dd, J=8.40, 1.20Hz, 1H), 7.01 (ddd, J=8.40, 7.80, 1.20Hz, 1H), 7.20 (brs, 1H), 7.57 (dd, J=7.80, 7.80Hz, 1H), 7.76 (d, J=7.80Hz, 1H), 8.03 - 8.05 (m, 2H), 13.20 (brs, 1H) |
| 50 | 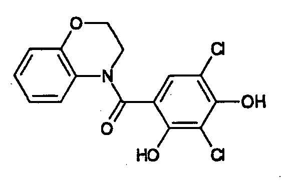 | (400 MHz, DMSO-d6) 3.78 (t, J=4.10Hz, 2H), 4.29 (t, J=4.10Hz, 2H), 6.79 (dd, J=7.70, 7.70Hz, 1H), 6.89 (d, J=8.4Hz, 1H), 7.02 (dd, J=7.70, 7.70Hz, 1H), 7.30 (s, 1H), 7.52 (brs, 1H), 10.14 (s, 1H), 10.41 (s, 1H) |
| 51 | 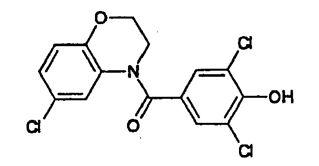 | (400 MHz, DMSO-d6) 3.84 (t, J=4.30Hz, 2H), 4.27 (t, J=4.30Hz, 2H), 6.95 (d, J=8.80Hz, 1H), 7.08 (dd, J=8.80, 2.60Hz, 1H), 7.53 - 7.58 (m, 1H), 7.59 (s, 2H), 10.84 (s, 1H) |
| 52 | 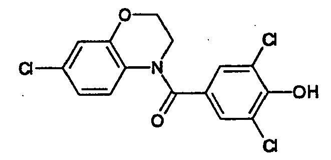 | (400 MHz, DMSO-d6) 3.86 (t, J=4.60Hz, 2H), 4.32 (t, J=4.60Hz, 2H), 6.86 (dd, J=8.90, 2.60Hz, 1H), 7.02 (d, J=2.60Hz, 1H), 7.27 - 7.38 (m, 1H), 7.57 (s, 2H), 10.81 (s, 1H) |

**Table 11**

| Ex. No. | Structural Formula | NMR |
|---|---|---|
| 53 | 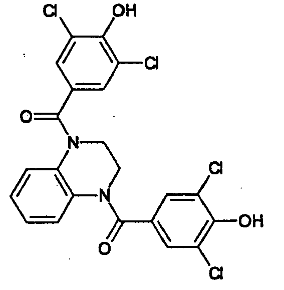 | (400 MHz, DMSO-d6) 3.99 (s, 4H) , 6.84 - 6.91 (m, 4H) , 7.47 (s, 4H), 10.75 (s, 2H) |
| 54 | 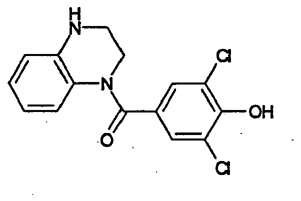 | (400 MHz, DMSO-d6) 3.38 (t, J=4.80Hz, 2H), 3.74 (t, J=4.80Hz, 2H), 6.20 (s, 1H), 6.29 (ddd, J=8.10, 7.70, 1.50Hz, 1H), 6.61 (dd, J=8 .10 , 1.50Hz, 1H), 6.65 (s, 1H), 6.82 (ddd, J=8.10, 7.70, 1.50Hz, 1H), 7.35 (s, 2H), 10.67 (s, 1H) |
| 55 | 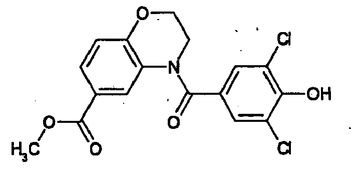 | (400 MHz, DMSO-d6) 3.78 (s, 3H), 3.88 (t, J=4.50Hz, 2H), 4.35 (t, J=4.50Hz, 2H), 7.02 (d, J=8.60Hz, 1H), 7.61 - 7.64 (m, 3H), 8.12 (s, 1H), 10.81 (s, 1H) |
| 56 | 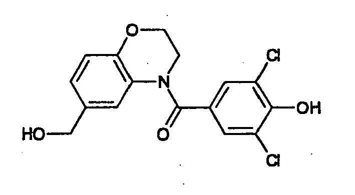 | (400 MHz, DMSO-d6) 3.84 (t, J=4.40Hz, 2H), 4.27 (t, J=4.40Hz, 2H), 4.27 - 4.35 (m, 2H), 4.97 - 5.09 (m, 1H), 6.85 (d, J=8.30Hz, 1H), 6.97 (dd, J=8.30, 1.90Hz, 1H), 7.30 - 7.41 (m, 1H), 7.55 (s, 2H), 10.78 (s, 1H) |
| 57 | 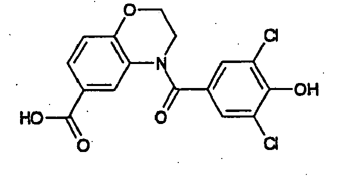 | (400 MHz, DMSO-d6) 3.88 (t, J=4.50Hz, 2H), 4.35 (t, J=4.50Hz, 2H), 7.00 (d, J=2.60Hz, 1H), 7.59 - 7.62 (m, 3H), 8.06 (s, 1H), 10.80 (s, 1H), 12.60 (s, 1H) |

**Table 12**

| Ex. No. | Structural Formula | NMR |
|---|---|---|
| 58 | 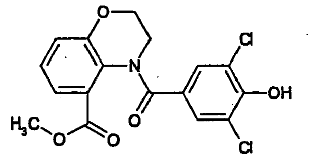 | (400 MHz, DMSO-d6) 3.56 (s, 3H), 3. 79 - 4.02 (m, 2H), 4.13 - 4 .35 (m, 2H), 7.15 - 7.54 (m, 5H), 10.83 (s, 1H) |
| 59 | 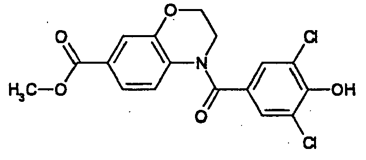 | (400 MHz, DMSO-d6) 3.82 (s, 3H), 3.90 (t, J=4.30Hz, 2H), 4.35 (t, J=4.30Hz, 2H), 7.37 - 7.47 (m, 3H), 7.60 (s, 2H), 10.86 (s, 1H) |
| 60 | 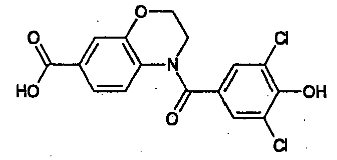 | (400 MHz, DMSO-d6) 3.89 (t, J=4.50Hz, 2H), 4.34 (t, J=4.50Hz, 2H), 7.34 - 7.41 (m, 3H), 7.59 (s, 2H), 10.86 (s, 1H), 12.85 (s, 1H) |
| 61 | 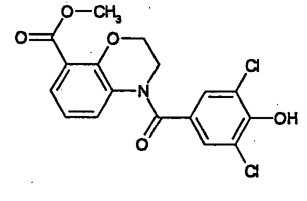 | (400 MHz, DMSO-d6) 3.79 (s, 3H), 3.90 (t, J=4.50Hz, 2H), 4.39 (t, J=4.50Hz, 2H), 6.84 (t, J=8.00Hz, 1H), 7. 34 - 7.41 (m, 1H), 7.40 (dd, J=7.70, 1.60Hz, 1H), 7.56 (s, 2H), 10.83 (s, 1H) |
| 62 | 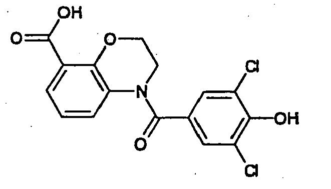 | (400 MHz, DMSO-d6) 3.89 (t, J=4.60Hz, 2H), 4.39 (t, J=4.60Hz, 2H), 6.77 - 6.84 (m, 1H), 7.33 (s, 1H), 7.38 (dd, J=7.90, 1.60Hz, 1H), 7.55 (s, 2H), 10.83 (s, 1H), 12.73 (s, 1H) |
| 63 | 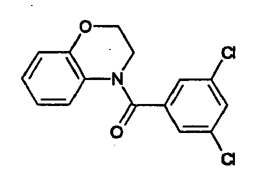 | (400 MHz, DMSO-d6) 3.84 (t, J=4.10Hz, 2H), 4.34 (t, J=4.10Hz, 2H), 6.78 (t, J=7.50Hz, 1H), 6.92 (dd, J=8.20, 1.40Hz, 1H), 7.00 - 7.08 (m, 1H), 7.60 (d, J=1.80Hz, 2H), 7.75 (t, J=1.80Hz, 1H) |

**Table 13**

| Ex. No. | Structural Formula | NMR |
|---|---|---|
| 64 | 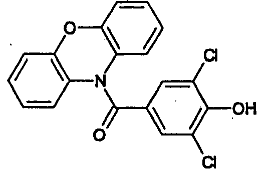 | (400 MHz, DMSO-d6) 7.02 - 7. 11 (m, 2H), 7.22 - 7.28 (m, 4H), 7.34 (s, 2H), 7.40 - 7.48 (m, 2H), 10.85 (s, 1H) |
| 65 | 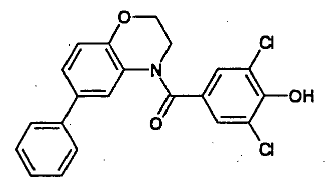 | (400 MHz, DMSO-d6) 3.92 (t, J=4.30Hz, 2H), 4.36 (t, J=4.30Hz, 2H), 6.99 (d, J=8.50Hz, 1H), 7.26 - 7.39 (m, 6H), 7.48 (s, 1H), 7.60 (s, 2H), 10.81 (s, 1H) |
| 66 | 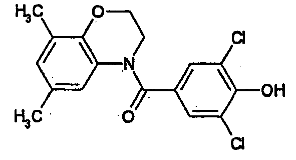 | (400 MHz, DMSO-d6) 2.06 (s, 3H), 2.12 (s, 3H), 3.82 (t, J=4.50Hz, 2H), 4.30 (t, J=4.50Hz, 2H), 6.72 (s,1H), 6.89 (s, 1H), 7.52 (s, 2H), 10.76 (s, 1H) |
| 67 | 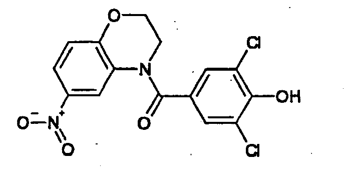 | (400 MHz, DMSO-d6) 3.91 (t, J=4.60Hz, 2H), 4.41 (t, J=4.60Hz, 2H), 7.15 (d, J=9.10Hz, 1H), 7.64 (s, 2H), 7.95 (dd, J=9.10, 2.80Hz, 1H), 8.53 (s, 1H), 10.87 (s, 1H) |
| 68 | 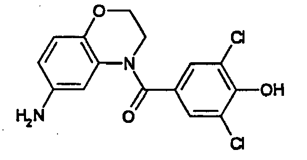 | (400 MHz, DMSO-d6) 3.78 (t, J=4.40Hz, 2H), 4.16 (t, J=4.40Hz, 2H), 6.30 (dd, J=8.70, 2.70Hz, 1H), 6.56 (s, 1H), 6.60 (d, J=8.70Hz, 1H), 7.51 (s, 2H) |
| 69 | 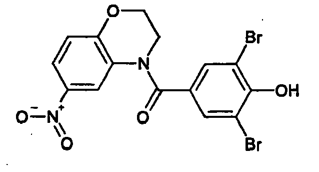 | (400 MHz, DMSO-d6) 3.91 (t, J=4.50Hz, 2H), 4.41 (t, J=4.50Hz, 2H), 7.15 (d, J=9.00Hz, 1H), 7.80 (s, 2H), 7.95 (dd, J=9.00, 2.70Hz, 1H), 8.53 (s, 1H), 10.62 (s, 1H) |
| 70 | 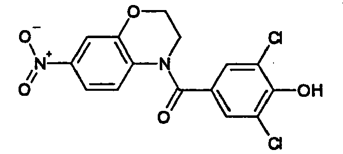 | (400 MHz, DMSO-d6) 3.93 (t, J=4.30Hz, 2H), 4.39 (t, J=4.30Hz, 2H), 7.61 - 7.74 (m, 3H), 7.65 (s, 2H), 10.84 (s, 1H) |

**Table 14**

| Ex. No. | Structural Formula | NMR |
|---|---|---|
| 71 | 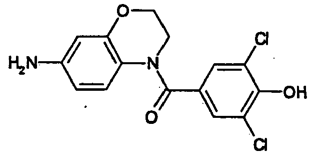 | (400 MHz, DMSO-d6) 3.79 (t, J=3.70Hz, 2H), 4.22 (t, J=3.70Hz, 2H), 5.90 - 6.04 (m, 1H), 6.04 - 6.16 (m, 1H), 6.86 (s, 1H), 7.47 (s, 1H) |
| 72 | 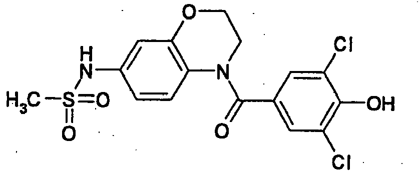 | (400 MHz, DMSO-d6) 2.95 (s, 3H), 3.85 (t, J=4.20Hz, 2H), 4.29 (t, J=4.20Hz, 2H), 6.63 (dd, J=8.60, 2.30Hz, 1H), 6.77 (d, J=2.60Hz, 1H), 7.24 (s, 1H), 7.53 (s, 2H), 9.64 (s, 1H) , 10.75 (s, 1H) |
| 73 | 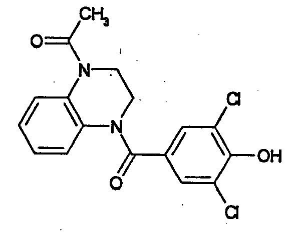 | (400 MHz, DMSO-d6) 2.25 (s, 3H), 3.92 (s, 4H), 6.87 (d, J=7.90Hz, 1H), 6.94 - 7.03 (m, 1H), 7.09 - 7.21 (m, 1H), 7.32 (s, 2H), 7.68 (s, 1H), 10.77 (s, 1H) |
| 74 | 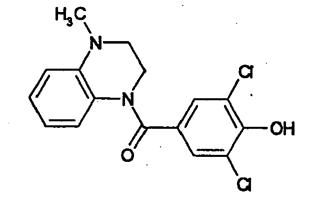 | (400 MHz, DMSO-d6) 2.94 (s, 3H), 3.40 (t, J=5. 40Hz, 2H), 3.84 (t, J=5.40Hz, 2H), 6.35 - 6.43 (m, 1H), 6.70 (s, 1H), 6.74 (dd, J=8.40, 1.20Hz, 1H), 6.92 - 6.99 (m, 1H) , 7.33 (s, 2H), 10.70 (s, 1H) |
| 75 | 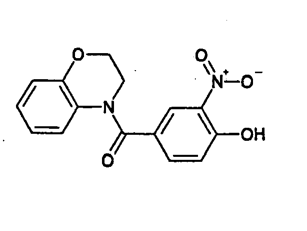 | (400 MHz, DMSO-d6) 3.89 (t, J=4.40Hz, 2H), 4.32 (t, J=4.40Hz, 2H), 6.75 (dd, J=7.64, 7.64Hz, 1H), 6.91 (dd, J=8.10, 1.62Hz, 1H), 7.01 (ddd, J=8.10, 7.64, 1.62Hz, 1H), 7.14 (d, J=8.57Hz, 1H), 7.18 (brs, 1H), 7.68 (dd, J=8.57, 2.20Hz, 1H), 8.05 (d, J=2.20Hz, 1H), 11.61 (brs, 1H) |

**Table 15**

| Ex. No. | Structural Formula | NMR |
|---|---|---|
| 76 | 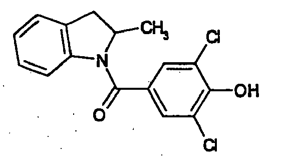 | (400 MHz, DMSO-d6) 1.07 (s, 3H), 2.56 - 2.70 (m, 1H), 3.38 - 3.51 (m, 1H), 4.54 - 4.69 (m, 1H), 6.96 - 7.07 (m, 1H), 7.07 - 7.20 (m, 1H), 7.24 - 7.32 (m, 1H), 7.42 (s, 1H), 7.62 (s, 2H), 10.74 (s, 1H) |
| 77 | 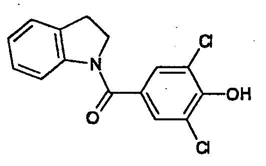 | (400 MHz, DMSO-d6) 3.08 (t, J=8.30Hz, 2H), 4.05 (t, J=8.30Hz, 2H), 6.98 - 7.09 (m, 1H), 7.11 - 7.22 (m, 1H), 7.24 - 7.34 (m, 1H), 7.63 (s, 2H), 7.88 (s, 1H), 10.73 (s, 1H) |
| 78 | 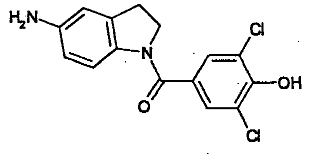 | (400 MHz, DMSO-d6) 3.06 (t, J=8.10Hz, 2H), 4.06 (t, J=8.10Hz, 2H), 6.90 (d, J=8.30Hz, 1H), 6.99 (s, 1H), 7.12 - 7.27 (m, 1H), 7.62 (s, 2H) |
| 79 | 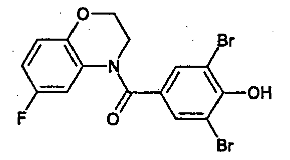 | (400 MHz, DMSO-d6) 3.84 (t, J=4.19Hz, 2H), 4.25 (t, J=4.19Hz, 2H), 6.87 - 6.95 (m, 2H), 7.33 (d, J=10.65Hz, 1H), 7.75 (s, 2H), 10.60 (brs, 1H) |
| 80 | 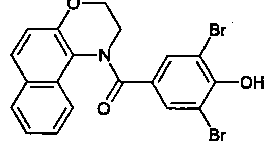 | (400 MHz, DMSO-d6) 3.70 - 4.50 (m, 4H), 7.10 - 7.45 (m, 5H), 7.74 - 8.05 (m, 3H), 10.73 (brs, 1H) |
| 81 | 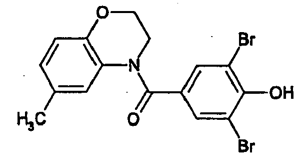 | (400 MHz, DMSO-d6) 2.11 (s, 3H), 3.86 (t, J=4.17Hz, 2H), 4.25 (t, J=4. 17Hz, 2H), 6.78 - 6.85 (m, 2H), 7.15 (brs, 1H), 7.70 (s, 2H), 10.54 (brs, 1H) |
| 82 | 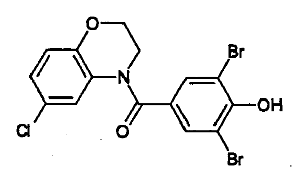 | (400 MHz, DMSO-d6) 3.84 (t, J=4.50Hz, 2H), 4.27 (t, J=4.50Hz, 2H), 6.95 (d, J=8.80Hz, 1H), 7.08 (dd, J=8.80, 2.50Hz, 1H), 7.55 (s, 1H), 7.75 (s, 2H), 10.60 (s, 1H) |

**Table 16**

| Ex. No. | Structural Formula | NMR |
|---|---|---|
| 83 | 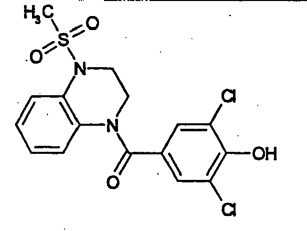 | (400 MHz, DMSO-d6) 3.20 (s, 3H), 3.87 - 4.00 (m, 4H), 6.94 - 7.03 (m, 1H), 7.09 - 7.18 (m, 2H), 7.41 (s, 2H), 7.69 (d, J=8. 40Hz , 1H), 10.81 (s, 1H) |
| 84 | 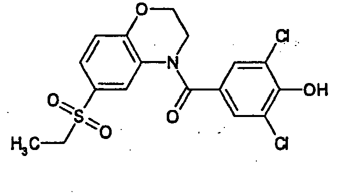 | (400 MHz, DMSO-d6) 0.99 (t, J=7.30Hz, 3H), 3.09 (q, J=7.30Hz, 2H), 3.92 (t, J=4.50Hz, 2H), 4.42 (t, J=4.50Hz, 2H), 7.16 (d, J=8.70Hz, 1H), 7.50 (dd, J=8.70, 2.20Hz, 1H), 7.61 (s, 2H) , 7.80 (s, 1H) , 10.86 (s, 1H) |
| 85 | 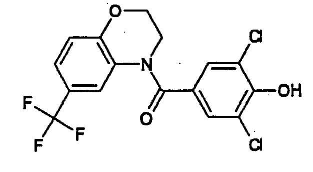 | (400 MHz, DMSO-d6) 3.90 (t, J=4.50Hz, 2H), 4.35 (t, J=4.50Hz, 2H), 7.11 (d, J=8.50Hz, 1H), 7.38 (dd, J=8.50, 2.10Hz, 1H), 7.60 (s, 2H), 7.83 (s, 1H), 10.85 (s, 1H) |
| 86 | 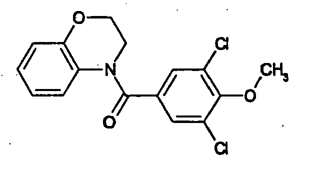 | (400 MHz, DMSO-d6) 3.85 (t, J=4.40Hz, 2H), 3.87 (s, 3H), 4.32 (t, J=4.40, 2H), 6.79 (dd, J=7.99, 7.99Hz, 1H), 6.92 (dd, J=8.10, 1.39Hz, 1H), 7.04 (ddd, J=8.10, 7.18, 1.39Hz, 1H), 7.37 (brs, 1H), 7.68 (s, 2H) |
| 87 | 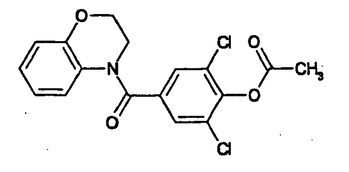 | (400 MHz, DMSO-d6) 2.44 (s, 3H), 3.85 (t, J=4.18Hz, 2H), 4.33 (t, J=4.18Hz, 2H), 6.80 (dd, J=7.41, 7.41Hz, 1H), 6.92 (dd, J=8.34, 1.39Hz, 1H), 7.05 (ddd, J=8.10, 7.18, 1.39Hz, 1H), 7.42 (brs, 1H), 7.80 (s, 2H) |

**Table 17**

| Ex. No. | Structural Formula | NMR |
|---|---|---|
| 88 | 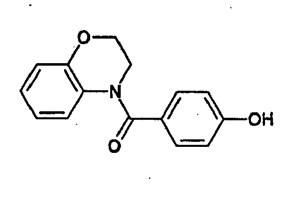 | (400 MHz, DMSO-d6) 3.87 (t, J=4.40Hz, 2H); 4.28 (t, J=4.40Hz, 2H), 6.72 (ddd, J=8.10, 7.18, 1.62Hz, 1H), 6.78 (d, J=8.57Hz, 2H), 6.88 (dd, J=8.10, 1.62Hz, 1H), 6.97 (ddd, J=8.22, 7.18, 1.62Hz, 1H) , 7.13 (d, J=8.10Hz, 1H), 7.39 (d, J=8.57Hz, 2H), 10.06 (brs, 1H) |
| 89 | 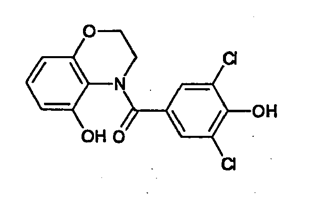 | (400 MHz, DMSO-d6) 3.85 (brs, 2H), 4.31 (t, J=4.75Hz, 2H), 6.24 (dd, J=8.11, 1.16Hz, 1H), 6.38 (dd, J=8.11, 1.16Hz, 1H), 6.65 (s, 1H) , 6.87 (dd, J=8.11, 8.11Hz, 1H), 7.39 (s, 1H), 9.39 (s, 1H), 10.69 (brs, 1H) |
| 90 | 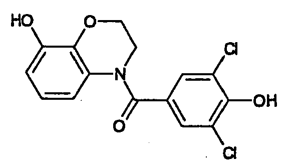 | (400 MHz, DMSO-d6) 3.86 (t, J=4.55Hz, 2H), 4.35 (t, J=4.55Hz, 2H), 6.42 - 6.59 (m, 3H), 7.51 (s, 2H), 9.14 (s, 1H) , 10.77 (brs, 1H) |
| 91 | 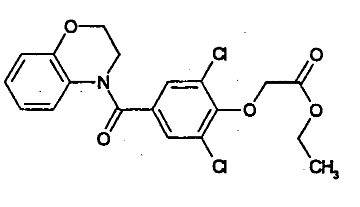 | (400 MHz, DMSO-d6) 1.24 (t, J=7.20Hz, 3H), 3.86 (t, J=4.20Hz, 2H), 4.22 (q, J=7.20Hz, 2H), 4.34 (t, J=4.20Hz, 2H), 4.76 (s, 2H), 6.78 - 6.82 (m, 1H), 6.92 - 6.94 (m, 1H), 7.03 - 7. 07 (m, 1H) , 7.36 (brs, 1H), 7.69 (s, 2H) |
| 92 | 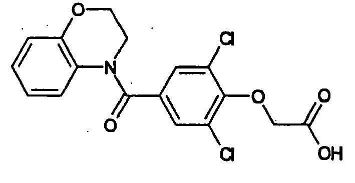 | (400 MHz, DMSO-d6) 3.85 (t, J=4.40Hz, 2H), 4.32 (t, J=4.40Hz, 2H), 4.64 (s, 2H), 6.80 (ddd, J=8.00, 7.60, 1.60Hz, 1H), 6.93 (dd, J=7.60, 1.60Hz, 1H), 7.05 (ddd, J=8.00, 7.60, 1.60Hz, 1H), 7.38 (brs, 1H), 7.68 (s, 2H), 13.14 (brs, 1H) |

**Table 18**

| Ex. No. | Structural Formula | NMR |
|---|---|---|
| 93 | 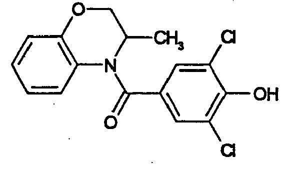 | (400 MHz, DMSO-d6) 1.16 (d, J=6.80Hz, 3H), 4.19 - 4.22 (m, 1H), 4.34 - 4.37 (m, 1H), 4.54 - 4.56 (m, 1H), 6.71 - 6.75 (m, 1H), 6.91 - 7.04 (m, 3H), 7.50 (s, 2H), 10.78 (s, 1H) |
| 94 | 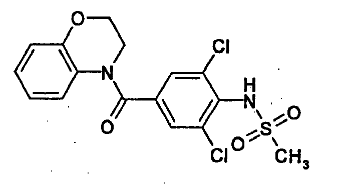 | (400 MHz, DMSO-d6) 3.20 (s, 3H), 3.80 - 3.89 (m, 2H), 4.28 - 4.38 (m, 2H), 6.73 - 6.85 (m, 1H), 6.89 - 6.96 (m, 1H), 7. 00 - 7.08 (m, 1H) , 7.42 (brs, 1H) , 7.73 (s, 2H), 9.76 (s, 1H) |
| 95 | 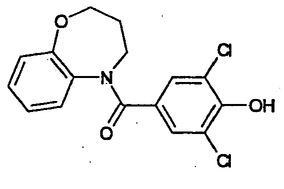 | (400 MHz, DMSO-d6) 1.72 - 2.12 (m, 2H), 2.71 - 4.99 (m, 4H), 6. 81 - 6.92 (m, 2H), 7.05 (s, 2H), 7.11 - 7.22 (m, 2H) |

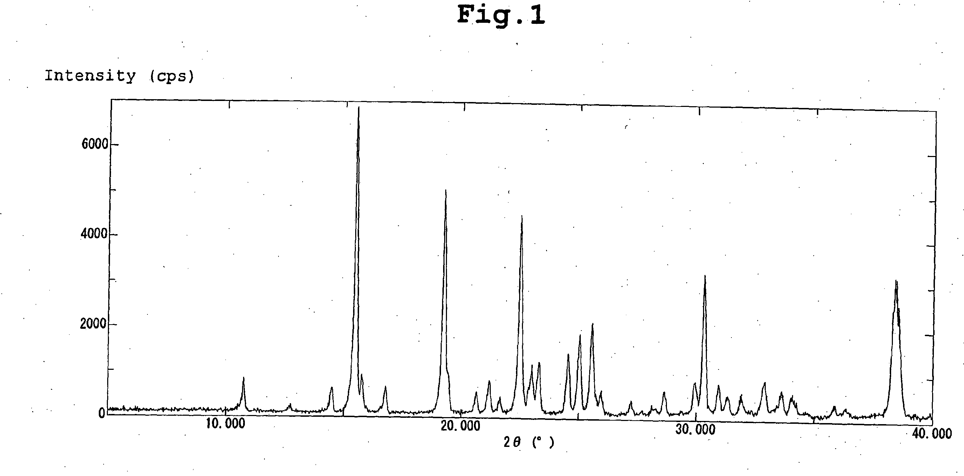

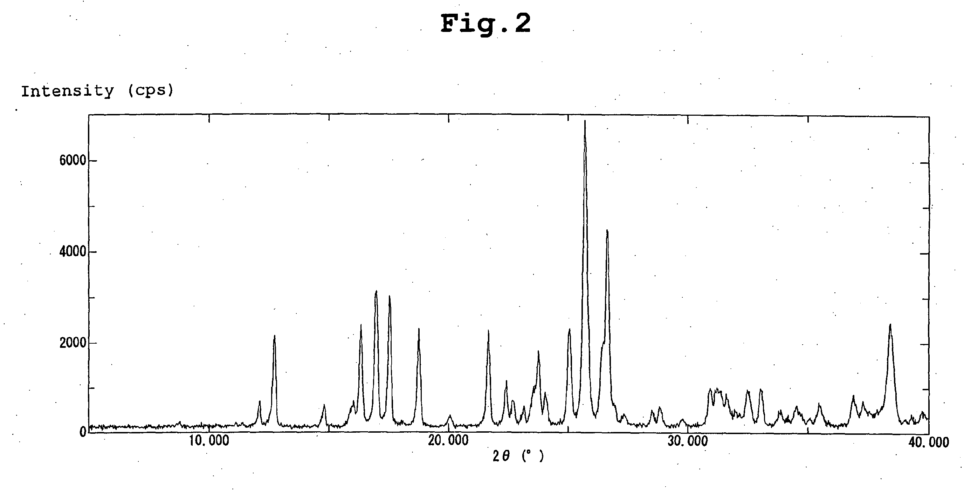

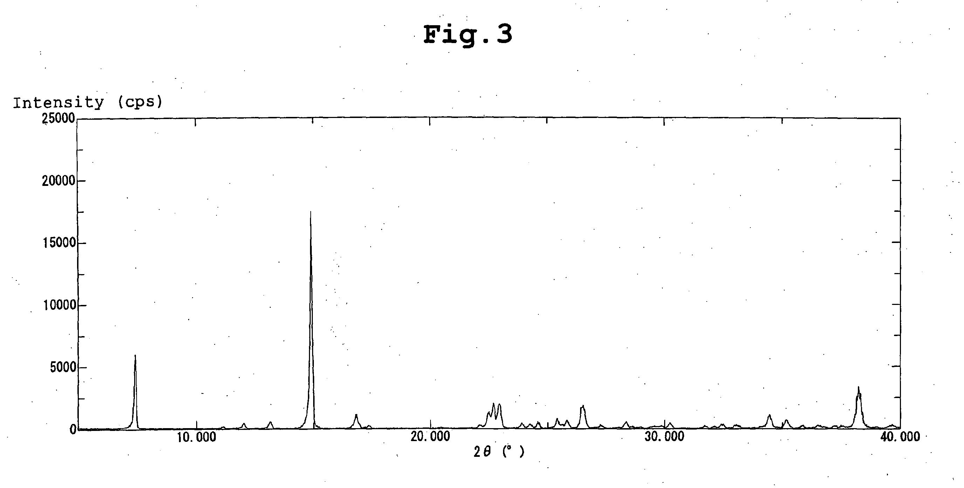

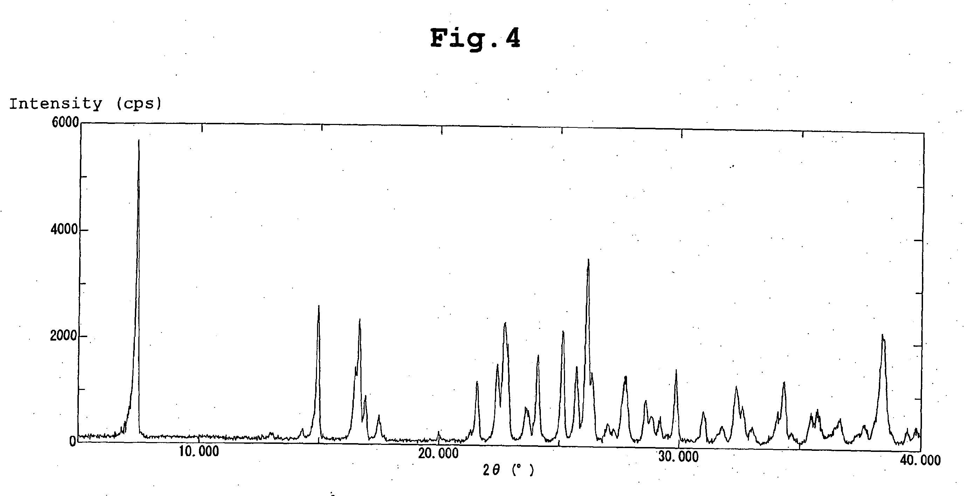

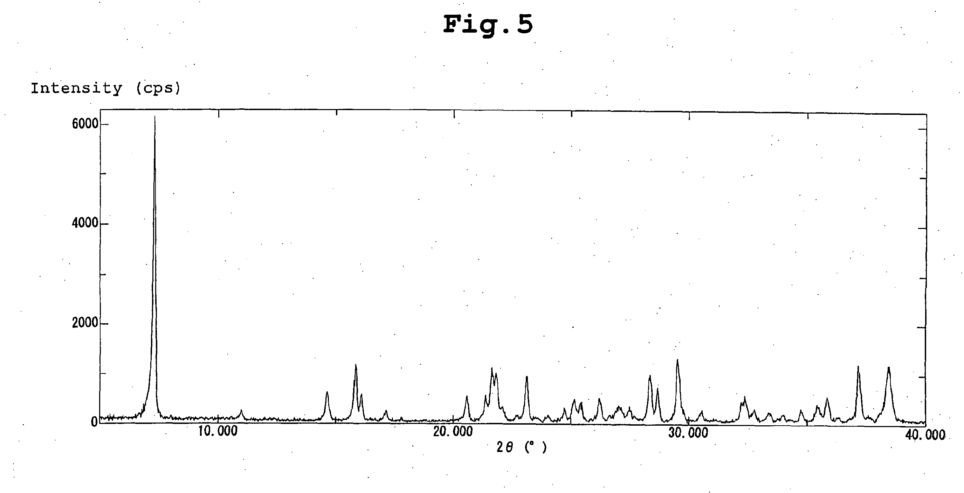

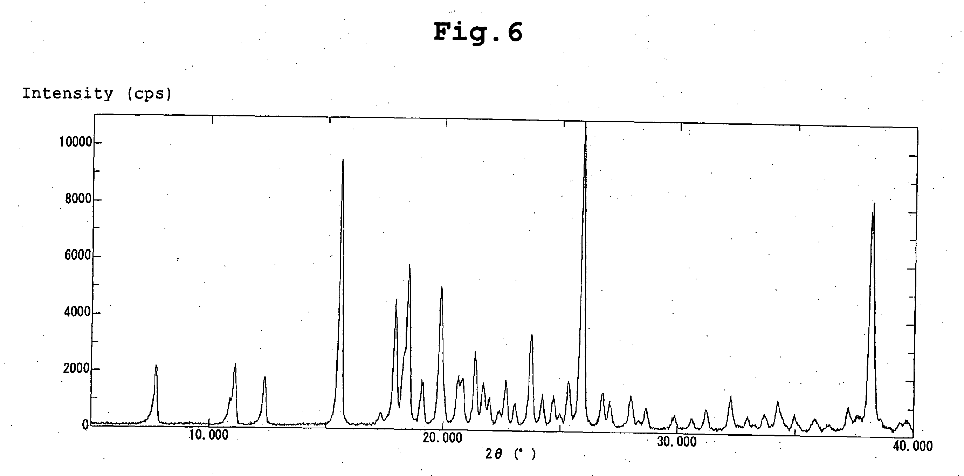

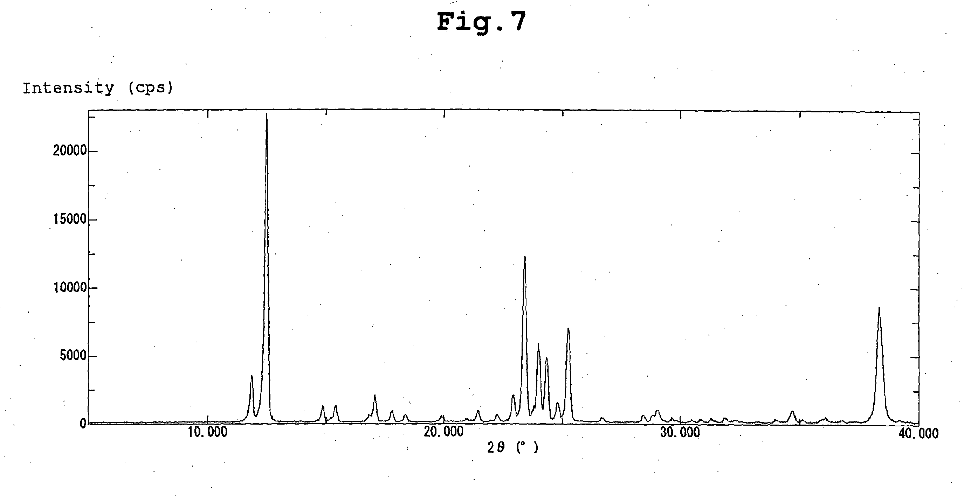

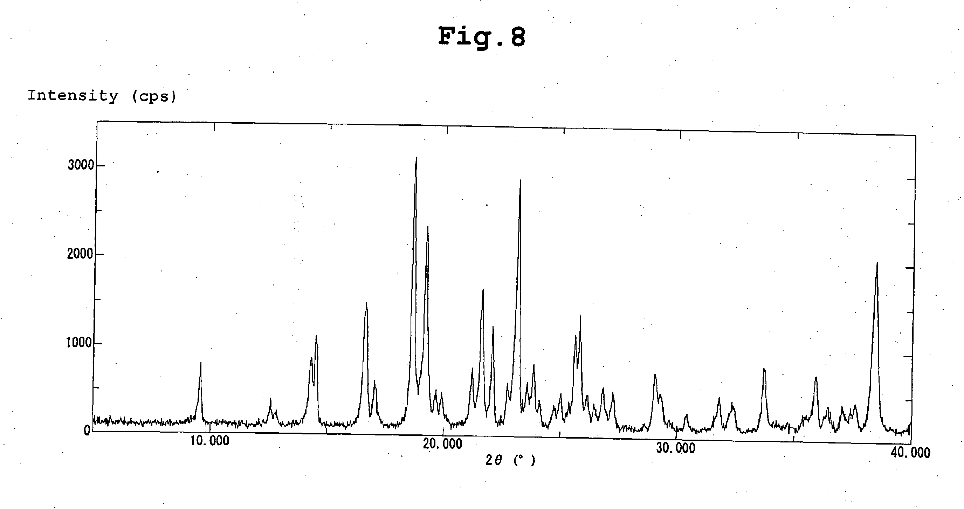

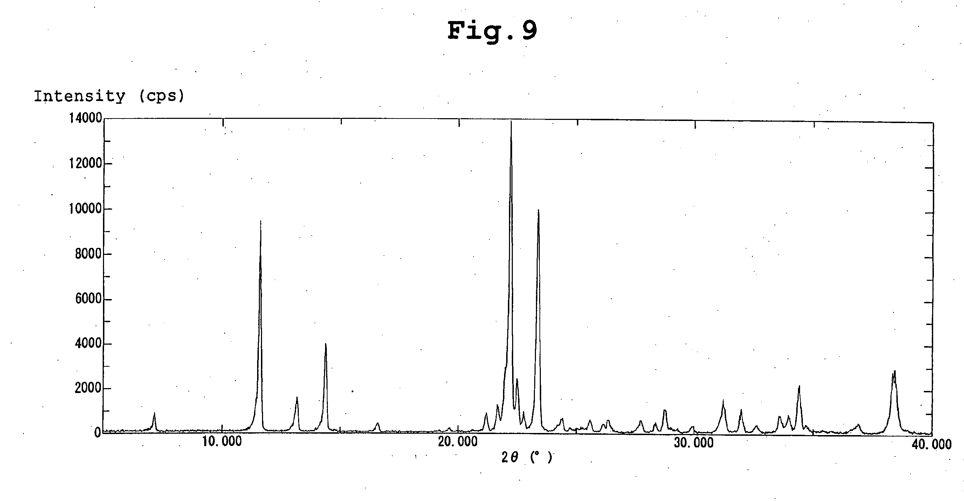

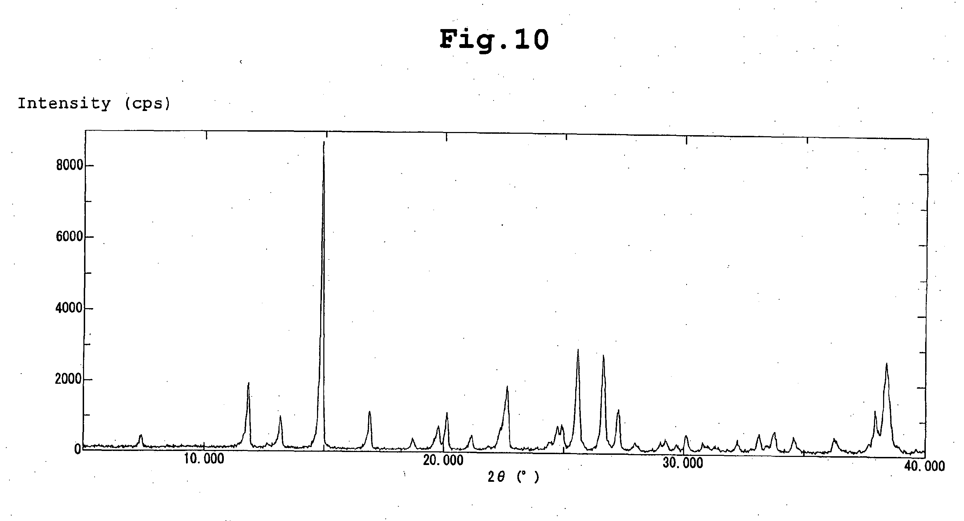

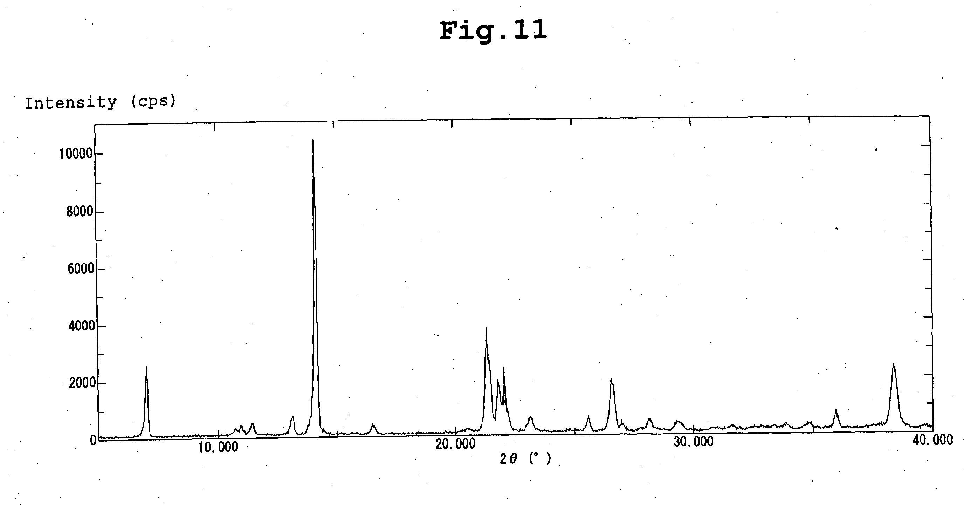

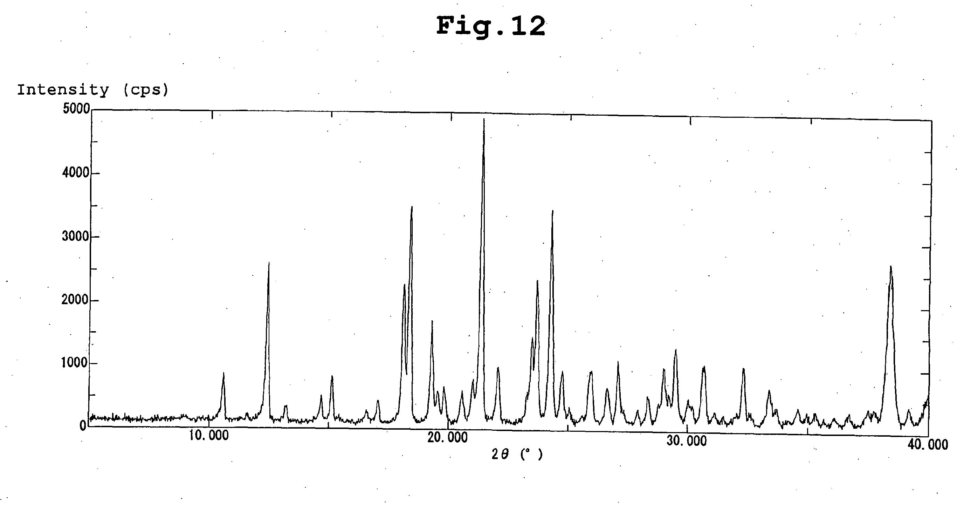

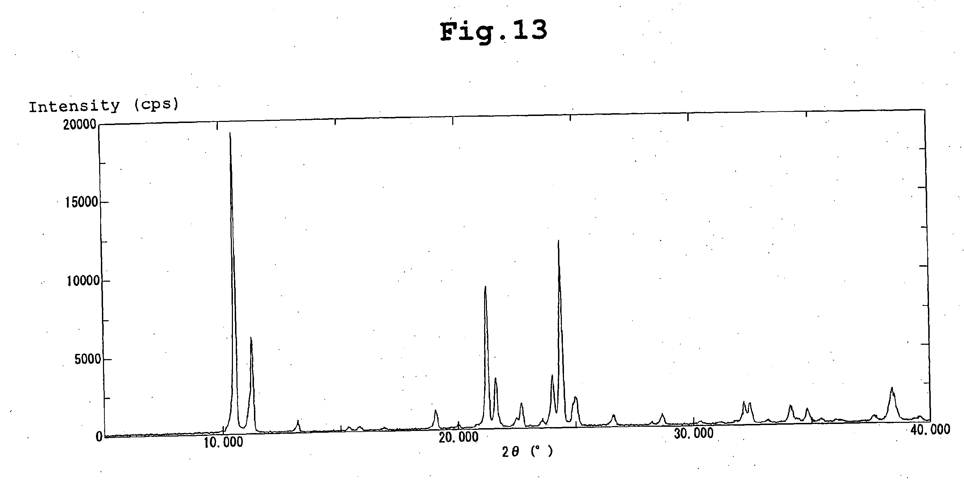

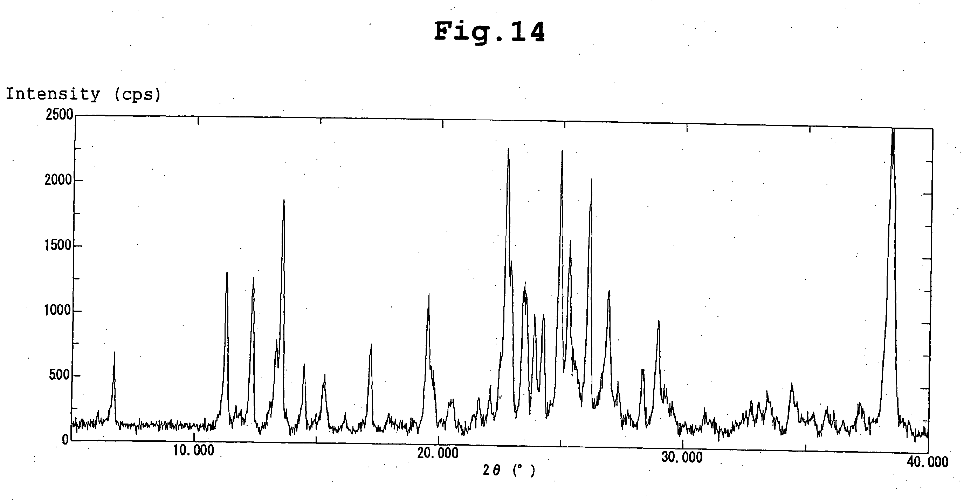

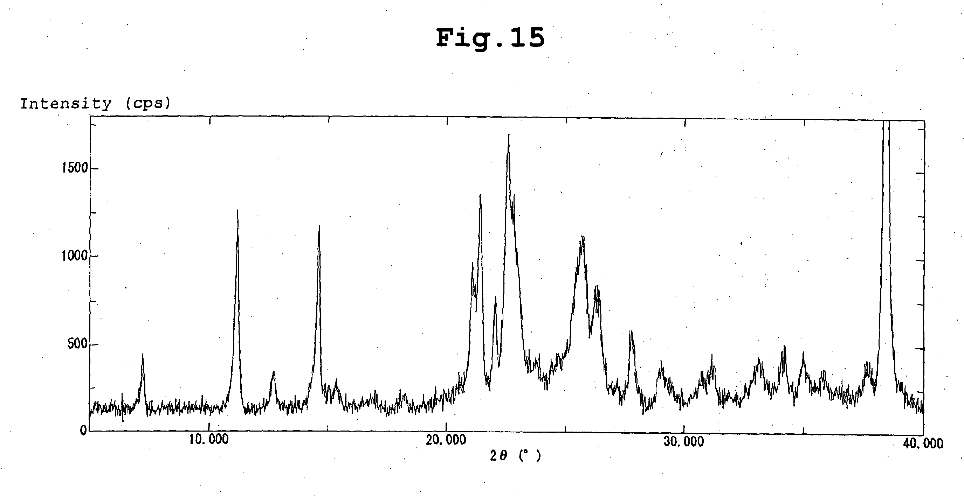

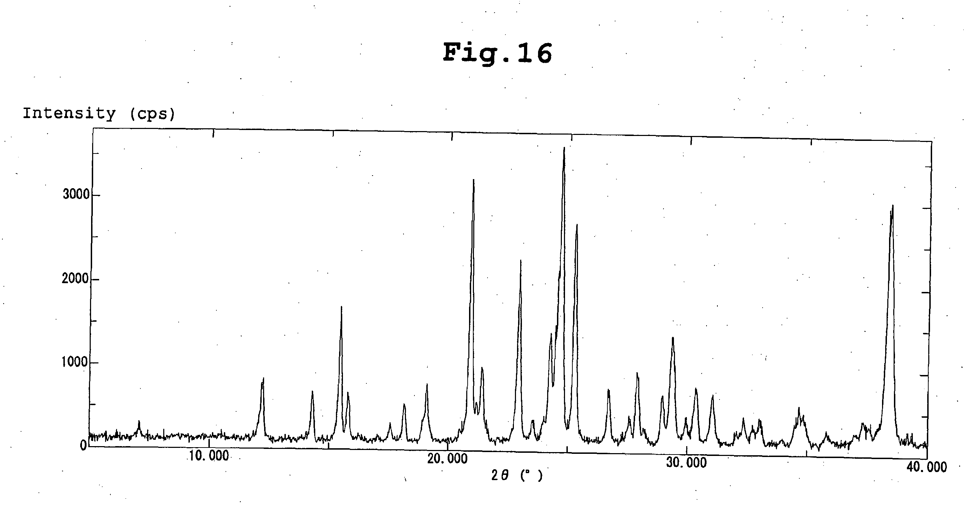

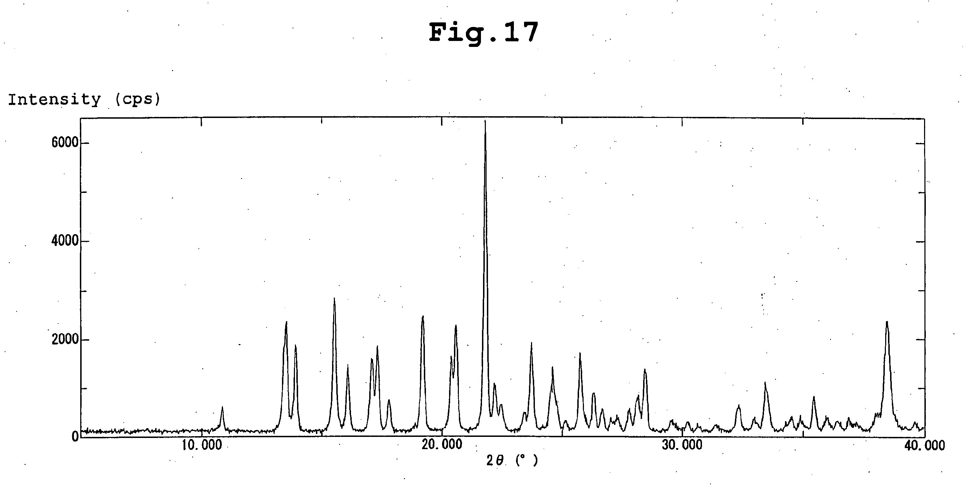

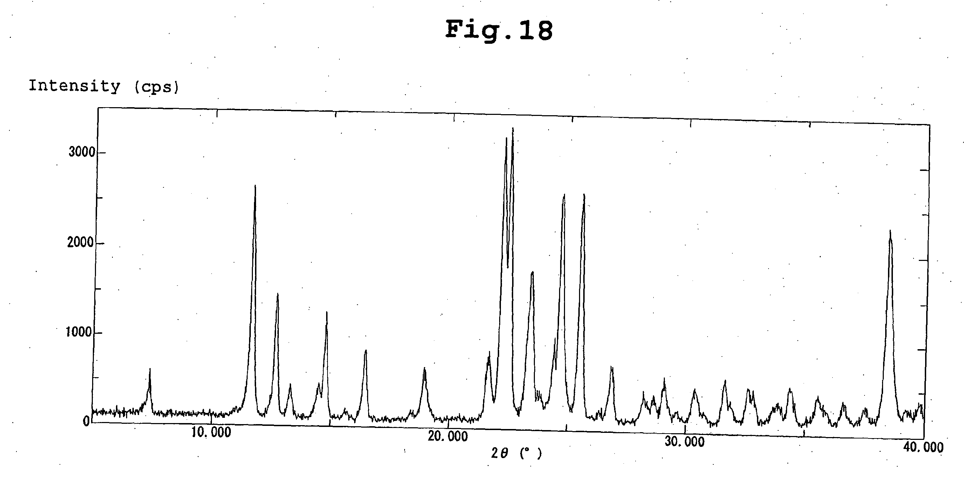

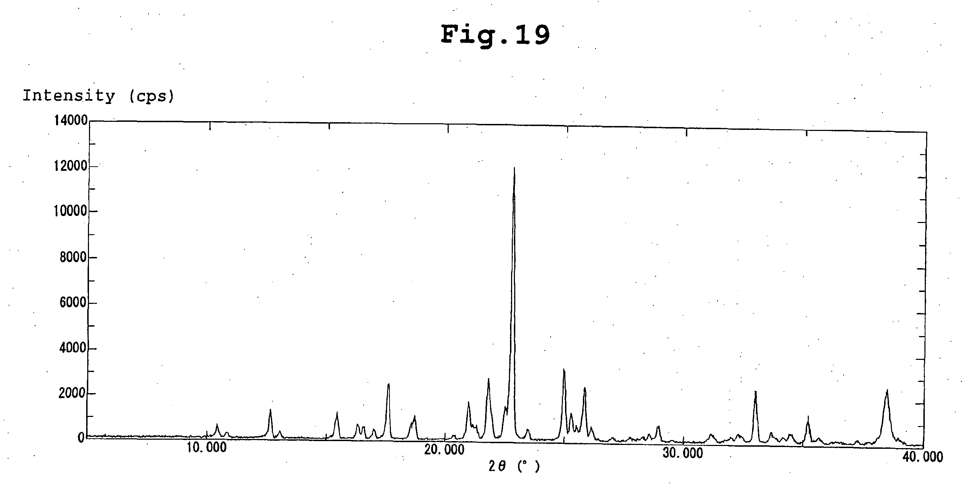

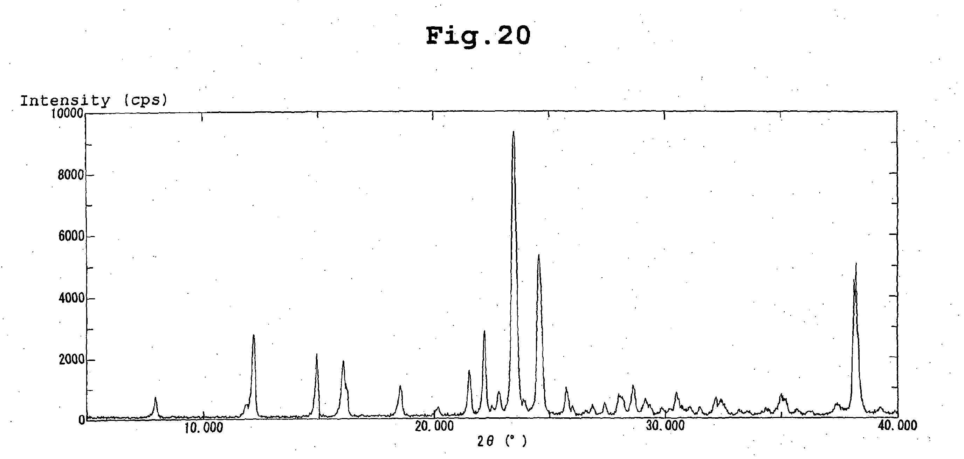

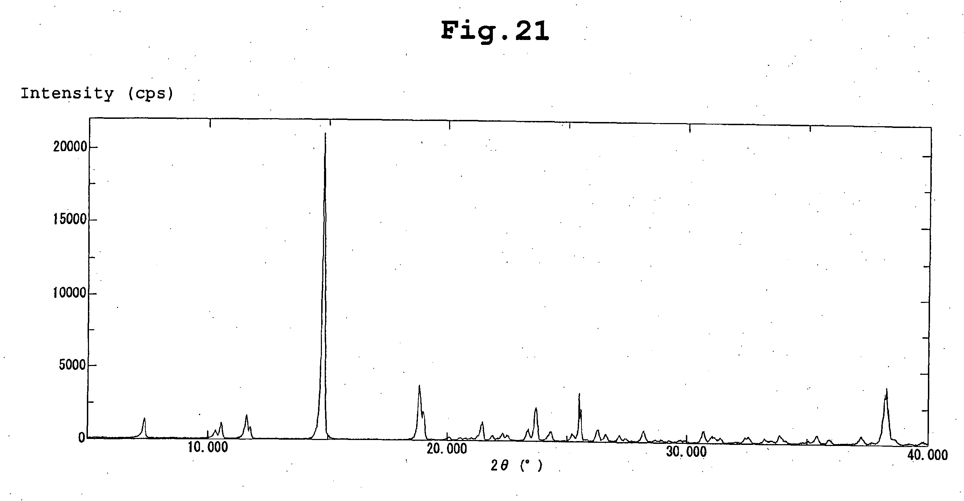

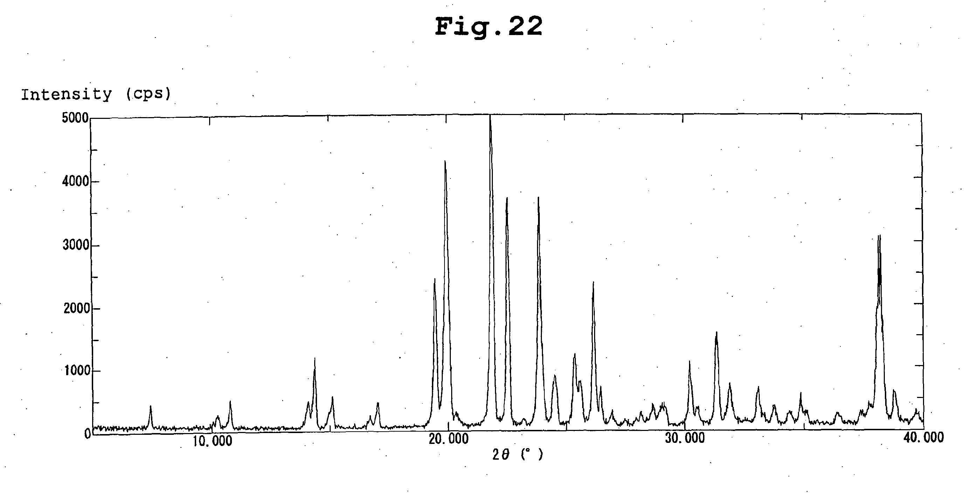

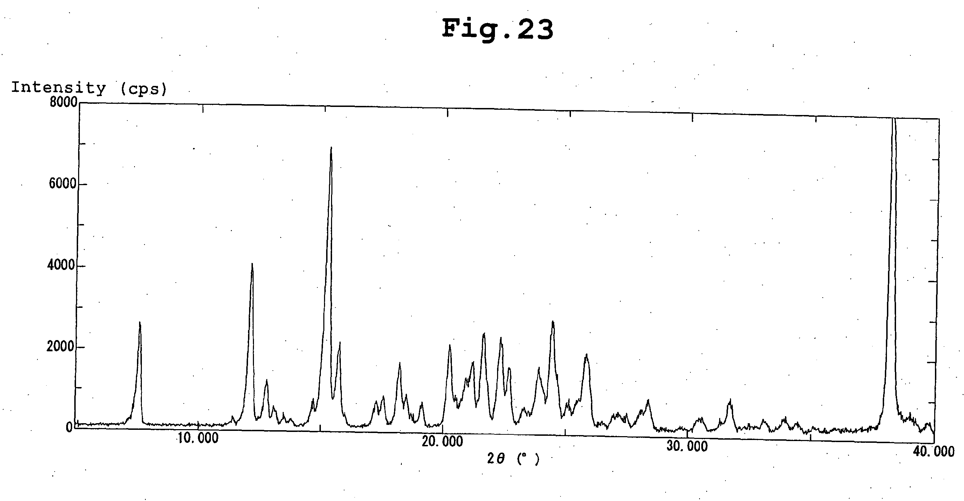

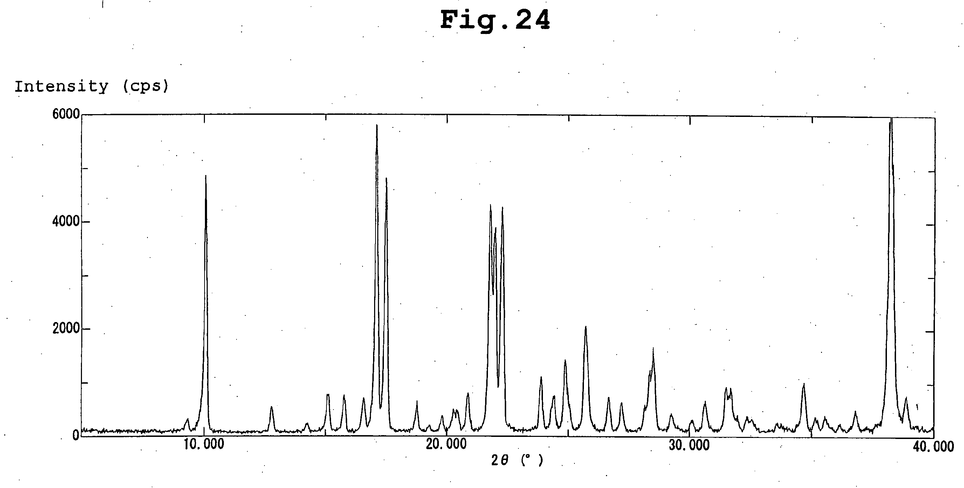

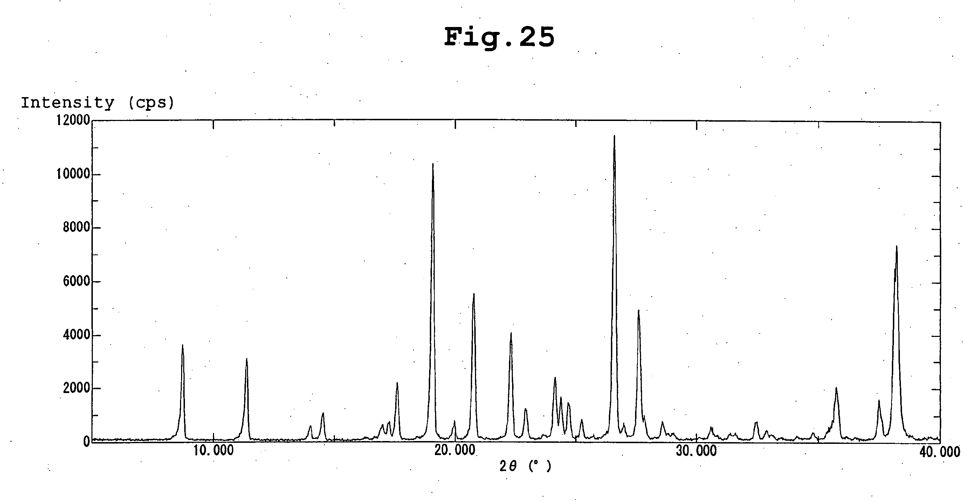

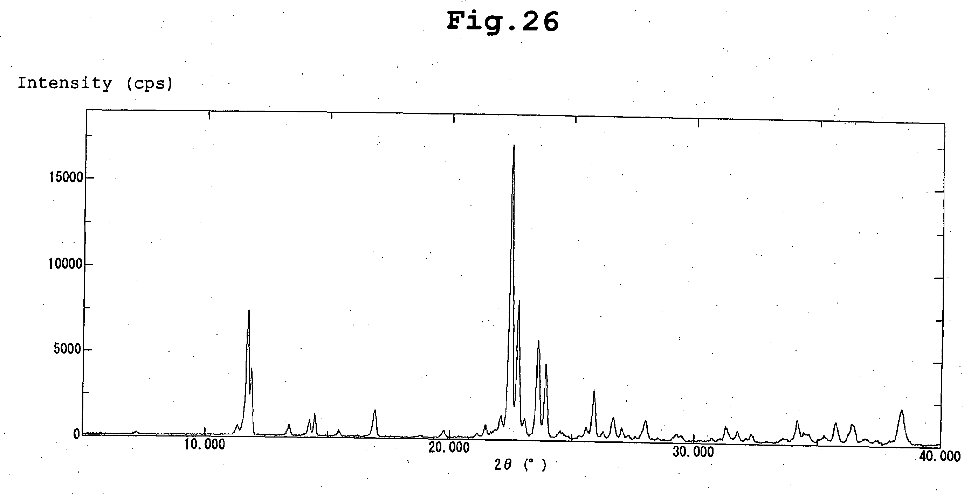

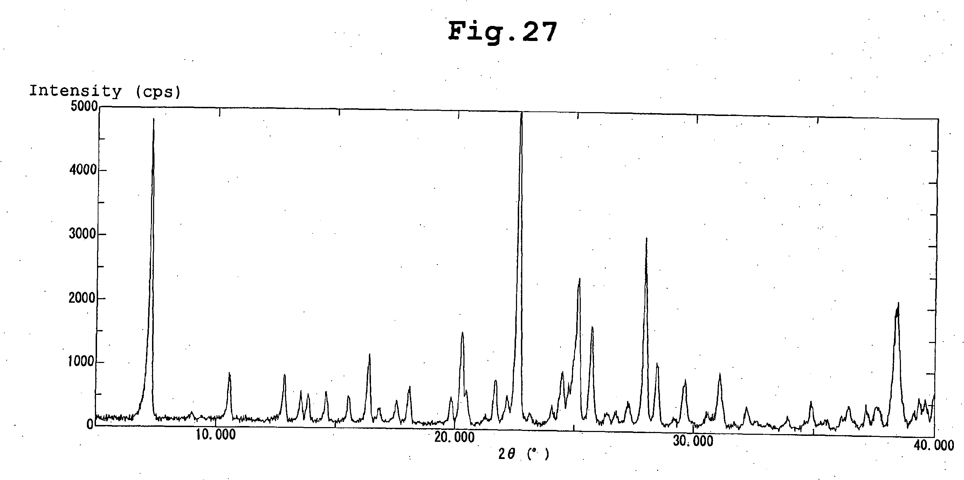

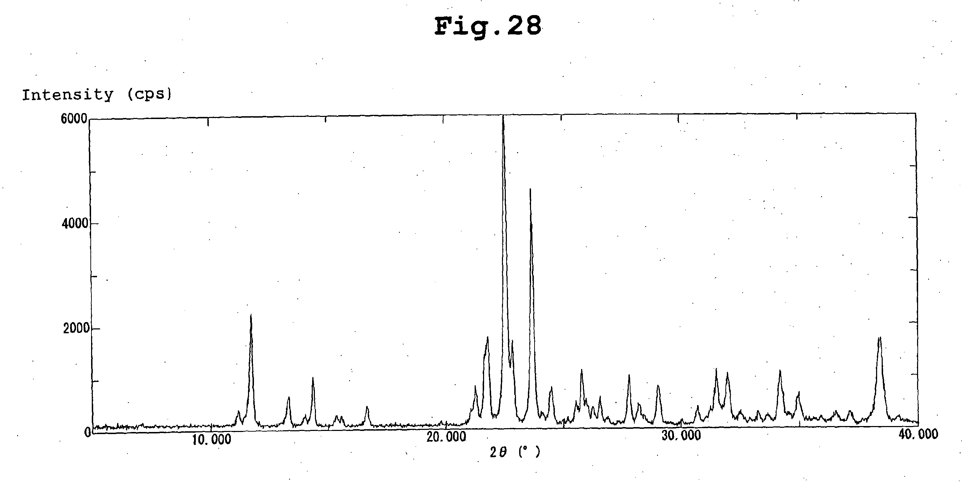

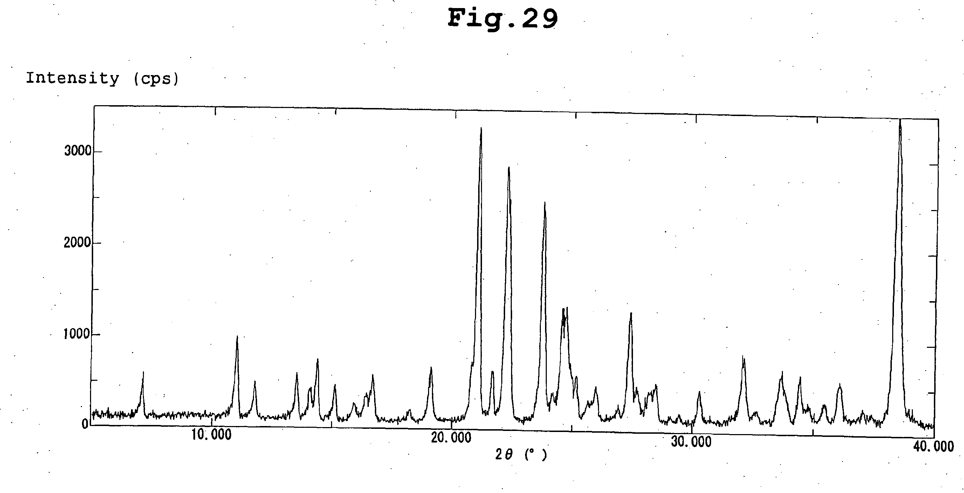

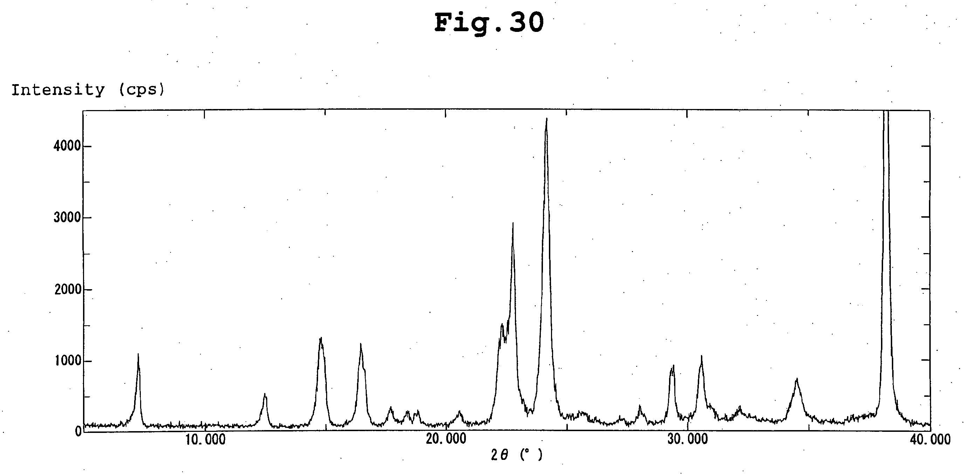

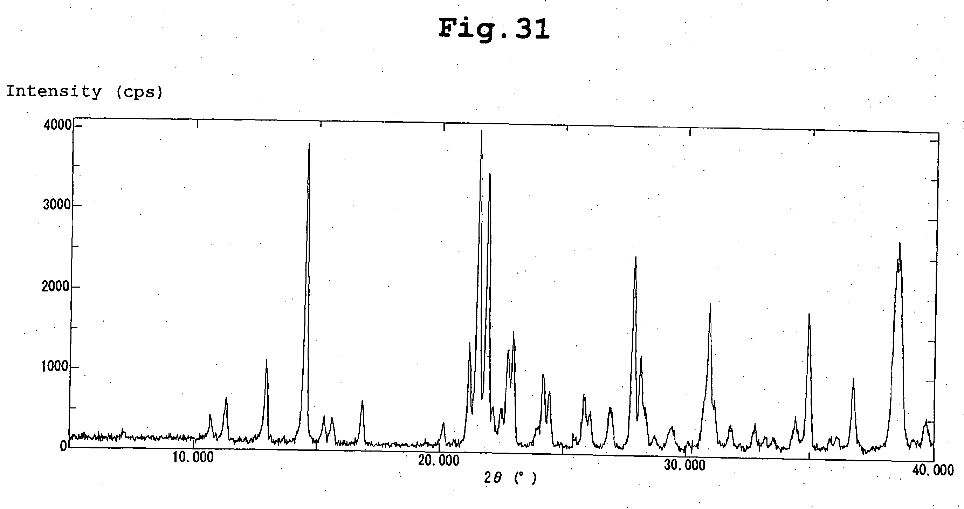

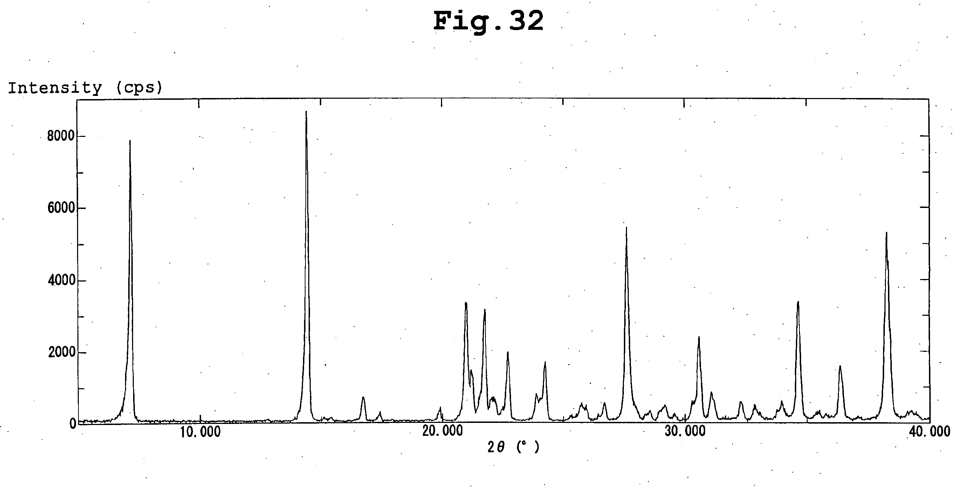

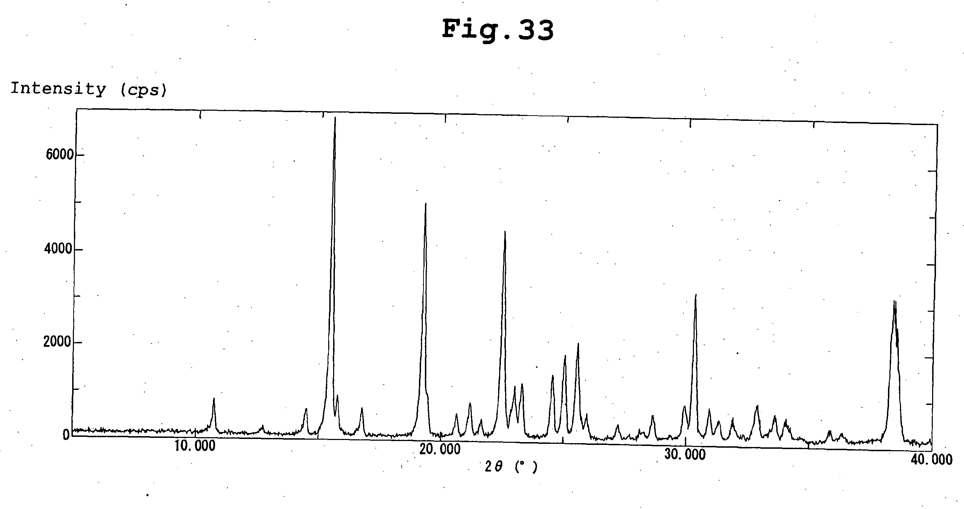

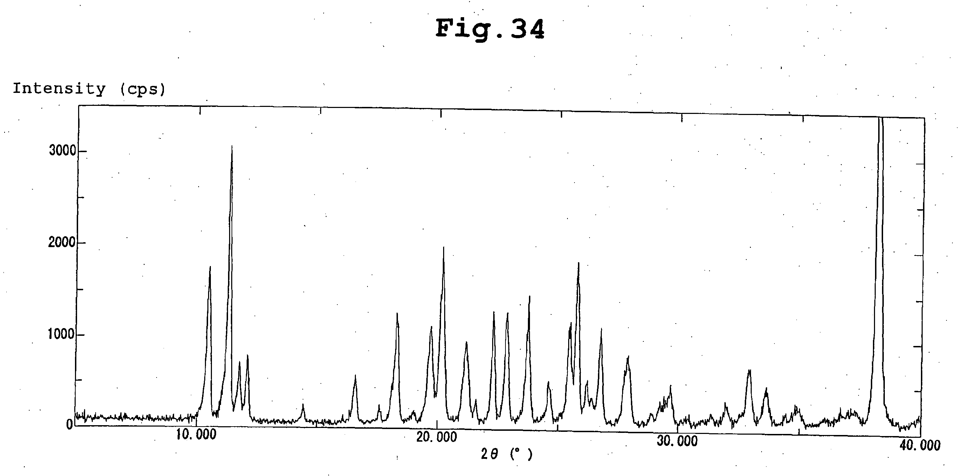

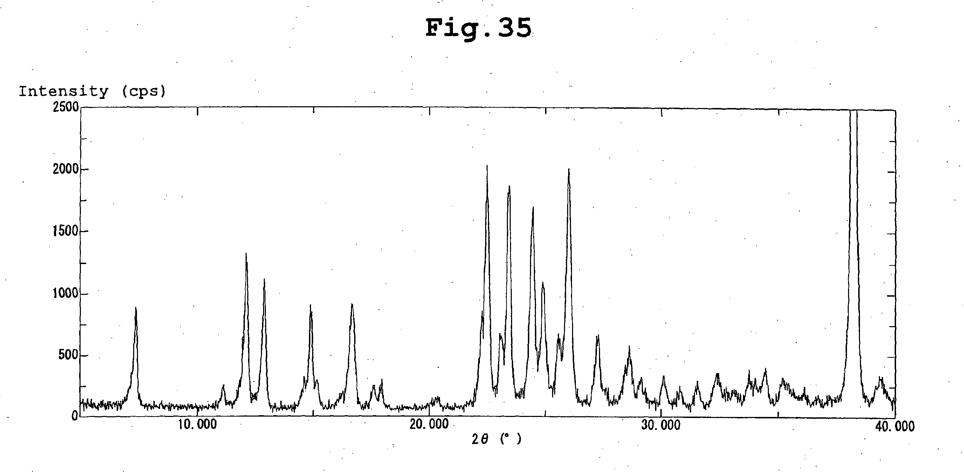

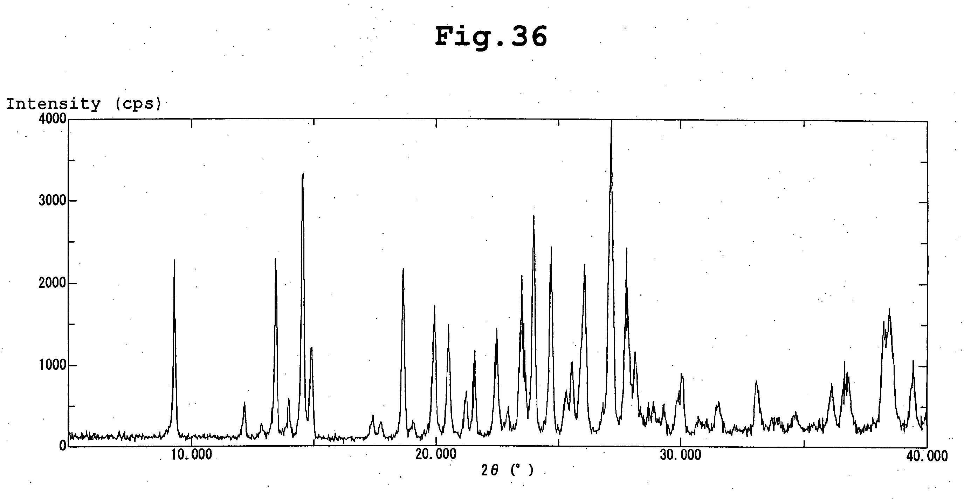

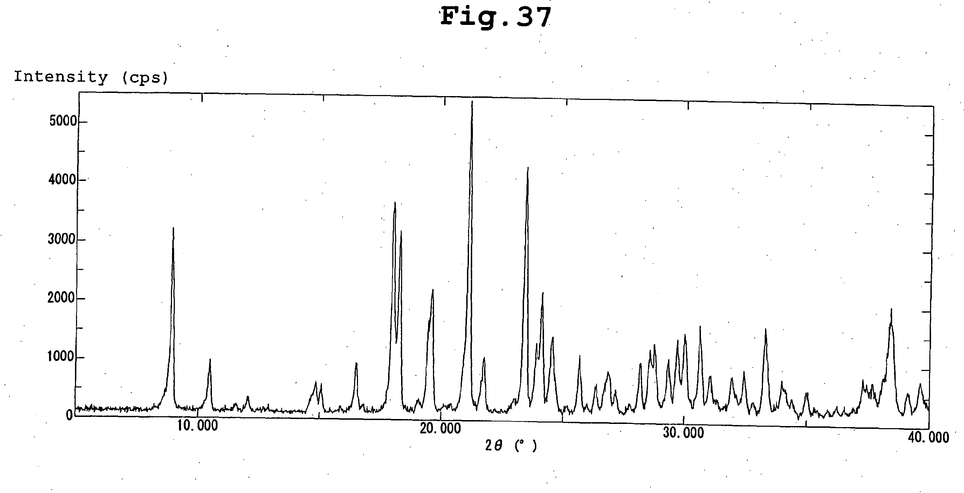

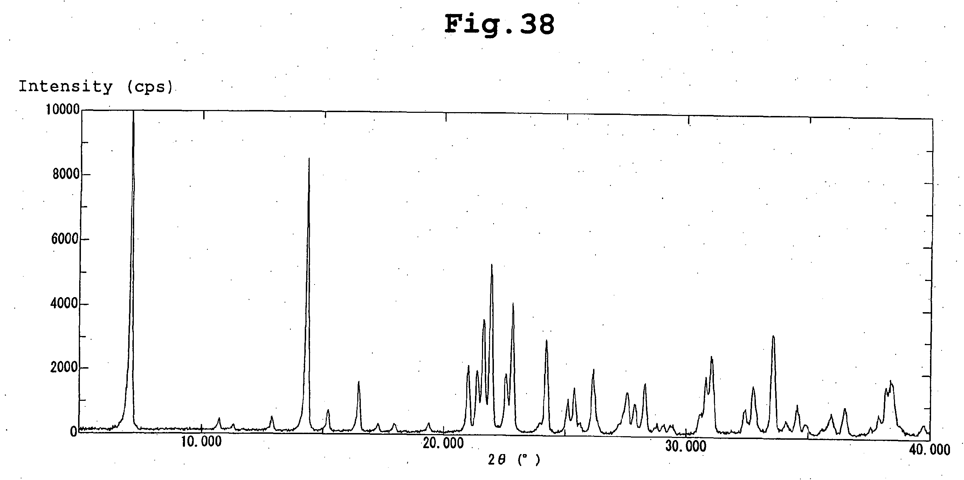

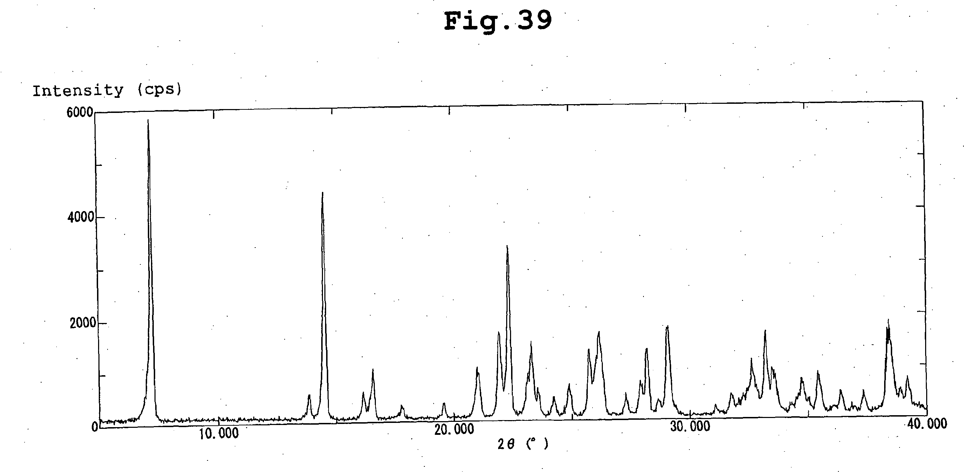

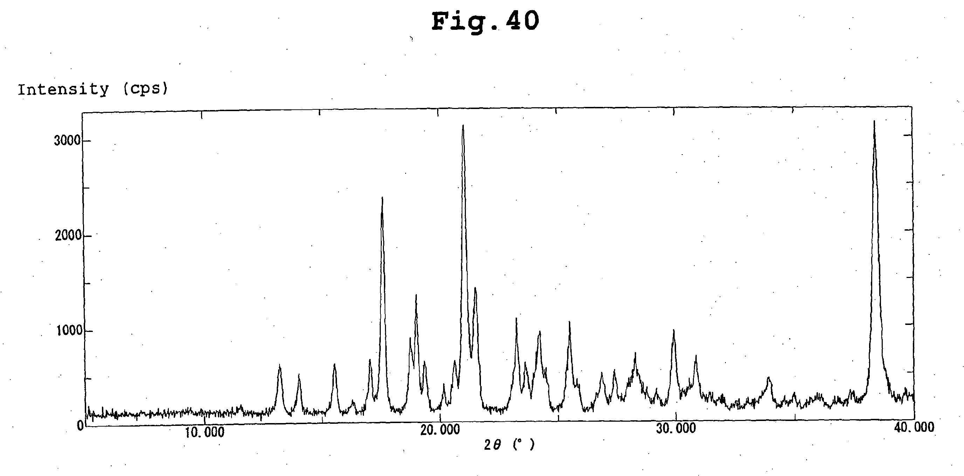

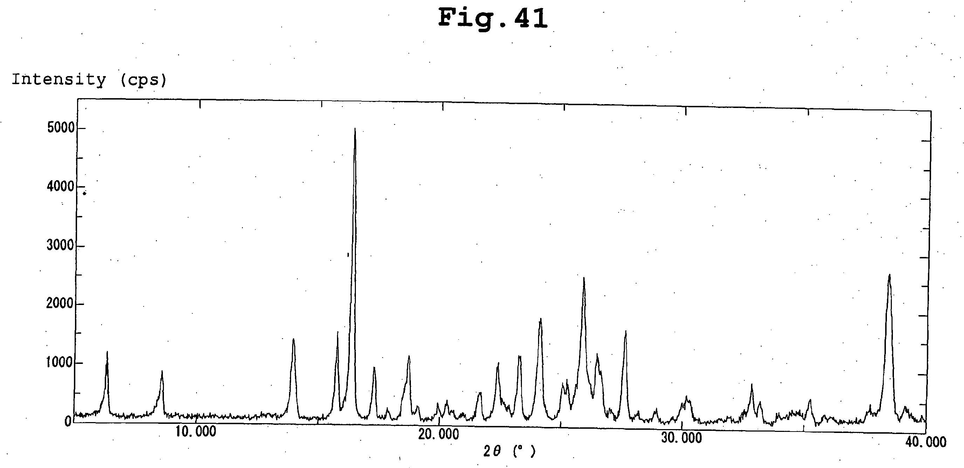

The powder X-ray diffraction patterns of the crystals of the present invention are shown in Fig. 1 - Fig. 41, wherein the horizontal axis shows the diffraction angle (2θ) and the vertical axis shows the peak intensity (cps).

Based on the powder X-ray diffraction patterns, the diffraction angles (2θ) of the characteristic diffraction peaks of each crystal are listed in Table 19 -Table 22.

Each sample crystal of the present invention was mounted on an aluminum cell, and the powder X-ray diffraction pattern was measured using a powder X-ray diffractometer (RINT2100 Ultima+, manufactured by Rigaku) at X-ray radiation: Cu-Kα1 ray, applied voltage: 40 kV, applied electric current: 40 mA, scanning rate: 5° per min, scanning step: 0.02°, scanning range: 5°-40°. Since a diffraction peak due to aluminum cell is observed at around 38.20°-38.40°, the diffraction peaks near this range are not listed as a characteristic diffraction peak of each crystal.

In general, diffraction angle (2θ) and peak intensity (cps) of powder X-ray diffraction pattern may vary depending on a measurement device, measurement conditions, and so on. The crystals in the present specification may show diffraction angle (2θ) and peak intensity of powder X-ray diffraction pattern different from those described in the present specification, as long as it is within a general error range.

Because the compounds of the present invention are superior in physical and chemical stability in the form of a crystal, it is advantageous that they are capable of retaining the quality for a long-time, and easily preserved. In addition, advantages such as easy handling during production of various pharmaceutical compositions and bulk drug and decreased production cost can be attained. Therefore, the crystals of the present invention are extremely useful as a medicine.

**Table 19**

| Ex. No. | Diffraction angles 2θ (°) of main diffraction peaks (characteristic diffraction peaks are underlined) | Fig. |
|---|---|---|
| 1 | 8.86, 12.94, 16.66, 17.36, 18.26, 18.94, 21.60, 22.24, 23.12, 23.50, 25.30, 26.10, 26.94, 28.88, 31.54, 32.34, 33.28, 35.12 | 1 |
| 2 | 12.08, 12.72, 14.80, 16.00, 16.32, 16.96, 17.54, 18.74, 21.66, 22.38, 22.68, 23.14, 23.56, 23.74, 24.04, 25.04, 25.68, 26.40, 26.62, 26.92, 28.50, 28.82, 30.90, 31.34, 31.62, 32.46, 33.02, 33.82, 34.48, 35.46, 36.84, 37.26 | 2 |
| 3 | 7.40, 12.00, 13.14, 14.90, 16.80, 22.46, 22.68, 22.92, 23.88, 24.58, 25.38,25.80, 26.46, 27.24, 28.32, 30.20, 34.42, 35.14, 36.48 | 3 |
| 4 | 7.40, 14.92, 16.64, 16.90, 17.46, 21.56, 22.40, 22.68, 22.82, 23.62, 24.08, 25.12, 25.70, 26.12, 26.36, 27.02, 27.74, 28.58, 28.82, 29.18, 29.84, 30.98, 31.78, 32.32, 32.62, 34.04, 34.30, 35.42, 35.70, 36.44, 36.62, 37.62, 39.40 | 4 |
| 5 | 7.28, 14.64, 15.84, 16.08, 20.56, 21.62, 21.82, 23.10, 25.12, 25.42, 26.20, 27.48, 28.34, 28.66, 29.54, 32.40, 35.44, 35.84, 37.16 | 5 |
| 6 | 7.72, 11.08, 12.34, 15.58, 17.30, 17.92, 18.48, 19.08, 19.86, 20.60, 20.80, 21.34, 21.68, 21.96, 22.36, 22.64, 23.04, 23.72, 24.20, 24.68, 25.32, 25.90, 26.78, 27.10, 27.98, 28.64, 29.84, 30.58, 31.20, 32.24, 32.94, 33.66, 34.24, 34.92, 35.78, 37.20, 37.58, 39.38, 39.68 | 6 |
| 7 | 11.84, 12.46, 14.86, 15.40, 17.06, 17.80, 18.36, 19.90, 21.44, 22.24, 22.92, 23.38, 23.98, 24.32, 24.78, 25.24, 28.42, 28.80, 29.02, 29.64, 31.28, 31.86, 34.68, 36.08 | 7 |
| 10 | 9.56, 14.28, 14.50, 16.62, 17.04, 18.64, 19.20, 19.90, 21.20, 21.60, 22.06, 22.72, 23.14, 23.84, 25.62, 25.82, 26.80, 27.24, 29.06, 29.32, 31.80, 33.72, 35.90, 37.02, 37.58 | 8 |
| 12 | 7.14, 11.58, 13.14, 14.36, 16.58, 21.16, 21.66, 22.18, 22.48, 22.74, 23.36, 24.38, 25.58, 26.12, 27.72, 28.34, 28.74, 29.90, 31.22, 31.96, 32.60, 33.58, 33.96, 34.38, 34.70, 36.88 | 9 |
| 14 | 7.38, 11.82, 13.16, 14.88, 16.88, 18.70, 19.76, 20.10, 21.12, 22.62, 24.70, 24.92, 25.56, 26.62, 27.24, 29.24, 30.10, 33.10, 33.72, 34.56, 36.20, 37.90 | 10 |
| 15 | 7.02, 10.96, 11.44, 13.12, 14.14, 16.52, 21.30, 21.80, 22.04, 23.14, 25.56, 26.56, 28.12, 29.34, 35.92 | 11 |
| 16 | 10.58, 12.40, 14.66, 15.12, 17.04, 18.10, 18.36, 19.28, 19.54, 19.82, 20.54, 21.00, 21.34, 22.06, 23.46, 23.66, 24.24, 24.72, 25.92, 26.62, 27.08, 28.34, 28.98, 29.46, 29.98, 30.64, 32.30, 33.38, 39.16 | 12 |
| 17 | 10.54, 11.24, 13.16, 19.04, 20.00, 21.24, 21.60, 22.68, 24.00, 24.38, 24.98, 26.58, 28.66, 32.14, 32.38, 34.08, 34.78, 37.58 | 13 |
| 18 | 6.70, 11.26, 12.32, 13.30, 13.52, 14.46, 15.28, 17.18, 19.50, 22.70, 23.42, 23.54, 23.88, 24.22, 24.88, 25.26, 26.10, 26.88, 28.32, 28.94, 34.40 | 14 |
| 19 | 7.22, 11.16, 14.58, 21.06, 21.38, 22.02, 22.54, 22.76, 22.98, 25.70, 26.24, 26.40, 27.76 | 15 |
| 20 | 12.20, 14.30, 15.46, 15.80, 18.14, 19.08, 20.90, 21.38, 22.92, 24.22, 24.68, 25.24, 26.68, 27.88, 28.92, 29.34, 30.34, 31.02, 32.34, 33.00 | 16 |
| 21 | 10.84, 13.48, 13.90, 15.52, 16.06, 17.06, 17.30, 17.78, 19.18, 20.36, 20.58, 21.80, 22.18, 22.44 23.40, 23.70, 24.58, 25.74, 26.30, 26.64, 27.78, 28.10, 28.44, 32.30, 33.44, 35.40, 36.84, 37.98 | 17 |
| 26 | 7.38, 11.66, 12.68, 13.28, 14.48, 14.78, 16.44, 18.96, 21.66, 22.20, 22.48, 23.42, 24.68, 25.52, 26.84, 29.06, 30.36, 31.62, 32.60, 32.82, 34.34, 35.50, 36.56 | 18 |
| 27 | 10.42, 12.64, 15.44, 16.32, 16.56, 17.00, 17.60, 18.70, 20.96, 21.28, 21.78, 22.78, 23.46, 24.96, 25.28, 25.82, 26.12, 28.54, 28.92, 31.12, 32.24, 32.98, 33.64, 34.42, 35.18, 35.62 | 19 |
| 31 | 7.94, 11.84, 12.16, 14.90, 16.04, 18.52, 21.50, 22.16, 22.78, 23.46, 23.88, 24.52, 25.70, 26.82, 27.36, 27.96, 28.60, 29.10, 30.46, 32.14, 32.38, 34.96, 35.16, 37.34 | 20 |
| 36 | 7.34, 10.30, 10.54, 11.58, 14.78, 18.80, 21.42, 21.88, 22.28, 22.52, 23.34, 23.66, 24.30, 25.22, 25.52, 26.26, 26.60, 27.16, 28.18, 30.68, 31.04, 31.40, 32.40, 33.22, 33.84, 35.38, 35.88, 37.20 | 21 |
| 38 | 7.42, 10.78, 14.04, 14.34, 15.08, 17.00, 19.46, 19.94, 21.86, 22.52, 23.84, 24.50, 25.32, 25.56, 26.14, 26.42, 30.20, 31.34, 31.86, 33.04, 34.84, 37.70 | 22 |
| 39 | 7.58, 12.10, 12.76, 14.64, 15.24, 15.70, 17.24, 17.54, 18.18, 18.48, 19.12, 20.22, 20.88, 21.16, 21.60, 22.30, 22.66, 23.86, 24.40, 25.12, 25.46, 25.80, 26.92, 27.48, 28.36, 30.58, 31.30, 31.66, 33.88 | 23 |
| 40 | 10.06, 12.80, 15.10, 15.76, 16.56, 17.10, 17.48, 18.76, 20.26, 20.44, 20.86, 21.78, 22.26, 23.86, 24.38, 24.86, 25.70, 26.66, 27.18, 28.14, 28.52, 29.24, 30.62, 31.68, 34.66, 35.52, 36.78 | 24 |
| 41 | 8.72, 11.34, 13.98, 14.50, 16.96, 17.24, 17.58, 19.06, 19.94, 20.76, 22.30, 22.90, 24.12, 24.36, 24.70, 25.22, 26.58, 26.98, 27.60, 28.58, 30.58, 31.34, 32.44, 32.86, 35.72, 37.48 | 25 |
| 44 | 11.30, 11.70, 13.40, 14.24, 14.48, 15.46, 16.92, 19.74, 21.44, 22.08, 22.44, 22.74, 23.04, 23.58, 23.90, 25.56, 25.88, 26.66, 27.02, 28.00, 29.46, 26 31.30, 31.76, 32.32, 34.18, 34.64, 35.28, 35.74, 36.42 | 26 |
| 50 | 7.26, 10.54, 12.84, 13.54, 13.84, 14.60, 15.54, 16.36, 17.54, 18.08, 19.82, 20.26, 21.66, 22.18, 22.62, 24.48, 25.12, 25.70, 27.92, 28.44, 29.60, 31.06, 32.18, 34.88, 36.42, 37.14, 37.56, 39.36, 39.60 | 27 |
| 51 | 11.72, 13.28, 14.34, 16.62, 21.24, 21.66, 21.78, 22.54, 22.82, 23.68, 24.46, 25.50, 25.78, 26.22, 26.54, 27.78, 28.20, 29.02, 31.48, 31.96, 34.16, 34.94 | 28 |
| 56 | 7.12, 11.02, 11.78, 13.52, 14.06, 14.36, 15.10, 16.70, 19.10, 21.04, 21.66, 22.22, 23.74, 24.54, 24.72, 25.14, 25.98, 27.38, 28.46, 30.26, 32.10, 33.64, 34.42, 36.04 | 29 |
| 66 | 7.26, 12.48, 14.80, 16.44, 22.28, 22.76, 24.14, 29.36, 30.56, 34.50 | 30 |
| 67 | 10.68, 11.28, 12.92, 14.52, 15.28, 15.62, 16.84, 21.18, 21.52, 21.88, 22.48, 22.72, 22.96, 24.18, 24.44, 25.84, 26.08, 26.90, 27.84, 28.14, 28.34, 30.92, 31.14, 34.42, 34.92, 36.72, 39.62 | 31 |
| 69 | 7.16, 14.40, 16.72, 20.96, 21.72, 22.14, 22.68, 23.86, 24.22, 25.70, 26.66, 27.58, 29.18, 30.56, 31.04, 32.28, 32.84, 33.94, 34.62, 36.34 | 32 |
| 73 | 10.74, 14.48, 15.48, 15.78, 16.78, 19.24, 20.64, 21.18, 21.64, 22.48, 22.98, 23.28, 24.52, 25.02, 25.54, 25.92, 27.20, 28.62, 29.92, 30.30, 30.94, 31.32, 31.88, 32.86, 33.60, 34.02 | 33 |
| 76 | 10.48, 11.32, 11.76, 12.08, 16.58, 18.30, 19.68, 20.18, 21.16, 22.28, 22.84, 23.74, 24.62, 25.46, 25.76, 26.18, 26.74, 27.90, 29.64, 32.88, 33.60 | 34 |
| 77 | 7.38, 12.10, 12.88, 14.88, 16.66, 22.48, 23.04, 23.40, 24.42, 24.88, 25.52, 25.98, 27.24, 28.62 | 35 |
| 79 | 9.30, 13.44, 13.96, 14.52, 14.90, 18.64, 19.90, 20.50, 21.22, 21.56, 22.46, 23.46, 23.96, 24.68, 25.28, 25.54, 26.04, 27.12, 27.74, 28.12, 29.84, 30.06, 33.06, 36.10, 36.56, 39.40 | 36 |
| 80 | 8.92, 10.46, 14.82, 15.06, 16.48, 17.98, 18.26, 19.58, 21.10, 21.72, 23.40, 24.10, 24.52, 25.66, 26.32, 26.88, 27.16, 28.16, 28.54, 29.32, 29.68, 30.00, 30.62, 31.02, 31.92, 32.44, 33.28, 33.96, 34.98, 37.28, 37.66, 39.10, 39.60 | 37 |
| 81 | 7.12, 10.72, 12.90, 14.32, 15.20, 16.48, 20.98, 21.36, 21.62, 21.92, 22.54, 22.80, 24.20, 25.08, 25.36, 26.12, 27.54, 27.86, 28.28, 30.54, 31.02, 32.38, 32.74, 33.54, 34.08, 34.54, 34.88, 35.94, 36.48, 37.88, 39.72 | 38 |
| 82 | 7.22, 13.84, 14.54, 16.16, 16.56, 19.56, 21.02, 21.92, 22.34, 23.10, 23.30, 23.56, 24.24, 24.86, 25.72, 26.18, 27.30, 27.92, 28.18, 29.08, 31.76, 32.64, 33.22, 34.76, 35.42, 36.36, 37.32, 39.18 | 39 |
| 85 | 13.20, 14.00, 15.52, 17.04, 17.58, 18.74, 19.00, 19.34, 20.62, 21.04, 21.52, 23.24, 23.60, 24.20, 25.48, 26.84, 27.38, 29.90, 30.82, 33.92 | 40 |
| 87 | 6.32, 8.56, 13.94, 15.76, 16.38, 17.30, 18.72, 21.64, 22.38, 23.28, 24.12, 25.06, 25.26, 25.88, 26.46, 27.62, 30.16, 32.84, 33.18, 35.22 | 41 |

The present invention is explained in detail by referring to the following Preparation Examples, which are not to be construed as limitative.

### Formulation Example 1 (production of capsule)

| | |
|---|---|
| 1) compound of Example 1 | 30 mg |
| 2) microcrystalline cellulose | 10 mg |
| 3) lactose | 19 mg |
| 4) magnesium stearate | 1 mg |

1), 2), 3) and 4) are admixed and filled in a gelatin capsule.

### Formulation Example 2 (production of tablet)

| | |
|---|---|
| 1) compound of Example 1 | 30 g |
| 2) lactose | 50 g |
| 3) cornstarch | 15 g |
| 4) carboxymethylcellulose calcium | 44 g |
| 5) magnesium stearate | 1 mg |

The total amount of 1), 2) and 3) and 30 g of 4) are kneaded with water, vacuum dried and sized. This sized powder is admixed with 14 g of 4) and 1 g of 5) and the mixture is punched with a tableting machine. In this way, 1000 tablets containing 30 mg of the compound of Example 1 per tablet are obtained.

Capsule or tablet can be produced for the compounds of Examples 2 to 95 in the same manner as in Example the above-mentioned Formulation Example 1 or Formulation Example 2.

### Experimental Example 1 uric acid transport inhibitory test using human URAT1-expressing cells

Human URAT1 full length cDNA was subcloned to expression vector pcDNA3.1 and human URAT1 gene was transfected into human embryonic kidney derived cell line (HEK293 cells) by liposome method using Lipofectamine2000. Simultaneously, HEK293 cells transfected with expression vector pcDNA3.1 alone (hereinafter mock cells) were also produced. HEK293 cells expressing human URAT1 gene or mock cells were selected with geneticin resistance as an index. The functional expression of human URAT1 gene was confirmed by a method similar to the following method, using transport of uric acid labeled with ¹⁴C into the cells as an index.

Human URAT1 expressing HEK293 cells or mock cells were cultured in Dulbecco's modified Eagle's MEM medium (high glucose) containing 10% fetal bovine serum, 0.5 mg/mL geneticin sulfate, 100 units/mL penicillin and 100 µg/mL streptomycin under the conditions of 37°C and 5% CO₂ in an incubator. The cells were plated in a 96 well plate (poly-D-Lysine coated) at 1x10⁵ cells/well and the following uric acid transport inhibitory test was performed 24 hr later. This test was performed at room temperature.

After the medium was removed by aspiration from each well, the cells were washed once with Hank's Balanced Salt Solution (HBSS) and preincubated with HBSS (100 mL/well) for 5 min. HBSS was aspirated and an assay buffer (wherein NaCl in the above-mentioned HBSS had been substituted with Na-gluconate) containing various concentrations of the Example compound and a radioactive ligand (uric acid labeled with 14C; final concentration 50 mM) was added to each well at 50 mL/well and an uptake reaction was carried out for 5 min. After the reaction, the cells were washed twice with ice-cold HBSS (150 mL/well), and Microscin ti TM 20 (PerkinElmer) was added at 50 mL/well. The cells were lysed by stirring and the radioactivity of each well was measured in a liquid scintillation counter (TOP COUNT, Packard).

The uric acid transport rate (%) of the Example compound at each concentration was calculated relative to the radioactivity (difference in radioactivity between human URAT1 expressing HEK293 cells and mock cells without addition of Example compound (DMSO addition)) showing URAT1 specific uric acid transport as 100%, and the concentration (IC₅₀) of the Example compound necessary for inhibiting the uric acid transport rate by 50% was determined. The results are shown in Table 23 - Table 24. In Table - Table , "+++" means IC₅₀ value of less than 100 nM, "++" means IC₅₀ value of 100 nM to less than 1000 nM, and "+" means IC₅₀ value of 1000 nM to less than 3000 nM

**Table 23**

| Ex.No. | hURAT1 |
|---|---|
| 1 | +++ |
| 2 | +++ |
| 3 | +++ |
| 4 | +++ |
| 5 | +++ |
| 6 | ++ |
| 7 | ++ |
| 8 | ++ |
| 9 | + |
| 10 | ++ |
| 11 | +++ |
| 12 | +++ |
| 13 | ++ |
| 14 | +++ |
| 15 | +++ |
| 16 | +++ |
| 17 | +++ |
| 18 | ++ |
| 19 | ++ |
| 20 | ++ |
| 21 | ++ |
| 22 | + |
| 23 | + |
| 24 | ++ |
| 26 | ++ |
| 27 | ++ |
| 28 | ++ |
| 29 | + |
| 30 | ++ |
| 31 | +++ |
| 33 | ++ |
| 35 | + |
| 36 | ++ |
| 38 | ++ |
| 39 | +++ |
| 40 | ++ |
| 41 | ++ |
| 44 | +++ |

**Table 24**

| Ex. No. | hURAT1 |
|---|---|
| 50 | ++ |
| 51 | +++ |
| 52 | ++ |
| 54 | ++ |
| 55 | ++ |
| 56 | ++ |
| 58 | ++ |
| 59 | + |
| 61 | + |
| 64 | + |
| 65 | + |
| 66 | ++ |
| 67 | +++ |
| 68 | ++ |
| 69 | +++ |
| 70 | ++ |
| 71 | + |
| 73 | ++ |
| 74 | ++ |
| 75 | ++ |
| 76 | ++ |
| 77 | ++ |
| 78 | ++ |
| 79 | +++ |
| 80 | +++ |
| 81 | +++ |
| 82 | +++ |
| 83 | ++ |
| 84 | + |
| 85 | +++ |
| 86 | + |
| 87 | ++ |
| 89 | +++ |
| 90 | ++ |
| 91 | + |
| 93 | ++ |
| 94 | ++ |
| 95 | ++ |

### Experimental Example 2 CYP inhibitory test using human liver microsome

Human liver microsome (20 mg protein/mL, 5 _{µ}L, Xenotech LLC, purchased from Lenexa KS) was suspended in 100 mM potassium phosphate buffer (70 _{µ}L, pH 7.4), mixed with a solution (0.5 _{µ}L) of the Example compound dissolved in DMSO and preincubated at 37°C for 5 min. NADPH producing system coenzyme solution (β-nicotinamide adenine dinucleotide phosphate: 5.2 mM, D-glucose-6-phosphate: 13.2 mM, magnesium chloride: 13.2 mM, glucose-6-phosphate dehydrogenase: 1.8 U/mL) 25 _{µ}L, and a model substrate (CYP3A4: midazolam 1 mM, CYP2D6: bufuralol 1 mM, CYP2C9: diclofenac 2 mM) 0.5 _{µ}L dissolved in DMSO were added to start the reaction. After incubation at 37°C for 10 min., acetonitrile (200 _{µ}L) containing an internal standard substance (propranolol 1 _{µ}M) was added and the mixture was centrifuged (room temperature, 3000 rpm, 20 min). The amount of the metabolite produced from each model substrate in the supernatant was measured by high performance liquid chromatography/mass spectrometry (LC/MS/MS) and each CYP enzyme activity was determined. The concentration showing 50% inhibition (IC50) was calculated relative to the enzyme activity without addition of Example compound (DMSO 0.5 _{µ}L added) as 100%. The results are shown in Table 25 - Table 27 .

**Table 25**

| Ex. No. | CYP2C9 inhibition IC50 (µM) |
|---|---|
| 1 | >50 |
| 2 | 37 |
| 3 | 27 |
| 4 | 9 |
| 5 | 2 |
| 6 | >50 |
| 7 | 4 |
| 8 | 19 |
| 9 | 39 |
| 10 | >50 |
| 11 | 12 |
| 12 | 22 |
| 13 | 18 |
| 14 | 16 |
| 15 | 6 |
| 16 | 10 |
| 17 | 33 |
| 18 | 9 |
| 19 | >50 |
| 20 | 13 |
| 21 | 17 |
| 22 | 1 |
| 23 | 19 |
| 24 | >50 |
| 25 | >50 |
| 26 | 13 |
| 27 | 21 |
| 28 | 24 |
| 29 | >50 |
| 30 | >50 |
| 31 | 20 |
| 32 | 10 |
| 33 | 4 |
| 34 | 11 |
| 35 | 14 |
| 36 | 16 |
| 37 | >50 |
| 38 | 5 |
| 39 | 21 |
| 40 | >50 |

**Table 26**

| Ex. No. | CYP2C9 inhibition IC50 (µM) |
|---|---|
| 41 | >50 |
| 42 | >50 |
| 43 | >50 |
| 44 | 31 |
| 45 | >50 |
| 46 | >50 |
| 47 | >50 |
| 48 | >50 |
| 49 | >50 |
| 50 | 15 |
| 51 | 16 |
| 52 | 11 |
| 53 | 6 |
| 54 | 48 |
| 55 | 19 |
| 56 | >50 |
| 57 | >50 |
| 58 | 21 |
| 59 | 8 |
| 60 | >50 |
| 61 | 43 |
| 62 | >50 |
| 63 | >50 |
| 64 | 6 |
| 65 | 14 |
| 66 | 12 |
| 67 | 49 |
| 68 | 27 |
| 69 | 21 |
| 70 | 20 |
| 71 | 15 |
| 72 | 13 |
| 73 | >50 |
| 74 | 33 |
| 75 | >50 |
| 76 | 28 |
| 77 | 18 |
| 78 | 18 |
| 79 | 13 |
| 80 | 5 |

**Table 27**

| Ex. No. | CYP2C9 inhibition IC50 (µM) |
|---|---|
| 81 | 13 |
| 82 | 11 |
| 83 | >50 |
| 84 | 15 |
| 85 | 19 |
| 86 | >50 |
| 87 | 31 |
| 88 | >50 |
| 89 | 11 |
| 90 | 21 |
| 91 | >50 |
| 92 | >50 |
| 93 | >50 |
| 94 | 6 |
| 95 | >50 |

As is clear from the above-mentioned Experimental Example 1 (uric acid transport inhibitory test using human URAT1 expression cells), the compound of the present invention and a pharmaceutically acceptable salt thereof have a superior inhibitory action on URAT1 activity. As is clear from Experimental Example 2 (CYP inhibitory test), the compound of the present invention and a pharmaceutically acceptable salt thereof have no or extremely low CYP inhibitory action.

They indicate that the compound of the present invention and a pharmaceutically acceptable salt thereof have an effect of strong suppression of reabsorption of uric acid, and very low fear of side effect since they do not substantially inhibit CYP.

Therefore, the compound of the present invention inhibits reabsorption of uric acid by inhibiting URAT1 activity, whereby affords a drug for the prophylaxis or treatment of pathology showing involvement of uric acid, such as hyperuricemia, gouty tophus, acute gouty arthritis, chronic gouty arthritis, gouty kidney, urolithiasis, renal function disorder, coronary artery disease, ischemic heart disease and the like.

Moreover, as the causal disease of the above-mentioned pathology showing involvement of uric acid, complications or diseases highly possibly complicated, for example, gout arthritis, urolithiasis, hypertension or hypertensive complications, hyperlipidemia or hyperlipidemic complications, diabetes or diabetic complications, obesity or obesity complications, decreased uric acid excretion secondary hyperuricemia and the like can be mentioned. A combined use of an agent for the prophylaxis or treatment of these diseases including hyperuricemia and a pharmaceutically effective amount of the compound of the present invention or a pharmaceutically acceptable salt thereof is effective for the prophylaxis or treatment of these diseases. In addition, a combined use of a pharmaceutical agent that increases the blood uric acid level and a pharmaceutically effective amount of the compound of the present invention or a pharmaceutically acceptable salt thereof is effective for suppressing the increase in the blood uric acid level.

### Industrial Applicability

The present invention is useful for the prophylaxis or treatment of pathology showing involvement of uric acid, such as hyperuricemia, gouty tophus, acute gouty arthritis, chronic gouty arthritis, gouty kidney, urolithiasis, renal function disorder, coronary artery disease, ischemic heart disease and the like.

This application is based on a patent application No. 2004/344563 filed in Japan and a patent application No. 60/634,223 filed in USA the contents of which are hereby incorporated by reference.

## Claims

1. A URAT1 activity inhibitor comprising a substance that inhibits URAT1 activity and does not substantially inhibit CYP.

2. The inhibitor of claim 1, wherein CYP is CYP2C9.

3. The inhibitor of claim 1 or 2, wherein the concentration of the substance necessary for inhibiting CYP2C9 by 50% is not less than 1 _{µ}M.

4. A URAT1 activity inhibitor comprising a nitrogen-containing fused ring compound represented by the following formula [1]: 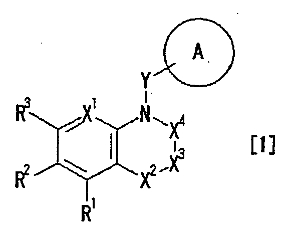 wherein
R¹, R² and R³ are the same or different and each is
1) a hydrogen atom, or
2) a group selected from group A below, or
3) R¹ and R² may form, together with the carbon atoms they are bonded to, a saturated or unsaturated carbon ring having 3 to 14 carbon atoms optionally substituted by one or more, the same or different substituents selected from group A below, or
4) R² and R³ may form, together with the carbon atoms they are bonded to, a saturated or unsaturated carbon ring having 3 to 14 carbon atoms optionally substituted by one or more, the same or different substituents selected from group A below;
Y is
1) -CO-,
2) -CS-, or
3) -S (=O)₂-;
X¹ is
1) a nitrogen atom, or
2) CR⁴ wherein R⁴ is
(a) a hydrogen atom, or
(b) a group selected from group A below, or
(c) R³ and R⁴ may form, together with the carbon atoms they are bonded to, a saturated or unsaturated carbon ring having 3 to 14 carbon atoms optionally substituted by one or more, the same or different substituents selected from group A below;
X² is
1) an oxygen atom,
2) -N(R⁵)- wherein R⁵ is
(a) a hydrogen atom, or
(b) a C₁₋₆ alkyl group optionally substituted by one or more, the same or different substituents selected from group B below,
3) -N(COR⁶)- wherein R⁶ is
(a) a hydroxyl group,
(b) a C₁₋₆ alkyl group optionally substituted by one or more, the same or different substituents selected from group B below,
(c) a C₁₋₆ alkoxy group optionally substituted by one or more, the same or different substituents selected from (i) and (ii):
(i) a substituent selected from group B below,
(ii) a C₁₋₆ alkyl group optionally substituted by one or more, the same or different substituents selected from group B below,
(d) a saturated or unsaturated carbon ring group having 3 to 14 carbon atoms optionally substituted by one or more, the same or different substituents selected from group A below,
(e) a cycloalkylalkoxy group optionally substituted by one or more, the same or different substituents selected from group A below,
(f) an aralkyl group optionally substituted by one or more, the same or different substituents selected from group A below, or
(g) an aralkoxy group optionally substituted by one or more, the same or different substituents selected from group A below,
4) -N(S(=O)₂R⁶)- wherein R⁶ is as defined above,
5) -N(CONR⁷R⁸)- wherein R⁷ and R⁸ are the same or different and each is
(a) a hydrogen atom, or
(b) a C₁₋₆ alkyl group optionally substituted by one or more, the same or different substituents selected from group B below, or
(c) R⁷ and R⁸ may form, together with the nitrogen atom they are bonded to, a monocyclic nitrogen-containing saturated heterocycle optionally substituted by one or more, the same or different substituents selected from group A below,
6) a sulfur atom,
7) -S(=O)-,
8) -S (=O) ₂-, or
9) -CR⁹R¹⁰- wherein R⁹ and R¹⁰ are the same or different and each is
(a) a hydrogen atom, or
(b) a C₁₋₆ alkyl group optionally substituted by one or more, the same or different substituents selected from group B below, or
(c) R⁹ and R¹⁰ may in combination form an oxo group;
-X³-X⁴- is
- (CR¹¹R¹²)n- wherein n is an integer of 1 to 3, and R¹¹ and R¹² each in the number of n are the same or different and each is
(a) a hydrogen atom, or
(b) a C₁₋₆ alkyl group optionally substituted by one or more, the same or different substituents selected from group B below, or
(c) R¹¹ and R¹² bonded to a single carbon atom may in combination form an oxo group, or
(d) two of R¹¹ and R¹² each in the number of n, which are bonded to a single carbon atom or two adjacent carbon atoms, may form, together with the carbon atom(s), a saturated or unsaturated carbon ring having 3 to 14 carbon atoms optionally substituted by one or more, the same or different substituents selected from group A below; and
ring A is
1) a saturated or unsaturated carbon ring group having 3 to 14 carbon atoms optionally substituted by one or more, the same or different substituents selected from group A below, or .
2) a saturated or unsaturated heterocyclic group containing at least one heteroatom selected from a nitrogen atom, an oxygen atom and a sulfur atom, optionally substituted by one or more, the same or different substituents selected from group A below,
or a pharmaceutically acceptable salt thereof:
[group A]
1) a halogen atom,
2) -OR¹³ wherein R¹³ is
(a) a hydrogen atom,
(b) a C₁₋₆ alkyl group optionally substituted by one or more, the same or different substituents selected from group B below, or
(c) -COR¹⁴ wherein R¹⁴ is
a) a hydrogen atom,
b) a hydroxyl group,
c) a C₁_₆ alkyl group optionally substituted by one or more, the same or different substituents selected from group B below,
d) a C₁₋₆ alkoxy group optionally substituted by one or more, the same or different substituents selected from (i) and (ii):
(i) a substituent selected from group B below,
(ii) a C₁₋₆ alkyl group optionally substituted by one or more, the same or different substituents selected from group B below,
e) a saturated or unsaturated carbon ring group having 3 to 14 carbon atoms optionally substituted by one or more, the same or different substituents selected from (i) and (ii) :
(i) a substituent selected from group B below,
(ii) a C₁₋₆ alkyl group optionally substituted by one or more, the same or different substituents selected from group B below,
f) a cycloalkylalkoxy group optionally substituted by one or more, the same or different substituents selected from (i) and (ii) :
(i) a substituent selected from group B below,
(ii) a C₁₋₆ alkyl group optionally substituted by one or more, the same or different substituents selected from group B below,
g) an aralkyl group optionally substituted by one or more, the same or different substituents selected from (i) and (ii) :
(i) a substituent selected from group B below,
(ii) a C₁₋₆ alkyl group optionally substituted by one or more, the same or different substituents selected from group B below, or
h) an aralkoxy group optionally substituted by one or more, the same or different substituents selected from (i) and (ii) :
(i) a substituent selected from group B below,
(ii) a C₁₋₆ alkyl group optionally substituted by one or more, the same or different substituents selected from group B below,
3) a C₁₋₆ alkyl group optionally substituted by one or more, the same or different substituents selected from group B below,
4) a cycloalkylalkoxy group optionally substituted by one or more, the same or different substituents selected from (a) and (b) :
(a) a substituent selected from group B below,
(b) a C₁₋₆ alkyl group optionally substituted by one or more, the same or different substituents selected from group B below,
5) an aralkyl group optionally substituted by one or more, the same or different substituents selected from (a) and (b):
(a) a substituent selected from group B below,
(b) a C₁₋₆ alkyl group optionally substituted by one or more, the same or different substituents selected from group B below,
6) an aralkoxy group optionally substituted by one or more, the same or different substituents selected from (a) and (b):
(a) a substituent selected from group B below,
(b) a C₁₋₆ alkyl group optionally substituted by one or more, the same or different substituents selected from group B below,
7) -COR¹⁴ wherein R¹⁴ is as defined above,
8) -NR¹⁵R¹⁶ wherein R¹⁵ and R¹⁶ are the same or different and each is
(a) a hydrogen atom, or
(b) a C₁₋₆ alkyl group optionally substituted by one or more, the same or different substituents selected from group B below, or
(c) R¹⁵ and R¹⁶ may form, together with the nitrogen atom they are bonded to, a monocyclic nitrogen-containing saturated heterocycle optionally substituted by one or more, the same or different substituents selected from (i) and (ii):
(i) a substituent selected from group B below,
(ii) a C₁₋₆ alkyl group optionally substituted by one or more, the same or different substituents selected from group B below,
9) -CONR¹⁵R¹⁶ wherein R¹⁵ and R¹⁶ are as defined above,
10) -NR¹⁷COR¹⁴ wherein R¹⁴ is as defined above, and R¹⁷ is
(a) a hydrogen atom, or
(b) a C₁₋₆ alkyl group optionally substituted by one or more, the same or different substituents selected from group B below,
11) -NR¹⁷S(=O)₂R¹⁴ wherein R¹⁴ and R¹⁷ are as defined above,
12) -NR¹⁷CONR¹⁵R¹⁶ wherein R¹⁵, R¹⁶ and R¹⁷ are as defined above,
13) -SR¹³ wherein R¹³ is as defined above,
14) -S (=O) R¹⁴ wherein R¹⁴ is as defined above,
15) -S (=O)₂R¹⁴ wherein R¹⁴ is as defined above,
16) -S(=O)₂NR¹⁵R¹⁶ wherein R¹⁵ and R¹⁶ are as defined above,
17) a saturated or unsaturated carbon ring group having 3 to 14 carbon atoms optionally substituted by one or more, the same or different substituents selected from (a) and (b):
(a) a substituent selected from group B below,
(b) a C₁₋₆ alkyl group optionally substituted by one or more, the same or different substituents selected from group B below,
18) a saturated or unsaturated heterocyclic group containing at least one heteroatom selected from a nitrogen atom, an oxygen atom and a sulfur atom, optionally substituted by one or more, the same or different substituents selected from (a) and (b):
(a) a substituent selected from group B below,
(b) a C₁₋₆ alkyl group optionally substituted by one or more, the same or different substituents selected from group B below,
19) an aryloxy group optionally substituted by one or more, the same or different substituents selected from (a) and (b):
(a) a substituent selected from group B below,
(b) a C₁₋₆ alkyl group optionally substituted by one or more, the same or different substituents selected from group B below,
20) a cyano group, and
21) a nitro group,
[group B]
1) a halogen atom,
2) a hydroxyl group,
3) a C₁₋₆ alkoxy group,
4) -NR¹⁸R¹⁹ wherein R¹⁸ and R¹⁹ are the same or different and each is
(a) a hydrogen atom, or
(b) a C₁₋₆ alkyl group, or
(c) R¹⁸ and R¹⁹ may form, together with the nitrogen atom they are bonded to, a monocyclic nitrogen-containing saturated heterocycle,
5) -CONR¹⁸R¹⁹ wherein R¹⁸ and R¹⁹ are as defined above,
6) -COR²⁰ wherein R²⁰ is
(a) a hydrogen atom,
(b) a hydroxyl group,
(c) a C₁₋₆ alkyl group, or
(d) a C₁₋₆ alkoxy group,
7) -NR²¹COR²⁰ wherein R²⁰ is as defined above, and R²¹ is
(a) a hydrogen atom, or
(b) a C₁₋₆ alkyl group,
8) -NR²¹CONR¹⁸R¹⁹ wherein R¹⁸, R¹⁹ and R²¹ are as defined above,
9) -NR²¹S (=O)₂R²² wherein R²¹ is as defined above, and R²² is a C₁₋₆ alkyl group, and
10) -S(=O)₂R²² wherein R²² is as defined above,
wherein the C₁₋₆ alkyl group and C₁₋₆ alkoxy group in 3) to 10)
above are optionally further substituted by one or more, the same or different substituents selected from group B, and the monocyclic nitrogen-containing saturated heterocycle in 4), 5) and 8) above are optionally further substituted by one or more substituents selected from group B and a C₁₋₆ alkyl group.

5. A nitrogen-containing fused ring compound represented by the following formula [2]: 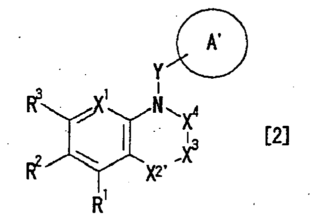 wherein R¹, R², R³, Y, X¹, X³ and X⁴ are as defined in claim 4, ring A' is
1) a saturated or unsaturated carbon ring group having 3 to 14 carbon atoms optionally substituted by one or more, the same or different substituents selected from the group C below, or
2) a saturated or unsaturated heterocyclic group containing at least one heteroatom selected from a nitrogen atom, an oxygen atom and a sulfur atom, optionally substituted by one or more, the same or different substituents selected from the group C below,
the ring A' is substituted by at least one -OR¹³_{,} wherein R¹³' is as defined in the group C below;
X²' is
1) an oxygen atom,
2) -N (R⁵) - wherein R⁵ is
(a) a hydrogen atom, or
(b) a C₁₋₆ alkyl group optionally substituted by one or more, the same or different substituents selected from group B below,
3) -N (COR⁶) - wherein R⁶ is
(a) a hydroxyl group,
(b) a C₁₋₆ alkyl group optionally substituted by one or more, the same or different substituents selected from group B below,
(c) a C₁₋₆ alkoxy group optionally substituted by one or more, the same or different substituents selected from (i) and (ii) :
(i) a substituent selected from group B below,
(ii) a C₁₋₆ alkyl group optionally substituted by one or more, the same or different substituents selected from group B below,
(d) a saturated or unsaturated carbon ring group having 3 to 14 carbon atoms optionally substituted by one or more, the same or different substituents selected from group A below,
(e) a cycloalkylalkoxy group optionally substituted by one or more, the same or different substituents selected from group A below,
(f) an aralkyl group optionally substituted by one or more, the same or different substituents selected from group A below, or
(g) an aralkoxy group optionally substituted by one or more, the same or different substituents selected from group A below,
4) -N(S(=O)₂R⁶)- wherein R⁶ is as defined above,
5) -N (CONR⁷R⁸) - wherein R⁷ and R⁸ are the same or different and each is
(a) a hydrogen atom, or
(b) a C₁₋₆ alkyl group optionally substituted by one or more, the same or different substituents selected from group B below, or
(c) R⁷ and R⁸ may form, together with the nitrogen atom they are bonded to, a monocyclic nitrogen-containing saturated heterocycle optionally substituted by one or more, the same or different substituents selected from group A below,
6) a sulfur atom,
7) -S(=O)-,
8) -S(=O)₂-, or
9) -CH₂-,
(provided that when X²' is -CH₂-,
then -X³-X⁴- should be
-(CR¹¹R¹²)n- wherein n is an integer of 1 to 3, and R¹¹ and R¹² each in the number of n are the same or different and each is
(a) a hydrogen atom, or
(b) R¹¹ and R¹² bonded to a single carbon atom may in combination form an oxo group, or
(c) two of R¹¹ and R¹² each in the number of n, which are bonded to a single carbon atom or two adjacent carbon atoms, may form, together with the carbon atom(s), a saturated or unsaturated carbon ring having 3 to 14 carbon atoms optionally substituted by one or more, the same or different substituents selected from group A below;
R¹³' should be a hydrogen atom; and
ring A' should be further substituted by at least one a halogen atom;
provided that when both R¹¹ and R¹² are hydrogen atoms, and n
is 2, then all of R¹, R² and R³ should be hydrogen atoms) ,
or a pharmaceutically acceptable salt thereof:
[group A]
1) a halogen atom,
2) -OR¹³ wherein R¹³ is
(a) a hydrogen atom,
(b) a C₁₋₆ alkyl group optionally substituted by one or more, the same or different substituents selected from group B below, or
(c) -COR¹⁴ wherein R¹⁴ is
a) a hydrogen atom,
b) a hydroxyl group,
c) a C₁₋₆ alkyl group optionally substituted by one or more, the same or different substituents selected from group B below,
d) a C₁₋₆ alkoxy group optionally substituted by one or more, the same or different substituents selected from (i) and (ii) :
(i) a substituent selected from group B below,
(ii) a C₁₋₆ alkyl group optionally substituted by one or more, the same or different substituents selected from group B below,
e) a saturated or unsaturated carbon ring group having 3 to 14 carbon atoms optionally substituted by one or more, the same or different substituents selected from (i) and (ii) :
(i) a substituent selected from group B below,
(ii) a C₁₋₆ alkyl group optionally substituted by one or more, the same or different substituents selected from group B below,
f) a cycloalkylalkoxy group optionally substituted by one or more, the same or different substituents selected from (i) and (ii) :
(i) a substituent selected from group B below,
(ii) a C₁₋₆ alkyl group optionally substituted by one or more, the same or different substituents selected from group B below,
g) an aralkyl group optionally substituted by one or more, the same or different substituents selected from (i) and (ii) :
(i) a substituent selected from group B below,
(ii) a C₁₋₆ alkyl group optionally substituted by one or more, the same or different substituents selected from group B below, or
h) an aralkoxy group optionally substituted by one or more, the same or different substituents selected from (i) and (ii):
(i) a substituent selected from group B below,
(ii) a C₁₋₆ alkyl group optionally substituted by one or more, the same or different substituents selected from group B below,
3) a C₁₋₆ alkyl group optionally substituted by one or more, the same or different substituents selected from group B below,
4) a cycloalkylalkoxy group optionally substituted by one or more, the same or different substituents selected from (a) and (b) :
(a) a substituent selected from group B below,
(b) a C₁₋₆ alkyl group optionally substituted by one or more, the same or different substituents selected from group B below,
5) an aralkyl group optionally substituted by one or more, the same or different substituents selected from (a) and (b):
(a) a substituent selected from group B below,
(b) a C₁₋₆ alkyl group optionally substituted by one or more, the same or different substituents selected from group B below,
6) an aralkoxy group optionally substituted by one or more, the same or different substituents selected from (a) and (b):
(a) a substituent selected from group B below,
(b) a C₁₋₆ alkyl group optionally substituted by one or more, the same or different substituents selected from group B below,
7) -COR¹⁴ wherein R¹⁴ is as defined above,
8) -NR¹⁵R¹⁶ wherein R¹⁵ and R¹⁶ are the same or different and each is
(a) a hydrogen atom, or
(b) a C₁₋₆ alkyl group optionally substituted by one or more, the same or different substituents selected from group B below, or
(c) R¹⁵ and R¹⁶ may form, together with the nitrogen atom they are bonded to, a monocyclic nitrogen-containing saturated heterocycle optionally substituted by one or more, the same or different substituents selected from (i) and (ii):
(i) a substituent selected from group B below,
(ii) a C₁₋₆ alkyl group optionally substituted by one or more, the same or different substituents selected from group B below,
9) -CONR¹⁵R¹⁶ wherein R¹⁵ and R¹⁶ are as defined above,
10) -NR¹⁷COR¹⁴ wherein R¹⁴ is as defined above, and R¹⁷ is
(a) a hydrogen atom, or
(b) a C₁₋₆ alkyl group optionally substituted by one or more, the same or different substituents selected from group B below,
11) -NR¹⁷S (=O)₂R¹⁴ wherein R¹⁴ and R¹⁷ are as defined above,
12) -NR¹⁷CONR¹⁵R¹⁶ wherein R¹⁵, R¹⁶ and R¹⁷ are as defined above,
13) -SR¹³ wherein R¹³ is as defined above,
14) -S (=O) R¹⁴ wherein R¹⁴ is as defined above,
15) -S (=O) ₂R¹⁴ wherein R¹⁴ is as defined above,
16) -S (=O) ₂NR¹⁵R¹⁶ wherein R¹⁵ and R¹⁶ are as defined above,
17) a saturated or unsaturated carbon ring group having 3 to 14 carbon atoms optionally substituted by one or more, the same or different substituents selected from (a) and (b):
(a) a substituent selected from group B. below,
(b) a C₁₋₆ alkyl group optionally substituted by one or more, the same or different substituents selected from group B below,
18) a saturated or unsaturated heterocyclic group containing at least one heteroatom selected from a nitrogen atom, an oxygen atom and a sulfur atom, optionally substituted by one or more, the same or different substituents selected from (a) and (b):
(a) a substituent selected from group B below,
(b) a C₁₋₆ alkyl group optionally substituted by one or more, the same or different substituents selected from group B below,
19) an aryloxy group optionally substituted by one or more, the same or different substituents selected from (a) and (b):
(a) a substituent selected from group B below,
(b) a C₁₋₆ alkyl group optionally substituted by one or more, the same or different substituents selected from group B below,
20) a cyano group, and
21) a nitro group,
[group B]
1) a halogen atom,
2) a hydroxyl group,
3) a C₁₋₆ alkoxy group,
4) -NR¹⁸R¹⁹ wherein R¹⁸ and R¹⁹ are the same or different and each is
(a) a hydrogen atom, or
(b) a C₁₋₆ alkyl group, or
(c) R¹⁸ and R¹⁹ may form, together with the nitrogen atom they are bonded to, a monocyclic nitrogen-containing saturated heterocycle,
5) -CONR¹⁸R¹⁹ wherein R¹⁸ and R¹⁹ are as defined above,
6) -COR²⁰ wherein R²⁰ is
(a) a hydrogen atom,
(b) a hydroxyl group,
(c) a C₁₋₆ alkyl group, or
(d) a C₁₋₆ alkoxy group,
7) -NR²¹COR²⁰ wherein R²⁰ is as defined above, and R²¹ is
(a) a hydrogen atom, or
(b) a C₁₋₆ alkyl group,
8) -NR²¹CONR¹⁸R¹⁹ wherein R¹⁸, R¹⁹ and R²¹ are as defined above,
9) -NR²¹S(=O)₂R²² wherein R²¹ is as defined above, and R²² is a C₁₋₆ alkyl group, and
10) -S(=O)₂R²² wherein R²² is as defined above,
wherein the C₁₋₆ alkyl group and C₁₋₆ alkoxy group in 3) to 10) above are optionally further substituted by one or more, the same or different substituents selected from group B, and the monocyclic nitrogen-containing saturated heterocycle in 4), 5) and 8) above are optionally further substituted by one or more substituents selected from group B and a C₁₋₆ alkyl group,
[group C]
1) a halogen atom,
2) -OR¹³' wherein R¹³' is
(a) a hydrogen atom,
(b) a C₁₋₆ alkyl group optionally substituted by one or more, the same or different substituents selected from group B above, or
(c) -COR¹⁴' wherein R¹⁴' is
a) a hydrogen atom,
b) a hydroxyl group,
c) a C₁₋₆ alkyl group optionally substituted by one or more, the same or different substituents selected from group B above,
d) a C₁₋₆ alkoxy group optionally substituted by one or more, the same or different substituents selected from (i) and (ii) :
(i) a substituent selected from group B above,
(ii) a C₁₋₆ alkyl group optionally substituted by one or more, the same or different substituents selected from group B above,
e) a saturated or unsaturated carbon ring group having 3 to 14 carbon atoms optionally substituted by one or more, the same or different substituents selected from (i) and (ii) :
(i) a substituent selected from group B above,
(ii) a C₁₋₆ alkyl group optionally substituted by one or more, the same or different substituents selected from group B above,
f) a cycloalkylalkoxy group optionally substituted by one or more, the same or different substituents selected from (i) and (ii) :
(i) a substituent selected from group B above,
(ii) a C₁₋₆ alkyl group optionally substituted by one or more, the same or different substituents selected from group B above,
g) an aralkyl group optionally substituted by one or more, the same or different substituents selected from (i) and (ii) :
(i) a substituent selected from group B above,
(ii) a C₁₋₆ alkyl group optionally substituted by one or more, the same or different substituents selected from group B above, or
h) an aralkoxy group optionally substituted by one or more, the same or different substituents selected from (i) and (ii):
(i) a substituent selected from group B above,
(ii) a C₁₋₆ alkyl group optionally substituted by one or more, the same or different substituents selected from group B above,
3) a C₁₋₆ alkyl group optionally substituted by one or more, the same or different substituents selected from group B above,
4) a cycloalkylalkoxy group optionally substituted by one or more, the same or different substituents selected from (a) and (b):
(a) a substituent selected from group B above,
(b) a C₁₋₆ alkyl group optionally substituted by one or more, the same or different substituents selected from group B above,
5) an aralkyl group optionally substituted by one or more, the same or different substituents selected from (a) and (b):
(a) a substituent selected from group B above,
(b) a C₁₋₆ alkyl group optionally substituted by one or more, the same or different substituents selected from group B above,
6) an aralkoxy group optionally substituted by one or more, the same or different substituents selected from (a) and (b):
(a) a substituent selected from group B above,
(b) a C₁₋₆ alkyl group optionally substituted by one or more, the same or different substituents selected from group B above,
7) -COR¹⁴' whereinR¹⁴' is as defined above,
8) -NR¹⁵' R¹⁶' wherein R¹⁵' and R¹⁶' are the same or different and each is
(a) a hydrogen atom, or
(b) a C₁₋₆ alkyl group optionally substituted by one or more, the same or different substituents selected from group B above, or
(c) R¹⁵' and R¹⁶' may form, together with the nitrogen atom they are bonded to, a monocyclic nitrogen-containing saturated heterocycle optionally substituted by one or more, the same or different substituents selected from (i) and (ii):
(i) a substituent selected from group B above,
(ii) a C₁₋₆ alkyl group optionally substituted by one or more, the same or different substituents selected from group B above,
9) -NR¹⁷'COR¹⁴' wherein R¹⁴' is as defined above, and R¹⁷' is
(a) a hydrogen atom, or
(b) a C₁₋₆ alkyl group optionally substituted by one or more, the same or different substituents selected from group B above,
10) -NR¹⁷'S(=O)₂R¹⁴' wherein R¹⁴' and R¹⁷' are as defined above,
11) -NR¹⁷'CONR¹⁵'R¹⁶' wherein R¹⁵', R¹⁶' and R¹⁷' are as defined above,
12) -SR¹³' wherein R¹³' is as defined above,
13) -S (=O) R¹⁴' wherein R¹⁴' is as defined above,
14) -S (=O)₂R¹⁴' wherein R¹⁴' is as defined above,
15) *-*S(=O)₂NR¹⁵'R¹⁶' wherein R¹⁵' and R¹⁶' are as defined above,
16) a saturated or unsaturated carbon ring group having 3 to 14 carbon atoms optionally substituted by one or more, the same or different substituents selected from (a) and (b):
(a) a substituent selected from group B above,
(b) a C₁₋₆ alkyl group optionally substituted by one or more, the same or different substituents selected from group B above,
17) a saturated or unsaturated heterocyclic group containing at least one heteroatom selected from a nitrogen atom, an oxygen atom and a sulfur atom, optionally substituted by one or more, the same or different substituents selected from (a) and (b):
(a) a substituent selected from group B above,
(b) a C₁₋₆ alkyl group optionally substituted by one or more, the same or different substituents selected from group B above,
18) an aryloxy group optionally substituted by one or more, the same or different substituents selected from (a) and (b):
(a) a substituent selected from group B above,
(b) a C₁₋₆ alkyl group optionally substituted by one or more, the same or different substituents selected from group B above,
19) a cyano group, and
20) a nitro group.

6. The nitrogen-containing fused ring compound of claim 5,
wherein
(A) when X²' is
1) -N(R⁵)- wherein R⁵ is
(a) a hydrogen atom, or
(b) a C₁₋₆ alkyl group optionally substituted by one or more, the same or different substituents selected from group B above,
2) -N(COR⁶)- wherein R⁶ is
(a) a hydroxyl group,
(b) a C₁₋₆ alkyl group optionally substituted by one or more, the same or different substituents selected from group B above,
(c) a C₁_₆ alkoxy group optionally substituted by one or more, the same or different substituents selected from (i) and (ii) :
(i) a substituent selected from group B above,
(ii) a C₁₋₆ alkyl group optionally substituted by one or more, the same or different substituents selected from group B above,
(d) a saturated or unsaturated carbon ring group having 3 to 14 carbon atoms optionally substituted by one or more, the same or different substituents selected from group A above,
(e) a cycloalkylalkoxy group optionally substituted by one or more, the same or different substituents selected from group A above,
(f) an aralkyl group optionally substituted by one or more, the same or different substituents selected from group A above, or
(g) an aralkoxy group optionally substituted by one or more, the same or different substituents selected from group A above,
3) -N(S (=O)₂R⁶) - wherein R⁶ is as defined above, or
4) -N (CONR⁷R⁸) - wherein R⁷ and R⁸ are the same or different and each is
(a) a hydrogen atom, or
(b) a C₁₋₆ alkyl group optionally substituted by one or more, the same or different substituents selected from group B above, or
(c) R⁷and R⁸ may form, together with the nitrogen atom they are bonded to, a monocyclic nitrogen-containing saturated heterocycle optionally substituted by one or more, the same or different substituents selected from group A above,
then -X³-X⁴- should be -CH₂-CH₂-; and
R¹³' should be a hydrogen atom;
(B) when X²_{,} is
1) -S (=O) -, or
2) -S(=O)₂-,
then -X³-X⁴- should be -CH₂-CH₂-;
(C) when X²_{,} is a sulfur atom, and
Y is
1) -CO-, or
2) -CS-,
then -X³-X⁴- should be -CH₂-CH₂-; and
R¹³' should be a hydrogen atom;
(D) when X²' is an oxygen atom, and
Y is
1) -CO-, or
2) -CS-,
then R¹, R² and R³ should be the same or different and each is
1) a hydrogen atom, or
2) a group selected from group D below, or
3) R¹ and R² may form, together with the carbon atoms they are bonded to, a saturated or unsaturated carbon ring having 3 to 14 carbon atoms optionally substituted by one or more, the same or different substituents selected from group D below, or
4) R² and R³ may form, together with the carbon atoms they are bonded to, a saturated or unsaturated carbon ring having 3 to 14 carbon atoms optionally substituted by one or more, the same or different substituents selected from group D below; and
-X³-X⁴- should be -CH₂-CH₂-;
(E) when X²' is
1) an oxygen atom, or
2) a sulfur atom, and
Y is -S (=O)₂-,
then -X³-X⁴- should be -CH₂-CH₂-; and
R¹³' should be a hydrogen atom; or
(F) when X^{2,} is -CH₂-,
then -X³-X⁴- should be
-(CR¹¹R¹²)n- wherein n is an integer of 1 to 3, and R¹¹ and R¹² each in the number of n are the same or different and each is
(a) a hydrogen atom, or
(b) R¹¹ and R¹² bonded to a single carbon atom may in combination form an oxo group, or
(c) two of R¹¹ and R¹² each in the number of n, which are bonded to a single carbon atom or two adjacent carbon atoms, may form, together with the carbon atom(s), a saturated or unsaturated carbon ring having 3 to 14 carbon atoms optionally substituted by one or more, the same or different substituents selected from group A above;
R¹³' should be a hydrogen atom; and
ring A' should be further substituted by at least one a halogen atom;
provided that in (F), when both R¹¹ and R¹² are hydrogen atoms, and n is 2, then all of R¹, R² and R³ should be hydrogen atoms, or a pharmaceutically acceptable salt thereof:
[group D]
1) a halogen atom,
2) -OR¹³'' wherein R¹³'' is
(a) a hydrogen atom,
(b) a C₁₋₆ alkyl group optionally substituted by one or more, the same or different substituents selected from group B above, or
(c) -COR¹⁴'' wherein R¹⁴'' is
a) a hydrogen atom,
b) a hydroxyl group,
c) a C₁₋₆ alkyl group optionally substituted by one or more, the same or different substituents selected from group B above,
d) a C₁₋₆ alkoxy group optionally substituted by one or more, the same or different substituents selected from (i) and (ii) :
(i) a substituent selected from group B above,
(ii) a C₁₋₆ alkyl group optionally substituted by one or more, the same or different substituents selected from group B above,
e) a saturated or unsaturated carbon ring group having 3 to 14 carbon atoms optionally substituted by one or more, the same or different substituents selected from (i) and (ii) :
(i) a substituent selected from group B above,
(ii) a C₁₋₆ alkyl group optionally substituted by one or more, the same or different substituents selected from group B above,
f) a cycloalkylalkoxy group optionally substituted by one or more, the same or different substituents selected from (i) and (ii) :
(i) a substituent selected from group B above,
(ii) a C₁₋₆ alkyl group optionally substituted by one or more, the same or different substituents selected from group B above,
g) an aralkyl group optionally substituted by one or more, the same or different substituents selected from (i) and (ii):
(i) a substituent selected from group B above,
(ii) a C₁₋₆ alkyl group optionally substituted by one or more, the same or different substituents selected from group B above, or
h) an aralkoxy group optionally substituted by one or more, the same or different substituents selected from (i) and (ii):
(i) a substituent selected from group B above,
(ii) a C₁₋₆ alkyl group optionally substituted by one or more, the same or different substituents selected from group B above,
3) a C₁₋₆ alkyl group optionally substituted by one or more, the same or different substituents selected from group B above,
4) a cycloalkylalkoxy group optionally substituted by one or more, the same or different substituents selected from (a) and (b):
(a) a substituent selected from group B above,
(b) a C₁₋₆ alkyl group optionally substituted by one or more, the same or different substituents selected from group B above,
5) an aralkyl group optionally substituted by one or more, the same or different substituents selected from (a) and (b):
(a) a substituent selected from group B above,
(b) a C₁₋₆ alkyl group optionally substituted by one or more, the same or different substituents selected from group B above,
6) an aralkoxy group optionally substituted by one or more, the same or different substituents selected from (a) and (b):
(a) a substituent selected from group B above,
(b) a C₁₋₆ alkyl group optionally substituted by one or more, the same or different substituents selected from group B above,
7) -COR¹⁴" wherein R¹⁴" is as defined above,
8) -NR¹⁵''¹⁶''wherein R¹⁵''and R¹⁶''are the same or different and each is
(a) a hydrogen atom, or
(b) a C₁₋₆ alkyl group optionally substituted by one or more, the same or different substituents selected from group B above, or
(c) R¹⁵" and R¹⁶" may form, together with the nitrogen atom they are bonded to, a monocyclic nitrogen-containing saturated heterocycle optionally substituted by one or more, the same or different substituents selected from (i) and (ii) :
(i) a substituent selected from group B above,
(ii) a C₁₋₆ alkyl group optionally substituted by one or more, the same or different substituents selected from group B above,
9) -CONR¹⁵''¹⁶''wherein R¹⁵''and R¹⁶'' are as defined above,
10) -NR¹⁷''COR¹⁴''wherein R¹⁴'' is as defined above, and R¹⁷'' is
(a) a hydrogen atom, or
(b) a C₁₋₆ alkyl group optionally substituted by one or more, the same or different substituents selected from group B above,
11) -NR¹⁷''S (=O)₂R¹⁴'' wherein R¹⁴, and R¹⁷'' are as defined above,
12) -NR¹⁷''ONR¹⁵''¹⁶''wherein R¹⁵'' R¹⁶'' and R¹⁷'' are as defined above,
13) -SR¹³'' wherein R¹³'' is as defined above,
14) -S(=O)R¹⁴'' wherein R¹⁴'' is as defined above,
15) -S(=O)₂R¹⁴'' wherein R¹⁴'' is as defined above,
16) -S(=O)₂NR¹⁵'' ¹⁶'' wherein R¹⁵'' and R¹⁶'' are as defined above,
17) a saturated or unsaturated carbon ring group having 3 to 14 carbon atoms optionally substituted by one or more, the same or different substituents selected from (a) and (b):
(a) a substituent selected from group B above,
(b) a C₁₋₆ alkyl group optionally substituted by one or more, the same or different substituents selected from group B above,
18) an aryloxy group optionally substituted by one or more, the same or different substituents selected from (a) and (b):
(a) a substituent selected from group B above,
(b) a C₁₋₆ alkyl group optionally substituted by one or more, the same or different substituents selected from group B above,
19) a cyano group, and
20) a nitro group.

7. The nitrogen-containing fused ring compound of claim 5,
wherein
X²' is
1) -N(R⁵)- wherein R⁵ is
(a) a hydrogen atom, or
(b) a C₁₋₆ alkyl group optionally substituted by one or more, the same or different substituents selected from group B above,
2) -N(COR⁶)- wherein R⁶ is
(a) a hydroxyl group,
(b) a C₁₋₆ alkyl group optionally substituted by one or more, the same or different substituents selected from group B above,
(c) a C₁₋₆ alkoxy group optionally substituted by one or more, the same or different substituents selected from (i) and (ii):
(i) a substituent selected from group B above,
(ii) a C₁₋₆ alkyl group optionally substituted by one or more, the same or different substituents selected from group B above,
(d) a saturated or unsaturated carbon ring group having 3 to 14 carbon atoms optionally substituted by one or more, the same or different substituents selected from group A above,
(e) a cycloalkylalkoxy group optionally substituted by one or more, the same or different substituents selected from group A above,
(f) an aralkyl group optionally substituted by one or more, the same or different substituents selected from group A above, or
(g) an aralkoxy group optionally substituted by one or more, the same or different substituents selected from group A above,
3) -N(S(=O)₂R⁶)- wherein R⁶ is as defined above, or
4) -N(CONR⁷R⁸)- wherein R⁷ and R⁸ are the same or different and each is
(a) a hydrogen atom, or
(b) a C₁₋₆ alkyl group optionally substituted by one or more, the same or different substituents selected from group B above, or
(c) R⁷ and R⁸ may form, together with the nitrogen atom they are bonded to, a monocyclic nitrogen-containing saturated heterocycle optionally substituted by one or more, the same or different substituents selected from group A above;
-X³-X⁴- is -CH₂-CH₂-; and
R¹³, is a hydrogen atom;
or a pharmaceutically acceptable salt thereof.

8. The nitrogen-containing fused ring compound of claim 7,
wherein ring A' is 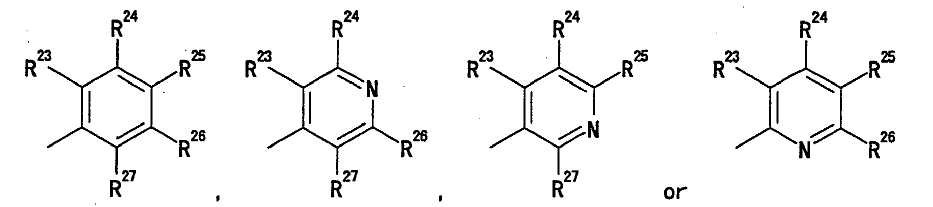 wherein
R²³ to R²⁷ are the same or different and each is
1) a hydrogen atom, or
2) a group selected from group C above, and
at least one of R²³ to R²⁷ is a hydroxyl group;
or a pharmaceutically acceptable salt thereof.

9. The nitrogen-containing fused ring compound of claim 7,
wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -COOH,
7) -CO-C₁₋₆ alkoxy group,
8) an amino group,
9) -NHS (=O)₂-C₁₋₆ alkyl group,
10) -S (=O)₂-C₁₋₆ alkyl group ,
11) -S (=O)₂-NR15R¹⁶ wherein R¹⁵ and R¹⁶ are the same or different and each is
(a) a hydrogen atom, or
(b) a C₁₋₆ alkyl group, or
(c) R¹⁵ and R¹⁶ may form, together with the nitrogen atom they are bonded to, a monocyclic nitrogen-containing saturated heterocycle,
12) a saturated or unsaturated carbon ring group having 3 to 14 carbon atoms, or
13) a nitro group;
Y is
1) -CO-,
2) -CS-, or
3) -S(=O)₂-;
X¹ is
1) a nitrogen atom, or
2) CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X²' is
1) -N (R⁵) - wherein R⁵ is
(a) a hydrogen atom, or
(b) a C₁₋₆ alkyl group,
2) -N (COR⁶) - wherein R⁶ is
(a) a C₁₋₆ alkyl group, or
(b) a saturated or unsaturated carbon ring group having 3 to 14 carbon atoms optionally substituted by one or more, the same or different substituents selected from a halogen atom and a hydroxyl group, or
3) -N (S (=O)₂-C₁₋₆ alkyl group)-;
-X³-X⁴- is -CH₂-CH₂-; and
ring A' is 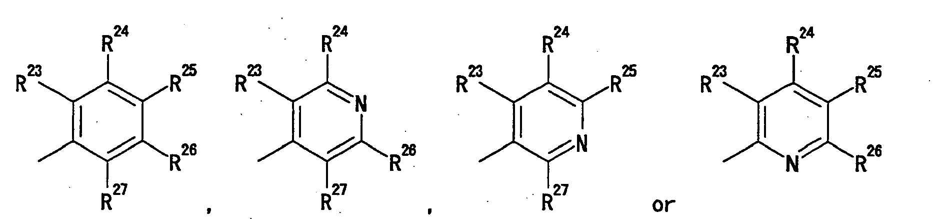 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms,
5) -COOH ,
6) -CO-C₁₋₆ alkoxy group,
7) an amino group,
8) -NHS(=O)₂-C₁₋₆ alkyl group, or
9) a nitro group, and
at least one of R²³ to R²⁷ is a hydroxyl group;
or a pharmaceutically acceptable salt thereof.

10. The nitrogen-containing fused ring compound of claim 7,
wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -CO-C₁₋₆ alkoxy group,
7) -S (=O)₂-NR¹⁵R¹⁶ wherein R¹⁵ and R¹⁶ are the same or different,
(a) a C₁₋₆ alkyl group, or
(b) R¹⁵ and R¹⁶ may form, together with the nitrogen atom they are bonded to, a monocyclic nitrogen-containing saturated heterocycle, or
8) a nitro group;
Y is
1) -CO-, or
2) -CS-;
X¹ is
CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X², is
1) -N(R⁵)- wherein R⁵ is
(a) a hydrogen atom, or
(b) a C₁₋₆ alkyl group,
2) -N(COR⁶)-wherein R⁶ is
(a) a C₁₋₆ alkyl group, or
(b) a saturated or unsaturated carbon ring group having 3 to 14 carbon atoms optionally substituted by one or more, the same or different substituents selected from a halogen atom and a hydroxyl group, or
3) -N (S (=O)₂-C₁₋₆ alkyl group) -;
-X³-X⁴- is -CH₂-CH₂-; and
ring A' is 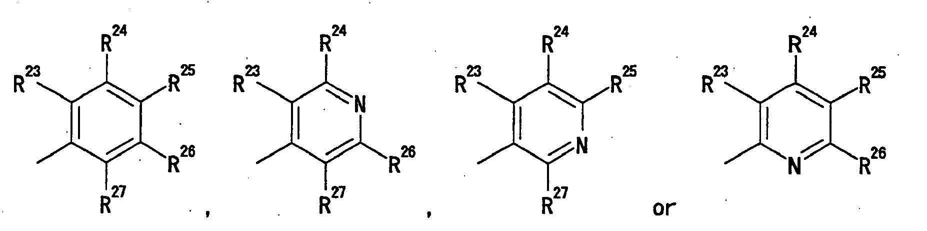 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms, or
5) a nitro group, and
at least one of R²³ to R²⁷ is a hydroxyl group;
or a pharmaceutically acceptable salt thereof.

11. The nitrogen-containing fused ring compound of claim 5,
wherein
X², is
1) -S(=O)-, or
2) -S (=O) ₂-; and
-X³-X⁴- is -CH₂-CH₂-;
or a pharmaceutically acceptable salt thereof.

12. The nitrogen-containing fused ring compound of claim 11, wherein ring A' is 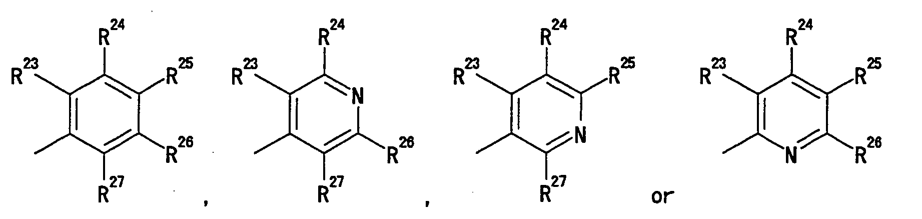 wherein
R²³ to R²⁷ are the same or different and each is
1) a hydrogen atom, or
2) a group selected from group C above, and at least one of R²³ to R²⁷ is -OR¹³_{'} wherein R¹³' is as defined in claim 5;
or a pharmaceutically acceptable salt thereof.

13. The nitrogen-containing fused ring compound of claim 11,
wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -COOH,
7) -CO-C₁₋₆ alkoxy group,
8) an amino group,
9) -NHS(=O)₂-C₁₋₆ alkyl group,
10) -S(=O)₂-C₁₋₆ alkyl group,
11) -S(=O)₂-NR¹⁵R¹⁶ wherein R¹⁵ and R¹⁶ are the same or different and each is
(a) a hydrogen atom, or
(b) a C₁₋₆ alkyl group, or
(c) R¹⁵ and R¹⁶ may form, together with the nitrogen atom they are bonded to, a monocyclic nitrogen-containing saturated heterocycle,
12) a saturated or unsaturated carbon ring group having 3 to 14 carbon atoms, or
13) a nitro group;
Y is
1) -CO-,
2) -CS-, or
3) -S (=O)₂-;
X¹ is
1) a nitrogen atom, or
2) CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X²' is
1) -S(=O)-, or
2) -S(=O)₂-;
-X³-X⁴- is -CH₂-CH₂-; and
ring A' is 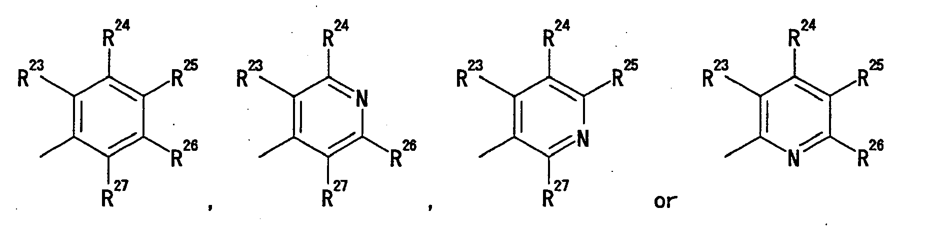 wherein
R²³ to R²⁷ are the same or different and each is
1) a hydrogen atom,
2) a halogen atom,
3) a hydroxyl group,
4) a C₁₋₆ alkoxy group optionally substituted by one or more, the same or different substituents selected from - COOH and -CO-C₁₋₆ alkoxy group,
5) -O-CO-C₁₋₆ alkyl group,
6) a C₁₋₆ alkyl group optionally substituted by one or more, the same or different halogen atoms,
7) -COOH,
8) -CO-C₁₋₆ alkoxy group,
9) an amino group,
10) -NHS (=O) ₂-C₁₋₆ alkyl group, or
11) a nitro group, and
at least one of R²³ to R²⁷ is a group selected from a hydroxyl group, a C₁₋₆ alkoxy group optionally substituted by one or more, the same or different substituents selected from -COOH and -CO-C₁₋₆ alkoxy group, and -O-CO-C₁₋₆ alkyl group;
or a pharmaceutically acceptable salt thereof.

14. The nitrogen-containing fused ring compound of claim 11, wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -CO-C₁₋₆ alkoxy group,
7) -S(=O)₂-NR¹⁵R¹⁶ wherein R¹⁵ and R¹⁶ are the same or different,
(a) a C₁₋₆ alkyl group, or
(b) R¹⁵ and R¹⁶ may form, together with the nitrogen atom they are bonded to, a monocyclic nitrogen-containing saturated heterocycle, or
8) a nitro group;
Y is
1) -CO-, or
2) -CS-;
X¹ is
CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X²' is
1) -S (=O)-, or
2) -S(=O)₂-;
-X³-X⁴- is -CH₂-CH₂-; and
ring A' is 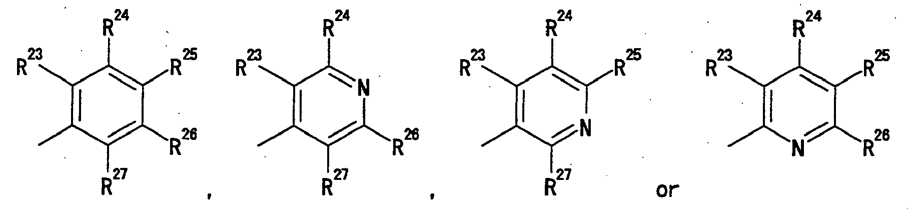 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms, or
5) a nitro group, and
at least one of R²³ to R²⁷ is a hydroxyl group;
or a pharmaceutically acceptable salt thereof.

15. The nitrogen-containing fused ring compound of claim 5,
wherein
Y is
1) -CO-, or
2) -CS-,
X^{2,} is a sulfur atom;
-X³-X⁴- is -CH₂-CH₂-; and
R¹³' is a hydrogen atom;
or a pharmaceutically acceptable salt thereof.

16. The nitrogen-containing fused ring compound of claim 15, wherein ring A' is 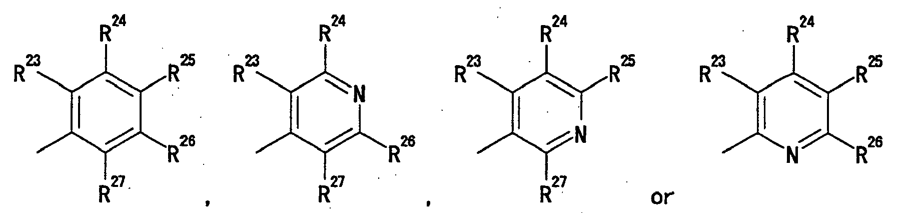 wherein
R²³ to R²⁷ are the same or different and each is
1) a hydrogen atom, or
2) a group selected from group C above, and
at least one of R²³ to R²⁷ is a hydroxyl group;
or a pharmaceutically acceptable salt thereof.

17. The nitrogen-containing fused ring compound of claim 15, wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -COOH,
7) -CO-C₁₋₆ alkoxy group,
8) an amino group,
9) -NHS (=O)₂-C₁₋₆ alkyl group,
10) -S (=O)₂-C₁₋₆ alkyl group,
11) -S (=O) ₂-NR¹⁵R¹⁶ wherein R¹⁵ and R¹⁶ are the same or different and each is
(a) a hydrogen atom, or
(b) a C₁₋₆ alkyl group, or
(c) R¹⁵ and R¹⁶ may form, together with the nitrogen atom they are bonded to, a monocyclic nitrogen-containing saturated heterocycle,
12) a saturated or unsaturated carbon ring group having 3 to 14 carbon atoms, or
13) a nitro group;
Y is
1) -CO-, or
2) -CS-,
X¹ is
1) a nitrogen atom, or
2) CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X^{2'} is a sulfur atom;
-X³-X⁴- is -CH₂-CH₂-; and
ring A' is 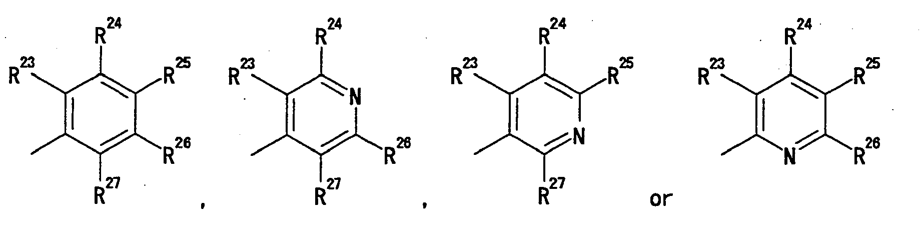 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms,
5) -COOH,
6) -CO-C₁₋₆ alkoxy group,
7) an amino group,
8) -NHS(=O)₂-C₁₋₆ alkyl group, or
9) a nitro group, and
at least one of R²³ to R²⁷ is a hydroxyl group;
or a pharmaceutically acceptable salt thereof.

18. The nitrogen-containing fused ring compound of claim 15, wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -CO-C₁₋₆ alkoxy group,
7) -S(=O)₂-NR¹⁵R¹⁶ wherein R¹⁵ and R¹⁶ are the same or different,
(a) a C₁₋₆ alkyl group, or
(b) R¹⁵ and R¹⁶ may form, together with the nitrogen atom they are bonded to, a monocyclic nitrogen-containing saturated heterocycle, or
8) a nitro group;
Y is
1) -CO-, or
2) -CS-;
X¹ is
CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X², is a sulfur atom;
-X³-X⁴- is -CH₂-CH₂-; and
ring A' is 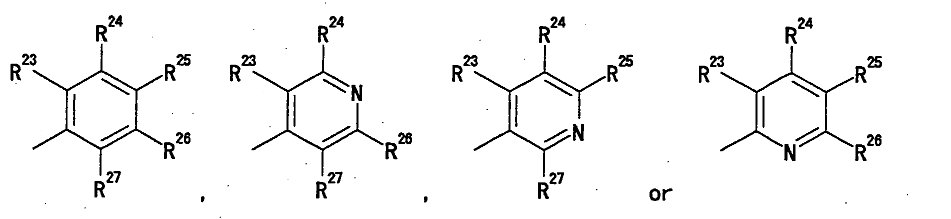 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms, or
5) a nitro group, and
at least one of R²³ to R²⁷ is a hydroxyl group;
or a pharmaceutically acceptable salt thereof.

19. The nitrogen-containing fused ring compound of claim 5, wherein
R¹, R² and R³ are the same or different and each is
1) a hydrogen atom, or
2) a group selected from group D below, or
3) R¹ and R² may form, together with the carbon atoms they are bonded to, a saturated or unsaturated carbon ring having 3 to 14 carbon atoms optionally substituted by one or more, the same or different substituents selected from group D below, or
4) R² and R³ may form, together with the carbon atoms they are bonded to, a saturated or unsaturated carbon ring having 3 to 14 carbon atoms optionally substituted by one or more, the same or different substituents selected from group D below;
Y is
1) -CO-, or
2) -CS-;
X²' is an oxygen atom; and
-X³-X⁴- is -CH₂-CH₂-;
or a pharmaceutically acceptable salt thereof:
[group D]
1) a halogen atom,
2) -OR¹³'' wherein R¹³'' is
(a) a hydrogen atom,
(b) a C₁₋₆ alkyl group optionally substituted by one or more, the same or different substituents selected from group B above, or
(c) -COR¹⁴_{''} wherein R¹_{''} is
a) a hydrogen atom,
b) a hydroxyl group,
c) a C₁₋₆ alkyl group optionally substituted by one or more, the same or different substituents selected from group B above,
d) a C₁₋₆ alkoxy group optionally substituted by one or more, the same or different substituents selected from (i) and (ii):
(i) a substituent selected from group B above,
(ii) a C₁₋₆ alkyl group optionally substituted by one or more, the same or different substituents selected from group B above,
e) a saturated or unsaturated carbon ring group having 3 to 14 carbon atoms optionally substituted by one or more, the same or different substituents selected from (i) and (ii):
(i) a substituent selected from group B above,
(ii) a C₁₋₆ alkyl group optionally substituted by one or more, the same or different substituents selected from group B above,
f) a cycloalkylalkoxy group optionally substituted by one or more, the same or different substituents selected from (i) and (ii) :
(i) a substituent selected from group B above,
(ii) a C₁₋₆ alkyl group optionally substituted by one or more, the same or different substituents selected from group B above,
g) an aralkyl group optionally substituted by one or more, the same or different substituents selected from (i) and (ii) :
(i) a substituent selected from group B above,
(ii) a C₁₋₆ alkyl group optionally substituted by one or more, the same or different substituents selected from group B above, or
h) an aralkoxy group optionally substituted by one or more, the same or different substituents selected from (i) and (ii):
(i) a substituent selected from group B above,
(ii) a C₁₋₆ alkyl group optionally substituted by one or more, the same or different substituents selected from group B above,
3) a C₁₋₆ alkyl group optionally substituted by one or more, the same or different substituents selected from group B above,
4) a cycloalkylalkoxy group optionally substituted by one or more, the same or different substituents selected from (a) and (b) :
(a) a substituent selected from group B above,
(b) a C₁₋₆ alkyl group optionally substituted by one or more, the same or different substituents selected from group B above,
5) an aralkyl group optionally substituted by one or more, the same or different substituents selected from (a) and (b):
(a) a substituent selected from group B above,
(b) a C₁₋₆ alkyl group optionally substituted by one or more, the same or different substituents selected from group B above,
6) an aralkoxy group optionally substituted by one or more, the same or different substituents selected from (a) and (b):
(a) a substituent selected from group B above,
(b) a C₁₋₆ alkyl group optionally substituted by one or more, the same or different substituents selected from group B above,
7) -COR¹⁴'' wherein R¹⁴'' is as defined above,
8) -NR¹⁵''R¹⁶'' wherein R¹⁵'' and R¹⁶'' are the same or different and each is
(a) a hydrogen atom, or
(b) a C₁₋₆ alkyl group optionally substituted by one or more, the same or different substituents selected from group B above, or
(c) R¹⁵'' and R¹⁶'' may form, together with the nitrogen atom they are bonded to, a monocyclic nitrogen-containing saturated heterocycle optionally substituted by one or more, the same or different substituents selected from (i) and (ii) :
(i) a substituent selected from group B above,
(ii) a C₁₋₆ alkyl group optionally substituted by one or more, the same or different substituents selected from group B above,
9) -CONR¹⁵''R¹⁶'' wherein R¹⁵'' and R¹⁶'' are as defined above,
10) -NR¹⁷''OR^{14''}wherein R¹⁴'' is as defined above, and R¹⁷'' is
(a) a hydrogen atom, or
(b) a C₁₋₆ alkyl group optionally substituted by one or more, the same or different substituents selected from group B above,
11) -NR¹⁷''S(=O)₂R¹⁴'' wherein R¹⁴'' and R¹⁷'' are as defined above,
12) -NR¹⁷''CONR¹⁵''R¹⁶'' wherein R¹⁵'', R¹⁶''and R¹⁷'' are as defined above,
13) -SR¹³'' wherein R¹³'' is as defined above,
14) -S(=O)R¹⁴'' wherein R¹⁴'' is as defined above,
15) -S (=O)₂R¹⁴'' wherein R¹⁴'' is as defined above,
16) -S(=O)₂NR¹⁵''¹⁶'' wherein R¹⁵'' and R¹⁶'' are as defined above,
17) a saturated or unsaturated carbon ring group having 3 to 14 carbon atoms optionally substituted by one or more, the same or different substituents selected from (a) and (b):
(a) a substituent selected from group B above,
(b) a C₁₋₆ alkyl group optionally substituted by one or more, the same or different substituents selected from group B above,
18) an aryloxy group optionally substituted by one or more, the same or different substituents selected from (a) and (b):
(a) a substituent selected from group B above,
(b) a C₁₋₆ alkyl group optionally substituted by one or more, the same or different substituents selected from group B above,
19) a cyano group, and
20) a nitro group.

20. The nitrogen-containing fused ring compound of claim 19, wherein ring A' is 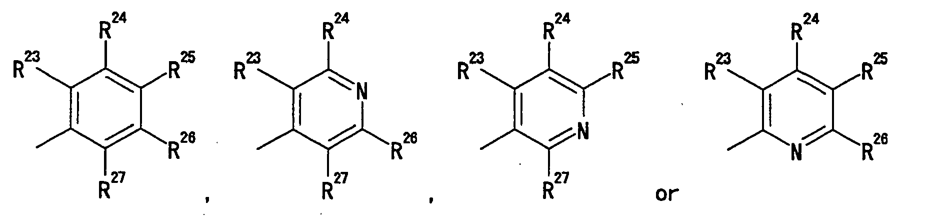 wherein
R²³ to R²⁷ are the same or different and each is
1) a hydrogen atom, or
2) a group selected from group C above, and at least one of R²³ to R²⁷ is -OR¹³_{'} wherein R¹³' is as defined in claim 5;
or a pharmaceutically acceptable salt thereof.

21. The nitrogen-containing fused ring compound of claim 19, wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -COOH,
7) -CO-C₁₋₆ alkoxy group,
8) an amino group,
9) -NHS(=O)₂-C₁₋₆ alkyl group,
10) -S (=O)₂-C₁₋₆ alkyl group,
11) -S(=O)₂-NR¹⁵''R¹⁶'' wherein R¹⁵'' and R¹⁶'' are the same or different and each is
(a) a hydrogen atom, or
(b) a C₁₋₆ alkyl group, or
(c) R¹⁵'' and R¹⁶'' may form, together with the nitrogen atom they are bonded to, a monocyclic nitrogen-containing saturated heterocycle,
12) a saturated or unsaturated carbon ring group having 3 to 14 carbon atoms, or
13) a nitro group;
Y is
1) -CO-, or
2) -CS-;
X¹ is
1) a nitrogen atom, or
2) CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X², is an oxygen atom;
-X³-X⁴- is -CH₂-CH₂-; and
ring A' is 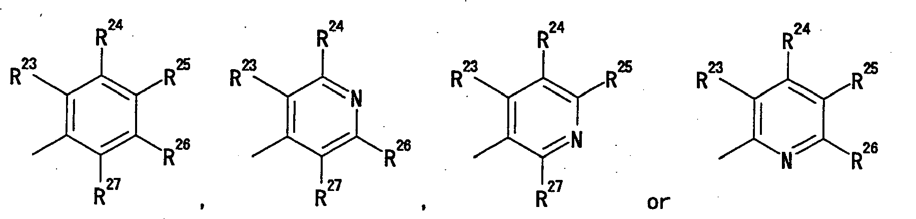 wherein
R²³ to R²⁷ are the same or different and each is
1) a hydrogen atom,
2) a halogen atom,
3) a hydroxyl group,
4) a C₁₋₆ alkoxy group optionally substituted by one or more, the same or different substituents selected from - COOH and -CO-C₁₋₆ alkoxy group,
5) -O-CO-C₁₋₆ alkyl group,
6) a C₁₋₆ alkyl group optionally substituted by one or more, the same or different halogen atoms,
7) -COOH,
8) -CO-C₁₋₆ alkoxy group,
9) an amino group,
10) -NHS (=O)₂-C₁₋₆ alkyl group, or
11) a nitro group, and
at least one of R²³ to R²⁷ is a group selected from a hydroxyl group, a C₁₋₆ alkoxy group optionally substituted by one or more, the same or different substituents selected from -COOH and -CO-C₁₋₆ alkoxy group, and -O-CO-C₁₋₆ alkyl group;
or a pharmaceutically acceptable salt thereof.

22. The nitrogen-containing fused ring compound of claim 19, wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -CO-C₁₋₆ alkoxy group,
7) -S(=O)₂-NR¹⁵''R¹⁶''wherein R¹⁵'' and R¹⁶''are the same or different,
(a) a C₁₋₆ alkyl group, or
(b) R¹⁵''and R¹⁶'' may form, together with the nitrogen atom they are bonded to, a monocyclic nitrogen-containing saturated heterocycle, or
8) a nitro group;
Y is
1) -CO-, or
2) -CS-;
X¹ is
CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X²' is an oxygen atom;
-X³-X⁴- is -CH₂-CH₂-; and
ring A' is 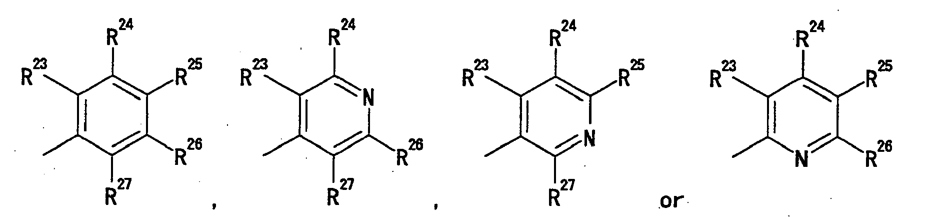 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms, or
5) a nitro group, and
at least one of R²³ to R²⁷ is a hydroxyl group;
or a pharmaceutically acceptable salt thereof.

23. The nitrogen-containing fused ring compound of claim 5, wherein
Y is -S(=O)₂-,
X², is
1) an oxygen atom, or
2) a sulfur atom;
-X³-X⁴- is -CH₂-CH₂-; and
R¹³' is a hydrogen atom;
or a pharmaceutically acceptable salt thereof.

24. The nitrogen-containing fused ring compound of claim 23, wherein ring A' is 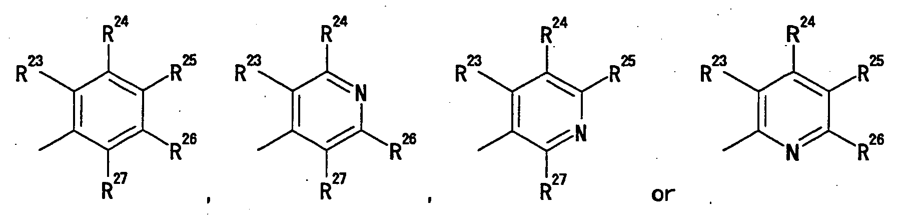 wherein
R²³ to R²⁷ are the same or different and each is
1) a hydrogen atom, or
2) a group selected from group C above, and
at least one of R²³ to R²⁷ is a hydroxyl group;
or a pharmaceutically acceptable salt thereof.

25. The nitrogen-containing fused ring compound of claim 23, wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -COOH,
7) -CO-C₁₋₆ alkoxy group,
8) an amino group,
9) -NHS (=O) ₂-C₁₋₆ alkyl group,
10) -S (=O)₂-C₁₋₆ alkyl group,
11) -S (=O) ₂-NR¹⁵R¹⁶ wherein R¹⁵ and R¹⁶ are the same or different and each is
(a) a hydrogen atom, or
(b) a C₁₋₆ alkyl group, or they are bonded to, a monocyclic nitrogen-containing saturated heterocycle,
12) a saturated or unsaturated carbon ring group having 3 to 14 carbon atoms, or
13) a nitro group;
Y is -S (=O) ₂-,
X¹ is
1) a nitrogen atom, or
2) CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁹ may form, together with the carbon atoms they are bonded to, a saturated or unsaturated carbon ring having 3 to 14 carbon atoms;
X²' is
1) an oxygen atom, or
2) a sulfur atom;
-X³-X⁴- is -CH₂-CH₂-; and
ring A' is 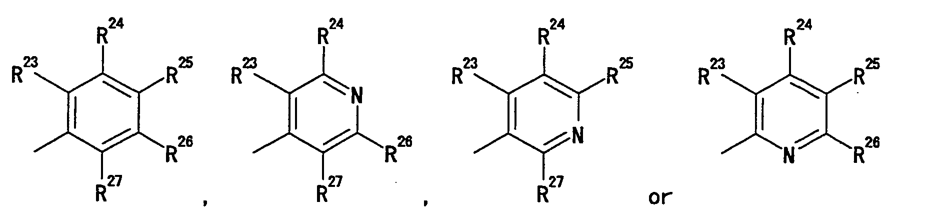 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms,
5) -COOH,
6) -CO-C₁₋₆ alkoxy group,
7) an amino group,
8) -NHS(=O)₂-C₁₋₆ alkyl group, or
9) a nitro group, and
at least one of R²³ to R²⁷ is a hydroxyl group;
or a pharmaceutically acceptable salt thereof.

26. The nitrogen-containing fused ring compound of claim 23, wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -CO-C₁₋₆ alkoxy group,
7) -S(=O)₂-NR¹⁵R¹⁶ wherein R¹⁵ and R¹⁶ are the same or different,
(a) a C₁₋₆ alkyl group, or
(b) R¹⁵ and R¹⁶ may form, together with the nitrogen atom they are bonded to, a monocyclic nitrogen-containing saturated heterocycle, or
8) a nitro group;
Y is -S (=O) ₂-,
X¹ is
CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X², is
1) an oxygen atom, or
2) a sulfur atom;
-X³-X⁴- is -CH₂-CH₂-; and
ring A' is 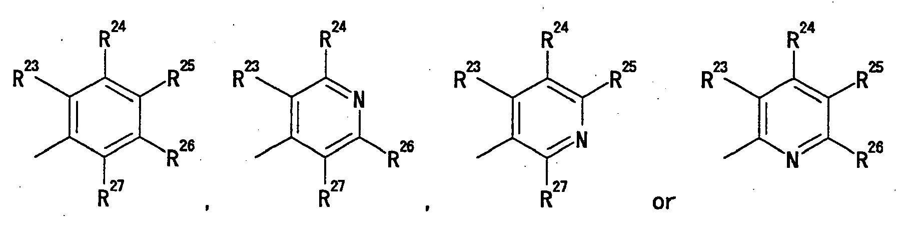 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms, or
5) a nitro group, and
at least one of R²³ to R²⁷ is a hydroxyl group;
or a pharmaceutically acceptable salt thereof.

27. The nitrogen-containing fused ring compound of claim 5,
wherein
X²' is -CH₂-;
-X³-X⁴- is
-(CR¹¹R¹²)n- wherein n is an integer of 1 to 3, and R¹¹ and R¹² each in the number of n are the same or different and each is
(a) a hydrogen atom, or
(b) R¹¹ and R¹² bonded to a single carbon atom may in combination form an oxo group, or
(c) two of R¹¹ and R¹² each in the number of n, which are bonded to a single carbon atom or two adjacent carbon atoms, may form, together with the carbon atom(s), a saturated or unsaturated carbon ring having 3 to 14 carbon atoms optionally substituted by one or more, the same or different substituents selected from group A above;
R¹³' is a hydrogen atom; and
ring A' is further substituted by at least one a halogen atom; provided that when both R¹¹ and R¹² are hydrogen atoms, and n is 2, then all of R¹, R² and R³ should be hydrogen atoms,
or a pharmaceutically acceptable salt thereof.

28. The nitrogen-containing fused ring compound of claim 27, wherein ring A' is 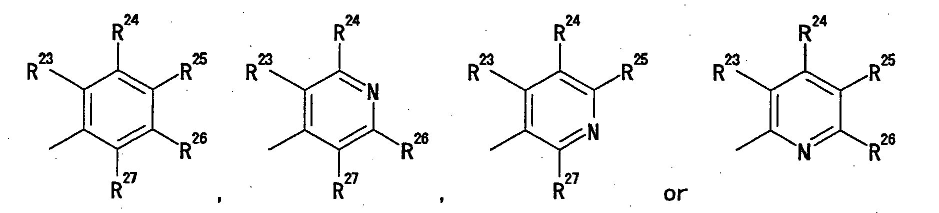 wherein
R²³ to R²⁷ are the same or different and each is
1) a hydrogen atom, or
2) a group selected from group C above, and
at least one of R²³ to R²⁷ is a hydroxyl group, and at least one of R²³ to R²⁷ is a halogen atom;
or a pharmaceutically acceptable salt thereof.

29. The nitrogen-containing fused ring compound of claim 27, wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -COOH,
7) -CO-C₁₋₆ alkoxy group,
8) an amino group,
9) -NHS(=O)₂-C₁₋₆ alkyl group,
10) -S(=O)₂-C₁₋₆ alkyl group,
11) -S(=O)₂-NR¹⁵R¹⁶ wherein R¹⁵ and R¹⁶ are the same or different and each is
(a) a hydrogen atom, or
(b) a C₁₋₆ alkyl group, or
(c) R¹⁵ and R¹⁶ may form, together with the nitrogen atom they are bonded to, a monocyclic nitrogen-containing saturated heterocycle,
12) a saturated or unsaturated carbon ring group having 3 to 14 carbon atoms, or
13) a nitro group;
Y is
1) -CO-,
2) -CS-, or
3) -S(=O)₂-;
X¹ is
1) a nitrogen atom, or
2) CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X²' is -CH₂-;
-X³-X⁴- is
-(CR¹¹R¹²)n- wherein n is an integer of 1 to 3, and R¹¹ and _{R}¹² each in the number of n are the same or different and each is
(a) a hydrogen atom, or
(b) R¹¹ and R¹² bonded to a single carbon atom may in combination form an oxo group, or
(c) two of R¹¹ and R¹² each in the number of n, which are bonded to a single carbon atom or two adjacent carbon atoms, may form, together with the carbon atom(s), a saturated or unsaturated carbon ring having 3 to 14 carbon atoms optionally substituted by one or more, the same or different substituents selected from group A above; and
ring A' is 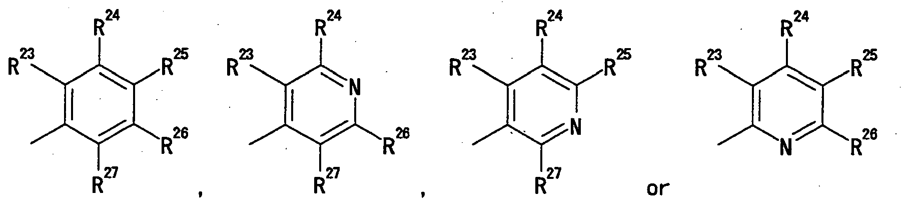 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms,
5) -COOH,
6) -CO-C₁₋₆ alkoxy group,
7) an amino group,
8) -NHS (=O)₂-C₁₋₆ alkyl group, or
9) a nitro group, and
at least one of R²³ to R²⁷ is a hydroxyl group, and at least one of R²³ to R²⁷ is a halogen atom;
provided that when both R¹¹ and R¹² are hydrogen atoms, and n is 2, then all of R¹, R² and R³ should be hydrogen atoms, or a pharmaceutically acceptable salt thereof.

30. The nitrogen-containing fused ring compound of claim 27, wherein
R¹, R² and R³ are the same or different and each is
1) a hydrogen atom,
2) a halogen atom,
3) a hydroxyl group,
4) a C₁₋₆ alkoxy group,
5) a C₁₋₆ alkyl group optionally substituted by one or more, the same or different substituents selected from a halogen atom and a hydroxyl group,
6) -CO-C₁₋₆ alkoxy group,
7) -S (=O)₂-NR¹⁵R¹⁶ wherein R¹⁵ and R¹⁶ are the same or different,
(a) a C₁₋₆ alkyl group, or
(b) R¹⁵ and R¹⁶ may form, together with the nitrogen atom they are bonded to, a monocyclic nitrogen-containing saturated heterocycle, or
8) a nitro group;
Y is
1) -CO-, or
2) -CS-;
X¹ is
CR⁴ wherein R⁴ is
(a) a hydrogen atom,
(b) a hydroxyl group,
(c) a C₁₋₆ alkyl group, or
(d) -CO-C₁₋₆ alkoxy group, or
(e) R³ and R⁴ may form, together with the carbon atoms they are bonded to, a saturated or unsaturated carbon ring having 3 to 14 carbon atoms;
X²' is -CH₂-;
-X³-X⁴- is
-(CR¹¹R¹²)n- wherein n is an integer of 1 to 3, and R¹¹ and R¹² each in the number of n are the same or different and each
is
(a) a hydrogen atom, or
(b) R¹¹ and R¹² bonded to a single carbon atom may in combination form an oxo group, or
(c) two of R¹¹ and R¹² each in the number of n, which are bonded to a single carbon atom or two adjacent carbon atoms, may form, together with the carbon atom(s), a saturated or unsaturated carbon ring having 3 to 14 carbon atoms optionally substituted by one or more, the same or different substituents selected from group A above; and
ring A' is 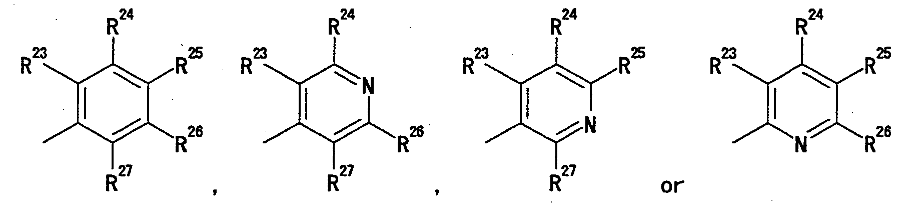 wherein
R²³ to R²⁷ are the same or different and each is
1) a hydrogen atom,
2) a halogen atom,
3) a hydroxyl group,
4) a C₁₋₆ alkyl group optionally substituted by one or more, the same or different halogen atoms, or
5) a nitro group, and
at least one of R²³ to R²⁷ is a hydroxyl group, and at least one of R²³ to R²⁷ should be a halogen atom;
provided that when both R¹¹ and R¹² are hydrogen atoms, and n is 2, then all of R¹, R² and R³ should be hydrogen atoms, or a pharmaceutically acceptable salt thereof.

31. The nitrogen-containing fused ring compound of claim 5, which is selected from the group consisting of
(1) (3-chloro-4-hydroxyphenyl)-(2,3-dihydrobenzo[1,4]oxazin-4-yl)-methanone,
(2) (3-bromo-4-hydroxyphenyl)-(2,3-dihydrobenzo[1,4]oxazin-4-yl)-methanone,
(3) (3,5-dichloro-4-hydroxyphenyl)-(2,3-dihydrobenzo[1,4]oxazin-4-yl)-methanone,
(4) (3,5-dibromo-4-hydroxyphenyl)-(2,3-dihydrobenzo[1,4]oxazin- . 4-yl)-methanone,
(5) (2,3-dihydrobenzo[1,4]oxazin-4-yl)-(4-hydroxy-3,5-diiodophenyl)-methanone,
(6) (3,5-difluoro-4-hydroxyphenyl)-(2,3-dihydrobenzo[1,4]oxazin-4-yl)-methanone,
(7) (2,3-dihydrobenzo[1,4]oxazin-4-yl)-(4-hydroxy-3,5-dimethylphenyl)-methanone,
(8) (3,5-dichloro-4-hydroxyphenyl)-(2,3-dihydrobenzo[1,4]thiazin-4-yl)-methanone,
(9) (3,5-dichloro-4-hydroxyphenyl)-(1-oxo-2,3-dihydro-1H-1λ⁴-benzo[1,4]thiazin-4-yl)-methanone,
(10) (3,5-dichloro-4-hydroxyphenyl)-(1,1-dioxo-2,3-dihydro-1H-1λ⁶-benzo[1,4]thiazin-4-yl)-methanone,
(11) (3,5-dichloro-4-hydroxyphenyl)-(2,3-dihydrobenzo[1,4]oxazin-4-yl)-methanethione,
(12) (3,5-dichloro-4-hydroxyphenyl)-(6-methyl-2,3-dihydrobenzo[1,4]oxazin-4-yl)-methanone,
(13) (3,5-dichloro-4-hydroxyphenyl)-(7-methyl-2,3-dihydrobenzo[1,4]oxazin-4-yl)-methanone,
(14) (3,5-dichloro-4-hydroxyphenyl)-(5-methyl-2,3-dihydrobenzo[1,4]oxazin-4-yl)-methanone,
(15) (3,5-dichloro-4-hydroxyphenyl)-(8-methyl-2,3-dihydrobenzo[1,4]oxazin-4-yl)-methanone,
(16) (3,5-dichloro-4-hydroxyphenyl)-(2,3-dihydronaphtho[2,1-b] [1,4]oxazin-1-yl)-methanone,
(17) (3,5-dichloro-4-hydroxyphenyl)-(6-methoxy-2,3-dihydrobenzo[1,4]oxazin-4-yl)-methanone,
(18) (3,5-dichloro-4-hydroxyphenyl)-(7-methoxy-2,3-dihydrobenzo[1,4]oxazin-4-yl)-methanone,
(19) (3,5-dichloro-4-hydroxyphenyl)-(6-hydroxy-2,3-dihydrobenzo[1,4]oxazin-4-yl)-methanone,
(20) (3,5-dichloro-4-hydroxyphenyl)-(7-hydroxy-2,3-dihydrobenzo[1,4]oxazin-4-yl)-methanone,
(21) 4-(3,5-dichloro-4-hydroxybenzoyl)-3,4-dihydro-2H-benzo[1,4]oxazine-6-sulfonic acid diethylamide,
(22) 2,6-dichloro-4-(2,3-dihydrobenzo[1,4]oxazine-4-sulfonyl)phenol,
(23) (6-tert-butyl-2,3-dihydrobenzo[1,4]oxazin-4-yl)-(3,5-dichloro-4-hydroxyphenyl)-methanone,
(24) 4-(3,5-dichloro-4-hydroxybenzoyl)-4H-benzo[1,4]oxazin-3-one,
(25) 4-(3,5-dichloro-4-hydroxybenzoyl)-3,4-dihydro-2H-benzo[1,4]oxazine-6-sulfonamide,
(26) (3,5-dichloro-4-hydroxyphenyl)-(3,4-dihydro-2H-quinolin-1-yl)-methanone,
(27) (3,5-dichloro-4-hydroxyphenyl)-(2,3,4,5-tetrahydrobenzo[b]azepin-1-yl)-methanone,
(28) (4-amino-3,5-dichlorophenyl)-(2,3-dihydrobenzo[1,4]oxazin-4-yl)-methanone,
(29) (5-chloro-6-hydroxypyridin-3-yl)-(2,3-dihydrobenzo[1,4]oxazin-4-yl)-methanone,
(30) (2,3-dihydrobenzo[1,4]oxazin-4-yl)-(4-hydroxy-3,5-dinitrophenyl)-methanone,
(31) (3-chloro-4-hydroxy-5-nitrophenyl) - (2, 3-dihydrobenzo[1,4]oxazin-4-yl)-methanone,
(32) (3,5-dichloro-4-hydroxyphenyl)-(2,8-diisopropyl-2,3-dihydrobenzo[1,4]oxazin-4-yl)-methanone,
(33) (3,4-dichloro-4-hydroxyphenyl)-[6-(pyrrolidine-1-sulfonyl)-2,3-dihydrobenzo[1,4]oxazin-4-yl]-methanone,
(34) 4-(3,5-dichloro-4-hydroxybenzoyl)-3,4-dihydro-2H-benzo[1,4]oxazine-6-sulfonic acid ethylamide,
(35) 4-(3,5-dichloro-4-hydroxybenzoyl)-3,4-dihydro-2H-benzo[1,4]oxazine-6-sulfonic acid dimethylamide,
(36) (3,5-dichloro-4-hydroxyphenyl)-(2,3-dihydropyrido[3,2-b][1,4]oxazin-4-yl)-methanone,
(37) 5-(3,5-dichloro-4-hydroxybenzoyl)-1,3,4,5-tetrahydrobenzo[b][1,4]diazepin-2-one,
(38) (3,5-dichloro-2-hydroxyphenyl)-(2,3-dihydrobenzo[1,4]oxazin-4-yl)-methanone,
(39) (2,3-dihydrobenzo[1,4]oxazin-4-yl)-(4-hydroxy-3-trifluoromethylphenyl)-methanone,
(40) (3-chloro-4-hydroxy-5-methoxyphenyl)-(2,3-dihydrobenzo[1,4]oxazin-4-yl)-methanone,
(41) (4-chloro-3-hydroxyphenyl)-(2,3-dihydrobenzo[1,4]oxazin-4-yl)-methanone,
(42) (2,6-dichloropyridin-4-yl)-(2,3-dihydrobenzo[1,4]oxazin-4-yl)-methanone,
(43) (2,3-dihydrobenzo[1,4]oxazin-4-yl)-(4-nitrophenyl)-methanone,
(44) (3,5-dichloro-4-hydroxyphenyl)-(6-fluoro-2,3-dihydrobenzo[1,4]oxazin-4-yl)-methanone,
(45) 2-(2,3-dihydrobenzo[1,4]oxazine-4-carbonyl)benzoic acid,
(46) methyl 4-(2,3-dihydrobenzo[1,4]oxazine-4-carbonyl)benzoate,
(47) 4-(2,3-dihydrobenzo[1,4]oxazine-4-carbonyl)benzoic acid,
(48) methyl 3-(2,3-dihydrobenzo[1,4]oxazine-4-carbonyl)benzoate,
(49) 3-(2,3-dihydrobenzo[1,4]oxazine-4-carbonyl)benzoic acid,
(50) (3,5-dichloro-2,4-dihydroxyphenyl)-(2,3-dihydrobenzo[1,4]oxazin-4-yl)-methanone,
(51) (6-chloro-2,3-dihydrobenzo[1,4]oxazin-4-yl)-(3,5-dichloro-4-hydroxyphenyl)-methanone,
(52) (7-chloro-2,3-dihydrobenzo[1,4]oxazin- 4-yl)-(3,5-dichloro-4-hydroxyphenyl)-methanone,
(53) [4-(3,5-dichloro-4-hydroxybenzoyl)-3,4-dihydro-2H-quinoxalin-1-yl]-(3,5-dichloro-4-hydroxyphenyl)-methanone,
(54) (3,5-dichloro-4-hydroxyphenyl)-(3,4-dihydro-2H-quinoxalin-1-yl)-methanone,
(55) methyl 4-(3,5-dichloro-4-hydroxybenzoyl)-3,4-dihydro-2H-benzo[1,4]oxazine-6-carboxylate,
(56) (3,5-dichloro-4-hydroxyphenyl)-(6-hydroxymethyl-2,3-dihydrobenzo[1,4]oxazin-4-yl)-methanone,
(57) 4-(3,5-dichloro-4-hydroxybenzoyl)-3,4-dihydro-2H-benzo[1,4]oxazine-6-carboxylic acid,
(58) methyl 4-(3,5-dichloro-4-hydroxybenzoyl)-3,4-dihydro-2H-benzo[1,4]oxazine-5-carboxylate,
(59) methyl 4-(3,5-dichloro-4-hydroxybenzoyl)-3,4-dihydro-2H-benzo[1,4]oxazine-7-carboxylate,
(60) 4-(3,5-dichloro-4-hydroxybenzoyl)-3,4-dihydro-2H-benzo[1,4]oxazine-7-carboxylic acid,
(61) methyl 4-(3,5-dichloro-4-hydroxybenzoyl)-3,4-dihydro-2H-benzo[1,4]oxazine-8-carboxylate,
(62) 4-(3,5-dichloro-4-hydroxybenzoyl)-3,4-dihydro-2H-benzo[1,4]oxazine-8-carboxylic acid,
(63) (3,5-dichlorophenyl)-(2,3-dihydrobenzo[1,4]oxazin-4-yl)-methanone,
(64) (3,5-dichloro-4-hydroxyphenyl)-(phenoxazin-10-yl)-methanone,
(65) (3,5-dichloro-4-hydroxyphenyl)-(6-phenyl-2,3-dihydrobenzo[1,4]oxazin-4-yl)-methanone,
(66) (3,5-dichloro-4-hydroxyphenyl)-(6,8-dimethyl-2,3-dihydrobenzo[1,4]oxazin-4-yl)-methanone,
(67) (3,5-dichloro-4-hydroxyphenyl)-(6-nitro-2,3-dihydrobenzo[1,4]oxazin-4-yl)-methanone,
(68) (6-amino-2,3-dihydrobenzo[1,4]oxazin-4-yl)-(3,5-dichloro-4-hydroxyphenyl)-methanone,
(69) (3,5-dibromo-4-hydroxyphenyl)-(6-nitro-2,3-dihydrobenzo[1,4]oxazin-4-yl)-methanone,
(70) (3,5-dichloro-4-hydroxyphenyl)-(7-nitro-2,3-dihydrobenzo[1,4]oxazin-4-yl)-methanone,
(71) (7-amino-2,3-dihydrobenzo[1,4]oxazin-4-yl)-(3,5-dichloro-4-hydroxyphenyl)-methanone,
(72) N-[4-(3,5-dichloro-4-hydroxybenzoyl)-3,4-dihydro-2H-benzo[1,4]oxazin-7-yl]-methanesulfonamide,
(73) 1-[4-(3,5-dichloro-4-hydroxybenzoyl)-3,4-dihydro-2H-quinoxalin-1-yl]-ethanone,
(74) (3,5-dichloro-4-hydroxyphenyl)-(4-methyl-3,4-dihydro-2H-quinoxalin-1-yl)-methanone,
(75) (2,3-dihydrobenzo[1,4]oxazin-4-yl)-(4-hydroxy-3-nitrophenyl)-methanone,
(76) (3,5-dichloro-4-hydroxyphenyl)-(2-methyl-2,3-dihydroindol-1-yl)-methanone,
(77) (3,5-dichloro-4-hydroxyphenyl)-(2,3-dihydroindol-1-yl)-methanone,
(78) (5-amino-2,3-dihydroindol-1-yl)-(3,5-dichloro-4-hydroxyphenyl)-methanone,
(79) (3,5-dibromo-4-hydroxyphenyl)-(6-fluoro-2,3-dihydrobenzo[1,4]oxazin-4-yl)-methanone,
(80) (3,5-dibromo-4-hydroxyphenyl)-(2,3-dihydronaphtho[2,1-b][1,4]oxazin-1-yl)-methanone,
(81) (3,5-dibromo-4-hydroxyphenyl)-(6-methyl-2,3-dihydrobenzo[1,4]oxazin-4-yl)-methanone,
(82) (6-chloro-2,3-dihydrobenzo[1,4]oxazin-4-yl)-(3,5-dibromo-4-hydroxyphenyl)-methanone,
(83) (3,5-dichloro-4-hydroxyphenyl)-(4-methanesulfonyl-3,4-dihydro-2H-quinoxalin-1-yl)-methanone,
(84) (3,5-dichloro-4-hydroxyphenyl)-(6-ethanesulfonyl-2,3-dihydrobenzo[1,4]oxazin-4-yl)-methanone,
(85) (3,5-dichloro-4-hydroxyphenyl)-(6-trifluoromethyl-2,3-dihydrobenzo[1,4]oxazin-4-yl)-methanone,
(86) (3,5-dichloro-4-methoxyphenyl)-(2,3-dihydrobenzo[1,4]oxazin-4-yl)-methanone,
(87) 2,6-dichloro-4-(2,3-dihydrobenzo[1,4]oxazine-4-carbonyl)phenyl acetate,
(88) (2,3-dihydrobenzo[1,4]oxazin-4-yl)-(4-hydroxyphenyl)-methanone,
(89) (3,5-dichloro-4-hydroxyphenyl)-(5-hydroxy-2,3-dihydrobenzo[1,4]oxazin-4-yl)-methanone,
(90) (3,5-dichloro-4-hydroxyphenyl)-(8-hydroxy-2,3-dihydrobenzo[1,4]oxazin-4-yl)-methanone,
(91) ethyl [2,6-dichloro-4-(2,3-dihydrobenzo[1,4]oxazine-4-carbonyl)phenoxy]acetate,
(92) [2,6-dichloro-4-(2,3-dihydrobenzo[1,4]oxazine-4-carbonyl)phenoxy]acetic acid
(93) (3,5-dichloro-4-hydroxyphenyl)-(3-methyl-2,3-dihydrobenzo[1,4]oxazin-4-yl)-methanone,
(94) N-[2,6-dichloro-4-(2,3-dihydrobenzo[1,4]oxazine-4-carbonyl)phenyl]methanesulfonamide, and
(95) (3,5-dichloro-4-hydroxyphenyl)-(7,8-dihydro-6H-5-oxa-9-azabenzocyclohepten-9-yl)-methanone, or a pharmaceutically acceptable salt thereof.

32. A pharmaceutical composition comprising a compound of any of claims 5 to 31 or a pharmaceutically acceptable salt thereof.

33. A URAT1 activity inhibitor comprising a compound of any of claims 5 to 31 or a pharmaceutically acceptable salt thereof.

34. An agent for lowering a blood uric acid level comprising the substance in any of claims 1 to 3.

35. An agent for lowering a blood uric acid level comprising the compound in claim 4 or a pharmaceutically acceptable salt thereof.

36. An agent for lowering a blood uric acid level comprising a compound of any of claims 5 to 31 or a pharmaceutically acceptable salt thereof.

37. An agent for the prophylaxis or treatment of pathology showing involvement of uric acid, comprising the substance in any of claims 1 to 3.

38. An agent for the prophylaxis or treatment of pathology showing involvement of uric acid, which comprises the compound in claim 4 or a pharmaceutically acceptable salt thereof.

39. An agent for the prophylaxis or treatment of pathology showing involvement of uric acid, which comprises a compound of any of claims 5 to 31 or a pharmaceutically acceptable salt thereof.

40. The agent of any of claims 37 to 39, wherein the pathology showing involvement of uric acid is hyperuricemia, gouty tophus, acute gouty arthritis, chronic gouty arthritis, gouty kidney, urolithiasis, renal function disorder, coronary artery disease or ischemic heart disease.

41. A method for the prophylaxis or treatment of pathology showing involvement of uric acid, which comprises administering a pharmaceutically effective amount of the substance in any of claims 1 to 3.

42. A method for the prophylaxis or treatment of pathology showing involvement of uric acid, which comprises administering a pharmaceutically effective amount of the compound in claim 4 or a pharmaceutically acceptable salt thereof.

43. A method for the prophylaxis or treatment of pathology showing involvement of uric acid, which comprises administering a pharmaceutically effective amount of a compound of any of claims 5 to 31 or a pharmaceutically acceptable salt thereof.

44. The method of any of claims 41 to 43, wherein the pathology showing involvement of uric acid is hyperuricemia, gouty tophus, acute gouty arthritis, chronic gouty arthritis, gouty kidney, urolithiasis, renal function disorder, coronary artery disease or ischemic heart disease.

45. A method for inhibiting URAT1 activity, which comprises administering a pharmaceutically effective amount of the substance in any of claims 1 to 3.

46. A method for inhibiting URAT1 activity, which comprises administering a pharmaceutically effective amount of the compound in claim 4 or a pharmaceutically acceptable salt thereof.

47. A method for inhibiting URAT1 activity, which comprises administering a pharmaceutically effective amount of a compound of any of claims 5 to 31 or a pharmaceutically acceptable salt thereof.

48. A method of lowering a blood uric acid level, which comprises administering a pharmaceutically effective amount of the substance in any of claims 1 to 3.

49. A method of lowering a blood uric acid level, which comprises administering a pharmaceutically effective amount of the compound in claim 4 or a pharmaceutically acceptable salt thereof.

50. A method of lowering a blood uric acid level, which comprises administering a pharmaceutically effective amount of a compound of any of claims 5 to 31 or a pharmaceutically acceptable salt thereof.

51. Use of the substance in any of claims 1 to 3 for the production of an agent for the prophylaxis or treatment of pathology showing involvement of uric acid.

52. Use of the compound in claim 4 or a pharmaceutically acceptable salt thereof for the production of an agent for the prophylaxis or treatment of pathology showing involvement of uric acid.

53. Use of a compound of any of claims 5 to 31 or a pharmaceutically acceptable salt thereof for the production of an agent for the prophylaxis or treatment of pathology showing involvement of uric acid.

54. Use of any of claims 51 to 53, wherein the pathology showing involvement of uric acid is hyperuricemia, gouty tophus, acute gouty arthritis, chronic gouty arthritis, gouty kidney, urolithiasis, renal function disorder, coronary artery disease or ischemic heart disease.

55. Use of the substance in any of claims 1 to 3 for the production of a URAT1 activity inhibitor.

56. Use of the compound in claim 4 or a pharmaceutically acceptable salt thereof for the production of a URAT1 activity inhibitor.

57. Use of a compound of any of claims 5 to 31 or a pharmaceutically acceptable salt thereof for the production of a URAT1 activity inhibitor.

58. Use of the substance in any of claims 1 to 3 for the production of an agent for lowering a blood uric acid level.

59. Use of the compound in claim 4 or a pharmaceutically acceptable salt thereof for the production of an agent for lowering a blood uric acid level.

60. Use of a compound of any of claims 5 to 31 or a pharmaceutically acceptable salt thereof for the production of an agent for lowering a blood uric acid level.

61. A pharmaceutical composition for the prophylaxis or treatment of pathology showing involvement of uric acid, which comprises the substance in any of claims 1 to 3.

62. A pharmaceutical composition for the prophylaxis or treatment of pathology showing involvement of uric acid, which comprises the compound in claim 4 or a pharmaceutically acceptable salt thereof.

63. A pharmaceutical composition for the prophylaxis or treatment of pathology showing involvement of uric acid, which comprises a compound of any of claims 5 to 31 or a pharmaceutically acceptable salt thereof.

64. The pharmaceutical composition of any of claims 61 to 63,
wherein the pathology showing involvement of uric acid is hyperuricemia, gouty tophus, acute gouty arthritis, chronic gouty arthritis, gouty kidney, urolithiasis, renal function disorder, coronary artery disease or ischemic heart disease.

65. A commercial package comprising the pharmaceutical composition of any of claims 61 to 64, and a written matter associated therewith, the written matter stating that the pharmaceutical composition can or should be used for the prophylaxis or treatment of a disease selected from hyperuricemia, gouty tophus, acute gouty arthritis, chronic gouty arthritis, gouty kidney, urolithiasis, renal function disorder, coronary artery disease and ischemic heart disease.

66. The pharmaceutical composition of claim 32, which is used in combination with 1 to 3 agents selected from groups (1) to (11):
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease that causes decreased uric acid excretion secondary hyperuricemia,
(10) an agent for the prophylaxis and/or treatment of kidney failure, cardiovascular disorder or cerebrovascular disorder caused by hyperuricemia, and
(11) a nucleic acid metabolic antagonist.

67. The inhibitor of any of claims 1 to 4 and 33, which is used in combination with 1 to 3 agents selected from groups (1) to (11) :
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease that causes decreased uric acid excretion secondary hyperuricemia,
(10) an agent for the prophylaxis and/or treatment of kidney failure, cardiovascular disorder or cerebrovascular disorder caused by hyperuricemia, and
(11) a nucleic acid metabolic antagonist.

68. The agent of any of claims 34 to 36, which is used in combination with 1 to 3 agents selected from groups (1) to (11):
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease that causes decreased uric acid excretion secondary hyperuricemia,
(10) an agent for the prophylaxis and/or treatment of kidney failure, cardiovascular disorder or cerebrovascular disorder caused by hyperuricemia, and
(11) a nucleic acid metabolic antagonist.

69. The agent of any of claims 37 to 40, which is used in combination with 1 to 3 agents selected from groups (1) to (11):
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease that causes decreased uric acid excretion secondary hyperuricemia,
(10) an agent for the prophylaxis and/or treatment of kidney failure, cardiovascular disorder or cerebrovascular disorder caused by hyperuricemia, and
(11) a nucleic acid metabolic antagonist.

70. The method of any of claims 41 to 44, which further comprises administering 1 to 3 agents selected from groups (1) to (11) :
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease that causes decreased uric acid excretion secondary hyperuricemia,
(10) an agent for the prophylaxis and/or treatment of kidney failure, cardiovascular disorder or cerebrovascular disorder caused by hyperuricemia, and
(11) a nucleic acid metabolic antagonist.

71. The method of any of claims 45 to 47, which further comprises administering 1 to 3 agents selected from groups (1) to (11) :
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease that causes decreased uric acid excretion secondary hyperuricemia,
(10) an agent for the prophylaxis and/or treatment of kidney failure, cardiovascular disorder or cerebrovascular disorder caused by hyperuricemia, and
(11) a nucleic acid metabolic antagonist.

72. The method of any of claims 48 to 50, which further comprises administering 1 to 3 agents selected from groups (1) to (11) :
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease that causes decreased uric acid excretion secondary hyperuricemia,
(10) an agent for the prophylaxis and/or treatment of kidney failure, cardiovascular disorder or cerebrovascular disorder caused by hyperuricemia, and
(11) a nucleic acid metabolic antagonist.

73. The use of any of claims 51 to 54, wherein the agent is used in combination with 1 to 3 agents selected from groups (1) to (11) :
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease that causes decreased uric acid excretion secondary hyperuricemia,
(10) an agent for the prophylaxis and/or treatment of kidney failure, cardiovascular disorder or cerebrovascular disorder caused by hyperuricemia, and
(11) a nucleic acid metabolic antagonist.

74. The use of any of claims 55 to 57, wherein the agent is used in combination with 1 to 3 agents selected from groups (1) to (11) :
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease that causes decreased uric acid excretion secondary hyperuricemia,
(10) an agent for the prophylaxis and/or treatment of kidney failure, cardiovascular disorder or cerebrovascular disorder caused by hyperuricemia, and
(11) a nucleic acid metabolic antagonist.

75. The use of any of claims 58 to 60, wherein the agent is used in combination with 1 to 3 agents selected from groups (1) to (11) :
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease that causes decreased uric acid excretion secondary hyperuricemia,
(10) an agent for the prophylaxis and/or treatment of kidney failure, cardiovascular disorder or cerebrovascular disorder caused by hyperuricemia, and
(11) a nucleic acid metabolic antagonist.

76. The pharmaceutical composition of claim 32, which is used for suppressing an increase in the blood uric acid level in combination with a pharmaceutical agent that increases the blood uric acid level.

77. The inhibitor of any of claims 1 to 4 and 33, which is used for suppressing an increase in the blood uric acid level in combination with a pharmaceutical agent that increases the blood uric acid level.

78. The method of any of claims 45 to 47, which further comprises administering a pharmaceutical agent that increases the blood uric acid level.

79. The use of any of claims 55 to 57 for the production of a URAT1 activity inhibitor, which is used for suppressing an increase in the blood uric acid level in combination with a pharmaceutical agent that increases the blood uric acid level.

80. A method for suppressing a blood uric acid level, which comprises administering the inhibitor of any of claims 1 to 4 and 33 in combination with a pharmaceutical agent that increases the blood uric acid level.
